# EUROPEAN PATENT APPLICATION

(11) **EP 4 790 048 A1**
(43) Date of publication of application: **12.08.2026**
(21) Application number: 24876521.6
(22) Date of filing: 08.10.2024
(51) Int. Cl.: C07D 205/04, C07D 207/09, C07D 211/28, C07C 251/48, A61K 31/397, A61K 31/4025, A61K 31/444, A61P 37/00

(54) **S1PR4 REGULATOR COMPOUND FOR AUTOIMMUNE DISEASE TREATMENT AND APPLICATION THEREOF**

(30) Priority: 08.10.2023 CN 202311292806; 14.09.2024 CN 202411294693
(71) Applicant: Shengyuan Zetong (Shanghai) Pharmaceutical Co., Ltd, Shanghai 201100 (CN)
(72) Inventor: LI, Honglin, Shanghai 200241 (CN); QIAN, Xuhong, Shanghai 200241 (CN); WANG, Rui, Shanghai 200237 (CN); ZHAO, Zhenjiang, Shanghai 200237 (CN); XU, Yufang, Shanghai 200237 (CN); HE, Huan, Shanghai 200241 (CN); GAO, Wei, Shanghai 200241 (CN); XIAO, Yunping, Shanghai 200237 (CN); YE, Tianyu, Shanghai 200237 (CN)
(74) Representative: Mewburn Ellis LLP
(86) International application number: PCT/CN2024/123499
(87) International publication number: WO 2025/077707

(57) **Abstract**

Disclosed in the present invention are a compound as shown in formula (I) and an application thereof as an S1PR regulator, especially an S1PR4 agonist. The compound of the present invention can treat related diseases mediated by S IPR4 and mutants thereof, especially autoimmune diseases.

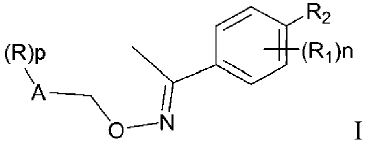

## Description

### Technical field

The present invention relates to the field of pharmacy. Specifically, the present invention relates to novel compounds of S1PR4 regulators for the prevention and treatment of autoimmune disease and uses thereof.

### Background

Sphingosine-1-phosphate (S1P) can mediate biological signaling to G protein-coupled receptors on biological membranes or act directly on intracellular receptors, thereby playing a unique role in mediating inflammatory and allergic responses, as well as participating in the migration, proliferation and differentiation of immune cells. Dysregulation of S1P signaling is associated with autoimmune diseases, cardiovascular diseases, neurological disorders and cancer. As a member of the G protein-coupled receptor family, five distinct S1P receptor subtypes have been identified in mammals, namely S1PR1, S1PR2, S1PR3, S1PR4 and S1PR5 (formerly known as EDG1, EDG5, EDG3, EDG6 and EDG8). Among them, S1PR1, S1PR2 and S1PR3 are widely expressed in various body tissues, S1PR4 is predominantly expressed in the lymphatic and hematopoietic systems, and S1PR5 is mainly expressed in the central nervous system. Current studies have confirmed that S1P can bind to S1PR1 on the cell surface, activate the β-arrestin signaling pathway to induce receptor internalization, thereby promoting lymphocyte homing and inhibiting lymphocyte egress from peripheral lymph nodes and secondary lymphoid organs, ultimately preventing lymphocyte migration to inflammatory sites and exerting favorable immunosuppressive regulatory effects. Targeted regulation of the S1PR1 signaling pathway can modulate a variety of autoimmune diseases, such as systemic lupus erythematosus, ulcerative colitis, Crohn's disease, multiple sclerosis, rheumatoid arthritis, psoriasis and ankylosing spondylitis. However, currently marketed drugs targeting S1PR1 exhibit potent agonist activity against other S1PR subtypes and lack selectivity; in particular, activation of S1PR3 causes off-target side effects such as bradycardia. Therefore, the development of selective S1PRs agonists is of great significance.

However, there are currently few reports on selective S1PR4 drugs. Almost all disclosed S1PR4 agonists/antagonists have been developed by Edward Roberts at the Scripps Research Institute in the United States. Nevertheless, these S1PR4 regulators have only been investigated as *in vitro* tool molecules, without systematic mechanistic studies or druggability evaluations. Therefore, the development of orally effective and selective S1PR4 agonists/antagonists for the treatment of autoimmune diseases is of significant scientific value.

### Summary of the invention

The purpose of the present invention is to provide related compounds as S1PR regulators, particularly S1PR4 agonists.

Another purpose of the present invention is to provide pharmaceutical compositions containing the above-mentioned compounds.

Yet another purpose of the present invention is to provide the use of the above-mentioned compounds or pharmaceutical compositions in the preparation of drugs for treating S1PR4-related diseases or regulating S1P receptors.

In the first aspect, the present invention provides a compound of formula I, or an optical isomer or a pharmaceutically acceptable salt thereof: 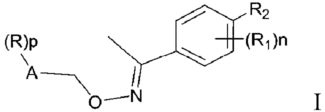
A is selected from: a substituted or unsubstituted C₅-C₈ aromatic ring (such as phenyl) or C₈-C₁₄ fused aromatic ring (such as naphthyl), substituted or unsubstituted five-membered or six-membered heterocyclic group containing 1-3 heteroatoms selected from N or O;
R is independently selected from the group consisting of a hydrogen, halogen, cyano, substituted or unsubstituted C₁-C₁₀ alkyl (for example, a methyl, trifluoromethyl, trifluoroethyl), substituted or unsubstituted C₃-C₈ cycloalkyl or cycloalkenyl, substituted or unsubstituted C₁-C₅ alkoxy, substituted or unsubstituted C₅-C₈ aryl (for example, phenyl), substituted or unsubstituted five-membered or six-membered heterocyclic group or heteroaryl containing 1-2 heteroatoms selected from N, O or S, substituted or unsubstituted C₁-C₁₀ alkyl formyl, substituted or unsubstituted C₅-C₈ aryl formyl; p is an integer from 1 to 3;
R¹ is selected from the group consisting of a hydrogen, halogen, cyano, substituted or unsubstituted C₁-C₅ alkoxy, substituted or unsubstituted C₁-C₁₀ alkyl, substituted or unsubstituted C₃-C₈ cycloalkyl, substituted or unsubstituted C₂-C₆ alkenyl, substituted or unsubstituted C₃-C₆ cycloalkenyl, substituted or unsubstituted C₃-C₈ lactone group, substituted or unsubstituted C₁-C₁₀ amide group, substituted or unsubstituted C₅-C₈ aryl, substituted or unsubstituted five-membered or six-membered heterocyclic group containing 1-2 heteroatoms selected from N, O or S, substituted or unsubstituted C₅-C₈ aromatic heterocyclic group;
n is an integer selected from 1-3;
R² is selected from the following group: 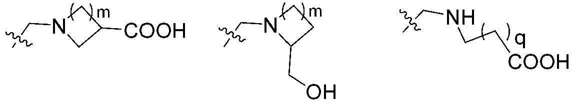
m is an integer selected from 1-4;
q is an integer selected from 1-2.

In a preferred embodiment, the compound is not the following compound:

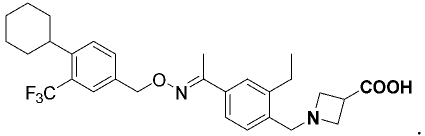

In a specific embodiment, the compound is represented by the following formula II: 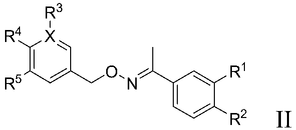 wherein,
R¹ is selected from the group consisting of a hydrogen, halogen (preferably F, Cl or Br), cyano, substituted or unsubstituted C₁-C₅ alkoxy (preferably methoxy), substituted or unsubstituted C₁-C₁₀ alkyl (preferably a substituted or unsubstituted C₁-C₆ alkyl, such as a methyl, ethyl, trifluoromethyl), substituted or unsubstituted C₃-C₈ cycloalkyl, substituted or unsubstituted C₂-C₆ alkenyl, substituted or unsubstituted C₃-C₆ cycloalkenyl, substituted or unsubstituted C₅-C₈ aryl, substituted or unsubstituted five-membered or six-membered heterocyclic group containing 1-2 heteroatoms selected from N, O or S, and substituted or unsubstituted C₅-C₈ aromatic heterocyclic group;
R² is selected from the following group: 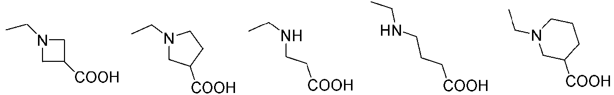
X is selected from C or N;
R³ is not present or is a substituent selected from the following group: a hydrogen, cyano, halogen, substituted or unsubstituted C₁-C₁₀ alkyl (for example, a methyl, trifluoromethyl, trifluoroethyl), substituted or unsubstituted C₃-C₈ cycloalkyl, cyano, substituted or unsubstituted C₁-C₅ alkoxy, substituted or unsubstituted C₁-C₁₀ alkyl formyl, substituted or unsubstituted C₅-C₈ aryl formyl, substituted or unsubstituted C₅-C₈ aryl, substituted or unsubstituted five-membered or six-membered heterocyclic group containing 1-2 heteroatoms selected from N, O or S;
R⁵ is selected from the group consisting of a hydrogen, cyano, halogen, substituted or unsubstituted C₁-C₁₀ alkyl (for example, a methyl, trifluoromethyl, trifluoroethyl), substituted or unsubstituted C₃-C₈ cycloalkyl, cyano, substituted or unsubstituted C₁-C₅ alkoxy, substituted or unsubstituted C₁-C₁₀ alkyl formyl, substituted or unsubstituted C₅-C₈ aryl formyl, substituted or unsubstituted C₅-C₈ aryl, substituted or unsubstituted five-membered or six-membered heterocyclic group containing 1-2 heteroatoms selected from N, O or S;
R⁴ is selected from the group consisting of a hydrogen, halogen, substituted or unsubstituted C₁-C₁₀ alkyl, substituted or unsubstituted C₃-C₈ cycloalkyl or cycloalkenyl, substituted or unsubstituted C₅-C₈ aryl, substituted or unsubstituted five-membered or six-membered heterocyclic group or heteroaryl groups containing 1-2 heteroatoms selected from N, O or S, substituted or unsubstituted C₁-C₁₀ alkyl formyl, and substituted or unsubstituted aryl formyl.

In a specific embodiment, the compound is represented by the following formula III: 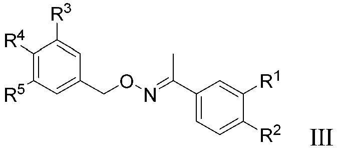 wherein,
R¹ is selected from the following group: a hydrogen, halogen (preferably F, Cl, or Br), cyano, substituted or unsubstituted C₁-C₅ alkoxy (preferably a methoxy), substituted or unsubstituted C₁-C₁₀ alkyl (preferably a substituted or unsubstituted C₁-C₆ alkyl, such as a methyl, ethyl, trifluoromethyl), substituted or unsubstituted C₃-C₈ cycloalkyl, substituted or unsubstituted C₂-C₆ alkenyl, substituted or unsubstituted C₃-C₆ cycloalkenyl, substituted or unsubstituted C₅-C₈ aryl, substituted or unsubstituted five-membered or six-membered heterocyclic group containing 1-2 heteroatoms selected from N, O, or S, substituted or unsubstituted C₅-C₈ aromatic heterocyclic group;
R² is selected from the following group: 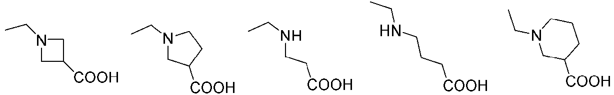
R³ is selected from the group consisting of a hydrogen, cyano, halogen, substituted or unsubstituted C₁-C₁₀ alkyl (for example, a methyl, trifluoromethyl, trifluoroethyl), substituted or unsubstituted C₃-C₈ cycloalkyl, cyano, substituted or unsubstituted C₁-C₅ alkoxy, substituted or unsubstituted C₁-C₁₀ alkyl formyl, substituted or unsubstituted C₅-C₈ aryl formyl, substituted or unsubstituted C₅-C₈ aryl, substituted or unsubstituted five-membered or six-membered heterocyclic group containing 1-2 heteroatoms selected from N, O or S;
R⁵ is selected from the following group: a hydrogen, cyano, halogen, substituted or unsubstituted C₁-C₁₀ alkyl (for example, a methyl, trifluoromethyl, trifluoroethyl), substituted or unsubstituted C₃-C₈ cycloalkyl, cyano, substituted or unsubstituted C₁-C₅ alkoxy, substituted or unsubstituted C₁-C₁₀ alkyl formyl, substituted or unsubstituted C₅-C₈ aryl formyl, substituted or unsubstituted C₅-C₈ aryl, substituted or unsubstituted five-membered or six-membered heterocyclic group containing 1-2 heteroatoms selected from N, O or S;
R⁴ is selected from the group consisting of a hydrogen, halogen, substituted or unsubstituted C₁-C₁₀ alkyl, substituted or unsubstituted C₃-C₈ cycloalkyl or cycloalkenyl, substituted or unsubstituted C₅-C₈ aryl, substituted or unsubstituted five-membered or six-membered heterocyclic group or heteroaryl containing 1-2 heteroatoms selected from N, O or S, substituted or unsubstituted C₁-C₁₀ alkyl formyl, and substituted or unsubstituted aryl formyl.

In a specific embodiment, the present invention provides a compound selected from the following group, or an optical isomer or a pharmaceutically acceptable salt thereof:

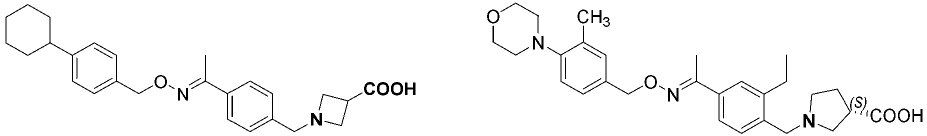

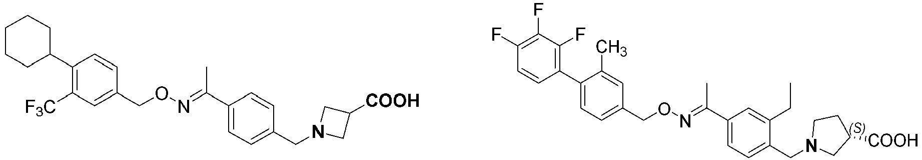

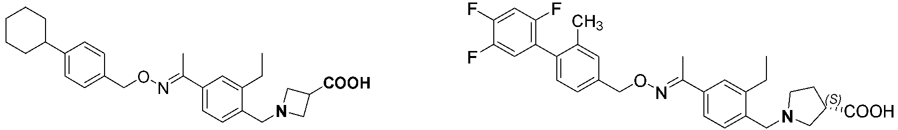

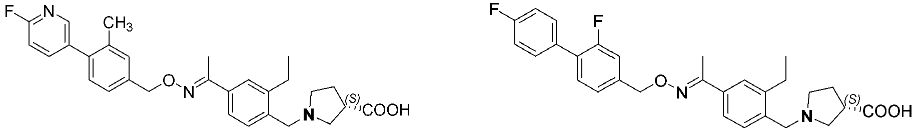

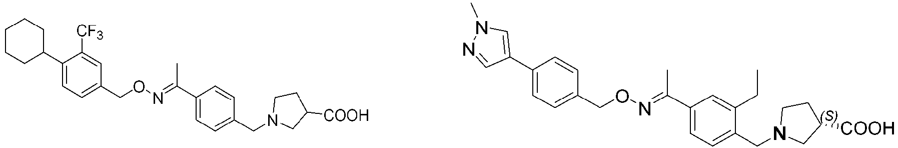

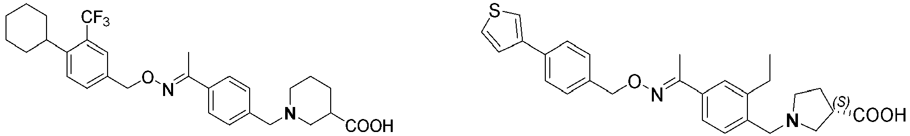

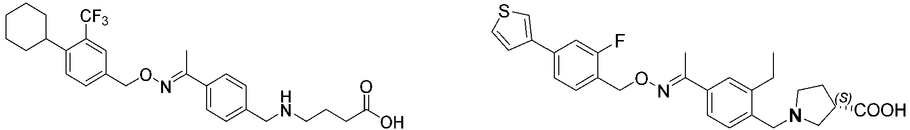

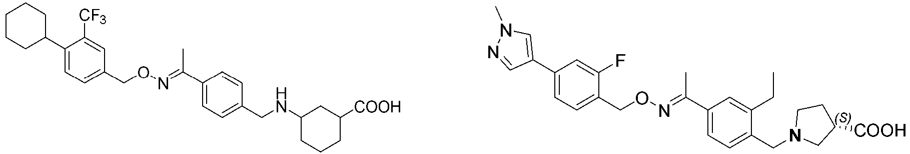

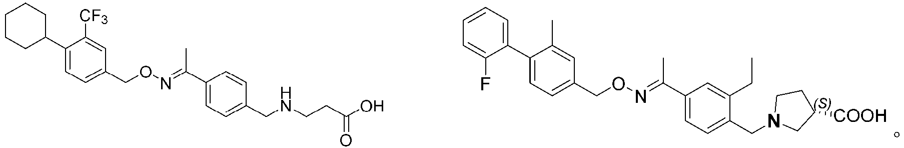

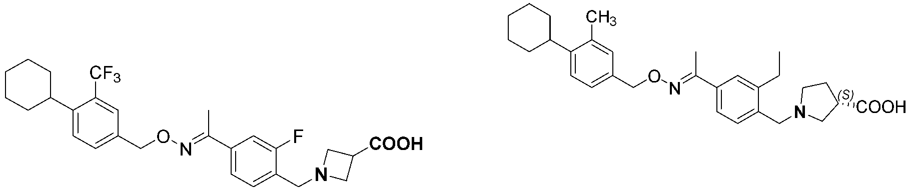

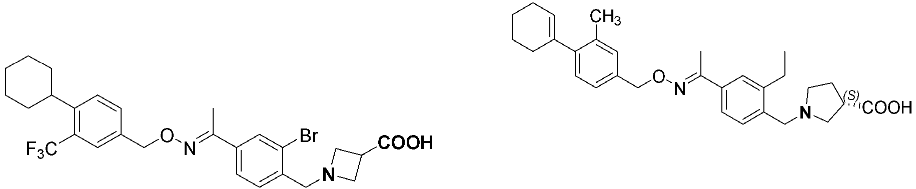

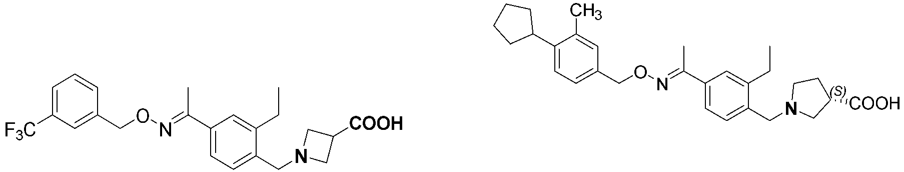

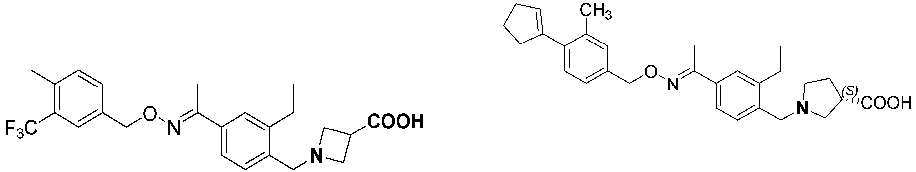

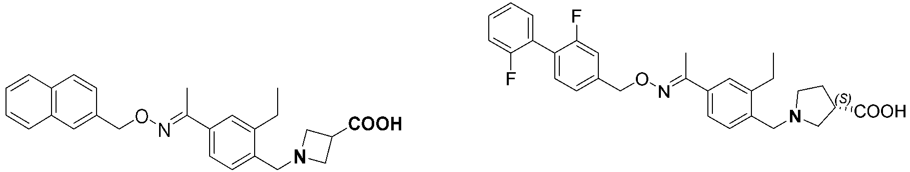

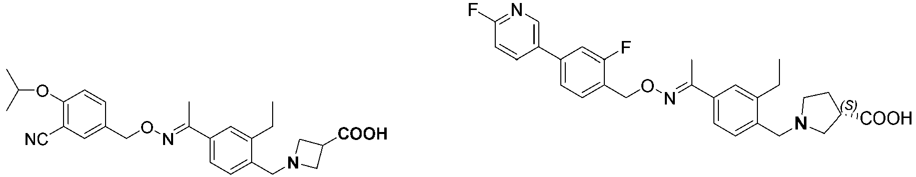

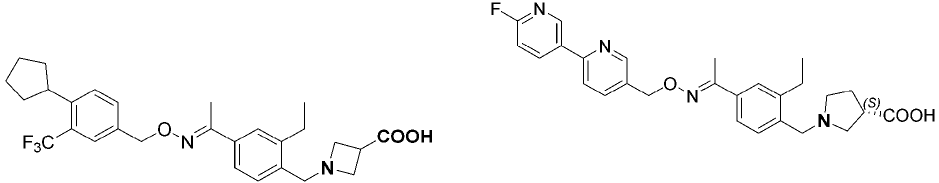

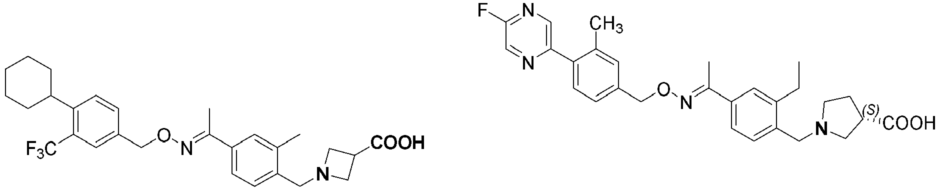

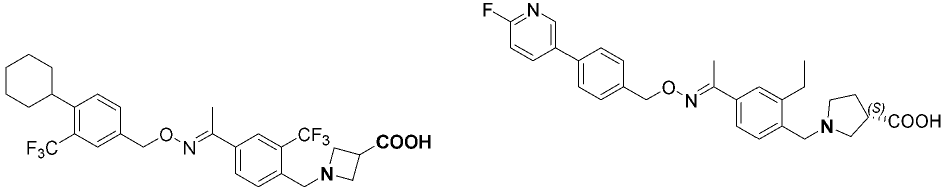

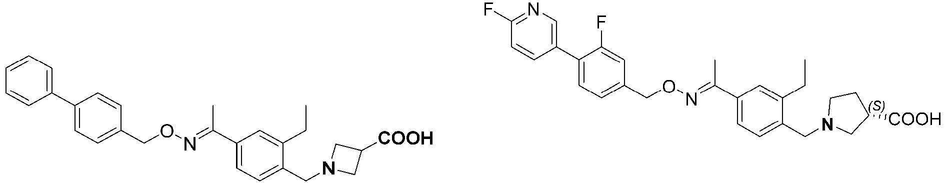

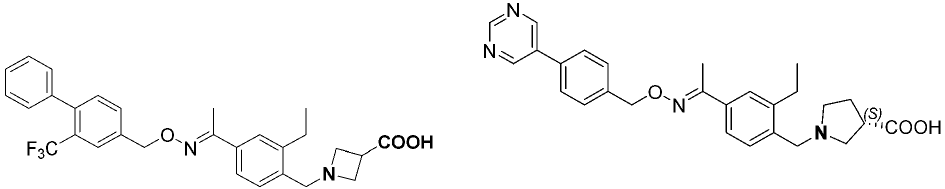

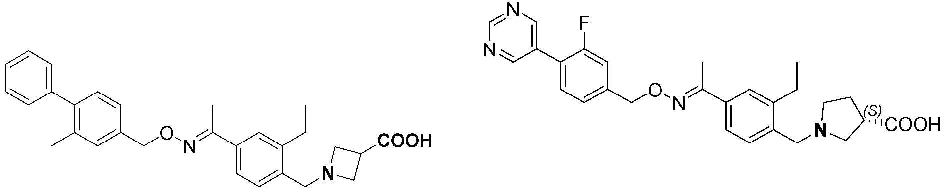

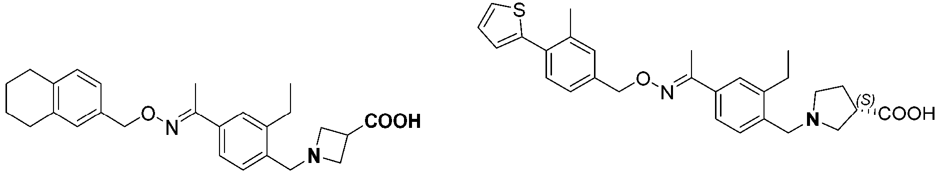

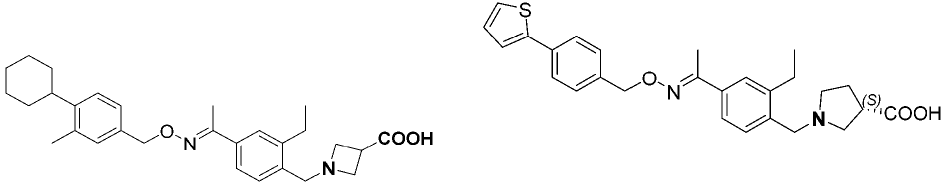

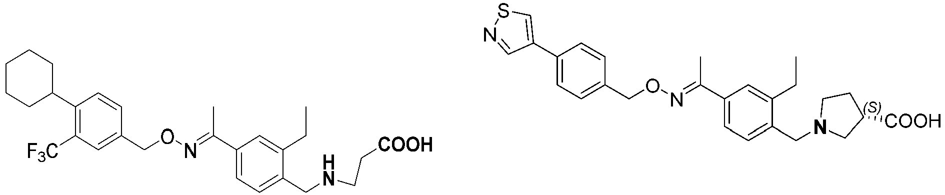

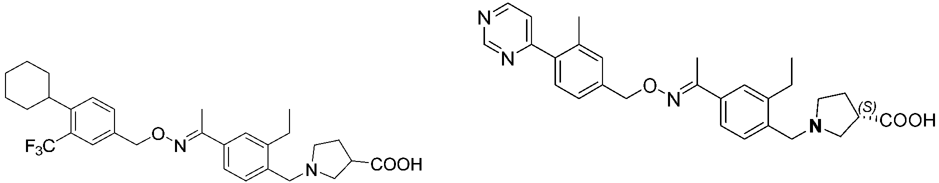

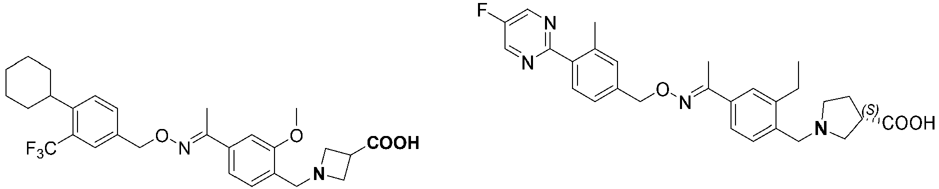

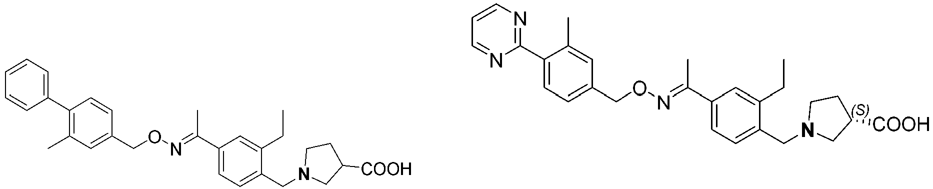

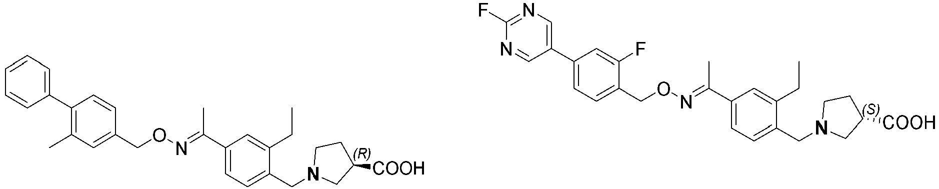

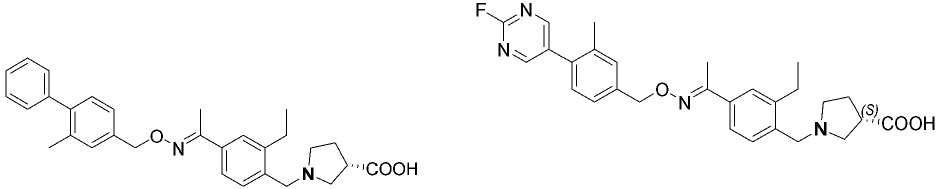

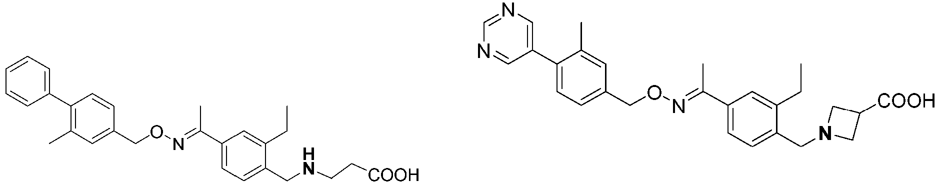

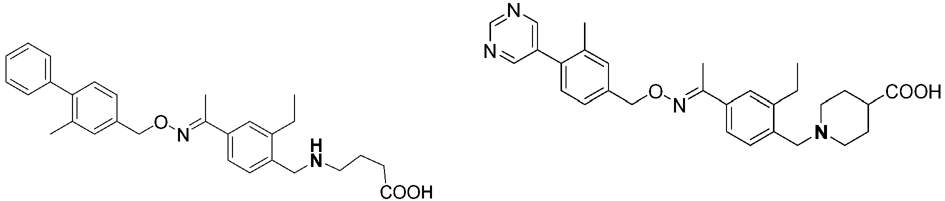

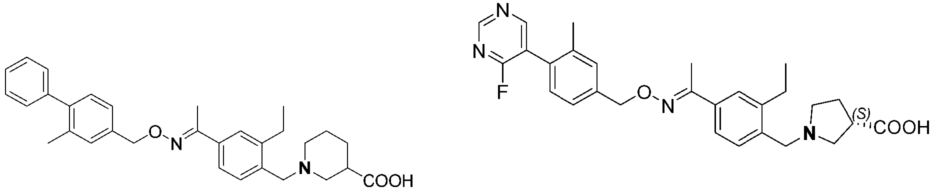

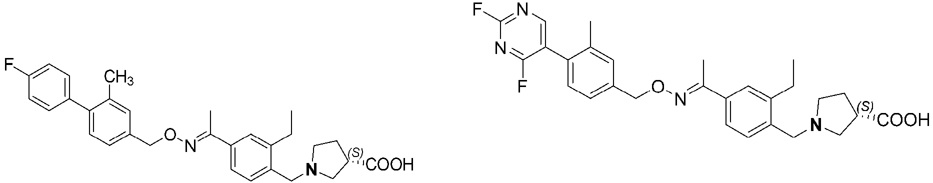

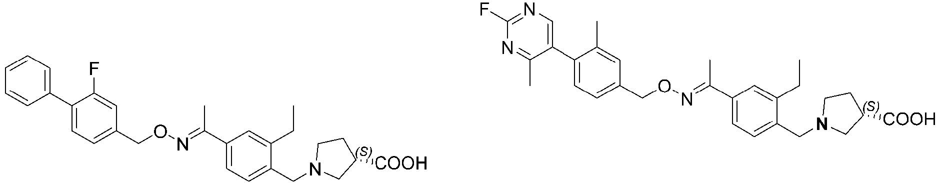

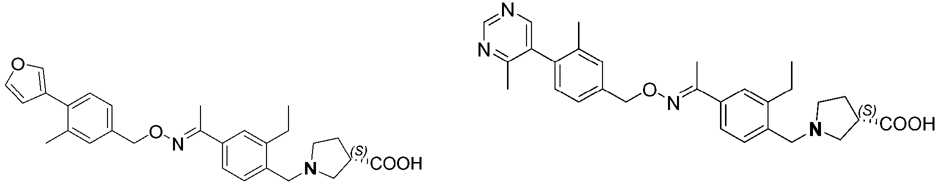

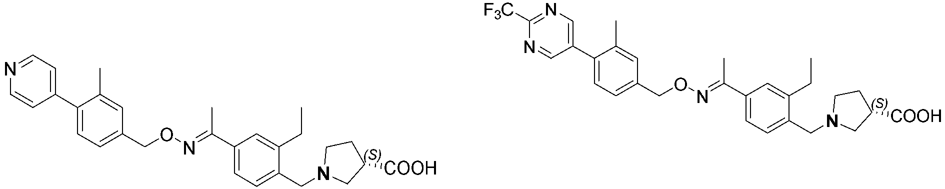

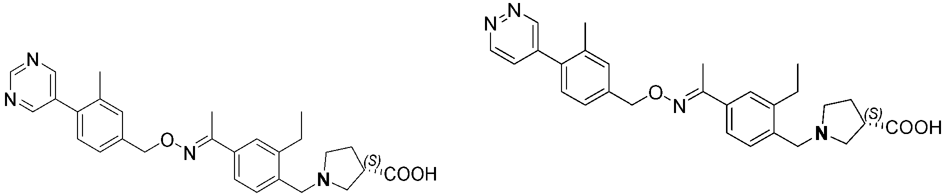

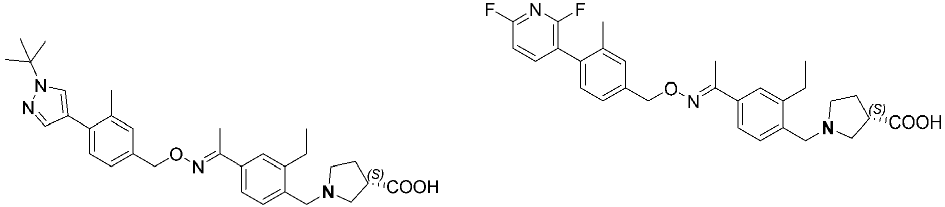

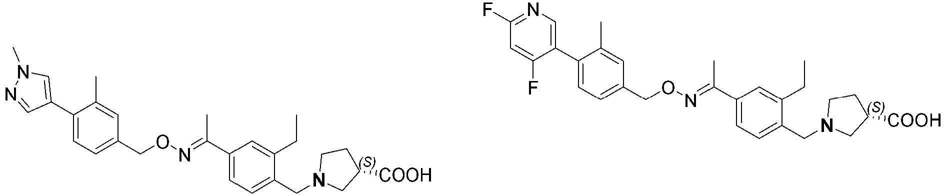

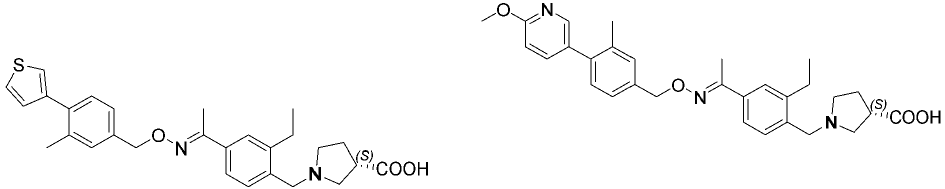

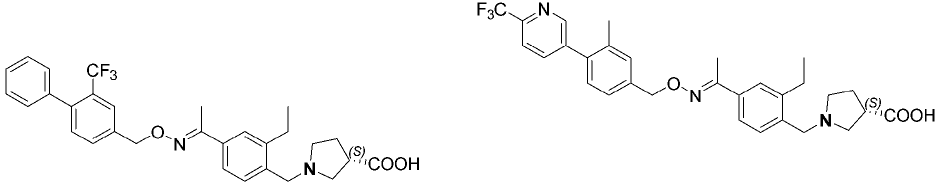

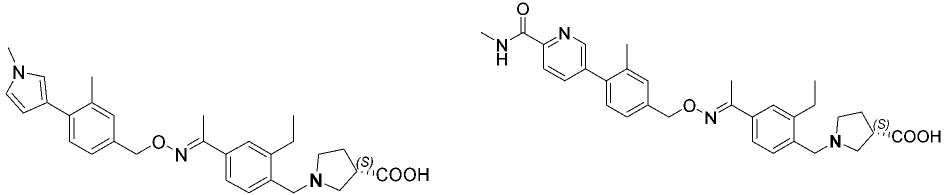

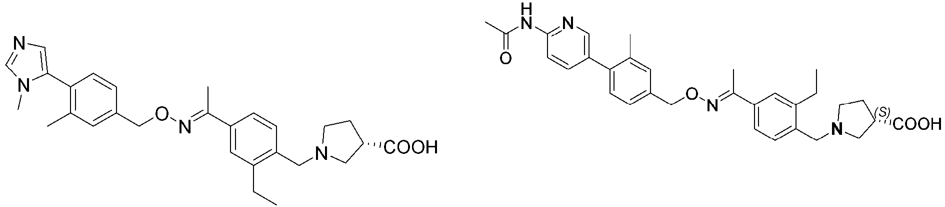

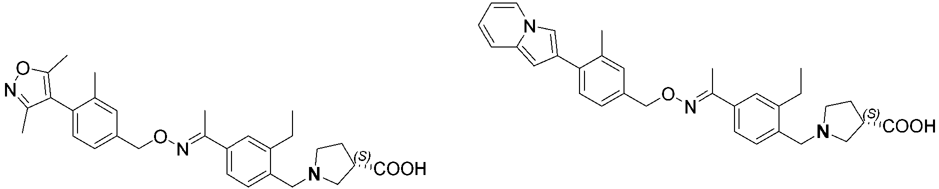

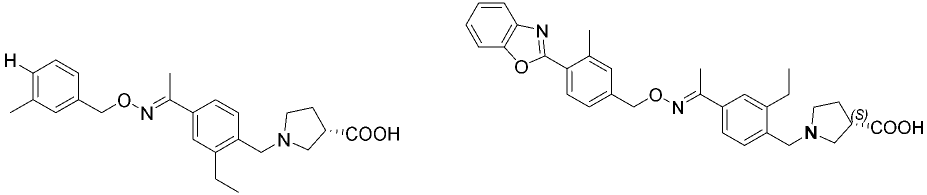

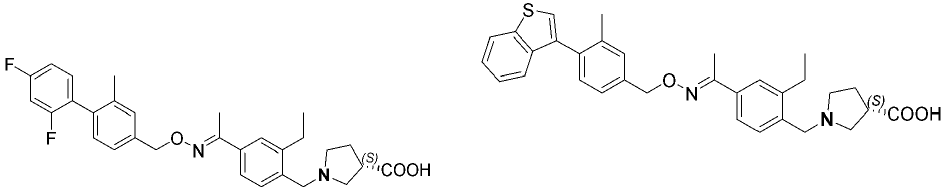

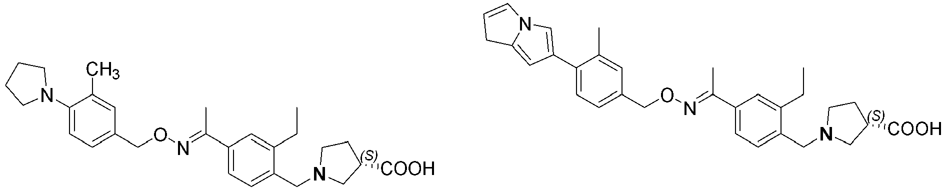

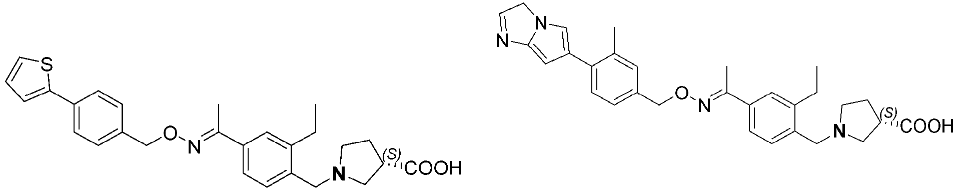

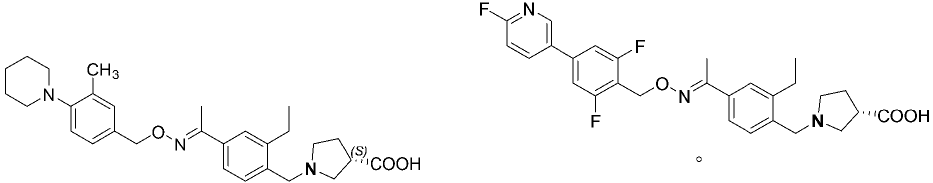

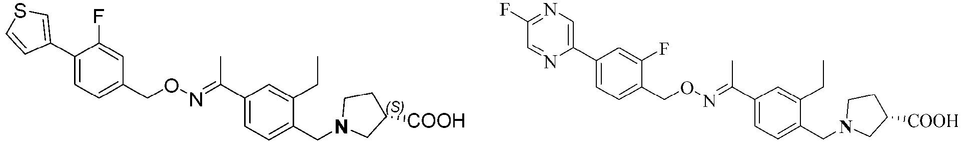

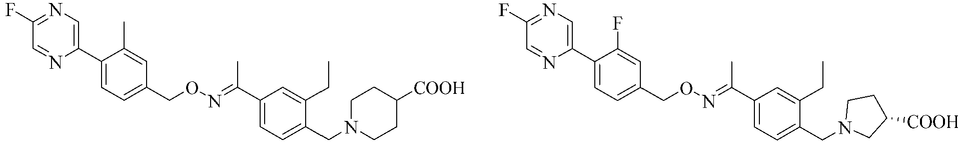

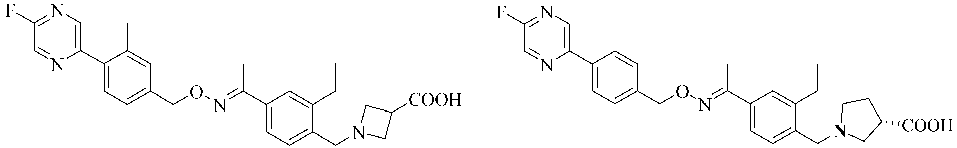

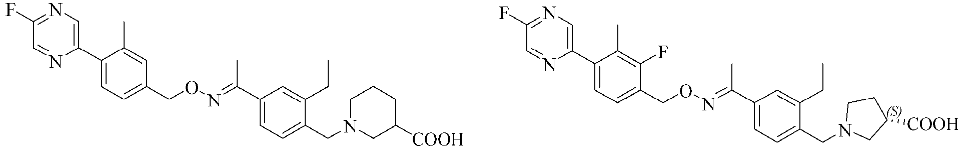

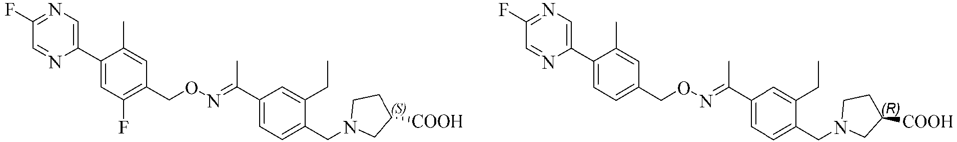

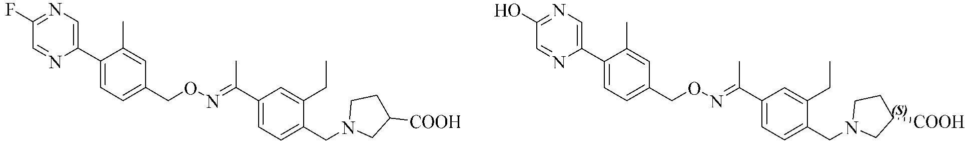

In a specific embodiment, in Formula III, R¹ is selected from the group consisting of: a hydrogen, halogen (preferably F, Cl, or Br), cyano, substituted or unsubstituted C₁-C₅ alkoxy (preferably a methoxy), substituted or unsubstituted C₁-C₆ alkyl (preferably a methyl, ethyl, propyl);
R² is: 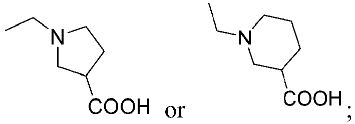
R³ and R⁵ are independently selected from the group consisting of a hydrogen, substituted or unsubstituted C₁-C₆ alkyl (preferably a methyl, ethyl or propyl), and halogen (preferably F);
R⁴ is selected from the group consisting of a substituted or unsubstituted C₅-C₈ aryl (preferably a phenyl or F-substituted phenyl), substituted or unsubstituted five-membered or six-membered heterocyclic group or heteroaryl containing 1-2 heteroatoms selected from N, O or S.

In a specific embodiment, the present invention provides a compound selected from the following group, or an optical isomer or a pharmaceutically acceptable salt thereof:

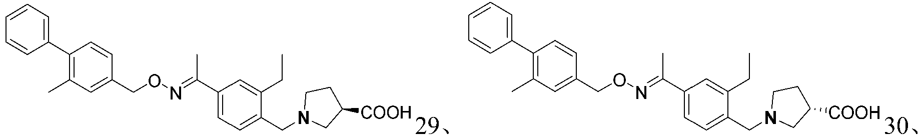

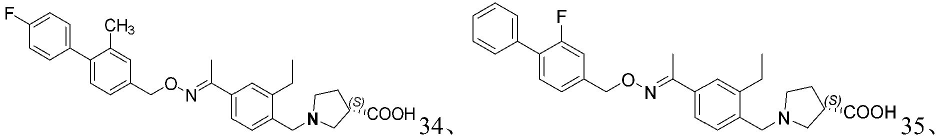

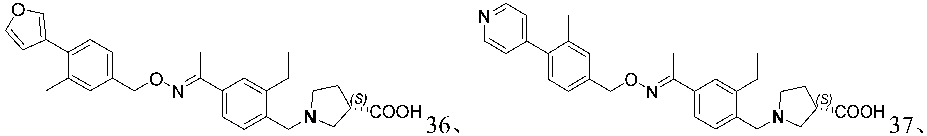

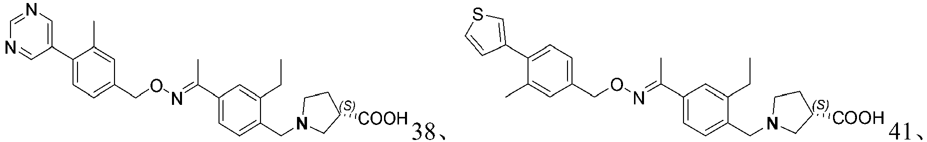

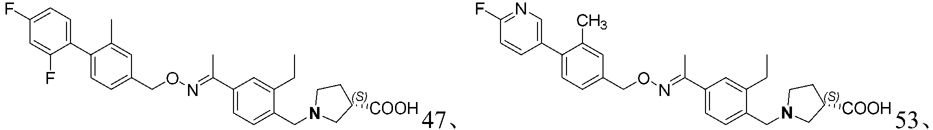

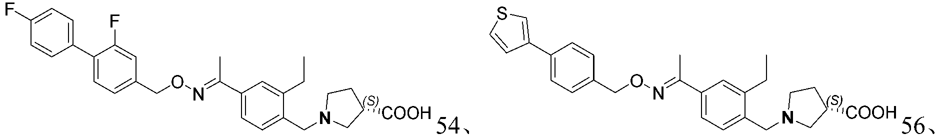

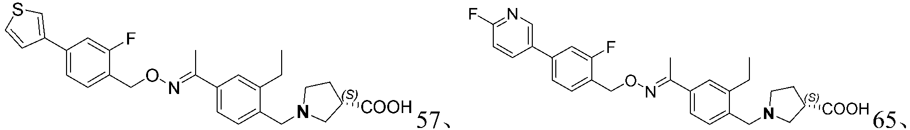

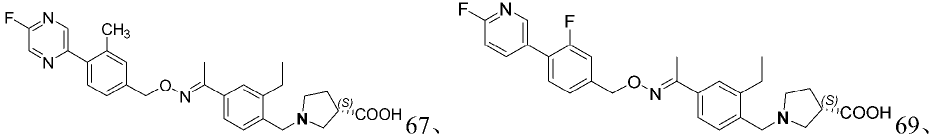

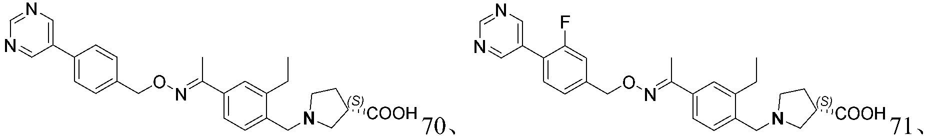

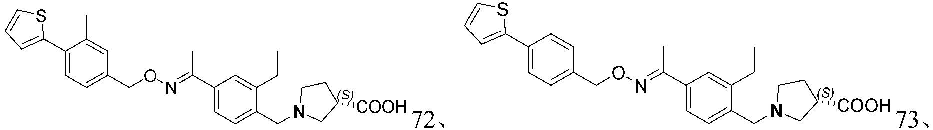

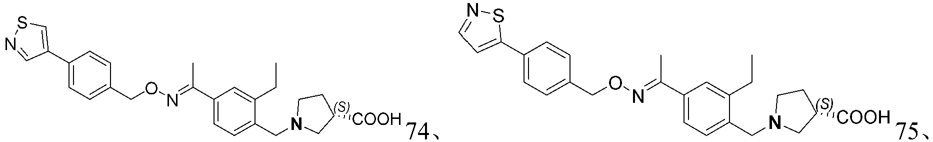

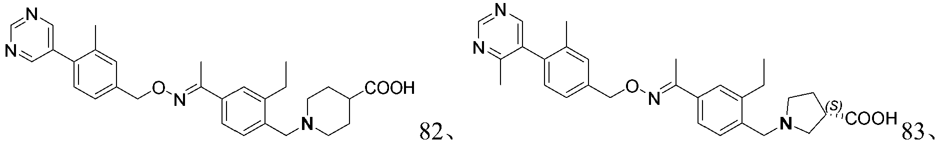

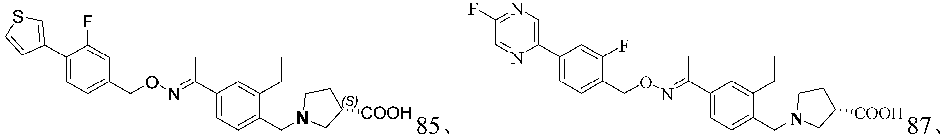

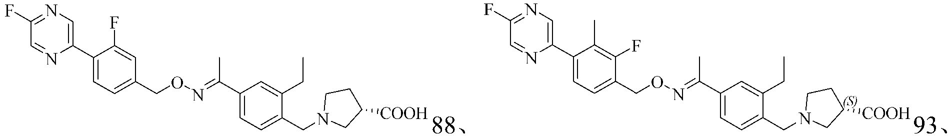

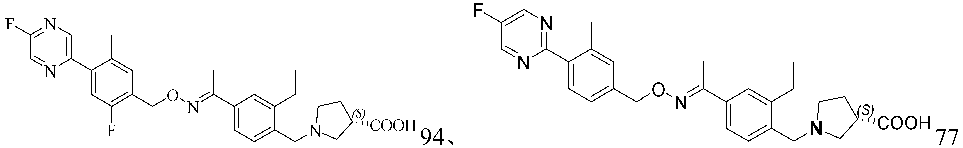

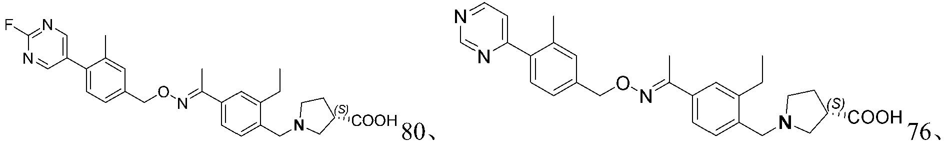

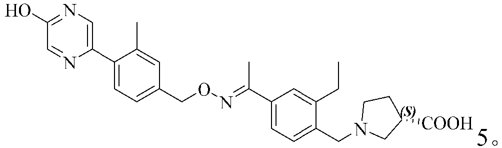

In a specific embodiment, in Formula III,
R¹ is selected from the group consisting of: a cyano, substituted or unsubstituted C₁-C₅ alkoxy (preferably a methoxy), substituted or unsubstituted C₁-C₆ alkyl (preferably a methyl, ethyl, propyl);
R² is: 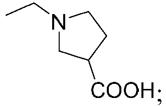
R³ and R⁵ are independently selected from the group consisting of a hydrogen, substituted or unsubstituted C₁-C₃ alkyl (preferably a methyl, ethyl or propyl), and halogen (preferably F);
R⁴ is selected from the group consisting of a substituted or unsubstituted C₅-C₈ aryl (preferably a phenyl or F-substituted phenyl), and substituted or unsubstituted five-membered or six-membered heteroaryl containing 1-2 heteroatoms selected from N, O or S.

In a specific embodiment, the present invention provides a compound selected from the following group, or an optical isomer or a pharmaceutically acceptable salt thereof:

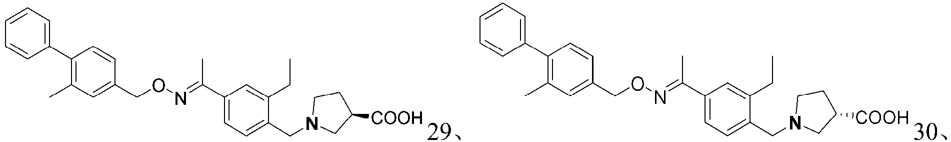

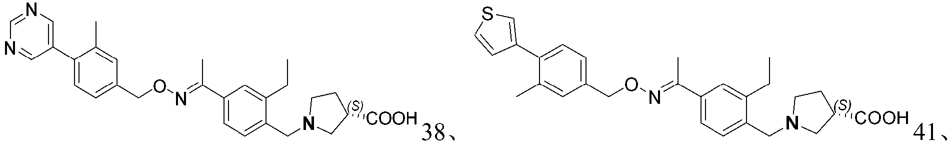

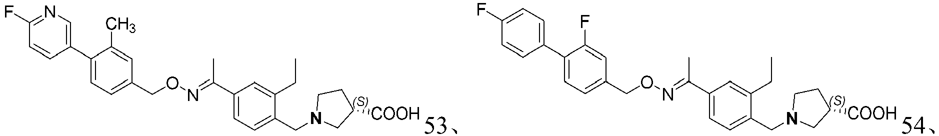

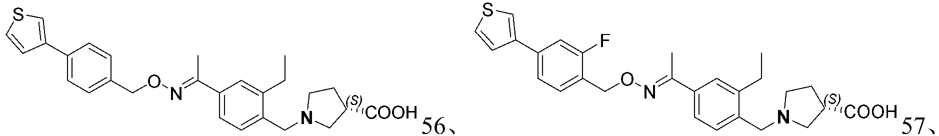

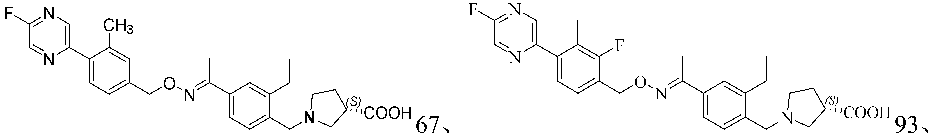

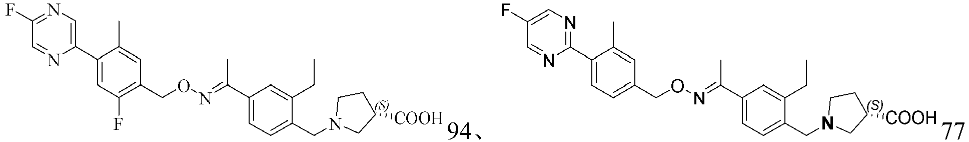

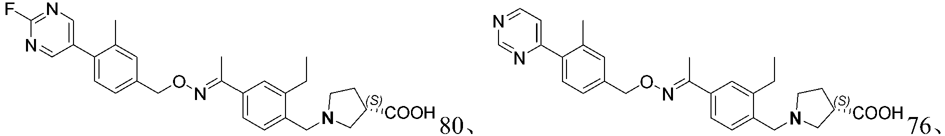

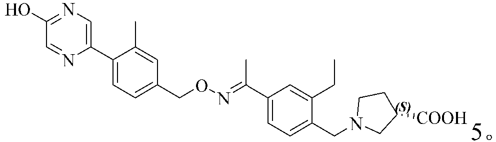

In a preferred embodiment, the compound is an S1PR regulator; preferably, the compound is an S1PR4 agonist; and more preferably, the compound has agonistic effects on S1PR4 but no agonistic effects on S1PR1.

In the second aspect, the present invention provides a pharmaceutical composition, comprising the compound described in the first aspect, or an optical isomer or a pharmaceutically acceptable salt thereof, as well as a pharmaceutically acceptable carrier or excipient.

In a preferred embodiment, the pharmaceutical composition is in a dosage form suitable for oral administration, including but not limited to tablets, solutions, suspensions, capsules, granules, and powders.

In a preferred embodiment, the pharmaceutical composition is used as an S1PR4 agonist or an S1PR4 mutant agonist.

In a preferred embodiment, the pharmaceutical composition is used for treating or preventing diseases mediated by S1PR4.

In a preferred embodiment, the S1PR4-mediated diseases include autoimmune diseases.

In a preferred embodiment, the autoimmune diseases include, but are not limited to: systemic lupus erythematosus, ulcerative colitis, Crohn's disease, multiple sclerosis, rheumatoid arthritis, psoriasis, ankylosing spondylitis, etc.

In the third aspect, the present invention provides the use of the compound described in the first aspect, or an optical isomer thereof or a pharmaceutically acceptable salt thereof, in the preparation of an S1PR regulator. In a preferred embodiment, the S1PR regulator is a selective agonist of S1PR4 or a selective agonist of S1PR4 mutant.

In a preferred embodiment, the S1PR regulator is an S1PR4 agonist or an S1PR4 mutant agonist.

In a preferred embodiment, the S1PR4 agonist or an S1PR4 mutant agonist is a medicament for treating or preventing diseases mediated by S1PR4.

In a preferred embodiment, the S1PR4-mediated diseases include autoimmune diseases.

In a preferred embodiment, the autoimmune diseases include, but are not limited to: systemic lupus erythematosus, ulcerative colitis, Crohn's disease, multiple sclerosis, rheumatoid arthritis, psoriasis, ankylosing spondylitis, etc.

In the fourth aspect, the present invention provides a method for regulating S1PR in a subject, comprising the step of administering an effective amount of the compound according to the first aspect, or an optical isomer thereof, or a pharmaceutically acceptable salt thereof, or the pharmaceutical composition according to the second aspect to a subject in need thereof.

In a preferred embodiment, the method is a method for activating S1PR4 in a subject; and more preferably, the method is a method for activating S1PR4 in a subject without activating S1PR1.

In a preferred embodiment, the method is used for treating or preventing diseases mediated by S1PR4.

In a preferred embodiment, the S1PR4-mediated diseases include autoimmune diseases.

In a preferred embodiment, the autoimmune diseases include, but are not limited to: systemic lupus erythematosus, ulcerative colitis, Crohn's disease, multiple sclerosis, rheumatoid arthritis, psoriasis, ankylosing spondylitis, etc.

In the fifth aspect, the present invention provides the compound described in the first aspect, or an optical isomer, pharmaceutically acceptable salt thereof, or the pharmaceutical composition described in the second aspect, for treating or preventing diseases mediated by S1PR4.

In a preferred embodiment, the S1PR4-mediated diseases include autoimmune diseases.

In a preferred embodiment, the autoimmune diseases include, but are not limited to: systemic lupus erythematosus, ulcerative colitis, Crohn's disease, multiple sclerosis, rheumatoid arthritis, psoriasis, ankylosing spondylitis, etc.

In the sixth aspect, the present invention provides a medicament comprising the compound described in the first aspect, or an optical isomer thereof, a pharmaceutically acceptable salt thereof, or the pharmaceutical composition described in the second aspect, for treating or preventing diseases mediated by S1PR4.

In a preferred embodiment, the S1PR4-mediated diseases include autoimmune diseases.

In a preferred embodiment, the autoimmune diseases include, but are not limited to: systemic lupus erythematosus, ulcerative colitis, Crohn's disease, multiple sclerosis, rheumatoid arthritis, psoriasis, ankylosing spondylitis, etc.

It should be understood that within the scope of the present invention, the above-mentioned technical features of the present invention and the technical features specifically described below (such as in the examples) can be combined with each other to form new or preferred technical solutions. Due to the limited contents, they will not be described one by one here.

### Description of drawings

Figure 1 shows the recovery effect of Compound 30 after inducing S1PR4 internalization;
Figure 2 shows that Compound 30 reduces the mortality rate of animals with lupus erythematosus;
Figure 3 shows that Compound 30 dose-dependently reduces subcutaneous nodules and antinuclear antibody levels in SLE mice;
Figure 4 shows that Compound 30 regulates the number and ratio of T lymphocytes;
Figure 5 shows that Compound 30 improves renal pathological injury in SLE;
Figure 6 shows the survival curve of acute UC model mice administered with Compound 30;
Figure 7 shows the effect of Compound 30 on body weight in acute UC model mice;
Figure 8 shows the effect of Compound 30 on daily DAI scores in acute UC model mice;
Figure 9 shows the effect of Compound 30 on the total DAI score in acute UC model mice;
Figure 10 shows the colon length of each experimental group.
Figure 11 shows the colon weight of each experimental group;
Figure 12 shows the colon swelling rate of each experimental group;
Figure 13 shows the pathological changes of mouse colon detected by H&E staining;
Figure 14 shows the colon pathological score of each group of mice by H&E staining;
Figure 15 shows Occludin in the colon of acute UC mice detected by immunohistochemistry;
Figure 16 shows Occludin in the colon of acute UC mice detected by immunohistochemistry;
Figure 17 shows the body weight changes of each group during chronic UC modeling;
Figure 18 shows the body weight changes of each group after the start of administration;
Figure 19 shows the body weight change rate of each group before and after administration;
Figure 20 shows the daily DAI scores of the chronic UC model during modeling;
Figure 21 shows the daily DAI scores of the chronic UC model during administration;
Figure 22 shows the total daily DAI scores of each group of chronic UC models during administration;
Figure 23 shows the colon length of each group in chronic UC;
Figure 24 shows the colon weight of each group in chronic UC;
Figure 25 shows the colon swelling rate of each group in chronic UC;
Figure 26 shows the pathological changes of mouse colon detected by H&E staining;
Figure 27 shows the colon pathological scores of each group by H&E staining;
Figure 28 shows Occludin in the colon of chronic UC mice detected by immunohistochemistry;
Figure 29 shows Occludin in the colon of chronic UC mice detected by immunohistochemistry.
Figure 30 shows the efficacy of compounds 30/38/53/67/77/80 in a mouse acute UC model.
Figure 31 shows the experimental protocol for osteoarthritis (OA).
Figure 32 shows the statistical analysis of pain scores in each group in the osteoarthritis experiment of compound 30.
Figure 33 shows the statistical analysis of five-minute walking distance in each group in the osteoarthritis experiment of compound 30.
Figure 34 shows the statistical analysis of five-minute rearing times in each group in the osteoarthritis experiment of compound 30.
Figure 35 shows the anatomical images of knee joints in each group in the osteoarthritis experiment of compound 30.
Figure 36 shows the effects of Safranin O-Fast Green staining and OARSI scoring on rat knee joints in the osteoarthritis experiment of compound 30.
Figure 37 shows the statistical analysis of pain scores in each group in the osteoarthritis experiment of compound 67.
Figure 38 shows the statistical analysis of five-minute walking distance in each group in the osteoarthritis experiment of compound 67.
Figure 39 shows the statistical analysis of five-minute rearing times in each group in the osteoarthritis experiment of compound 67.
Figure 40 shows the anatomical images of knee joints in each group in the osteoarthritis experiment of compound 67.
Figure 41 shows the effects of Compound 67 on serum cytokines in rats in the osteoarthritis experiment;
Figure 42 shows the effects of Compound 67 on safranin O-fast green staining and OARSI scores of rat knee joints in the osteoarthritis experiment;
Figure 43 shows the body weight and survival rate of MRL/Lpr lupus model mice within 8 weeks in the systemic lupus erythematosus experiment of Compound 67;
Figure 44 shows that Compound 67 significantly reduces the urinary microalbumin level in mice in the systemic lupus erythematosus experiment of Compound 67;
Figure 45 shows that Compound 67 significantly reduces the anti-double-stranded DNA antibody level in mice in the systemic lupus erythematosus experiment of Compound 67;
Figure 46 shows that Compound 67 significantly reduces the serum creatinine level in mice in the systemic lupus erythematosus experiment of Compound 67;
Figure 47 shows that Compound 67 regulates the number and ratio of T lymphocytes in mice in the systemic lupus erythematosus experiment of Compound 67.
Figure 48 shows the systemic lupus erythematosus experiment of Compound 67;
Figure 49 shows the peripheral clinical symptom scores of mice in each group in the mouse ankylosing spondylitis model (8 weeks);
Figure 50 shows the peripheral clinical symptom scores of mice in each group in the mouse ankylosing spondylitis model (12 weeks);
Figure 51 shows typical photographs of foot inflammation in mice in each group in the mouse ankylosing spondylitis model;
Figure 52 shows the spinal CT imaging scores of mice in each group in the mouse ankylosing spondylitis model;
Figure 53 shows typical spinal CT photographs of mice in each group in the mouse ankylosing spondylitis model; and
Figure 54 shows the skin reactions of each group after IMQ modeling.

### Modes for carrying out the invention

After extensive and in-depth research, the inventors found that S1PR4 agonists have therapeutic effects on certain autoimmune diseases at the animal level. Furthermore, the inventors synthesized candidate compounds with S1PR4 receptor regulatory activity. The inventors optimized the structure of the obtained candidate compounds, designed and synthesized a series of selective S1PR4 compounds that have not been reported in the literature, and conducted structural characterization. The inventors performed activity tests on this series of compounds at the cellular level and obtained a batch of compounds with S1PR4 agonistic activity. These compounds can regulate the activity of S1P receptors, among which the EC₅₀ value for S1PR4 agonistic activity reaches the nM level, showing a good targeting effect on S1PR4. The present invention was completed on this basis.

### Definition on terms

The definitions on some groups involved herein are as follows:
As used herein, "alkyl" refers to a saturated branched, straight-chain, or cycloalkyl group with a carbon chain length of 1-10 carbon atoms. Preferred alkyls include those with 1-5, 1-2, 1-6, 1-4, or 3-8 carbon atoms, and the like. Examples of alkyls include, but are not limited to: methyl, ethyl, n-propyl, isopropyl, n-butyl, isobutyl, heptyl, etc. Alkyls can be substituted by one or more substituents, such as halogens or haloalkyls. For example, an alkyl can be an alkyl substituted by 1-4 fluorine atoms, or it can be an alkyl substituted by a fluoroalkyl.

As used herein, "alkenyl" generally refers to a monovalent hydrocarbon group with at least one double bond, usually containing 2-8 carbon atoms, preferably 2-6 carbon atoms, and can be straight-chain or branched-chain. Examples of alkenyls include, but are not limited to, vinyl, propenyl, isopropenyl, butenyl, isobutenyl, hexenyl, and the like.

As used herein, "ester group" usually refers to carboxylic acid derivatives with at least one ester group, typically containing 3-8 carbon atoms, preferably 3-6 carbon atoms, and can be straight-chain or branched-chain. Examples of ester groups include, but are not limited to, methyl formate, ethyl formate, methyl acetate, ethyl acetate, propyl acetate, and the like.

As used herein, "hydroxyl" refers to branched or straight-chain alcohols with a carbon chain length of 1-10 carbon atoms, usually containing 1-10 carbon atoms, preferably 1-6 carbon atoms, and can be straight-chain or branched. Examples of ester hydroxyl include, but are not limited to, 1-hydroxy-n-butyl, 1-hydroxy-isobutyl, and so on.

As used herein, "amido group" refers to a group with the structural formula "-R'-NH-C(O)-R", where R' can be selected from a hydrogen or alkyl, and R can be selected from an alkyl, alkenyl, alkynyl, alkyl substituted by NR_{c}R_{d}, alkenyl substituted by NR_{c}R_{d}, alkynyl substituted by NR_{c}R_{d}, alkyl substituted by a halogen, and alkenyl substituted by cyano. Among them, R_{c} and R_{d} can be selected from an alkyl or alkenyl.

As used herein, "aryl" refers to monocyclic, bicyclic, or tricyclic aromatic groups containing 6 to 14 carbon atoms, including phenyl, naphthyl, phenanthryl, anthryl, indenyl, fluorenyl, tetrahydronaphthyl, dihydroindenyl, etc. The aryl group may optionally be substituted with 1-5 (for example, 1, 2, 3, 4, or 5) substituents selected from: a halogen, C₁₋₄ aldehyde group, C₁₋₆ alkyl, cyano, nitro, amino, amide group, hydroxyl, hydroxymethyl, halogen-substituted alkyl (such as trifluoromethyl), halogen-substituted alkoxy (such as trifluoromethoxy), carboxyl, C₁₋₄ alkoxy, ethoxy formyl, N(CH₃), C₁₋₄ acyl, and the like, heterocyclic group, or heteroaryl, and the like.

As used herein, "heterocyclic group" includes, but is not limited to, 5-membered or 6-membered heterocyclic groups containing 1-3 heteroatoms selected from O, S or N, including but not limited to furyl, thienyl, pyrrolyl, pyrrolidinyl, pyrazolyl, imidazolyl, triazolyl, oxazolyl, pyranyl, pyridyl, pyrimidinyl, pyrazinyl, piperidinyl, morpholinyl, etc.

As used herein, "aromatic heterocyclic group" refers to a group containing 5-14 ring atoms, with 6, 10, or 14 electrons shared in the ring system. Moreover, the contained ring atoms are carbon atoms and 1-3 heteroatoms selected from oxygen, nitrogen, or sulfur. Useful aromatic heterocyclic groups include piperazinyl, morpholinyl, piperidinyl, pyrrolidinyl, thienyl, furyl, pyranyl, pyrrolyl, imidazolyl, pyrazolyl, pyridyl, including but not limited to pyrimidinyl, etc. The aromatic heterocyclic group may optionally be substituted with 1-5 (for example, 1, 2, 3, 4, or 5) substituents selected from: a halogen, C₁₋₄ aldehyde group, C₁₋₆ straight-chain or branched alkyl, cyano, nitro, amino, hydroxyl, hydroxymethyl, halogen-substituted alkyl (such as trifluoromethyl), halogen-substituted alkoxy (such as trifluoromethoxy), carboxyl, C₁₋₄ alkoxy, ethoxy formyl, N(CH₃), and C₁₋₄ acyl.

As used herein, "alkoxy" refers to an oxygen group substituted by an alkyl. Preferred alkoxys are those with 1-6 carbon atoms in length, and more preferred are those with 1-3 carbon atoms in length. Examples of alkoxy includes, but is not limited to: a methoxy, ethoxy, propoxy, etc. Alkoxys can be substituted by one or more substituents, such as a halogen or haloalkyl. For example, an alkoxy can be an alkyl group substituted by 1-4 fluorine atoms, or the alkyl can be an alkyl substituted by a fluoroalkyl.

As used herein, "halogen" refers to a fluorine, chlorine, bromine, or iodine.

As used herein, "optionally substituted" means that the substituent it modifies can be optionally substituted with 1-5 (for example, 1, 2, 3, 4, or 5) substituents selected from the following: a halogen, C₁₋₄ aldehyde group, C₁₋₆ straight-chain or branched alkyl, cyano, nitro, amino, hydroxyl, hydroxymethyl, halogen-substituted alkyl (such as a trifluoromethyl), halogen-substituted alkoxy (such as a trifluoromethoxy), carboxyl, C₁₋₄ alkoxy, ethoxy formyl, N(CH₃), and C₁₋₄ acyl.

### Compounds of the present invention

The inventors discovered that both males and females in a family began to exhibit muscular dystrophy phenotypes from the age of 50, and these phenotypes worsened with increasing age, showing strong characteristics of a familial hereditary disease. Through studies such as whole-genome sequencing of this family, it was found that mutations in NDUFA10 and S1PR4 are potential pathogenic gene mutations for muscular dystrophy.

Given that there are currently no medicaments targeting S1PR4 in clinical practice, the S1P receptor regulators that are on the market or under research mainly focus on targets related to S1PR1 and S1PR5. The inventors have designed and synthesized a series of compounds with entirely new structures that have selectivity among different subtypes of S1PR. Therefore, the compounds of the present invention have different regulatory effects on different subtypes of S1PR; in other words, the compounds of the present invention are regulators of S1PR. In a specific embodiment, the compounds of the present invention are S1PR4 agonists. In a preferred embodiment, the compounds of the present invention have selectivity between S1PR4 and S1PR1; that is, the compounds of the present invention have an agonistic effect on S1PR4 but no agonistic effect on S1PR1.

In a specific embodiment, the compound of the present invention is a compound represented by the following formula I, or an optical isomer or a pharmaceutically acceptable salt thereof, 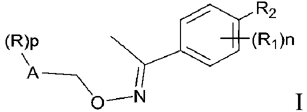 wherein A, R, R¹, n, R², m, and q are described as above.

In a preferred embodiment, the following compound is not included in the compound of the present invention:

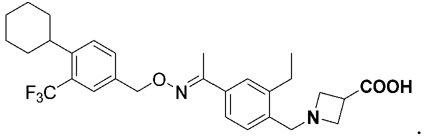

In a preferred embodiment, the compound of the present invention is represented by the following formula II: 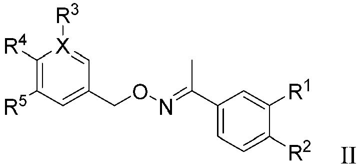 wherein R¹, R², X, R³, R⁴, and R⁵ are described as above.

In a preferred embodiment, the compound of the present invention is represented by the following formula III: 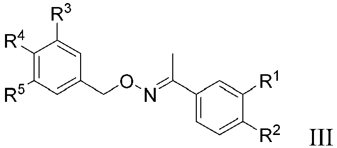 wherein R¹, R², R³, R⁴, and R⁵ are described as above.

Specifically, the present invention provides compounds selected from the following group, or an optical isomer or pharmaceutically acceptable salt thereof:

| No of compound | Structural formula |
|---|---|
| 1 | 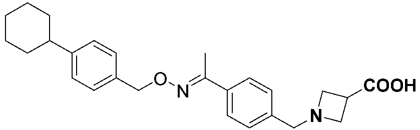 |
| 2 | 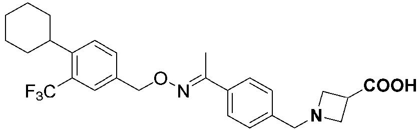 |
| 3 | 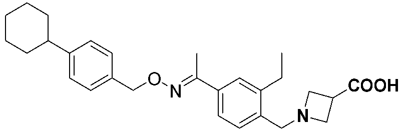 |
| 4 | 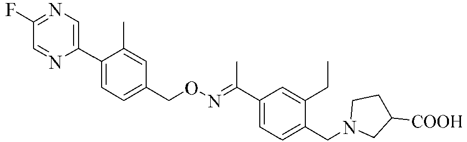 |
| 5 | 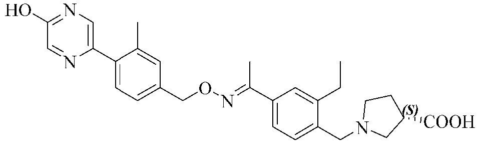 |
| 6 | 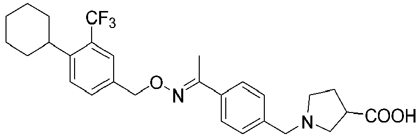 |
| 7 | 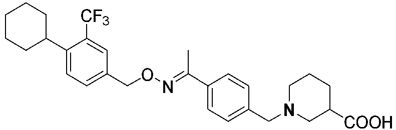 |
| 8 | 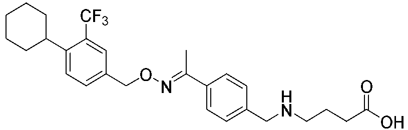 |
| 9 | 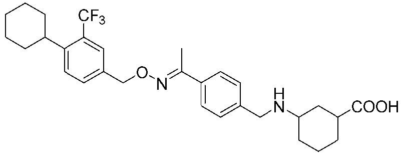 |
| 10 | 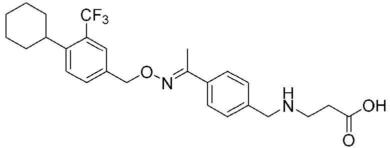 |
| 11 | 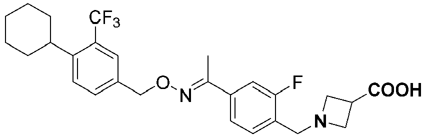 |
| 12 | 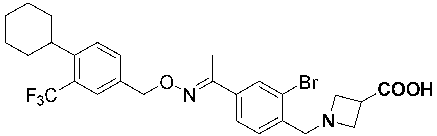 |
| 13 | 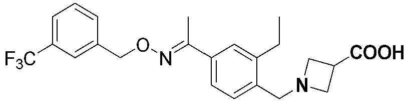 |
| 14 | 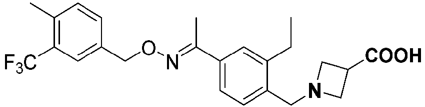 |
| 15 | 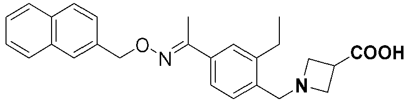 |
| 16 | 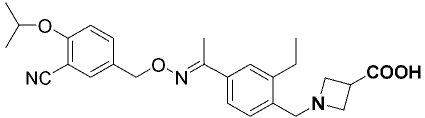 |
| 17 | 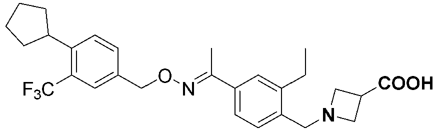 |
| 18 | 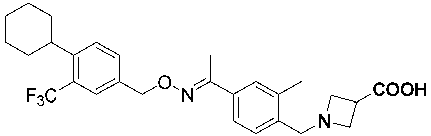 |
| 19 | 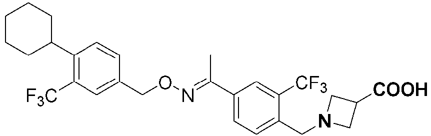 |
| 20 | 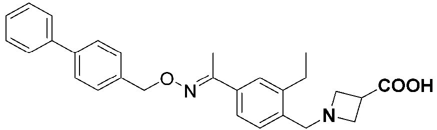 |
| 21 | 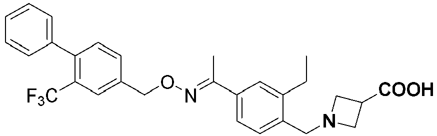 |
| 22 | 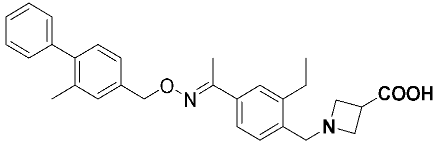 |
| 23 | 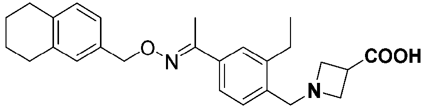 |
| 24 | 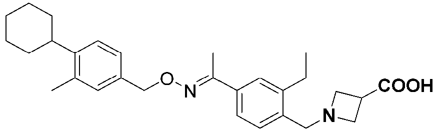 |
| 25 | 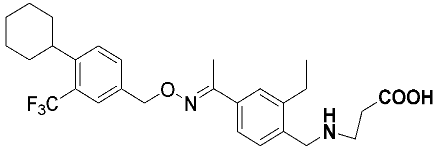 |
| 26 | 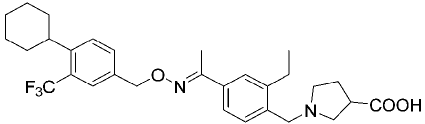 |
| 27 | 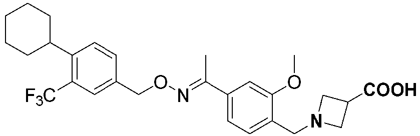 |
| 28 | 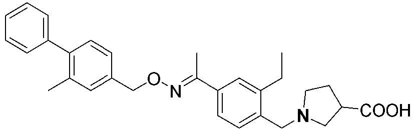 |
| 29 | 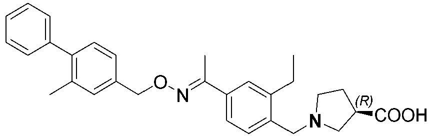 |
| 30 | 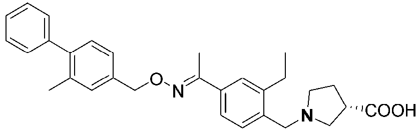 |
| 31 | 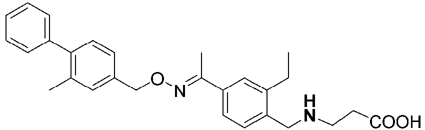 |
| 32 | 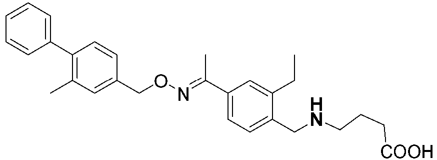 |
| 33 | 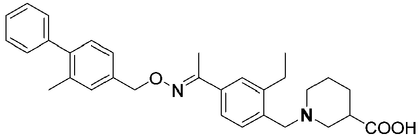 |
| 34 | 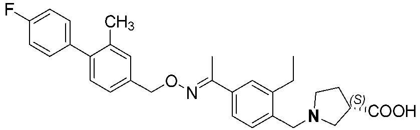 |
| 35 | 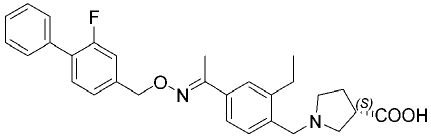 |
| 36 | 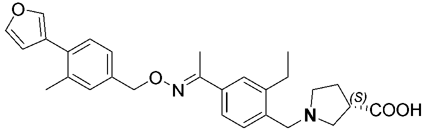 |
| 37 | 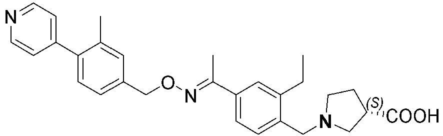 |
| 38 | 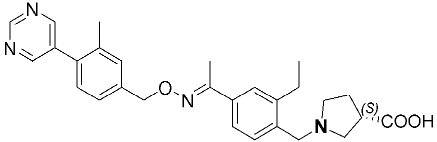 |
| 39 | 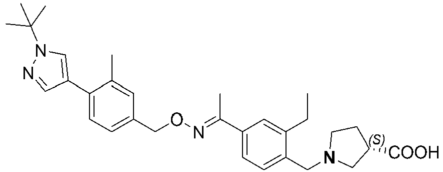 |
| 40 | 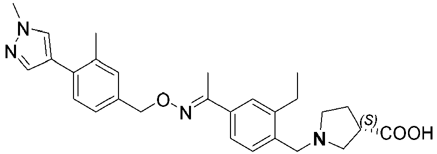 |
| 41 | 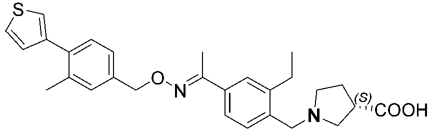 |
| 42 | 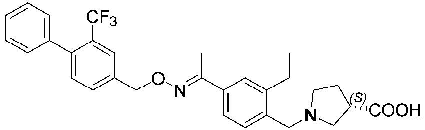 |
| 43 | 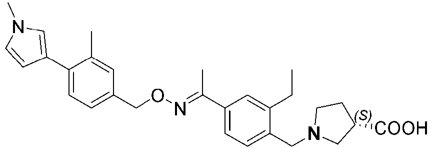 |
| 44 | 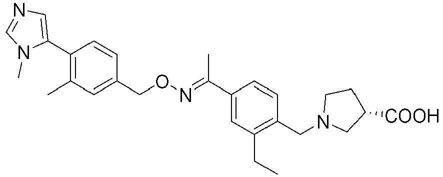 |
| 45 | 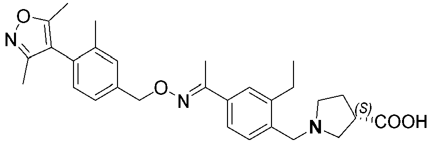 |
| 46 | 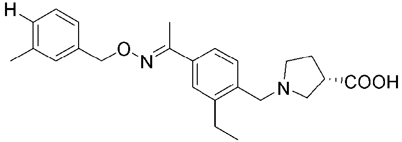 |
| 47 | 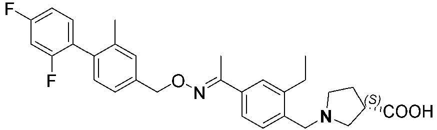 |
| 48 | 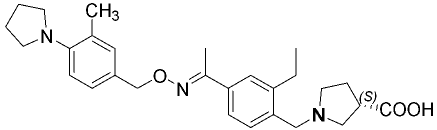 |
| 49 | 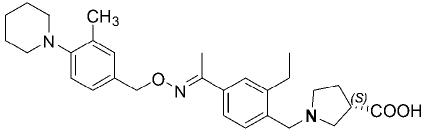 |
| 50 | 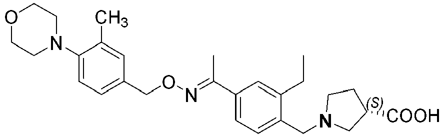 |
| 51 | 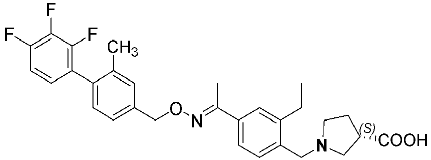 |
| 52 | 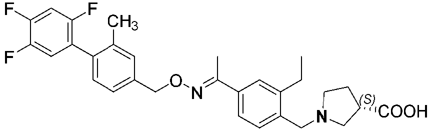 |
| 53 | 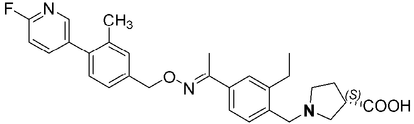 |
| 54 | 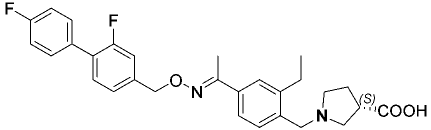 |
| 55 | 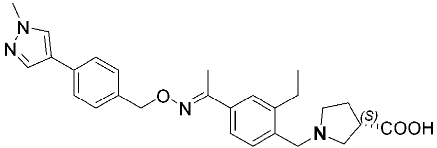 |
| 56 | 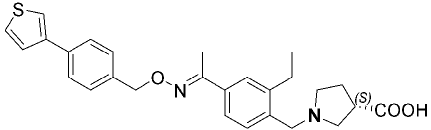 |
| 57 | 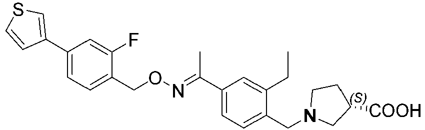 |
| 58 | 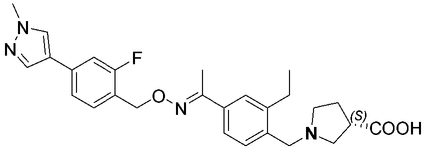 |
| 59 | 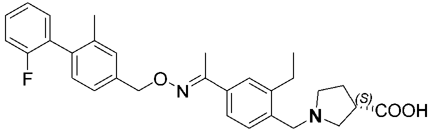 |
| 60 | 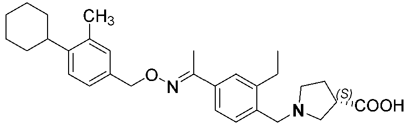 |
| 61 | 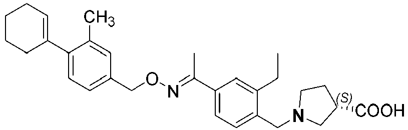 |
| 62 | 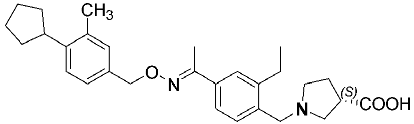 |
| 63 | 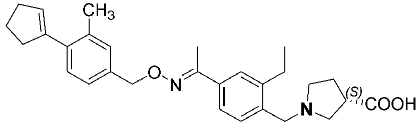 |
| 64 | 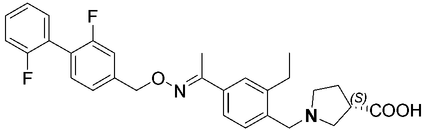 |
| 65 | 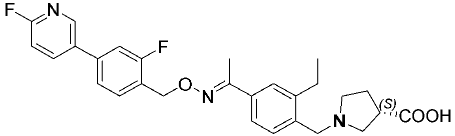 |
| 66 | 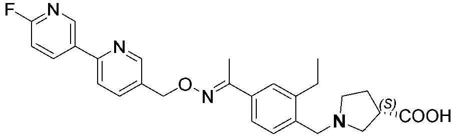 |
| 67 | 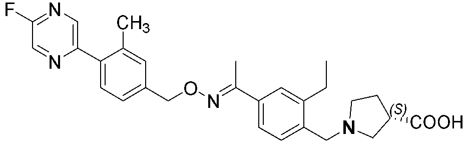 |
| 68 | 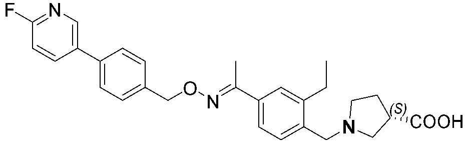 |
| 69 | 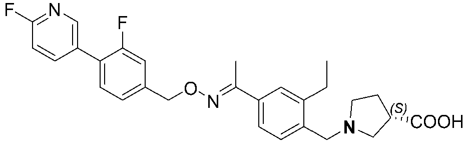 |
| 70 | 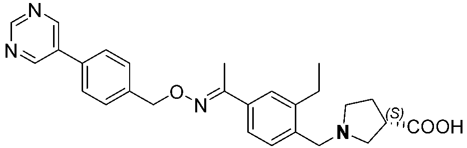 |
| 71 | 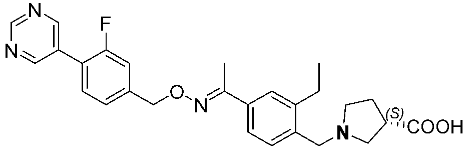 |
| 72 | 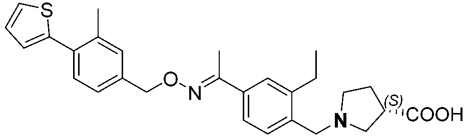 |
| 73 | 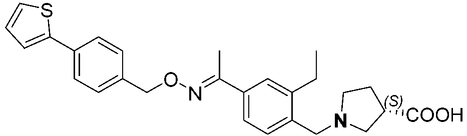 |
| 74 | 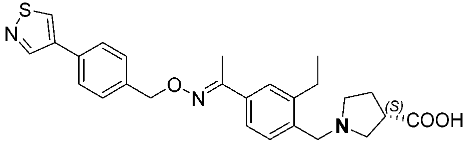 |
| 75 | 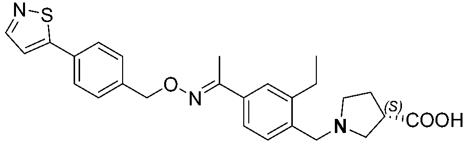 |
| 76 | 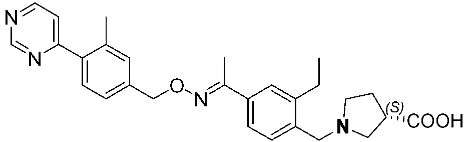 |
| 77 | 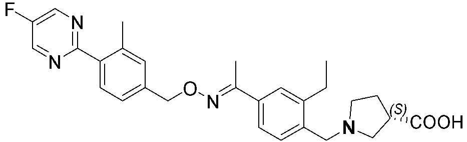 |
| 78 | 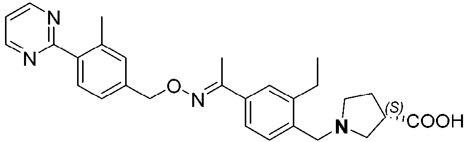 |
| 79 | 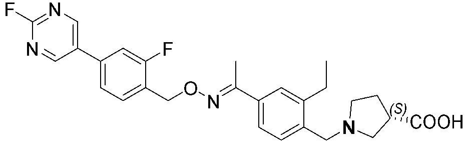 |
| 80 | 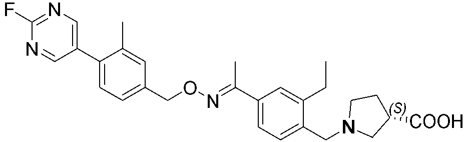 |
| 81 | 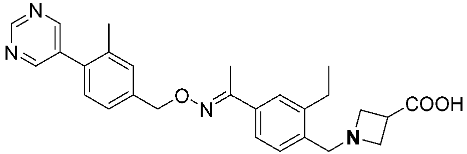 |
| 82 | 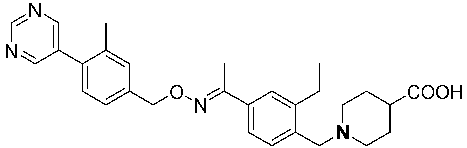 |
| 83 | 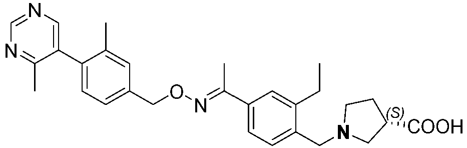 |
| 84 | 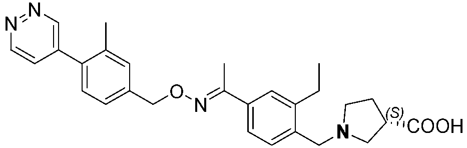 |
| 85 | 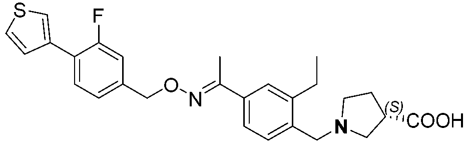 |
| 86 | 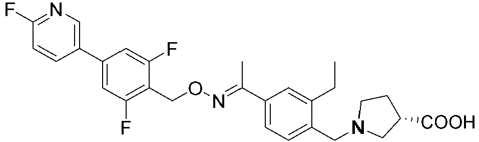 |
| 87 | 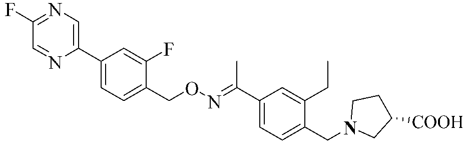 |
| 88 | 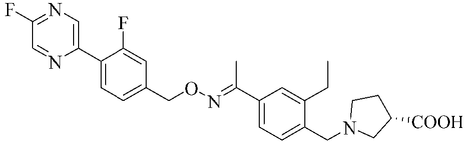 |
| 89 | 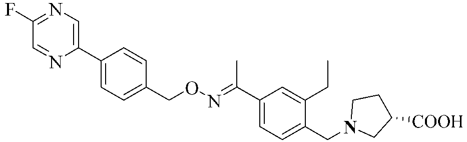 |
| 90 | 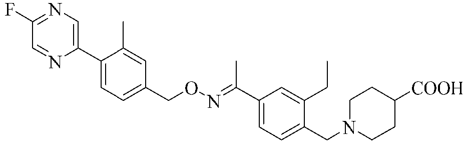 |
| 91 | 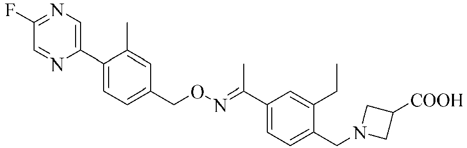 |
| 92 | 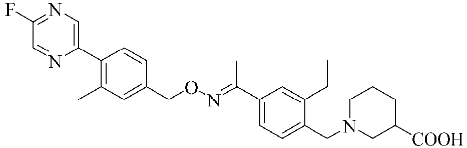 |
| 93 | 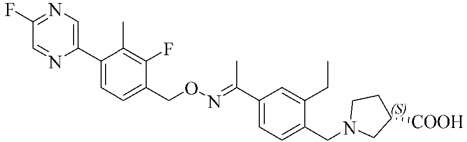 |
| 94 | 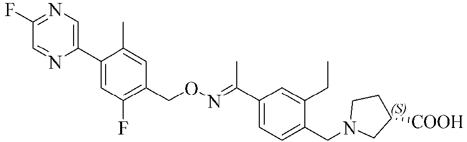 |
| 95 | 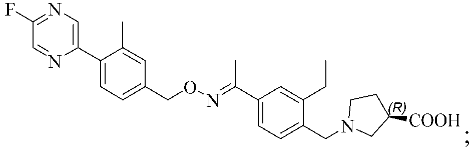 |

preferably, following compounds:

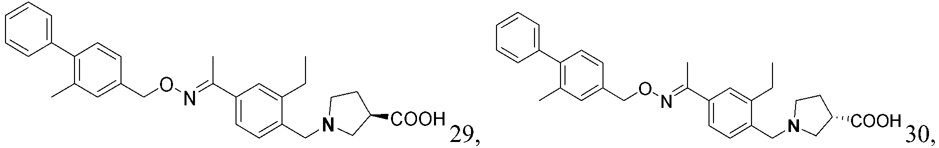

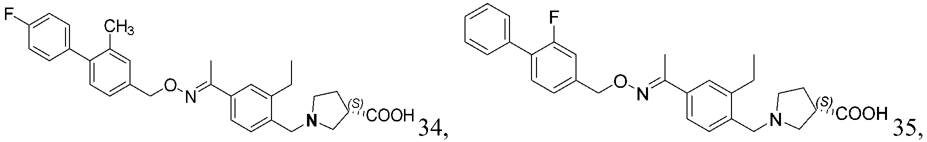

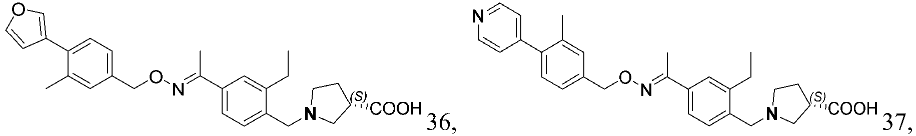

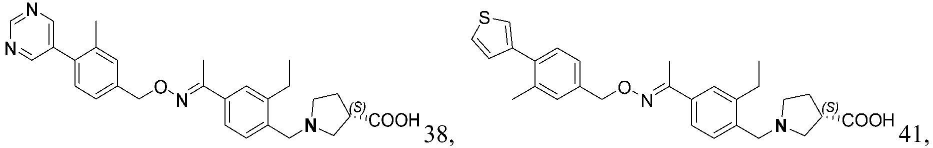

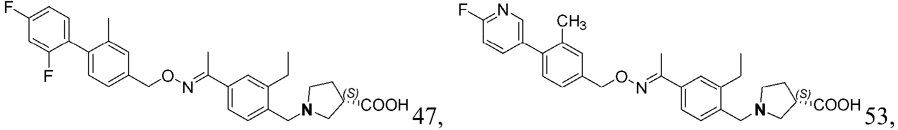

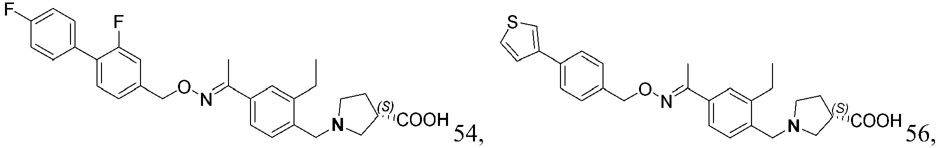

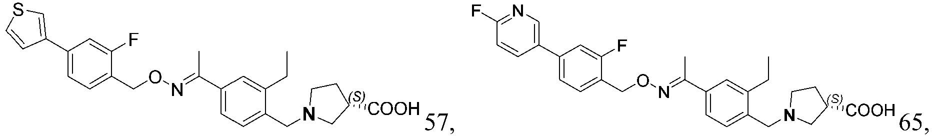

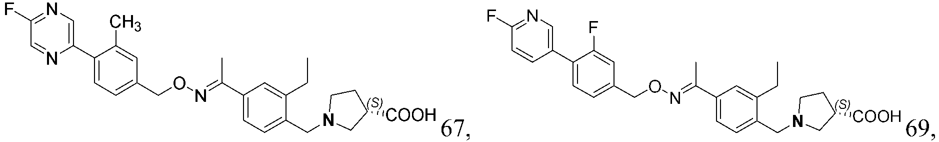

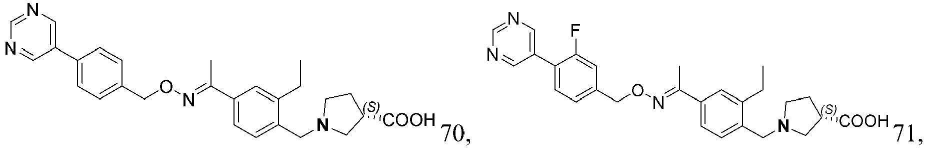

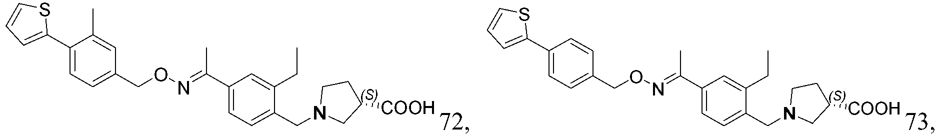

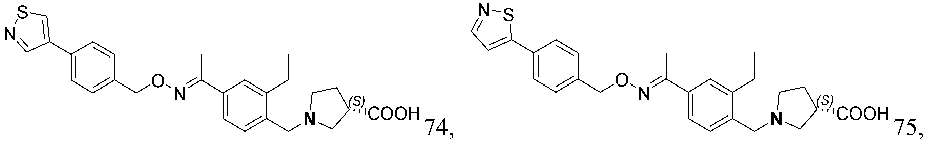

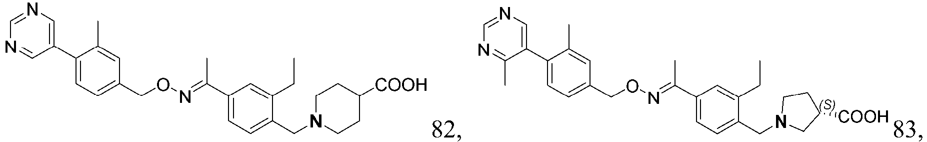

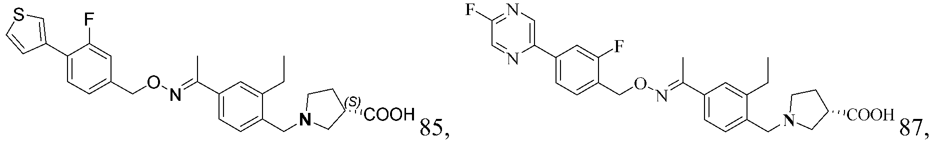

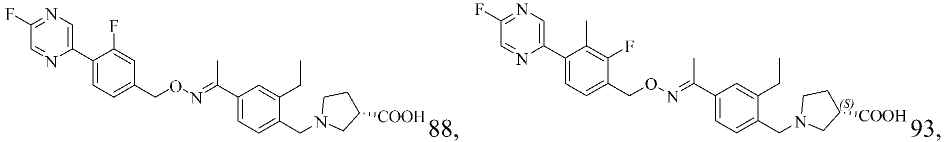

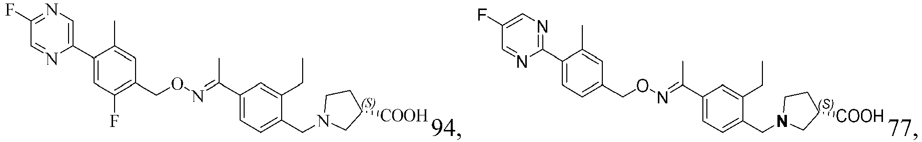

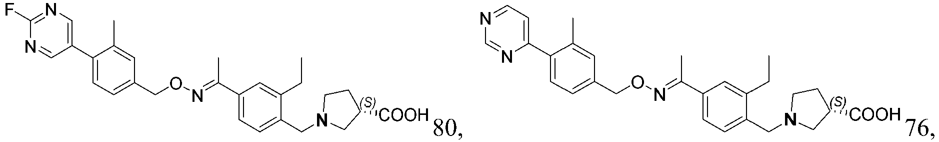

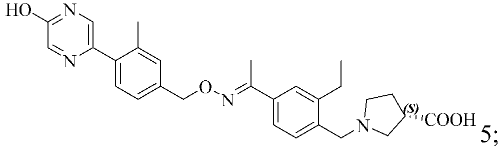

More preferably, following compounds:

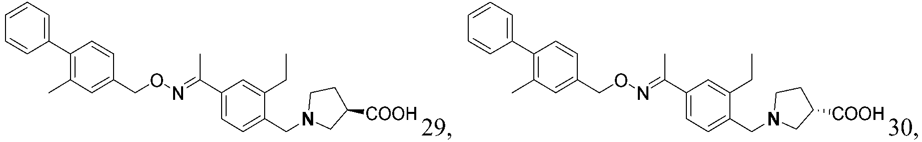

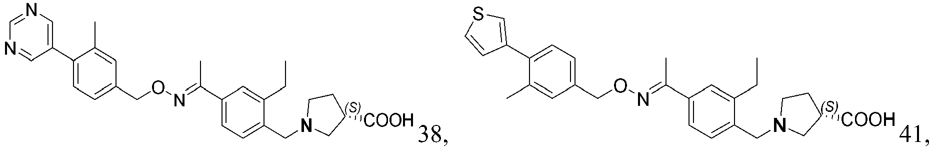

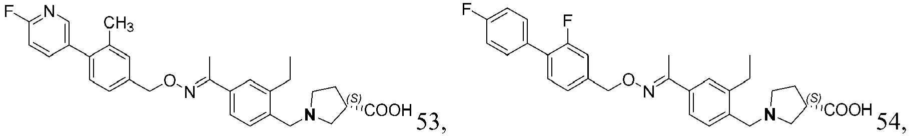

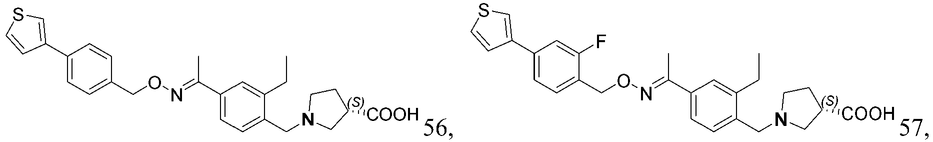

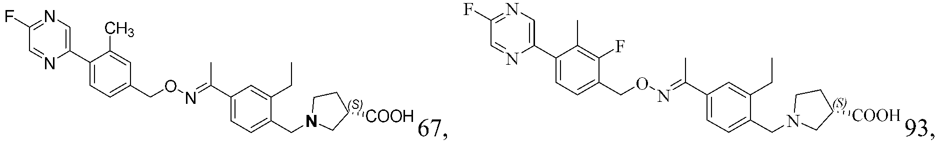

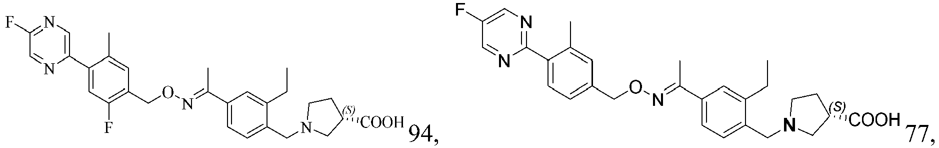

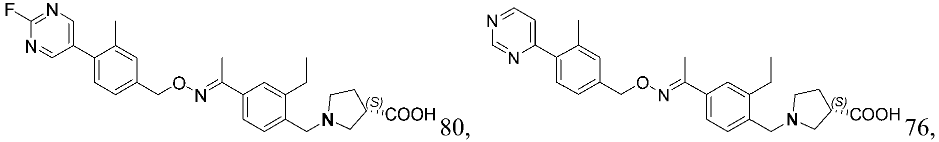

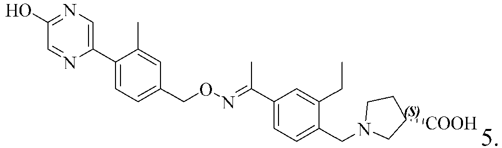

Based on the compounds of the present invention, the present invention provides a pharmaceutical composition, which contains a therapeutically effective amount of the compound of the present invention or a pharmaceutically acceptable salt thereof, as well as a pharmaceutically acceptable carrier or excipient.

Examples of pharmaceutically acceptable salts of the compounds of the present invention include, but are not limited to, inorganic and organic acid salts, such as hydrochloride, hydrobromide, sulfate, citrate, lactate, tartrate, maleate, fumarate, mandelate, and oxalate; as well as inorganic and organic base salts formed with bases, such as sodium hydroxide, tris(hydroxymethyl)aminomethane (TRIS, tromethamine), and N-methylglucamine.

The pharmaceutical composition of the present invention can be formulated into preparations suitable for various administration routes, including but not limited to those formulated for parenteral, subcutaneous, intravenous, intramuscular, intraperitoneal, transdermal, oral, intrathecal, intracranial, nasal, or topical administration, for the treatment of tumors and other diseases. The administered dosage is an amount effective to ameliorate or eliminate one or more conditions. For the treatment of a specific disease, an effective amount is a dosage sufficient to ameliorate or alleviate in some way the symptoms associated with the disease. Such an amount may be administered as a single dose or according to an effective treatment regimen. The administered dosage may cure the disease, but administration is usually intended to ameliorate the symptoms of the disease. Repeated administration is generally required to achieve the desired improvement in symptoms. The dosage will be determined according to the patient's age, health and weight, the type of concurrent treatment, the frequency of treatment, and the desired therapeutic effect.

The pharmaceutical preparation of the present invention can be administered to any mammal, as long as they can obtain the therapeutic effect of the compound of the present invention. Among these mammals, humans are the most important.

The compounds of the present invention or a pharmaceutical composition thereof can be used for the preparation of S1PR regulators. In a specific embodiment, the S1PR regulators are S1PR4 agonists and S1PR4 mutant agonists. Therefore, the compounds of the present invention or a pharmaceutical composition thereof can be used for treating various diseases mediated by S1PR4 (including its mutants), including autoimmune diseases. In a specific embodiment, autoimmune diseases mediated by said S1PR4 (including mutants thereof) include, but are not limited to, multiple sclerosis, rheumatoid arthritis, systemic lupus erythematosus, psoriasis, ulcerative colitis, Crohn's disease, etc.

The pharmaceutical preparations of the present invention can be manufactured in a known way. For example, they can be produced by traditional mixing, granulating, tabletting, dissolving, or freeze-drying processes. When manufacturing oral preparations, solid excipients can be combined with the active compound, and the mixture can be optionally ground. If needed or necessary, after adding an appropriate amount of auxiliaries, the granular mixture is processed to obtain tablets or dragee cores.

Suitable excipients are, especially fillers, such as sugars like lactose or sucrose, mannitol or sorbitol; cellulose preparations or calcium phosphates, such as tricalcium phosphate or dicalcium phosphate; and binders, such as starch pastes including corn starch, wheat starch, rice starch, potato starch, gelatin, tragacanth, methyl cellulose, hydroxypropyl methyl cellulose, sodium carboxymethyl cellulose, or polyvinylpyrrolidone. If necessary, disintegrants can be added, such as the starches mentioned above, as well as carboxymethyl starch, crospovidone, agar, or alginic acid or its salts, such as sodium alginate. Auxiliaries, especially flow regulators and lubricants, for example, silica, talc, stearates such as calcium magnesium stearate, stearic acid or polyethylene glycol. If needed, a suitable coating that can resist gastric juice can be provided for the tablet core. For this purpose, concentrated sugar solutions can be applied. This solution can contain gum arabic, talc, polyvinylpyrrolidone, polyethylene glycol and/or titanium dioxide, lacquer solutions and suitable organic solvents or solvent mixtures. To prepare gastric juice-resistant coatings, appropriate cellulose solutions can be used, such as cellulose acetate phthalate or hydroxypropyl methyl cellulose phthalate. Dyes or pigments can be added to the coating of tablets or tablet cores, for example, to identify or characterize combinations of active ingredient dosages.

Based on the above compounds and pharmaceutical compositions, the present invention further provides a method for treating diseases mediated by S1PR4, comprising administering the compound or pharmaceutical composition of the present invention to a subject in need thereof.

The administration methods include, but are not limited to, various administration methods well known in the art, which can be determined according to the actual situation of a patient. These methods include, but are not limited to, parenteral, subcutaneous, intravenous, intramuscular, intraperitoneal, transdermal, oral, intrathecal, intracranial, nasal or topical administration routes.

In view of the fact that the compound of the present invention or a pharmaceutical composition thereof can be used to prepare S1PR regulators, the present invention also provides a method for regulating S1PR in a subject, comprising administering an effective amount of the compound of the present invention, or an optical isomer, pharmaceutically acceptable salt or pharmaceutical composition thereof to a subject in need thereof. In a specific embodiment, the method is a method for activating S1PR4 in a subject; more preferably, the method activates S1PR4 in a subject without activating S1PR1.

### Advantages of the present invention:

1. The compound provided in the present invention is a novel S1PR regulator, especially a selective S1PR4 receptor regulator;
2. The compound provided in the present invention has excellent biological activity on S1PR4 and S1PR4 mutant proteins;
3. The compound provided in the present invention lays a foundation for the development of medicaments that can target the S1PR4 target, has great prospects for industrialization and commercialization as well as market value, and has significant economic benefits.

The following further describes the technical solution of the present invention in conjunction with specific implementation cases, however the following examples do not constitute a limitation to the present invention. All application methods adopted based on the principles and technical means of the present invention shall fall within the scope of the present invention. For the experimental methods in the following examples where specific conditions are not indicated, they are usually carried out in accordance with conventional conditions or the conditions suggested by the manufacturer. Unless otherwise specified, percentages and parts are calculated by weight.

### Material and method

### Compounds of the present invention are synthesized as follows:

### Example 1

### Synthesis of compounds

### Synthesis of 4-(bromomethyl)-2-methyl-1,1'-biphenyl

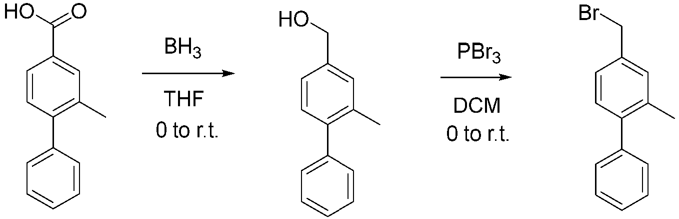

3-methyl-4-phenylbenzoic acid (2.00 g, 9.40 mmol) was dissolved in 10 mL of anhydrous tetrahydrofuran, and stirred in an ice bath. 1.0 M borane-tetrahydrofuran solution (14 mL, 14.20 mmol) was added dropwise. Upon the dropwise addition, the mixture was thawed to room temperature and stirred. After 2 hours, the reaction was complete, and quenched by adding water dropwise, then extracted with dichloromethane, washed with saturated brine. the organic phases were combined, dried over anhydrous sodium sulfate, and concentrated under a reduced pressure to obtain 1.50 g of a colorless oil with a yield of 80.6%. The product was directly used in the next step without further purification. LC-MS (ESI): m/z: 199.1 (M+H)+.

(2-methyl-[1,1'-biphenyl]-4-yl)methanol (1.50 g, 7.60 mmol) was dissolved in 10 mL of dichloromethane, and phosphorus tribromide (820 mg, 3.10 mmol) was added dropwise under ice bath. Upon the dropwise addition, the mixture was thawed to room temperature and stirred. After 0.5 hour, the reaction was complete, quenched with water, extracted with dichloromethane, and washed with saturated brine. The organic phases were combined, dried over anhydrous sodium sulfate, and concentrated under a reduced pressure to obtain 1.80 g of a colorless oil with a yield of 91.0%.

¹H NMR (400 MHz, DMSO-*d*₆) δ 7.45 (dd, *J* = 14.9, 7.9 Hz, 2H), 7.39-7.30 (m, 5H), 7.18 (d, *J* = 7.8 Hz, 1H), 4.72 (s, 2H), 2.21 (s, 3H). LC-MS (ESI): m/z: 261.3 (M+H)⁺.

### Synthesis of (E)-Ethyl N-((2-methyl-[1,1'-biphenyl]-4-yl)methoxy)acetamidocarbamate

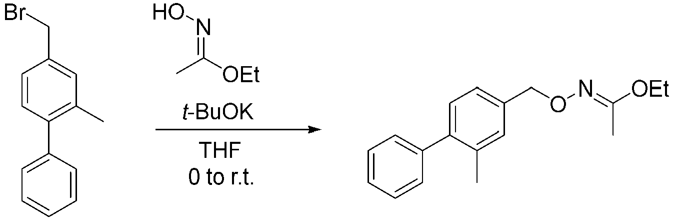

Ethyl acetohydroxamate (400 mg, 3.90 mmol) was dissolved in 10 mL of tetrahydrofuran solution and then placed in an ice bath. A 1.0 M solution of potassium tert-butoxide in tetrahydrofuran (15.2 mL, 15.20 mmol) was added dropwise to the reaction system. Upon the dropwise addition, the reaction system was stirred in the ice bath for another 0.5 hours. 4-(bromomethyl)-2-methyl-1,1'-biphenyl (1.01 g, 3.90 mmol) was dissolved in 5 mL of tetrahydrofuran and then added dropwise to the reaction system. Upon the dropwise addition, the mixture was thawed to room temperature and stirred, with the reaction progress monitored by TLC. After the reaction was completed, water was added to quench the reaction, followed by extraction with dichloromethane and washing with saturated brine. The combined organic phases were dried over anhydrous sodium sulfate, concentrated, and purified by silica gel column chromatography to obtain 821 mg of a colorless oily liquid with a yield of 74.4%.

¹H NMR (400 MHz, DMSO-*d*₆) δ 7.43 (t, *J* = 7.3 Hz, 2H), 7.38-7.30 (m, 3H), 7.27-7.16 (m, 3H), 4.89 (s, 2H), 3.95 (q, *J* = 7.5 Hz, 2H), 2.22 (s, 3H), 1.90 (s, 3H), 1.20 (t, *J* = 7.5 Hz, 3H).

### Synthesis of (E)-1-(3-ethyl-4-(hydroxymethyl)phenyl)ethan-1-one O-((2-methyl-[1,1'-biphenyl]-4-yl)methyl)oxime

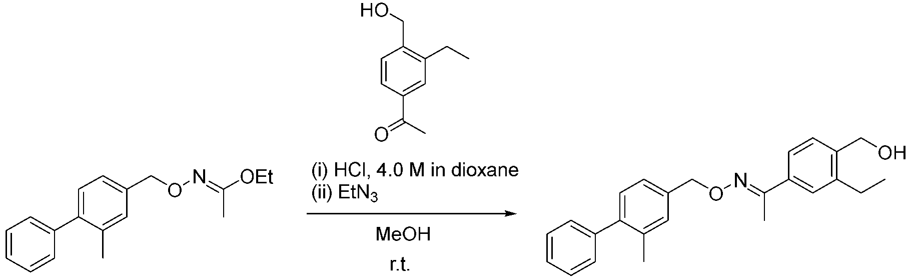

Ethyl (E)-*N*-((2-methyl-[1,1'-biphenyl]-4-yl)methoxy)acetimidate (200 mg, 0.70 mmol) was dissolved in 5 mL of methanol. A 4.0 M solution of hydrogen chloride in dioxane (0.4 mL, 0.30 mmol) was added dropwise to the reaction mixture. After stirring for 0.5 hours, triethylamine was added dropwise to adjust the pH to between 4 and 6. Then, 1-(3-ethyl-4-(hydroxymethyl)phenyl)ethanone (125 mg, 0.70 mmol) was dissolved in 2 mL of methanol and added dropwise to the reaction system. The progress of the reaction was monitored by TLC. After the reaction was completed, water was added to quench the reaction, followed by extraction with dichloromethane and washing with saturated brine. The combined organic phases were dried over anhydrous sodium sulfate, concentrated, and purified by silica gel column chromatography to obtain a colorless oily liquid (140 mg) with a yield of 53.6%.

¹H NMR (400 MHz, CDCl₃) δ 7.46 (s, 1H), 7.42 (dd, *J* = 8.0, 1.6 Hz, 1H), 7.36-7.27 (m, 4H), 7.29-7.18 (m, 3H), 7.18-7.06 (m, 2H), 5.18 (s, 2H), 4.66 (s, 2H), 2.65 (q, *J* = 7.5 Hz, 2H), 2.21 (s, 3H), 1.50 (s, 3H), 1.18 (t, *J* = 7.5 Hz, 3H).

### Synthesis of (E)-2-ethyl-4-(1-((2-methyl-[1,1'-biphenyl]-4-yl)methoxy)iminoethyl)benzaldehyde

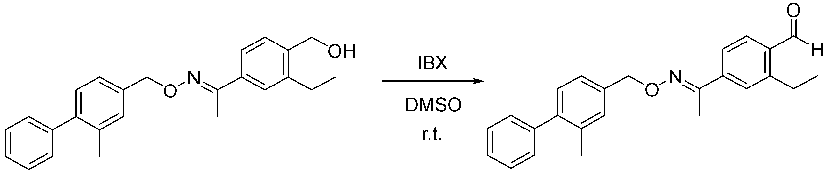

(*E*)-1-(3-ethyl-4-(hydroxymethyl)phenyl)ethan-1-one *O*-((2-methyl-[1,1'-biphenyl]-4-yl)methyl)oxime (112 mg, 0.30 mmol) was dissolved in 3 mL of DMSO solvent. 2-iodoxybenzoic acid (IBX) oxidant (84 mg, 0.30 mmol) was added. Then the reaction system was stirred at room temperature for approximately 2 hours. The reaction progress was monitored by TLC. After the reaction is completed, the reaction was quenched by adding water, extracted with dichloromethane, and washed with saturated brine. The organic phases were combined, dried over anhydrous sodium sulfate, and concentrated to obtain a colorless oily liquid (99 mg) with a yield of 89.1%.

¹H NMR (400 MHz, CDCl₃) δ 10.29 (s, 1H), 7.82 (d, *J* = 6.4 Hz, 1H), 7.64 (dd, *J* = 12.9, 5.5 Hz, 1H), 7.60 (d, *J* = 9.7 Hz, 1H), 7.40 (dd, *J* = 18.7, 11.4 Hz, 2H), 7.36-7.30 (m, 5H), 7.25-7.19 (m, 1H), 5.29 (s, 2H), 3.09 (q, *J* = 7.5 Hz, 2H), 2.31 (s, 3H), 2.30 (s, 3H), 1.33 (t, *J* = 7.5 Hz, 3H).

### Synthesis of (S, E)-1-(2-ethyl-4-(1-((2-methyl-[1,1'-biphenyl]-4-yl)methoxy)imino)ethyl)benzyl)pyrrolidine-3-carboxylic acid (Compound 30)

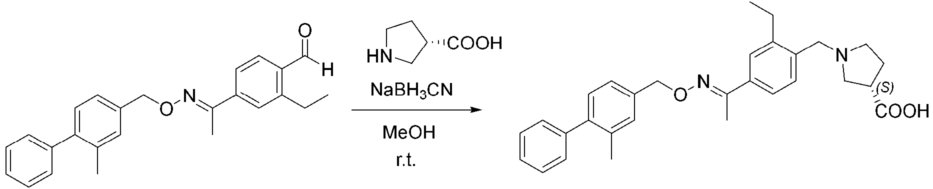

(*E*)-2-ethyl-4-(1-((2-methyl-[1,1'-biphenyl]-4-yl)methoxy)iminoethyl)benzaldehyde (74 mg, 0.20 mmol) was dissolved in 5 mL of methanol, 1 drop of acetic acid was added, and then *S-*pyrrolidine-3-carboxylic acid (35 mg, 0.30 mmol) was added. The reaction system was stirred at room temperature for 3 minutes, and then sodium cyanoborohydride (19 mg, 0.30 mmol) was added. The reaction progress was monitored by TLC. After the reaction is complete, the reaction system was distilled under a reduced pressure, extracted by adding water and dichloromethane, and washed with saturated brine. The organic phases were combined, dried over anhydrous sodium sulfate, concentrated, and pass through a silica gel column to obtain 29 mg of a white solid with a yield of 30.8%.

¹H NMR (400 MHz, CD₃OD) δ 7.57 (s, 1H), 7.51 (d, *J* = 8.0 Hz, 1H), 7.44-7.35 (m, 3H), 7.33-7.26 (m, 5H), 7.17 (d, *J* = 7.7 Hz, 1H), 5.20 (s, 2H), 4.02 (s, 2H), 3.19 (t, *J* = 8.6 Hz, 1H), 3.11-3.00 (m, 3H), 2.90 (dd, *J* = 17.2, 7.9 Hz, 1H), 2.79 (q, *J* = 7.5 Hz, 2H), 2.26 (s, 3H), 2.24 (s, 3H), 2.15 (dd, *J* = 14.6, 7.3 Hz, 2H), 1.23 (t, *J* = 7.5 Hz, 3H). ¹³C NMR (151 MHz, CD₃OD) δ 179.95, 154.70, 143.25, 141.79, 141.48, 137.08, 136.62, 134.91, 130.01, 129.84, 129.31, 128.79, 127.77, 126.49, 126.28, 125.37, 123.57, 75.60, 57.40, 55.54, 53.76, 44.56, 28.06, 25.20, 19.22, 14.48, 11.50. HRMS (ESI) (m/z): [M+H]⁺ calcd for C₃₀H₃₄N₂O₃, 471.2648; found: 471.2650. HPLC Purity: 98.16%, retention time: 1.52 min.

The synthetic route of representative compound 13 is as follows:

### Synthesis of (E)-Ethyl N-((3-(trifluoromethyl)benzyl)oxy)acetimidate

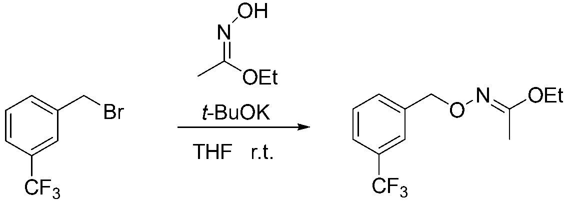

Ethyl acetohydroxamate (865 mg, 8.40 mmol) was dissolved in 10 mL of tetrahydrofuran solution and then placed in an ice bath. A 1.0 M solution of potassium tert-butoxide in tetrahydrofuran (17 mL, 16.70 mmol) was added dropwise to the reaction system. Upon the dropwise addition, the reaction system was stirred in the ice bath for another 0.5 hours. 3-(trifluoromethyl)benzyl bromide (1.99 g, 8.40 mmol) was dissolved in 5 mL of tetrahydrofuran and then added dropwise to the reaction system. Upon the dropwise addition, the mixture was stirred at room temperature, and the progress of the reaction was monitored by TLC. After the reaction was completed, water was added to quench the reaction, followed by extraction with dichloromethane and washing with saturated brine. The combined organic phases were dried over anhydrous sodium sulfate, concentrated, and passed through a silica gel column to obtain 1.81 g of a colorless oily liquid with a yield of 82.6%.

¹H NMR (400 MHz, CDCl₃) *δ* 7.63 (s, 1H), 7.54 (d, *J* = 7.1 Hz, 2H), 7.45 (t, *J* = 7.7 Hz, 1H), 4.97 (s, 2H), 3.98 (q, *J* = 7.1 Hz, 2H), 1.95 (s, 3H), 1.24 (t, *J* = 7.1 Hz, 3H).

### Synthesis of (E)-1-(3-ethyl-4-(hydroxymethyl)phenyl)ethan-1-one O-(3-(trifluoromethyl)benzyl)oxime

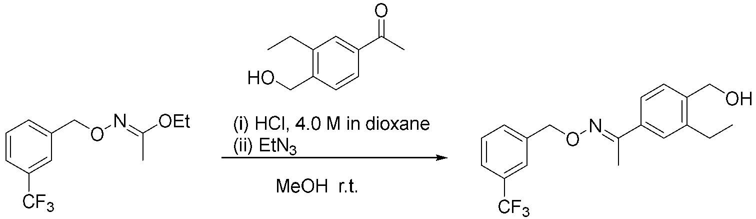

ethyl (*E*)-N-((3-(trifluoromethyl)benzyl)oxy)acetimidate (157 mg, 0.60 mmol) was dissolved in 5 mL of methanol. 0.4 mL of 4.0 M hydrogen chloride was added in dioxane solution dropwise to the reaction mixture. The reaction system was stirred for 0.5 hours, and then triethylamine was added dropwise to adjust the pH to between 4-6. Then, 1-(3-ethyl-4-(hydroxymethyl)phenyl)ethanone (107 mg, 0.60 mmol) was dissolved in 2 mL of methanol and added dropwise to the reaction system. The reaction progress was monitored by TLC. After the reaction is completed, the reaction was quenched by adding water, extracted with dichloromethane, and washed with saturated brine. The organic phases were combined, dried over anhydrous sodium sulfate, concentrated, and passed through a silica gel column to obtain 150 mg of a colorless oily liquid with a yield of 71.1%.

¹H NMR (400 MHz, CDCl₃) *δ* 7.69 (s, 1H), 7.59 (dd, *J* = 12.7, 7.9 Hz, 3H), 7.51-7.46 (m, 2H), 7.37 (d, *J* = 8.0 Hz, 1H), 5.28 (s, 2H), 4.74 (s, 1H), 4.72 (s, 2H), 2.71 (q, *J* = 7.6 Hz, 2H), 2.28 (s, 3H), 1.24 (t, *J* = 7.5 Hz, 3H).

### Synthesis of (E)-2-ethyl-4-(1-(((3-(trifluoromethyl)phenyl)oxy)imino)ethyl)benzaldehyde

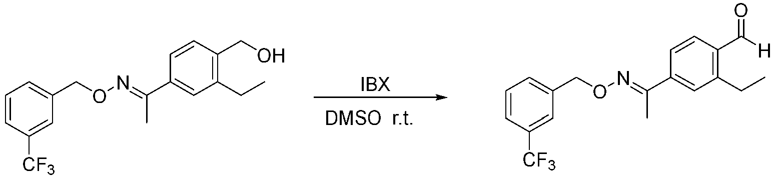

(*E*)-1-(3-ethyl-4-(hydroxymethyl)phenyl)ethan-1-one *O*-(3-(trifluoromethyl)benzyl)oxime (211 mg, 0.60 mmol) was dissolved in 3 mL of DMSO solvent. IBX oxidant (308 mg, 1.10 mmol) was added. Then, the mixture was stirred at room temperature for approximately 2 hours. The progress of the reaction was monitored by TLC. After the reaction was completed, water was added to quench the reaction, followed by extraction with dichloromethane and washing with saturated brine. The combined organic phases were dried over anhydrous sodium sulfate, concentrated, and then purified by silica gel column chromatography to obtain a colorless oily liquid (126 mg) with a yield of 60.0%.

¹H NMR (400 MHz, CDCl₃) *δ* 10.29 (s, 1H), 7.82 (d, *J* = 8.1 Hz, 1H), 7.69 (s, 1H), 7.64-7.53 (m, 4H), 7.50 (d, *J* = 7.7 Hz, 1H), 5.31 (s, 2H), 3.08 (d, *J* = 7.5 Hz, 2H), 2.30 (s, 3H), 1.28 (t, *J* = 7.5 Hz, 3H).

### Synthesis of (E)-1-(2-ethyl-4-(1-((3-(trifluoromethyl)phenyl)oxy)imino)ethyl)benzyl)azetidine-3-carboxylic acid (Compound 13)

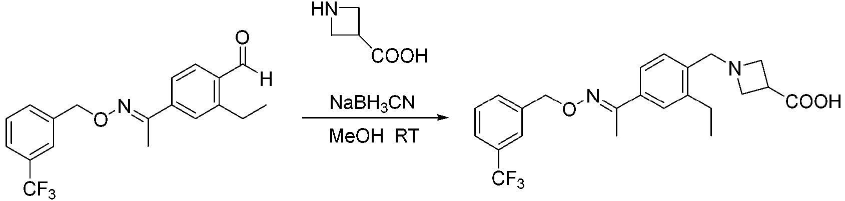

(*E*)-2-ethyl-4-(1-(((3-(trifluoromethyl)phenyl)oxy)imino)ethyl)benzaldehyde (105 mg, 0.30 mmol) was dissolved in 5 mL of methanol, 1 drop of acetic acid was added, and then 3-azetidinecarboxylic acid (61 mg, 0.60 mmol) added. After the reaction system was stirred at room temperature for 3 minutes, sodium cyanoborohydride (38 mg, 0.60 mmol) was added. The reaction progress was monitored by TLC. After the reaction is complete, the reaction system was distilled under a reduced pressure, an appropriate amount of water was added, extracted with dichloromethane, washed with saturated brine. The organic phases were combined, dried over anhydrous sodium sulfate, concentrated, and passed through a silica gel column to obtain 78 mg of an oily substance with a yield of 60.0%.

¹H NMR (400 MHz, CD₃OD) δ 7.71 (s, 1H), 7.67 (d, *J* = 7.4 Hz, 1H), 7.62-7.51 (m, 3H), 7.47 (dd, *J* = 8.0, 1.6 Hz, 1H), 7.31 (d, *J* = 8.0 Hz, 1H), 5.28 (s, 2H), 3.98 (s, 2H), 3.83 (t, *J* = 8.8 Hz, 2H), 3.67 (t, *J* = 8.4 Hz, 2H), 3.28 (dd, J = 15.2, 6.9 Hz, 1H), 2.73 (q, *J* = 7.5 Hz, 2H), 2.26 (s, 3H), 1.21 (t, *J* = 7.5 Hz, 3H). ¹³C NMR (151 MHz, CD₃OD) δ 178.20, 155.32, 142.88, 139.73, 136.16, 133.52, 131.41, 130.23 (q, *J* = 32.0 Hz), 128.87, 128.82, 126.17, 124.32, 124.31 (q, *J* = 272.0 Hz), 124.06, 123.61, 74.70, 57.68, 35.94, 25.11, 14.28, 11.47. HRMS (ESI) (m/z): [M+H]⁺ calcd for C₂₃H₂₅F₃N₂O₃, 435.1896; found: 435.1897. HPLC Purity: 99.79%, retention time: 1.17 min.

The synthetic route of the representative compound 16 is shown as follows:

### Synthesis of 5-(bromomethyl)-2-isopropoxybenzonitrile

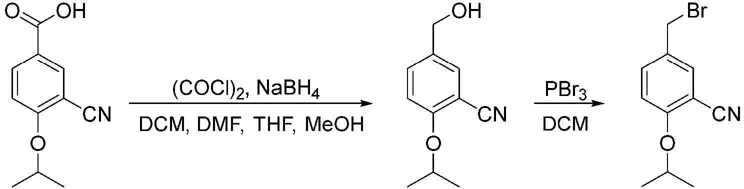

A 2.0 M solution of oxalyl chloride in dichloromethane (14.6 mL, 29.20 mmol) was added to 3-cyano-4-(isopropoxy)benzoic acid (2.01 g, 9.80 mmol) along with one drop of DMF. The reaction mixture was stirred at room temperature for 0.5 hours and then concentrated under a reduced pressure. 3-cyano-4-(isopropoxy)benzoyl chloride (923 mg, 4.50 mmol) was dissolved in 10 mL of tetrahydrofuran and cooled to 0°C. Sodium borohydride (414 mg, 11.20 mmol) was added, followed by 2 mL of methanol. The reaction mixture was stirred at 0°C for 20 minutes and then thawed to room temperature. After 2 hours, the reaction mixture was acidified to pH = 3 with 1.0 M hydrochloric acid. The aqueous layer was extracted with ethyl acetate, and the combined organic layers were dried over anhydrous sodium sulfate and concentrated to obtain a colorless oil (1.12 g) with a yield of 59.9%.

¹H NMR (400 MHz, CDCl₃) *δ* 7.48 (s, 1H), 7.47 (d, *J* = 12 Hz, 1H), 7.45 (s, 1H), 6.92 (d, *J* = 8.6 Hz, 1H), 4.61 (dt, *J* = 12.2, 6.2 Hz, 1H), 4.57 (s, 2H), 1.36 (d, *J* = 6.1 Hz, 6H).

5-(Hydroxymethyl)-2-isopropoxybenzonitrile (993 mg, 5.20 mmol) was dissolved in 10 mL of dichloromethane. Phosphorus tribromide (0.2 mL, 2.10 mmol) was added dropwise under an ice bath. Upon the dropwise addition, the mixture was stirred at room temperature, and the reaction was complete after 0.5 hours. The reaction was quenched by adding water, then extracted with dichloromethane and washed with saturated brine. The combined organic phases were dried over anhydrous sodium sulfate and concentrated to obtain 1.19 g of a colorless oil with a yield of 90.5%.

¹H NMR (400 MHz, CDCl₃) *δ* 7.56 (d, *J* = 2.1 Hz, 1H), 7.51 (dd, *J* = 8.7, 2.2 Hz, 1H), 6.92 (d, *J* = 8.7 Hz, 1H), 4.65 (dt, *J* = 12.0, 6.0 Hz, 1H), 4.42 (s, 2H), 1.39 (d, *J* = 6.1 Hz, 6H).

### Synthesis of Ethyl N-((3-cyano-4-(isopropoxy)benzyl)oxy)acetylcarbamate

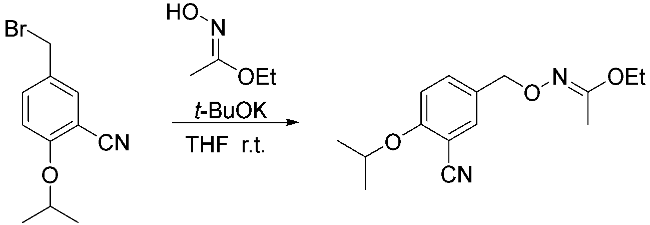

Ethyl acetohydroxamate (402 mg, 3.90 mmol) was dissolved in 10 mL of tetrahydrofuran solution and then placed in an ice bath. A 1.0 M solution of potassium tert-butoxide in tetrahydrofuran (8.0 mL, 8.00 mmol) was added dropwise to the reaction system. Upon the dropwise addition, the reaction system was stirred in the ice bath for another 0.5 hour. 3-cyano-4-isopropoxybenzyl bromide (987 mg, 3.90 mmol) was dissolved in 5 mL of tetrahydrofuran and then added dropwise to the reaction system. Upon the dropwise addition, the mixture was thawed to room temperature and stirred, with the reaction progress monitored by TLC. After the reaction was completed, water was added to quench the reaction, followed by extraction with dichloromethane and washing with saturated brine. The combined organic phases were dried over anhydrous sodium sulfate, concentrated, and purified by silica gel column chromatography to obtain 619 mg of a colorless oily liquid with a yield of 57.5%.

¹H NMR (400 MHz, CDCl₃) *δ* 7.54 (d, *J* = 2.0 Hz, 1H), 7.48 (dd, *J* = 8.7, 2.1 Hz, 1H), 6.92 (d, *J* = 8.7 Hz, 1H), 4.82 (s, 2H), 4.64 (dt, *J* = 12.1, 6.1 Hz, 1H), 3.97 (q, *J* = 7.1 Hz, 2H), 1.92 (s, 3H), 1.39 (d, *J* = 6.1 Hz, 6H), 1.24 (t, *J* = 7.1 Hz, 3H).

### Synthesis of (E)-1-(3-ethyl-4-(hydroxymethyl)phenyl)ethan-1-one-O-(3-cyano-4-(isopropoxy)benzyl)oxime

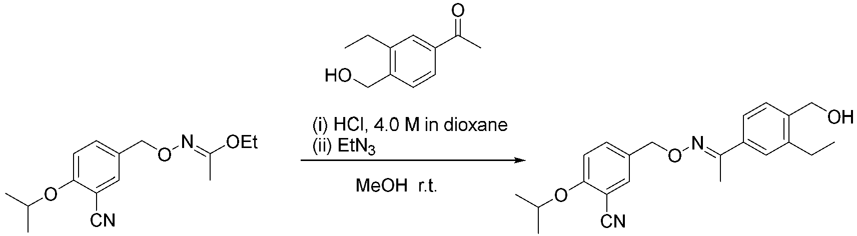

Ethyl *N*-((3-cyano-4-(isopropoxy)benzyl)oxy)acetylcarbamate (138 mg, 0.50 mmol) was dissolved in 5 mL of methanol. A 4.0 M solution of hydrogen chloride in dioxane (0.4 mL, 0.30 mmol) was added dropwise to the reaction mixture. After the reaction system was stirred for 0.5 hours, triethylamine was added dropwise to adjust the pH to between 4 and 6. Then, 1-(3-ethyl-4-(hydroxymethyl)phenyl)ethanone (90 mg, 0.50 mmol) was dissolved in 2 mL of methanol and added dropwise to the reaction system. The progress of the reaction was monitored by TLC. After the reaction was completed, water was added to quench the reaction, followed by extraction with dichloromethane and washing with saturated brine. The combined organic phases were dried over anhydrous sodium sulfate, concentrated, and passed through a silica gel column to obtain a colorless oily liquid (127 mg) with a yield of 69.3%.

¹H NMR (400 MHz, CDCl₃) *δ* 7.61 (d, *J* = 2.0 Hz, 1H), 7.54 (dd, *J* = 8.7, 2.1 Hz, 1H), 7.45 (dd, *J* = 12.1, 4.1 Hz, 2H), 7.38 (d, *J* = 7.9 Hz, 1H), 6.94 (d, *J* = 8.7 Hz, 1H), 5.13 (s, 2H), 4.73 (s, 2H), 4.64 (dt, *J* = 12.1, 6.0 Hz, 1H), 2.72 (q, *J* = 7.6 Hz, 2H), 2.24 (s, 3H), 1.40 (d, *J* = 6.1 Hz, 6H), 1.24 (t, *J* = 7.6 Hz, 3H).

### Synthesis of (E)-5-((((1-(3-ethyl-4-formylphenyl)ethylidene)amino)oxy)methyl)-2-isopropoxybenzonitrile

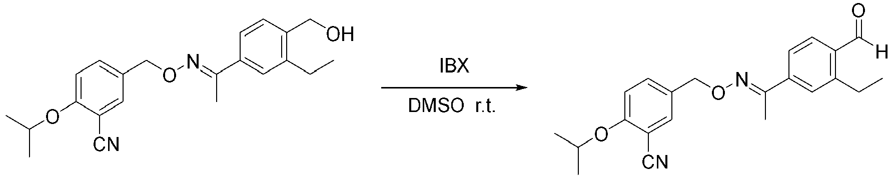

(*E*)-1-(3-ethyl-4-(hydroxymethyl)phenyl)ethan-1-one-*O*-(3-cyano-4-(isopropoxy)benzyl)oxime (147 mg, 0.40 mmol) was dissolved in 4 mL of DMSO solvent, and IBX oxidant (225 mg, 0.80 mmol) was added and stirred at room temperature for approximately 2 hours. The reaction progress was monitored by TLC. After the reaction is completed, the reaction was quenched by adding water, extracted with dichloromethane, and washed with saturated brine. The organic phases were combined, dried over anhydrous sodium sulfate, concentrated, and passed through a silica gel column to obtain 119 mg of a colorless oily liquid with a yield of 81.2%.

¹H NMR (400 MHz, CDCl₃) *δ* 10.28 (s, 1H), 7.82 (d, *J* = 8.1 Hz, 1H), 7.65 - 7.58 (m, 2H), 7.58 - 7.50 (m, 2H), 6.95 (d, *J* = 8.7 Hz, 1H), 5.16 (s, 2H), 4.65 (dt, *J* = 12.1, 6.1 Hz, 1H), 3.08 (q, *J* = 7.5 Hz, 2H), 2.26 (s, 3H), 1.40 (d, *J* = 6.1 Hz, 6H), 1.28 (t, *J* = 7.5 Hz, 3H).

### Synthesis of (E)-1-(2-ethyl-4-(1-((3-cyano-4-(isopropoxy)benzyl)oxy)imino)ethyl)benzyl)azetidine-3-carboxylic acid (Compound 16)

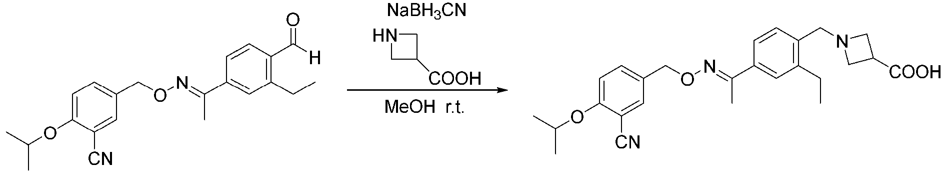

(*E*)-5-((((1-(3-ethyl-4-formylphenyl)ethylidene)amino)oxy)methyl)-2-isopropoxybenzonitrile (109 mg, 0.30 mmol) was dissolved in 5 mL of methanol, 1 drop of acetic acid was added, then 3-azetidinecarboxylic acid (61 mg, 0.60 mmol) was added. The reaction mixture was stirred at room temperature for 3 minutes, afterwards, sodium cyanoborohydride (19 mg, 0.30 mmol) was added. The reaction progress was monitored by TLC. After the reaction is complete, the reaction system was distilled under a reduced pressure, extracted by adding water and dichloromethane, and washed with saturated brine. The organic phases were combined, dried over anhydrous sodium sulfate, concentrated, and passed through a silica gel column to obtain 70 mg of a light-colored solid with a yield of 52.3%.

¹H NMR (400 MHz, CD₃OD) δ 7.64 (d, *J* = 6.7 Hz, 2H), 7.56 (s, 1H), 7.50 (dd, *J* = 8.0, 1.6 Hz, 1H), 7.33 (d, *J* = 8.1 Hz, 1H), 7.16 (d, *J* = 9.4 Hz, 1H), 5.14 (s, 2H), 4.78-4.73 (m, 1H), 4.18 (s, 2H), 3.99 (t, *J* = 9.3 Hz, 2H), 3.90 (t, *J* = 8.6 Hz, 2H), 3.40-3.33 (m, 1H), 2.76 (q, *J* = 7.5 Hz, 2H), 2.23 (s, 3H), 1.38 (s, 3H), 1.37 (s, 3H), 1.23 (t, *J* = 7.5 Hz, 3H). ¹³C NMR (151 MHz, CD₃OD) δ 177.23, 159.53, 154.92, 143.23, 136.96, 134.67, 133.44, 131.00, 129.23, 126.42, 123.80, 116.06, 113.66, 101.88, 74.22, 71.68, 57.49, 56.20, 35.40, 25.12, 20.71, 14.38, 11.39. HRMS (ESI) (m/z): [M+H]⁺ calcd for C₂₆H₃₁N₃O₄, 450.2396; found: 450.2395. HPLC Purity: 96.46%, retention time: 1.02 min.

The synthetic route of Compound 17 is shown as follows:

### Synthesis of (E)-Ethyl N-((4-cyclopentyl-3-(trifluoromethyl)benzyl)oxy)acetimidate

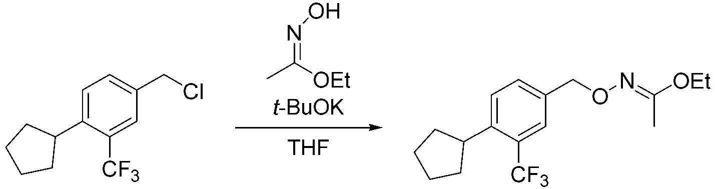

Ethyl acetohydroxamate (196 mg, 1.90 mmol) was dissolved in 5 mL of tetrahydrofuran solution and then placed in an ice bath. A 1.0 M solution of potassium tert-butoxide in tetrahydrofuran (4 mL, 3.90 mmol) was added dropwise to the reaction system. Upon the dropwise addition, the reaction system was stirred in the ice bath for another 0.5 hours. 4-chloromethyl-1-cyclopentyl-2-trifluoromethylbenzene (498 mg, 1.90 mmol) was dissolved in 3 mL of tetrahydrofuran and then added dropwise to the reaction system. Upon the dropwise addition, the mixture was stirred at room temperature, and the progress of the reaction was monitored by TLC. After the reaction was completed, water was added to quench the reaction, followed by extraction with dichloromethane and washing with saturated brine. The organic phases were combined, dried over anhydrous sodium sulfate, concentrated, and then passed through a silica gel column to obtain 532 mg of a colorless oily liquid with a yield of 85.1%.

¹H NMR (400 MHz, CDCl₃) *δ* 7.59 (s, 1H), 7.48 (d, *J* = 8.4 Hz, 1H), 7.43 (d, *J* = 8.1 Hz, 1H), 4.91 (s, 2H), 3.99 (q, *J* = 7.1 Hz, 2H), 3.41-3.28 (m, 1H), 2.07 (m, 2H), 1.94 (s, 3H), 1.89-1.80 (m, 2H), 1.73-1.68 (m, 2H), 1.63-1.56 (m, 2H), 1.25 (t, *J* = 7.1 Hz, 3H).

### Synthesis of (E)-1-(3-ethyl-4-(hydroxymethyl)phenyl)ethan-1-one-O-(4-cyclopentyl-3-(trifluoromethyl)benzyl)oxime

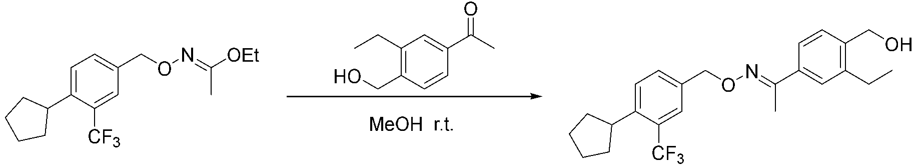

Ethyl (*E*)-N-((4-cyclopentyl-3-(trifluoromethyl)benzyl)oxy)acetimidate (461 mg, 1.40 mmol) was dissolved in 5 mL of methanol. A solution of 4.0 M hydrogen chloride in dioxane (1.0 mL, 0.70 mmol) was added dropwise to the reaction liquid. The reaction liquid was stirred for 0.5 hours, and then triethylamine was added dropwise to adjust the pH to between 4-6. Afterwards, 1-(3-ethyl-4-(hydroxymethyl)phenyl)ethanone (267 mg, 1.50 mmol) was dissolved in 2 mL of methanol and added dropwise to the reaction system. The reaction progress was monitored by TLC. After the reaction is completed, the reaction was quenched by adding water, extracted with dichloromethane, and washed with saturated brine. The organic phases were combined, dried over anhydrous sodium sulfate, concentrated, and passed through a silica gel column to obtain 489 mg of a colorless oily liquid with a yield of 83.4%.

¹H NMR (400 MHz, CDCl₃) *δ* 7.66 (s, 1H), 7.54 (d, *J* = 8.0 Hz, 1H), 7.49 (d, *J* = 6.2 Hz, 1H), 7.45 (d, *J* = 8.6 Hz, 2H), 7.38 (t, *J* = 8.6 Hz, 1H), 5.22 (s, 2H), 4.72 (s, 2H), 3.36 (p, *J* = 8.5 Hz, 1H), 2.71 (q, *J* = 7.6 Hz, 2H), 2.22 (s, 3H), 2.14-2.04 (m, 2H), 1.92-1.81 (m, 2H), 1.77-1.69 (m, 2H), 1.61-1.56 (m, 2H), 1.25 (t, *J* = 7.1 Hz, 3H).

### Synthesis of (E)-4-(1-((4-cyclopentyl-3-(trifluoromethyl)benzyl)oxy)imino)ethyl)-2-ethylbenzaldehyde

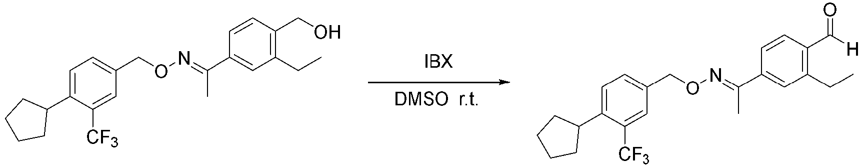

(*E*)-1-(3-ethyl-4-(hydroxymethyl)phenyl)ethan-1-one-*O*-(4-cyclopentyl-3-(trifluoromethyl)benzyl)oxime (503 mg, 1.20 mmol) was dissolved in 3 mL of DMSO solvent. IBX oxidant (952 mg, 2.40 mmol) was added, then stirred at room temperature for approximately 2 hours. The reaction progress was monitored by TLC. After the reaction is completed, the reaction was quenched by adding water, extracted with dichloromethane, and washed with saturated brine. The organic phases were combined, dried over anhydrous sodium sulfate, and passed through a silica gel column to obtain 302 mg of a colorless oily liquid with a yield of 60.3%.

¹H NMR (400 MHz, CDCl₃) *δ* 10.29 (s, 1H), 7.82 (d, *J* = 8.1 Hz, 1H), 7.69-7.59 (m, 2H), 7.59-7.52 (m, 2H), 7.47 (d, *J* = 8.1 Hz, 1H), 5.25 (s, 2H), 3.45-3.30 (m, 1H), 3.08 (q, *J* = 7.5 Hz, 2H), 2.28 (s, 3H), 2.07 (d, *J* = 12.3 Hz, 2H), 1.88-1.80 (m, 2H), 1.73-1.70 (m, 2H), 1.64-1.54 (m, 2H), 1.28 (t, *J* = 7.1 Hz, 3H).

### Synthesis of (E)-1-(4-(1-((4-cyclopentyl-3-(trifluoromethyl)benzyl)oxy)imino)ethyl)-2-ethylbenzyl)azetidine-3-carboxylic acid (Compound 17)

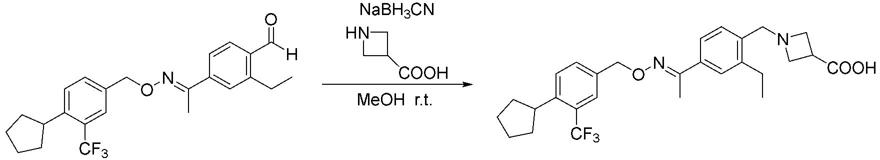

(*E*)-4-(1-((4-cyclopentyl-3-(trifluoromethyl)benzyl)oxy)imino)ethyl)-2-ethylbenzaldehyde (500 mg, 1.20 mmol) was dissolved in 5 mL of methanol, 1 drop of acetic acid was added, and then 3-azetidinecarboxylic acid (242 mg, 2.40 mmol) was added. After the reaction system was stirred at room temperature for 3 minutes, sodium cyanoborohydride (113 mg, 1.80 mmol) was added. The reaction progress was monitored by TLC. After the reaction is complete, the reaction system was distilled under a reduced pressure, and an appropriate amount of water was added, extracted with dichloromethane, and washed with saturated brine. The organic phases were combined, dried over anhydrous sodium sulfate, concentrated, and passed through a silica gel column to obtain 182 mg of a light-colored solid with a yield of 30.2%.

¹H NMR (400 MHz, CD₃OD) δ 7.64 (s, 1H), 7.53 (dt, *J* = 9.8, 5.1 Hz, 4H), 7.38 (t, *J* = 11.5 Hz, 1H), 5.22 (s, 2H), 4.25 (s, 2H), 4.02 (d, *J* = 8.3 Hz, 4H), 3.43-3.33 (m, 1H), 2.75 (dd, *J* = 15.0, 7.5 Hz, 2H), 2.29-2.14 (s, 3H), 2.03 (m, 2H), 1.93-1.81 (m, 2H), 1.78-1.57 (m, 4H), 1.20 (t, *J* = 7.1 Hz, 3H). ¹³C NMR (151 MHz, CD₃OD) δ 177.79, 154.79, 145.36, 143.28, 137.08, 136.13, 131.80, 130.92, 129.27, 127.96, 127.80 (q, *J* = 28.7 Hz), 126.46, 124.78 (q, *J* = 273.5 Hz), 124.76, 123.84, 74.79, 57.14, 40.91, 35.57, 25.62, 25.20, 14.36, 11.42. HRMS (ESI) (m/z): [M+H]⁺ calcd for C₂₈H₃₃F₃N₂O₃, 503.2522; found: 503.2523. HPLC Purity: 96.46%, retention time: 2.28 min.

Reaction conditions for other compounds are similar to those of the above compounds.

### (E)-1-(4-(1-((4-cyclohexylbenzyl)oxy)imino)ethyl)benzyl)azetidine-3-carboxylic acid

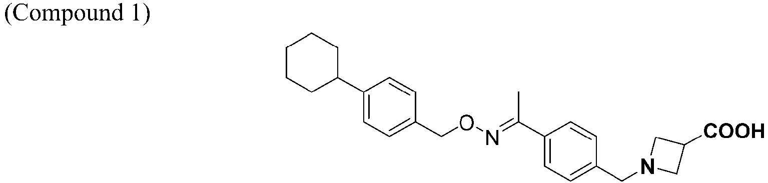

¹H NMR (400 MHz, Methanol-*d*₄) δ 7.60 (d, *J* = 8.3 Hz, 2H), 7.30 (dd, *J* = 8.2, 2.7 Hz, 4H), 7.19 (d, *J* = 8.1 Hz, 2H), 5.14 (s, 2H), 3.64 (d, *J* = 2.4 Hz, 2H), 3.53 (t, *J* = 8.1 Hz, 2H), 3.38 - 3.32 (m, 2H), 3.20 (dd, *J* = 16.6, 8.2 Hz, 1H), 2.57 - 2.45 (m, 1H), 2.22 (s, 3H), 1.84 (d, *J* = 9.2 Hz, 4H), 1.75 (d, *J* = 12.2 Hz, 1H), 1.43 (d, *J* = 4.4 Hz, 6H). ¹³C NMR (151 MHz, Methanol-*d*₄) δ 154.60, 147.52, 138.04, 135.64, 135.45, 128.59, 128.01, 126.36, 125.82, 75.68, 62.35, 57.58, 44.40, 36.48, 34.30, 29.46, 26.62, 25.86, 11.45. LC-MS (ESI): m/z: 421.1 (M+H)⁺.

### (E)-1-(4-(1-((4-cyclohexyl-3-(trifluoromethyl)benzyl)oxy)imino)ethyl)benzyl)azetidine-3-carboxylic acid (Compound 2)

¹H NMR (400 MHz, Methanol-*d*₄) δ 7.56 (d, *J* = 17.6 Hz, 1H), 7.53 (d, *J* = 7.4 Hz, 1H), 7.39 (t, *J* = 12.5 Hz, 1H), 7.30 (d, *J* = 7.9 Hz, 2H), 7.19 (d, *J* = 7.9 Hz, 2H), 5.11 (s, 2H), 4.31 (s, 2H), 4.10 (dd, *J* = 21.9, 12.7 Hz, 2H), 4.02 (t, *J* = 8.4 Hz, 2H), 3.35 (s, 3H), 2.78-2.70 (m, 2H), 1.49-1.36 (m, 9H). LC-MS (ESI): m/z: 431.1 (M+H)⁺.

### (E)-1-(4-(1-((4-cyclohexylbenzyl)oxy)imino)ethyl)-2-ethylbenzyl)azetidine-3-carboxylic acid (Compound 3)

¹H NMR (400 MHz, DMSO) δ 8.36 (s, 1H), 8.04 (dt, *J* = 28.8, 6.4 Hz, 4H), 7.71 - 7.46 (m, 4H), 4.38-4.33 (m, 2H), 4.10-4.01 (m, 2H), 3.59-3.55 (m, 2H), 2.85 - 2.76 (m, 2H), 2.79 (q, *J* = 7.5 Hz, 2H), 2.57 - 2.45 (m, 1H), 1.24-1.21 (m, 3H), 1.20 (t, *J* = 7.5 Hz, 3H). LC-MS (ESI): m/z: 449.1 (M+H)⁺.

### Structure of (E)-1-(2-ethyl-4-(1-(((4-(5-fluoropyrazin-2-yl)-3-methylbenzyl)oxy)imino)ethyl)benzyl)pyrrolidine-3-carboxylic acid (Compound 4)

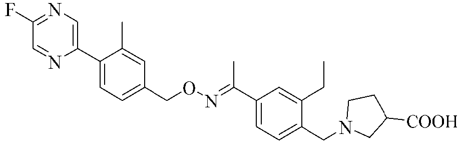

Pale yellow solid, yield 18.4%. ¹H NMR (500 MHz, Methanol-*d*₄) δ 8.58 (dd, *J* = 8.3, 1.3 Hz, 1H), 8.37 (d, *J* = 1.5 Hz, 1H), 7.64 (d, *J* = 1.9 Hz, 1H), 7.58 (dd, *J* = 8.1, 1.9 Hz, 1H), 7.49 (d, *J* = 8.1 Hz, 1H), 7.42 (d, *J* = 7.8 Hz, 1H), 7.40 - 7.35 (m, 2H), 5.26 (s, 2H), 4.40 (s, 2H), 3.53 (dd, *J* = 11.2, 5.8 Hz, 1H), 3.39 - 3.35 (m, 2H), 3.28 (dd, *J* = 11.0, 7.4 Hz, 1H), 3.15 - 3.06 (m, 1H), 2.82 (q, *J* = 7.5 Hz, 2H), 2.37 (s, 3H), 2.34 - 2.22 (m, 5H), 1.26 (t, *J* = 7.5 Hz, 3H). ¹³C NMR (126 MHz, Methanol-*d*₄) δ 177.85, 160.27, 158.28, 154.49, 152.90, 143.73, 141.29, 141.22, 139.46, 137.74, 136.37, 135.08, 131.94, 131.64, 130.48, 130.33, 130.21, 129.58, 126.66, 125.50, 123.97, 75.34, 56.55, 54.38, 53.52, 43.60, 27.72, 25.23, 19.04, 14.55, 11.40. MS (ESI) (m/z): [M+H]⁺ 491.25.

### Structure of (S, E)-1-(2-ethyl-4-(1-(((3-methyl-4-(5-hydroxypyrazin-2-yl)-benzyl)oxy)imino)ethyl)benzyl)pyrrolidine-3-carboxylic acid (Compound 5)

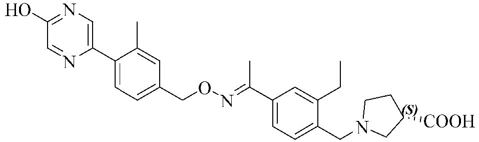

Yellow solid, yield 72.5%. ¹H NMR (500 MHz, Methanol-d4) δ 8.17 (d, *J* = 1.4 Hz, 1H), 7.67 (d, *J* = 1.8 Hz, 1H), 7.61 (dd, *J* = 8.1, 1.9 Hz, 1H), 7.57 (d, *J* = 8.1 Hz, 1H), 7.51 (d, *J* = 1.4 Hz, 1H), 7.35 (d, *J* = 8.1 Hz, 2H), 7.33 - 7.29 (m, 1H), 5.24 (s, 2H), 4.53 (s, 2H), 3.70 - 3.57 (m, 2H), 3.51 - 3.43 (m, 2H), 3.38 (q, *J* = 8.6, 8.2 Hz, 1H), 2.85 (q, *J* = 7.5 Hz, 2H), 2.49 - 2.41 (m, 1H), 2.38 (s, 3H), 2.36 - 2.30 (m, 1H), 2.29 (s, 3H), 1.27 (t, *J* = 7.5 Hz, 3H).¹³C NMR (126 MHz, Methanol-d4) δ 154.34, 143.95, 138.49, 138.17, 136.24, 135.20, 130.78, 130.24, 129.10, 129.01, 126.79, 125.48, 124.11, 75.49, 55.17, 54.38, 53.64, 27.07, 25.36, 19.14, 14.60, 11.39. MS(m/z): [M+H]⁺489.20

### (E)-1-(4-(1-((4-cyclohexyl-3-(trifluoromethyl)benzyloxy)imino)ethyl)benzyl)pyrrolidine-3-carboxylic acid (Compound 6)

¹H NMR (400 MHz, Methanol-d4) δ 7.70 - 7.63 (m, 3H), 7.57 (d, *J* = 16.3 Hz, 2 H), 7.44 (d, *J* = 8.2 Hz, 2H), 5.22 (s, 2H), 3.96 (d, *J* = 5.7 Hz, 2H), 3.35 (s, 2H), 3.16-3.12 (m, 1H), 3.05-2.98 (m, 2H), 2.95 - 2.82 (m, 2H), 2.26 (s, 3H), 2.16 (d, *J* = 8.0 Hz, 2H), 1.82-1.80 (m, , 2H), 1.78-1.75 (m, 3H), 1.58-1.45 (m, 6H). LC-MS (ESI): m/z: 503. 1 (M+H)⁺.

### (E)-1-(4-(1-((4-cyclohexyl-3-(trifluoromethyl)benzyl)oxy)imino)ethyl)benzyl)piperidine-3-carboxylic acid (Compound 7)

¹H NMR (400 MHz, Methanol-d4) δ 7.70 - 7.63 (m, 3H), 7.57 (d, *J* = 16.3 Hz, 2H), 7.44 (d, *J* = 8.2 Hz, 2H), 5.22 (s, 2H), 3.96 (d, *J* = 5.7 Hz, 2H), 3.35 (s, 2H), 3.16-3.12 (m, 1H), 3.05-2.98 (m, 2H), 2.95 - 2.82 (m, 2H), 2.26 (s, 3H), 2.16 (d, *J* = 8.0 Hz, 2H), 1.82-1.80 (m, , 2H), 1.78-1.75 (m, 3H), 1.58-1.45 (m, 8H). LC-MS (ESI): m/z: 517.2 (M+H)⁺.

### (E)-4-((4-((1-((4-cyclohexyl-3-(trifluoromethyl)benzyl)oxy)imino)ethyl)benzyl)amino)butanoic acid (Compound 8)

¹H NMR (400 MHz, Methanol-d4) δ 7.65 (d, *J* = 8.1 Hz, 3H), 7.58 (t, *J* = 10.4 Hz, 1H), 7.53 (d, *J* = 8.1 Hz, 1H), 7.41 (t, *J* = 10.5 Hz, 2H), 5.21 (s, 2H), 3.88 (s, 2H), 2.91 (t, *J* = 11.2 Hz, 1H), 2.76 (t, *J* = 7.1 Hz, 2H), 2.30 - 2.17 (m, 5H), 1.85-1.75 (m, 6H), 1.61 - 1.29 (m, 6H). LC-MS (ESI): m/z: 491.2 (M+H)⁺.

### (E)-3-((4-((1-((4-cyclohexyl-3-(trifluoromethyl)benzyl)oxy)imino)ethyl)benzyl)amino)cyclohexane-1-carboxylic acid (Compound 9)

¹H NMR (400 MHz, Methanol-d4) δ 7.77 - 7.32 (m, 7H), 5.21 (s, 2H), 3.91 (s, 2H), 2.91 (s, 1H), 2.68 (s, 1H), 2.25 (s, 3H), 2.18-2.15 (m, 2H), 1.88-1.70 (m, 9H), 1.65 - 1.33 (m, 8H). ¹³C NMR (151 MHz, MeOD) δ 182.91, 155.03, 146.14, 136.31, 135.59, 131.60, 128.55, 128.47, 128.10, 125.97, 124.84, 124.79, 74.73, 55.54, 49.13, 45.59, 40.06, 34.19, 31.24, 29.43, 26.60, 25.70, 11.40. LC-MS (ESI): m/z: 531.2 (M+H)⁺.

### (E)-3-((4-((1-((4-cyclohexyl-3-(trifluoromethyl)benzyl)oxy)imino)ethyl)benzyl)amino)propanoic acid (Compound 10)

¹H NMR (400 MHz, Methanol-d4) δ 7.65 (d, *J* = 7.4 Hz, 3H), 7.56 (dd, *J* = 24.3, 8.1 Hz, 2H), 7.39 (d, *J* = 8.0 Hz, 2H), 5.21 (s, 2H), 3.90 (s, 2H), 2.95-2.90 (m, 3H), 2.44 (t, *J* = 6.6 Hz, 2H), 2.25 (s, 3H), 1.87 (d, *J* = 16.6 Hz, 2H), 1.78 (d, *J* = 11.1 Hz, 3H), 1.48 (ddd, *J* = 33.8, 22.3, 10.1 Hz, 5H). ¹³C NMR (151 MHz, Methanol-d4) δ 178.44, 154.94, 146.14, 137.98, 136.30, 135.86, 131.60, 128.53, 128.10, 126.03, 124.84, 124.79, 74.74, 51.72, 45.19, 40.06, 35.04, 34.18, 26.59, 25.70, 11.38. LC-MS (ESI): m/z: 478.2 (M+H)⁺.

### Structure of (E)-1-(4-(1-((4-cyclohexyl-3-(trifluoromethyl)benzyl)oxy)imino)ethyl)-2-fluorobenzyl)azetidine-3-carboxylic acid (Compound 11)

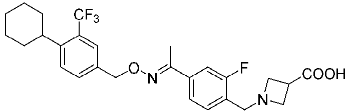

White solid, yield 40.6%, melting point: 144.6-144.9 °C. ¹H NMR (400 MHz, CDCl₃) δ 7.74-7.27 (m, 6H), 5.26 (s, 2H), 3.97 (s, 2H), 3.80 (d, *J* = 8.2 Hz, 2H), 3.72-3.67 (m, 3H), 2.94-2.90 (m, 1H), 2.27 (s, 3H), 1.94-1.82 (m, 2H), 1.80-1.75 (m, 2H), 1.56-1.50 (m, 2H), 1.45-1.40 (m, 4H). HRMS (ESI) (m/z): [M+H]⁺ calcd for C₂₇H₃₀F₄N₂O₃, 507.2271; found: 507.2270. HPLC Purity: 91.98%, retention time: 2.26 min.

### Structure of (E)-1-(2-bromo-4-(1-((4-cyclohexyl-3-(trifluoromethyl)benzyl)oxy)imino)ethyl)azetidine-3-carboxylic acid (Compound 12)

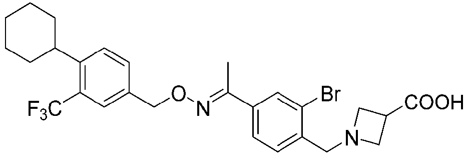

White solid, yield 40.6%, melting point: 131.6-131.9 °C. ¹H NMR (400 MHz, CD₃OD) δ 7.91 (d, *J* = 1.6 Hz, 1H), 7.69-7.63 (m, 2H), 7.63-7.49 (m, 2H), 7.45 (d, *J* = 8.1 Hz, 1H), 5.23 (s, 2H), 4.08 (s, 2H), 3.87 (t, *J* = 8.6 Hz, 2H), 3.75 (t, *J* = 8.2 Hz, 2H), 2.93-2.86 (m, 2H), 2.30 (s, 3H), 1.84-1.80 (m, 2H), 1.77-1.70 (m, 3H), 1.60-1.34 (m, 5H). ¹³C NMR (151 MHz, CD₃OD) δ 178.85, 154.09, 147.89, 146.25, 138.90, 131.73, 129.72, 129.46, 128.84, 128.53, 128.16, 127.33, 124.97, 121.07, 112.36, 90.94, 74.97, 61.53, 58.01, 40.07, 34.17, 26.69, 25.70, 12.97. HRMS (ESI) (m/z): [M+H]⁺ calcd for C₂₇H₃₀BrF₃N₂O₃, 567.1470; found: 567.1473. HPLC Purity: 90.25%, retention time: 2.87 min.

### Structure of (E)-1-(4-(1-((4-cyclohexyl-3-(trifluoromethyl)benzyl)oxy)imino)ethyl)-2-methylbenzyl)azetidine-3-carboxylic acid (Compound 18)

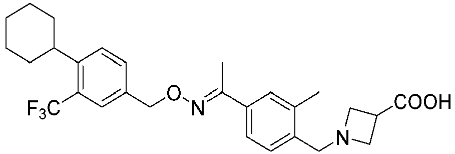

White solid, yield 40.6%, melting point: 155.1-155.6 °C. ¹H NMR (400 MHz, CD₃OD) δ 7.64 (d, *J* = 5.9 Hz, 1H), 7.59-7.42 (m, 4H), 7.37-7.29 (m, 1H), 5.20 (s, 2H), 4.05 (s, 2H), 3.91 (t, *J* = 8.9 Hz, 2H), 3.77 (dd, *J* = 17.0, 8.5 Hz, 2H), 3.60 (dd, *J* = 14.1, 7.1 Hz, 1H), 2.91 (t, *J* = 11.3 Hz, 1H), 2.39 (s, 3H), 2.23 (s, 3H), 1.88-1.80 (m, 2H), 1.79-1.70 (m, 3H), 1.46-1.39 (m, 5H). ¹³C NMR (151 MHz, CD₃OD) δ 178.49, 154.91, 146.15, 138.90, 136.99, 136.37, 131.61, 128.89, 128.52, 128.11, 127.91, 127.31, 124.88, 124.80 (q, *J* = 273.5 Hz), 123.68, 74.75, 57.59, 40.05, 35.70, 34.18, 34.13, 26.59, 25.70, 17.99, 16.97, 11.38. HRMS (ESI) (m/z): [M+H]+ calcd for C₂₈H₃₃F₃N₂O₃, 503.2522; found: 503.2523. HPLC Purity: 94.29%, retention time: 2.57 min.

### Structure of (E)-1-(4-(1-((4-cyclohexyl-3-(trifluoromethyl)benzyl)oxy)imino)ethyl)-2-methoxybenzyl)azetidine-3-carboxylic acid (Compound 27)

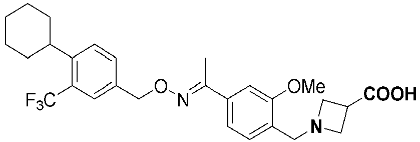

White solid, yield 40.6%, melting point: 145.2-145.6 °C. ¹H NMR (400 MHz, CD₃OD) δ 7.66 (s, 1H), 7.64-7.49 (m, 3H), 7.25 (t, *J* = 8.7 Hz, 2H), 5.21 (s, 2H), 3.84 (s, 3H), 3.70 (s, 2H), 3.59 (d, *J* = 8.2 Hz, 2H), 3.52 (t, *J* = 8.0 Hz, 1H), 3.42 (t, *J* = 8.2 Hz, 2H), 2.91 (d, *J* = 10.6 Hz, 1H), 2.23 (s, 3H), 1.92-1.83 (m, 2H), 1.79-1.87 (m, 3H), 1.63-1.48 (m, 5H). ¹³C NMR (151 MHz, CD₃OD) δ 175.87, 157.76, 154.59, 146.26, 139.82, 138.93, 136.16, 131.75, 128.55, 127.30, 127.01, 124.98, 124.80 (q, *J* = 273.5 Hz), 119.36, 118.66, 107.91, 74.92, 60.20, 57.19, 54.77, 40.06, 34.72, 34.18, 26.58, 25.69, 11.39. HRMS (ESI) (m/z): [M+H]⁺ calcd for C₂₈H₃₃F₃N₂O₄, 519.2471; found: 519.2473. HPLC Purity: 96.28%, retention time: 1.27 min.

### Structure of (E)-1-(4-(1-((4-cyclohexyl-3-(trifluoromethyl)benzyl)oxy)imino)ethyl)-2-(trifluoromethyl)benzyl)azetidine-3-carboxylic acid (Compound 19)

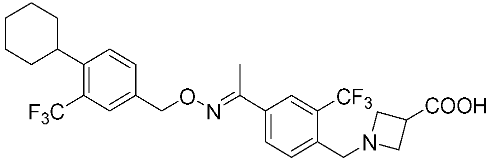

White solid, yield 40.6%, melting point: 135.6-138.9 °C. ¹H NMR (400 MHz, CD₃OD) δ 7.98 (s, 1H), 7.88 (d, *J* = 8.1 Hz, 1H), 7.69-7.51 (m, 4H), 5.24 (s, 2H), 3.96 (s, 2H), 3.73 (t, *J* = 8.3 Hz, 2H), 3.53 (t, *J* = 8.0 Hz, 2H), 3.29-3.19 (m, 1H), 2.92 (t, *J* = 11.3 Hz, 1H), 1.86-1.80 (m, 2H), 1.78-1.70 (m, 3H), 1.62-1.35 (m, 5H). ¹³C NMR (151 MHz, CD₃OD) δ 178.45, 153.61, 146.30, 138.92, 135.99, 135.83, 131.79, 129.85, 129.41, 128.52, 128.15, 127.29, 125.06, 124.77 (q, *J* = 273.5 Hz), 124.22 (q, *J* = 273.6 Hz), 123.02, 75.08, 58.10, 57.75, 40.06, 36.07, 34.17, 26.59, 25.70, 11.05. LC-MS (ESI): m/z: 557.2 (M+H)⁺. HRMS (ESI) (m/z): [M+H]⁺ calcd for C₂₈H₃₀F₆N₂O₃, 557.2239; found: 557.2237. HPLC Purity: 97.16%, retention time: 2.56 min.

### Structure of (E)-1-(4-(1-(([1,1'-biphenyl]-4-ylmethoxy)imino)ethyl)-2-ethylbenzyl)azetidine-3-carboxylic acid (Compound 20)

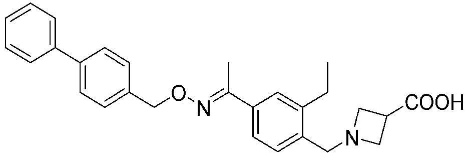

White solid, yield 40.6%, melting point: 120.3-121.9 °C. ¹H NMR (400 MHz, CD₃OD) δ 7.64-7.53 (m, 4H), 7.49-7.39 (m, 6H), 7.36-7.23 (m, 2H), 5.24 (s, 2H), 3.76 (s, 2H), 3.67-3.58 (m, 2H), 3.42 (t, *J* = 8.1 Hz, 2H), 3.29-3.15 (m, 1H), 2.72 (q, *J* = 7.5 Hz, 2H), 2.23 (s, 3H), 1.22 (t, *J* = 7.5 Hz, 3H). ¹³C NMR (151 MHz, CD₃OD) δ 179.26, 155.07, 142.50, 140.80, 140.59, 137.27, 135.67, 135.39, 129.45, 128.52, 128.44, 128.33, 126.93, 126.58, 126.55, 126.52, 126.50, 125.94, 123.39, 75.38, 58.84, 57.94, 36.49, 25.11, 14.23, 11.55. LC-MS (ESI): m/z: 443.2 (M+H)⁺. HRMS (ESI) (m/z): [M+H]⁺ calcd for C₂₈H₃₀N₂O₃, 443.2335; found: 443.2337. HPLC Purity: 98.03%, retention time: 1.30 min.

### Structure of (E)-1-(4-(1-((4-cyclopentyl-3-(trifluoromethyl)benzyl)oxy)imino)ethyl)-2-ethylbenzyl)azetidine-3-carboxylic acid (Compound 17)

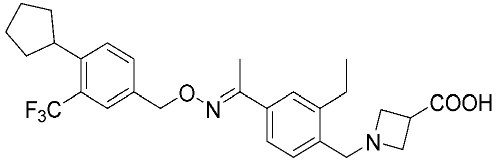

Light-colored solid, yield 30.2%, melting point: 131.7-133.2 °C. ¹H NMR (400 MHz, CD₃OD) δ 7.64 (s, 1H), 7.53 (dt, *J* = 9.8, 5.1 Hz, 4H), 7.38 (t, *J* = 11.5 Hz, 1H), 5.22 (s, 2H), 4.25 (s, 2H), 4.02 (d, *J* = 8.3 Hz, 4H), 3.43-3.33 (m, 1H), 2.75 (dd, *J* = 15.0, 7.5 Hz, 2H), 2.29-2.14 (s, 3H), 2.03 (m, 2H), 1.93-1.81 (m, 2H), 1.78-1.57 (m, 4H), 1.20 (t, *J* = 7.1 Hz, 3H). ¹³C NMR (151 MHz, CD₃OD) δ 177.79, 154.79, 145.36, 143.28, 137.08, 136.13, 131.80, 130.92, 129.27, 127.96, 127.80 (q, *J* = 28.7 Hz), 126.46, 124.78 (q, *J* = 273.5 Hz), 124.76, 123.84, 74.79, 57.14, 40.91, 35.57, 25.62, 25.20, 14.36, 11.42. HRMS (ESI) (m/z): [M+H]⁺ calcd for C₂₈H₃₃F₃N₂O₃, 503.2522; found: 503.2523. HPLC Purity: 96.46%, retention time: 2.28 min.

### Structure of (E)-1-(4-(1-((4-cyclohexyl-3-methylbenzyl)oxy)imino)ethyl)-2-ethylbenzyl)azetidine-3-carboxylic acid (Compound 24)

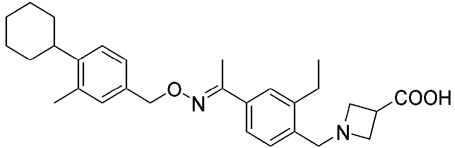

Yellow solid, yield 33.3%, melting point: 135.8-136.3 °C. ¹H NMR (400 MHz, CD₃OD) δ 7.53 (s, 1H), 7.47 (dd, *J* = 8.0, 1.5 Hz, 1H), 7.30 (d, *J* = 8.1 Hz, 1H), 7.15 (d, *J* = 8.5 Hz, 3H), 5.11 (s, 2H), 4.00 (s, 2H), 3.84 (t, *J* = 8.8 Hz, 2H), 3.69 (t, *J* = 8.4 Hz, 2H), 3.30-3.22 (m, 1H), 2.82-2.60 (m, 3H), 2.29 (s, 3H), 2.20 (s, 3H), 1.83-1.80 (m, 2H), 1.76-1.72 (m, 3H), 1.48-1.29 (m, 5H), 1.21 (t, *J* = 7.8 Hz, 3H). ¹³C NMR (151 MHz, CD₃OD) δ 178.12, 154.47, 145.22, 142.85, 136.54, 134.88, 134.61, 133.03, 129.89, 128.89, 126.20, 125.88, 124.96, 123.61, 75.80, 57.62, 57.34, 39.83, 35.88, 33.49, 26.85, 26.03, 25.14, 18.10, 14.33, 11.49. HRMS (ESI) (m/z): [M+H]⁺ calcd for C₂₉H₃₈N₂O₃, 463.2961; found: 463.2962. HPLC Purity: 98.19%, retention time: 2.39 min.

### (E)-3-((4-((1-((4-cyclohexyl-3-(trifluoromethyl)benzyl)oxy)imino)ethyl)-2-ethylbenzyl)amino)propanoic acid (Compound 25)

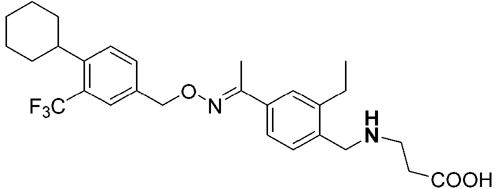

¹H NMR (400 MHz, CD₃OD) δ 7.65 (s, 1H), 7.58 (dd, *J* = 11.7, 7.9 Hz, 2H), 7.51 (t, *J* = 9.5 Hz, 2H), 7.46 - 7.35 (m, 1H), 5.21 (s, 2H), 4.19 (s, 2H), 3.18 (t, *J* = 6.3 H z, 2H), 3.07 (t, *J* = 6.4 Hz, 1H), 2.91 (t, *J* = 11.3 Hz, 1H), 2.78 (q, *J* = 7.5 Hz, 2H), 2. 51 (t, *J* = 6.3 Hz, 2H), 2.24 (s, 3H), 1.83-1.80 (m, 2H), 1.77-1.70 (m, 3H), 1.59 - 1.33 (m, 5H). ¹³C NMR (151 MHz, CD₃OD) δ 177.24, 154.83, 146.18, 143.23, 137.30, 136.22, 131.68, 131.45, 129.89, 128.13, 127.19, 127.00, 126.43, 124.96, 124.91, 123.96, 74.81, 44.8 4, 40.06, 34.19, 33.03, 32.12, 26.59, 25.70, 25.07, 14.37, 11.42. LC-MS (ESI): m/z: 505.2 (M+H)⁺.

### (E)-1-(4-(1-((4-cyclohexyl-3-(trifluoromethyl)benzyl)oxy)imino)ethyl)-2-ethylbenzyl)pyrroli dine-3-carboxylic acid (Compound 26)

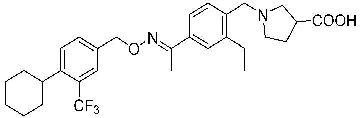

¹H NMR (400 MHz, CD₃OD) δ 7.65 (s, 1H), 7.59 (d, *J* = 7.2 Hz, 2H), 7.53 (d, *J* = 7.5 Hz, 2H), 7.45 (d, *J* = 8.1 Hz, 1H), 5.22 (s, 2H), 4.33 (s, 2H), 3.40-3.42 (m, 1H), 3.38 - 3.32 (m, 1H), 3.25 (t, *J* = 6.9 Hz, 2H), 3.11 - 3.00 (m, 1H), 2.91 (t, *J* = 11.3 Hz, 1H), 2.80 (q, *J* = 7.5 Hz, 2H), 2.24 (s, 3H), 1.85 (d, *J* = 11.7 Hz, 2H), 1.77 (d, *J* = 11.9 Hz, 3H), 1.59 - 1.50 (m, 2H), 1.46 - 1.31 (m, 3H), 1.27 (t, *J* = 7.1 Hz, 3H). ¹³C NMR (151 MHz, CD₃OD) δ 178.42, 154.79, 146.19, 143.65, 137.44, 136.23, 131.68, 130.98, 130.40, 128.13, 127.20, 127.01, 126.59, 125.71, 124.95, 124.91, 123.91, 74.82, 56.80, 54.66, 53.60, 43.92, 40.06, 34.18, 26.59, 25.69, 25.21, 14.51, 11.39. LC-MS (ESI): m/z: 531.3 (M+H)⁺.

### Structure of (E)-1-(2-ethyl-4-(1-((2-methyl-[1,1'-biphenyl]-4-yl)methoxy)imino)ethyl)azetidine-3-carboxylic acid (Compound 22)

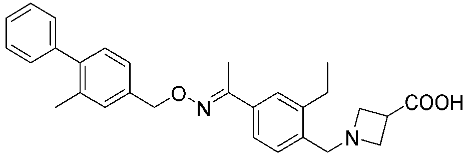

White solid, yield 40.6%, melting point: 154.2-154.8 °C. ¹H NMR (400 MHz, CD₃OD) δ 7.55 (s, 1H), 7.49 (d, *J* = 8.0 Hz, 1H), 7.38 (t, *J* = 7.3 Hz, 2H), 7.34-7.23 (m, 6H), 7.15 (d, *J* = 7.7 Hz, 1H), 5.17 (s, 2H), 4.00 (s, 2H), 3.84 (t, *J* = 8.7 Hz, 2H), 3.69 (t, *J* = 8.3 Hz, 2H), 3.27-3.20 (m, 1H), 2.74 (q, *J* = 7.5 Hz, 2H), 2.24 (s, 3H), 2.23 (s, 3H), 1.21 (q, *J* = 7.5 Hz, 3H). ¹³C NMR (151 MHz, CD₃OD) δ 178.15, 154.69, 142.88, 141.76, 141.46, 137.05, 136.48, 134.91, 133.09, 129.85, 129.32, 128.92, 128.79, 127.78, 126.51, 126.21, 125.39, 123.63, 75.61, 57.61, 57.33, 35.89, 25.15, 19.26, 14.34, 11.52. HRMS (ESI) (m/z): [M+H]⁺ calcd for C₂₉H₃₂N₂O₃, 457.2492; found: 457.2493. HPLC Purity: 91.98%, retention time: 2.30 min.

### Structure of (E)-1-(2-ethyl-4-(1-((2-(trifluoromethyl)-[1,1'-biphenyl]-4-yl)methoxy)imino)ethyl)azetidine-3-carboxylic acid (Compound 21)

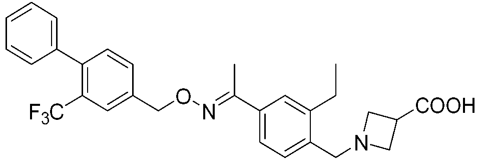

White solid, yield 40.6%, melting point: 155.5-155.9 °C. ¹H NMR (400 MHz, CD₃OD) δ 7.69 (d, *J* = 12.3 Hz, 1H), 7.56 (t, *J* = 8.1 Hz, 1H), 7.49 (s, 1H), 7.42 (d, *J* = 8.0 Hz, 1H), 7.26 (dd, *J* = 5.3, 2.9 Hz, 4H), 7.22 (d, *J* = 7.8 Hz, 1H), 7.19-7.13 (m, 2H), 5.21 (s, 2H), 4.11 (s, 2H), 3.93 (t, *J* = 9.1 Hz, 2H), 3.83 (t, *J* = 8.4 Hz, 2H), 3.29-3.22 (m, 1H), 2.66 (q, *J* = 7.5 Hz, 2H), 2.16 (s, 3H), 1.12 (t, *J* = 7.5 Hz, 3H). ¹³C NMR (151 MHz, CD₃OD) δ 177.22, 155.11, 143.30, 140.77, 139.59, 138.22, 136.91, 132.05, 131.31, 130.97, 129.29, 128.64, 127.87 (q, *J* = 29.8 Hz), 127.46, 127.37, 126.45, 125.32, 124.23 (q, *J* = 273.8 Hz), 123.86, 74.64, 57.34, 55.98, 35.39, 25.18, 14.40, 11.50. HRMS (ESI) (m/z): [M+H]⁺ calcd for C₂₉H₂₉F₃N₂O₃, 511.2209; found: 511.2211. HPLC Purity: 97.71%, retention time: 1.56 min.

### Structure of (E)-1-(2-ethyl-4-(1-((3-(trifluoromethyl)phenyl)oxy)imino)ethyl)benzyl)azetidine-3-carboxylic acid (Compound 13)

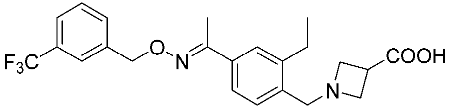

Pale yellow oily, yield 60.0%. ¹H NMR (400 MHz, CD₃OD) δ 7.71 (s, 1H), 7.67 (d, *J* = 7.4 Hz, 1H), 7.62-7.51 (m, 3H), 7.47 (dd, *J* = 8.0, 1.6 Hz, 1H), 7.31 (d, *J* = 8.0 Hz, 1H), 5.28 (s, 2H), 3.98 (s, 2H), 3.83 (t, *J* = 8.8 Hz, 2H), 3.67 (t, *J* = 8.4 Hz, 2H), 3.28 (dd, J = 15.2, 6.9 Hz, 1H), 2.73 (q, *J* = 7.5 Hz, 2H), 2.26 (s, 3H), 1.21 (t, *J* = 7.5 Hz, 3H). ¹³C NMR (151 MHz, CD₃OD) δ 178.20, 155.32, 142.88, 139.73, 136.16, 133.52, 131.41, 130.23 (q, *J* = 32.0 Hz), 128.87, 128.82, 126.17, 124.32, 124.31 (q, *J* = 272.0 Hz), 124.06, 123.61, 74.70, 57.68, 35.94, 25.11, 14.28, 11.47. HRMS (ESI) (m/z): [M+H]⁺ calcd for C₂₃H₂₅F₃N₂O₃, 435.1896; found: 435.1897. HPLC Purity: 99.79%, retention time: 1.17 min.

### Structure of (E)-1-(2-ethyl-4-(1-((4-methyl-3-(trifluoromethyl)benzyl)oxy)imino)ethyl)azetidine-3-carboxylic acid (Compound 14)

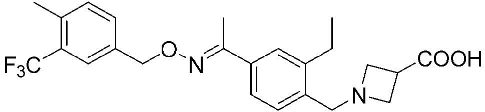

Pale yellow oily, yield 21.3%. ¹H NMR (400 MHz, CD₃OD) δ 7.67 (s, 1H), 7.57 (s, 1H), 7.52 (t, *J* = 7.9 Hz, 2H), 7.35 (dd, *J* = 12.5, 8.1 Hz, 2H), 5.22 (s, 2H), 4.22 (s, 2H), 3.97 (dd, J = 22.5, 8.5 Hz, 4H), 3.40-3.33 (m, 1H), 2.76 (q, *J* = 7.5 Hz, 2H), 2.47 (s, 3H), 2.25 (s, 3H), 1.22 (t, *J* = 7.5 Hz, 3H). ¹³C NMR (151 MHz, CD₃OD) δ 177.72, 155.00, 143.07, 136.62, 136.50, 135.81, 132.36, 131.84, 131.45, 129.07, 128.29 (q, *J* = 30.2 Hz), 126.30, 125.10, 124.70 (q, *J* = 273.3 Hz), 123.70, 74.70, 57.55, 56.78, 35.67, 25.12, 17.72, 14.32, 11.40. HRMS (ESI) (m/z): [M+H]⁺ calcd for C₂₄H₂₇F₃N₂O₃, 449.2052; found: 449.2050. HPLC Purity: 99.32%, retention time: 1.35 min.

### Structure of (E)-1-(2-ethyl-4-(1-((naphthalen-2-ylmethoxy)imino)ethyl)azetidine-3-carboxylic acid (Compound 15)

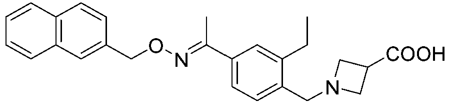

White solid, yield 25.9%, melting point: 131.5-131.7 °C. ¹H NMR (400 MHz, CD₃OD) δ 7.89-7.79 (m, 4H), 7.61-7.51 (m, 2H), 7.52-7.41 (m, 3H), 7.35-7.18 (m, 1H), 5.40 (s, 2H), 3.95 (s, 2H), 3.80 (t, *J* = 8.6 Hz, 2H), 3.61 (dt, *J* = 10.7, 5.9 Hz, 2H), 3.29-3.22 (m, 1H), 2.77-2.59 (m, 2H), 2.27 (s, 3H), 1.25-1.14 (m, 3H). ¹³C NMR (151 MHz, CD₃OD) δ 177.73, 154.96, 142.81, 136.31, 135.65, 133.40, 133.14, 128.83, 127.62, 127.55, 127.29, 126.58, 126.18, 125.78, 125.74, 125.59, 123.60, 75.84, 57.73, 36.00, 25.11, 14.28, 11.53. HRMS (ESI) (m/z): [M+H]⁺ calcd for C₂₆H₂₈N₂O₃, 417.2179; found: 417.2179. HPLC Purity: 98.77%, retention time: 1.24 min.

### Structure of (E)-1-(2-ethyl-4-(1-(((5,6,7,8-tetrahydronaphthalen-2-yl)methoxy)imino)ethyl)benzyl)azetidine-3-carboxylic acid (Compound 23)

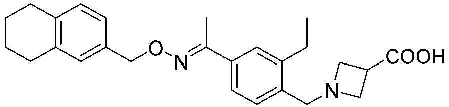

White solid, yield 10.2%, melting point: 135.3-138.9 °C. ¹H NMR (400 MHz, CD₃OD) δ 7.50 (s, 1H), 7.44 (dd, *J* = 8.0, 1.5 Hz, 1H), 7.28 (d, *J* = 8.0 Hz, 1H), 7.13-7.05 (m, 2H), 7.00 (d, *J* = 7.7 Hz, 1H), 5.09 (s, 2H), 3.83 (s, 2H), 3.70 (t, *J* = 8.3 Hz, 2H), 3.51 (t, *J* = 8.2 Hz, 2H), 3.24 (dd, *J* = 16.6, 8.3 Hz, 1H), 2.78-2.71 (m, 6H), 2.20 (s, 3H), 1.80-1.70 (m, 4H), 1.23 (t, J = 7.5 Hz, 3H). ¹³C NMR (151 MHz, CD₃OD) δ 178.90, 154.68, 142.58, 136.56, 136.32, 136.01, 134.99, 134.56, 128.69, 128.62, 128.61, 126.00, 125.26, 123.45, 75.81, 58.35, 57.83, 36.29, 29.01, 28.78, 25.11, 23.06, 14.25, 11.51. HRMS (ESI) (m/z): [M+H]⁺ calcd for C₂₆H₃₂N₂O₃, 421.2492; found: 421.2492. HPLC Purity: 98.54%, retention time: 1.48 min.

### Structure of (E)-1-(4-(1-(((3-cyano-4-isopropoxybenzyl)oxy)imino)ethyl)-2-ethylbenzyl)azetidine-3-carboxylic acid (Compound 16)

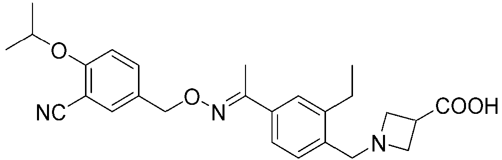

Pale yellow solid, yield 52.3%, melting point: 112.6-112.9 °C. ¹H NMR (400 MHz, CD₃OD) δ 7.64 (d, *J* = 6.7 Hz, 2H), 7.56 (s, 1H), 7.50 (dd, *J* = 8.0, 1.6 Hz, 1H), 7.33 (d, *J* = 8.1 Hz, 1H), 7.16 (d, *J* = 9.4 Hz, 1H), 5.14 (s, 2H), 4.78-4.73 (m, 1H), 4.18 (s, 2H), 3.99 (t, *J* = 9.3 Hz, 2H), 3.90 (t, *J* = 8.6 Hz, 2H), 3.40-3.33 (m, 1H), 2.76 (q, *J* = 7.5 Hz, 2H), 2.23 (s, 3H), 1.38 (s, 3H), 1.37 (s, 3H), 1.23 (t, *J* = 7.5 Hz, 3H). ¹³C NMR (151 MHz, CD₃OD) δ 177.23, 159.53, 154.92, 143.23, 136.96, 134.67, 133.44, 131.00, 129.23, 126.42, 123.80, 116.06, 113.66, 101.88, 74.22, 71.68, 57.49, 56.20, 35.40, 25.12, 20.71, 14.38, 11.39. HRMS (ESI) (m/z): [M+H]⁺ calcd for C₂₆H₃₁N₃O₄, 450.2396; found: 450.2395. HPLC Purity: 96.46%, retention time: 1.02 min.

### Structure of (E)-1-(2-ethyl-4-(1-((2-methyl-[1,1'-biphenyl]-4-yl)methoxy)imino)ethyl)benzyl)pyrrolidine-3-carboxylic acid (Compound 28)

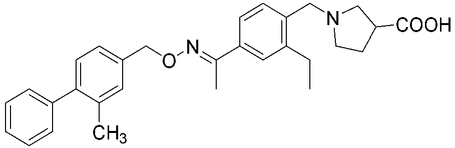

Greige solid, yield 30.1%, melting point: 144.3-146.4 °C. ¹H NMR (600 MHz, CD₃OD) δ 7.62 (s, 1H), 7.55 (dd, *J* = 8.0, 1.6 Hz, 1H), 7.47 (dd, *J* = 14.4, 6.3 Hz, 1H), 7.41-7.35 (m, 2H), 7.33-7.28 (m, 2H), 7.26 (dd, *J* = 5.0, 3.2 Hz, 3H), 7.14 (d, *J* = 7.7 Hz, 1H), 5.20 (s, 2H), 4.29 (s, 2H), 3.47-3.35 (m, 1H), 3.34-3.30 (m, 2H), 3.24 (t, *J* = 7.1 Hz, 2H), 3.03-3.09 (m, 1H), 2.80 (q, *J* = 7.5 Hz, 2H), 2.31-2.27 (m, 1H), 2.25 (s, 3H), 2.22 (s, 3H), 1.20 (t, *J* = 7.1 Hz, 3H). ¹³C NMR (151 MHz, CD₃OD) δ 178.58, 154.38, 143.61, 141.73, 141.47, 137.53, 137.03, 134.92, 130.99, 130.41, 129.86, 129.34, 128.79, 127.79, 126.57, 126.52, 125.40, 123.90, 75.69, 56.76, 54.66, 53.58, 43.99, 27.89, 25.26, 19.27, 14.57, 11.48. HRMS (ESI) (m/z): [M+H]⁺ calcd for C₃₀H₃₄N₂O₃, 471.2648; found: 471.2647. HPLC Purity: 96.16%, retention time: 1.66 min.

### Structure of (R, E)-1-(2-ethyl-4-(1-((2-methyl-[1,1'-biphenyl]-4-yl)methoxy)imino)ethyl)benzyl)pyrrolidine-3-carboxylic acid (Compound 29)

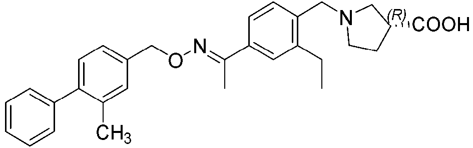

White solid, yield 14.9%, melting point: 149.6-150.6 °C. ¹H NMR (400 MHz, CD₃OD) δ 7.60 (s, 1H), 7.54 (d, *J* = 8.0 Hz, 1H), 7.45 (d, *J* = 8.0 Hz, 1H), 7.38 (t, *J* = 7.3 Hz, 2H), 7.34-7.18 (m, 5H), 7.14 (d, *J* = 7.7 Hz, 1H), 5.20 (s, 2H), 4.26 (s, 2H), 3.42-3.29 (m, 3H), 3.20 (t, *J* = 7.1 Hz, 2H), 3.09-2.97 (m, 1H), 2.79 (q, *J* = 7.5 Hz, 2H), 2.30-2.27 (m, 1H), 2.24 (s, 3H), 2.22 (s, 3H), 1.22 (t, *J* = 7.5 Hz, 3H). ¹³C NMR (151 MHz, CD₃OD) δ 178.63, 154.39, 143.55, 141.74, 141.47, 137.42, 137.04, 134.92, 131.32, 130.35, 129.85, 129.33, 128.79, 127.78, 126.53, 126.51, 125.39, 123.85, 75.68, 56.79, 54.73, 53.59, 44.01, 27.89, 25.24, 19.26, 14.55, 11.47. HRMS (ESI) (m/z): [M+H]⁺ calcd for C₃₀H₃₄N₂O₃, 471.2648; found: 471.2650. HPLC Purity: 97.51%, retention time: 1.45 min.

### Structure of (S, E)-1-(2-ethyl-4-(1-((2-methyl-[1,1'-biphenyl]-4-yl)methoxy)imino)ethyl)benzyl)pyrrolidine-3-carboxylic acid (Compound 30)

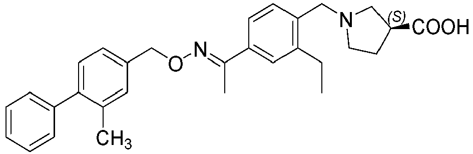

White solid, yield 31.8%, melting point: 145.6-148.9 °C. ¹H NMR (400 MHz, CD₃OD) δ 7.57 (s, 1H), 7.51 (d, *J* = 8.0 Hz, 1H), 7.44-7.35 (m, 3H), 7.33-7.26 (m, 5H), 7.17 (d, *J* = 7.7 Hz, 1H), 5.20 (s, 2H), 4.02 (s, 2H), 3.19 (t, *J* = 8.6 Hz, 1H), 3.11-3.00 (m, 3H), 2.90 (dd, *J* = 17.2, 7.9 Hz, 1H), 2.79 (q, *J* = 7.5 Hz, 2H), 2.26 (s, 3H), 2.24 (s, 3H), 2.15 (dd, *J* = 14.6, 7.3 Hz, 2H), 1.23 (t, *J* = 7.5 Hz, 3H). ¹³C NMR (151 MHz, CD₃OD) δ 179.95, 154.70, 143.25, 141.79, 141.48, 137.08, 136.62, 134.91, 130.01, 129.84, 129.31, 128.79, 127.77, 126.49, 126.28, 125.37, 123.57, 75.60, 57.40, 55.54, 53.76, 44.56, 28.06, 25.20, 19.22, 14.48, 11.50. HRMS (ESI) (m/z): [M+H]⁺ calcd for C₃₀H₃₄N₂O₃, 471.2648; found: 471.2650. HPLC Purity: 98.16%, retention time: 1.52 min.

### Structure of (E)-3-((2-ethyl-4-(1-((2-methyl-[1,1'-biphenyl]-4-yl)methoxy)imino)ethyl)benzyl)amine)propanoic acid (Compound 31)

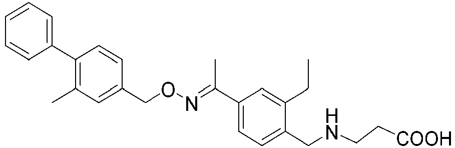

White solid, yield 22.4%, melting point: 149.6-149.8 °C. ¹H NMR (400 MHz, CD₃OD) δ 7.66 (s, 1H), 7.60 (d, *J* = 8.1 Hz, 1H), 7.43 (dd, *J* = 17.6, 7.9 Hz, 3H), 7.36-7.28 (m, 5H), 7.19 (d, *J* = 7.7 Hz, 1H), 5.24 (s, 2H), 4.28 (s, 2H), 3.25 (t, *J* = 6.2 Hz, 2H), 2.83 (q, *J* = 7.5 Hz, 2H), 2.60-2.47 (m, 2H), 2.28 (s, 3H), 2.25 (s, 3H), 1.29 (t, *J* = 7.5 Hz, 3H). ¹³C NMR (151 MHz, CD₃OD) δ 176.80, 154.30, 143.38, 141.76, 141.51, 137.84, 137.02, 134.93, 130.29, 130.06, 129.85, 129.31, 128.78, 127.78, 126.55, 126.52, 125.39, 124.05, 75.71, 44.64, 31.36, 25.08, 21.94, 19.22, 14.38, 11.40. HRMS (ESI) (m/z): [M+H]⁺ calcd for C₂₈H₃₂N₂O₃, 445.2491; found: 445.2490. HPLC Purity: 99.29%, retention time: 1.24 min.

### Structure of (E)-4-((2-ethyl-4-(1-((2-methyl-[1,1'-biphenyl]-4-yl)methoxy)imino)ethyl)benzyl)aminobutyric acid (Compound 32)

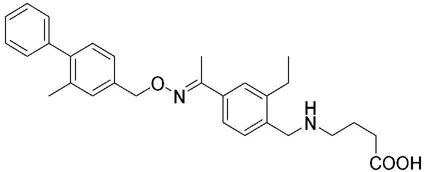

White solid, yield 13.2%, melting point: 133.3-136.3 °C. ¹H NMR (400 MHz, CD₃OD) δ 7.63 (s, 1H), 7.58 (d, *J* = 8.1 Hz, 1H), 7.49-7.36 (m, 3H), 7.36-7.28 (m, 5H), 7.17 (d, *J* = 7.7 Hz, 1H), 5.21 (s, 2H), 4.21 (s, 2H), 3.15 (t, *J* = 6.2 Hz, 2H), 2.79 (q, *J* = 7.5 Hz, 2H), 2.45-2.37 (m, 2H), 2.26 (s, 3H), 2.24 (s, 3H), 1.90-1.85 (m, 2H), 1.26 (t, *J* = 7.5 Hz, 3H). ¹³C NMR (151 MHz, CD₃OD) δ 176.86, 154.32, 143.24, 141.77, 141.51, 137.67, 137.04, 134.93, 130.46, 129.84, 129.80, 129.31, 128.78, 127.78, 126.52, 126.48, 125.38, 124.04, 75.70, 35.68, 25.21, 21.93, 21.44, 19.22, 14.36, 11.40. HRMS (ESI) (m/z): [M+H]⁺ calcd for C₂₉H₃₄N₂O₃, 459.2648; found: 459.2647. HPLC Purity: 98.69%, retention time: 1.34 min.

### Structure of (E)-1-(2-ethyl-4-(1-((2-methyl-[1,1'-biphenyl]-4-yl)methoxy)imino)ethyl)benzyl)piperidine-3-carboxylic acid (Compound 33)

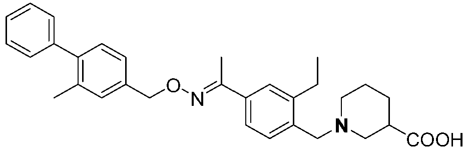

Pale yellow oily, yield 15.3%. ¹H NMR (400 MHz, CD₃OD) δ 7.61 (s, 1H), 7.56 (d, *J* = 8.0 Hz, 1H), 7.50-7.37 (m, 3H), 7.36-7.28 (m, 5H), 7.22-7.09 (m, 1H), 5.23 (s, 2H), 4.12 (s, 2H), 3.22-2.88 (m, 4H), 2.83 (q, *J* = 7.5 Hz, 2H), 2.61 (s, 1H), 2.27 (s, 3H), 2.25 (s, 3H), 1.79-1.70 (m, 4H), 1.31-1.19 (m, 3H). ¹³C NMR (151 MHz, CD₃OD) δ 178.19, 154.48, 143.99, 141.75, 141.48, 137.42, 137.05, 134.92, 130.86, 129.85, 129.32, 128.81, 128.79, 128.77, 127.79, 126.51, 125.39, 123.77, 75.67, 57.43, 55.07, 52.96, 25.22, 22.28, 19.25, 14.48, 11.48. HRMS (ESI) (m/z): [M+H]⁺ calcd for C₃₁H₃₆N₂O₃, 485.2804; found: 485.2802. HPLC Purity: 97.56%, retention time: 1.42 min.

### Structure of (S, E)-1-(2-ethyl-4-(1-(((4'-fluoro-2-methyl-[1,1'-biphenyl]-4-yl)methoxy)imino)ethyl)benzyl)pyrrolidine-3-carboxylic acid (Compound 34)

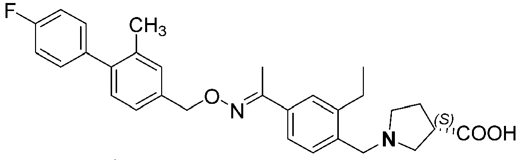

Pale yellow solid, yield 11.3%. ¹H NMR (600 MHz, CD₃OD) δ 7.64 (d, *J* = 1.9 Hz, 1H), 7.58 (dd, *J* = 8.1, 1.9 Hz, 1H), 7.47 (d, *J* = 8.1 Hz, 1H), 7.33-7.25 (m, 4H), 7.19-7.10 (m, 3H), 5.22 (s, 2H), 4.42-4.34 (m, 2H), 3.53- 3.47 (m, 1H), 3.38-3.32 (m, 2H), 3.29-3.24 (m, 1H), 3.12-3.04 (m, 1H), 2.82 (q, *J* = 7.5 Hz, 2H), 2.32 (dt, *J* = 14.2, 7.9 Hz, 1H), 2.27 (s, 3H), 2.24 (s, 3H), 2.23-2.18 (m, 1H), 1.26 (t, *J* = 7.5 Hz, 3H). ¹³C NMR (151 MHz, CD₃OD) δ 177.91, 162.81, 161.19, 154.29, 143.70, 140.39, 137.84, 137.27, 135.04, 130.62, 130.56, 130.44, 129.88, 129.37, 114.54, 114.40, 75.64, 56.64, 54.50, 53.58, 43.83, 27.74, 25.22, 19.19, 14.54, 11.38. MS (ESI) (m/z): [M+H]⁺ 489.25.

### Structure of (S, E)-1-(2-ethyl-4-(1-(((2-fluoro-[1,1'-biphenyl]-4-yl)methoxy)imino)ethyl)benzyl)pyrrolidine-3-carboxylic acid (Compound 35)

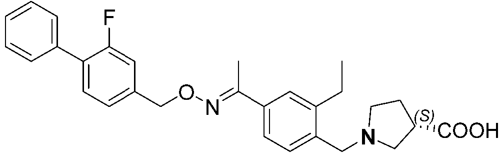

Pale yellow solid, yield 18.7%. ¹H NMR (600 MHz, CD₃OD) δ 7.63 (s, 1H), 7.57 (d, *J* = 6.1 Hz, 1H), 7.52 (d, *J* = 8.0 Hz, 2H), 7.48 - 7.40 (m, 4H), 7.35 (t, *J* = 7.4 Hz, 1H), 7.28 (d, *J* = 9.4 Hz, 1H), 7.22 (d, *J* = 11.5 Hz, 1H), 5.26 (s, 2H), 4.33 (d, *J* = 4.1 Hz, 2H), 3.46 (dd, *J* = 11.1, 5.8 Hz, 1H), 3.34 (d, *J* = 11.2 Hz, 2H), 3.25 (t, *J* = 9.0 Hz, 1H), 3.07 (ddd, *J* = 14.5, 8.6, 5.8 Hz, 1H), 2.81 (q, *J* = 7.5 Hz, 2H), 2.29 (s, 4H), 2.22 (dq, *J* = 13.0, 6.4 Hz, 1H), 1.24 (t, *J* = 7.5 Hz, 3H). ¹³C NMR (151 MHz, CD₃OD) δ 178.47, 159.53, 154.78, 143.69, 135.54, 130.42, 130.38, 128.61, 128.58, 128.09, 127.35, 126.66, 123.95, 123.69, 123.66, 115.10, 114.94, 74.70, 56.65, 54.60, 53.63, 43.70, 27.76, 25.22, 14.52, 11.42. MS (ESI) (m/z): [M+H]⁺ 475.30.

### Structure of (S, E)-1-(2-ethyl-4-(1-(((4-(furan-3-yl)-3-methylbenzyl)oxy)imino)ethyl)benzyl)pyrrolidine-3-carboxylic acid (Compound 36)

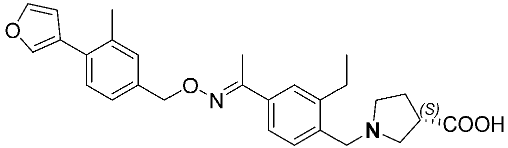

Pale yellow solid, yield 21.2%. ¹H NMR (600 MHz, CD₃OD) δ 7.61 (dd, *J* = 13.5, 1.8 Hz, 2H), 7.56 - 7.53 (m, 2H), 7.47 (d, *J* = 8.1 Hz, 1H), 7.30 (d, *J* = 7.8 Hz, 1H), 7.27 (s, 1H), 7.23 (dd, *J* = 7.8, 1.8 Hz, 1H), 6.62 (d, *J* = 1.9 Hz, 1H), 5.18 (s, 2H), 4.44 - 4.28 (m, 2H), 3.52 (dd, *J* = 11.3, 5.7 Hz, 1H), 3.40 - 3.36 (m, 1H), 3.35 - 3.32 (m, 1H), 3.27 (dt, *J* = 11.1, 7.4 Hz, 1H), 3.08 (ddd, *J* = 14.4, 8.8, 5.7 Hz, 1H), 2.80 (q, *J* = 7.5 Hz, 2H), 2.36 (s, 3H), 2.30 (dt, *J* = 15.6, 7.0 Hz, 1H), 2.24 (s, 3H), 2.22 (dd, *J* = 13.5, 6.2 Hz, 1H), 1.23 (t, *J* = 7.5 Hz, 3H). ¹³C NMR (151 MHz, CD₃OD) δ 177.76, 154.25, 143.72, 142.57, 139.97, 137.86, 136.85, 135.34, 131.69, 130.51, 130.15, 128.74, 126.66, 125.53, 123.98, 110.90, 75.65, 56.45, 54.32, 53.53, 43.55, 27.70, 25.24, 20.03, 14.56, 11.41.

### Structure of (S, E)-1-(2-ethyl-4-(1-(((3-methyl-4-(pyridin-4-yl)benzyl)oxy)imino)ethyl)benzyl)pyrrolidine-3-carboxylic acid (Compound 37)

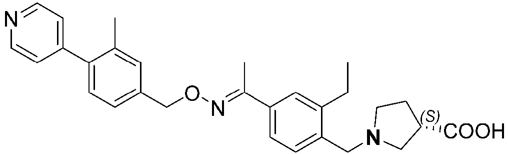

Pale yellow solid, yield 19.2%. ¹H NMR (600 MHz, CD₃OD) δ8.56 (d, *J* = 6.2 Hz, 2H), 7.63 (s, 1H), 7.56 (d, *J* = 8.1 Hz, 1H), 7.47 (d, *J* = 8.1 Hz, 1H), 7.40 (d, *J* = 6.2 Hz, 2H), 7.37 (s, 1H), 7.34 (d, *J* = 7.8 Hz, 1H), 7.22 (d, *J* = 7.8 Hz, 1H), 5.24 (s, 2H), 4.42 - 4.33 (m, 2H), 3.50 (dd, *J* = 11.2, 5.7 Hz, 1H), 3.38 (d, *J* = 7.8 Hz, 1H), 3.34 (s, 1H), 3.30 - 3.24 (m, 1H), 3.11 - 3.04 (m, 1H), 2.81 (q, *J* = 7.5 Hz, 2H), 2.32 (dd, *J* = 15.2, 7.1 Hz, 1H), 2.28 (s, 3H), 2.27 (s, 3H), 2.22 (dt, *J* = 13.6, 6.7 Hz, 1H), 1.24 (t, *J* = 7.5 Hz, 3H). ¹³C NMR (151 MHz, CD₃OD) δ 177.89, 154.44, 150.81, 148.56, 143.72, 138.78, 138.11, 137.73, 134.81, 130.48, 130.13, 128.98, 126.66, 125.69, 124.62, 123.97, 75.40, 56.56, 54.41, 53.57, 43.62, 27.73, 25.20, 19.02, 14.56, 11.38.

### Structure of (S, E)-1-(2-ethyl-4-(1-(((3-methyl-4-(pyrimidin-5-yl)benzyl)oxy)imino)ethyl)benzyl)pyrrolidine-3-carboxylic acid (Compound 38)

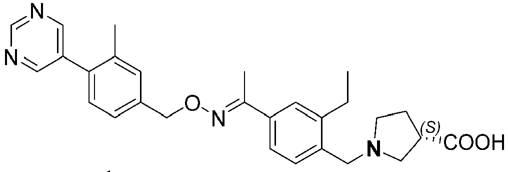

Pale yellow solid, yield 19.7%. ¹H NMR (600 MHz, CD₃OD) δ 9.14 (s, 1H), 8.78 (s, 2H), 7.62 (d, *J* = 1.9 Hz, 1H), 7.56 (dd, *J* = 8.1, 1.9 Hz, 1H), 7.47 (d, *J* = 8.1 Hz, 1H), 7.40 (d, *J* = 1.7 Hz, 1H), 7.37 (dd, *J* = 7.8, 1.8 Hz, 1H), 7.26 (d, *J* = 7.8 Hz, 1H), 5.24 (s, 2H), 4.42 - 4.32 (m, 2H), 3.49 (dd, *J* = 11.2, 5.8 Hz, 1H), 3.38 - 3.32 (m, 2H), 3.27 (dt, *J* = 10.8, 7.3 Hz, 1H), 3.08 (ddd, *J* = 14.4, 8.7, 5.8 Hz, 1H), 2.81 (q, *J* = 7.5 Hz, 2H), 2.35 - 2.31 (m, 1H), 2.29 (s, 3H), 2.27 (s, 3H), 2.25 - 2.19 (m, 1H), 1.24 (t, *J* = 7.5 Hz, 3H). ¹³C NMR (151 MHz, CD₃OD) δ 178.06, 156.57, 156.36, 154.49, 143.72, 139.33, 137.69, 135.64, 135.61, 133.41, 130.46, 130.18, 129.63, 126.65, 125.90, 123.96, 75.31, 56.55, 54.46, 53.59, 43.61, 27.73, 25.24, 18.95, 14.55, 11.39.

### Structure of (S, E)-1-(2-ethyl-4-(1-((4-(-1-(tert-butyl)-1H-pyrazol-4-yl)-3-methylbenzyl)oxy)imino)ethyl)benzyl)pyrrolidine-3-carboxylic acid (Compound 39)

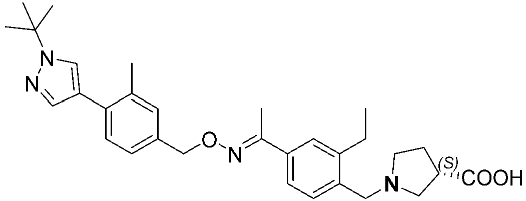

Pale yellow solid, yield 31.1%. ¹H NMR (600 MHz, CD₃OD) δ 7.85 (d, *J* = 0.8 Hz, 1H), 7.65 - 7.61 (m, 2H), 7.56 (dd, *J* = 8.1, 1.9 Hz, 1H), 7.45 (d, *J* = 8.1 Hz, 1H), 7.33 (d, *J* = 7.8 Hz, 1H), 7.29 - 7.26 (m, 1H), 7.23 (dd, *J* = 7.8, 1.8 Hz, 1H), 5.18 (s, 2H), 4.39 - 4.31 (m, 2H), 3.47 (dd, *J* = 11.1, 5.8 Hz, 1H), 3.33 (dd, *J* = 10.8, 6.1 Hz, 2H), 3.25 (d, *J* = 10.3 Hz, 1H), 3.07 (ddd, *J* = 14.7, 8.8, 5.9 Hz, 1H), 2.80 (q, *J* = 7.5 Hz, 2H), 2.39 (s, 3H), 2.33 - 2.26 (m, 1H), 2.25 (s, 3H), 2.24 - 2.19 (m, 1H), 1.62 (s, 9H), 1.24 (t, *J* = 7.5 Hz, 3H). ¹³C NMR (151 MHz, CD₃OD) δ 178.15, 154.25, 143.67, 137.80, 137.77, 136.36, 134.87, 131.84, 130.41, 130.20, 128.69, 126.65, 125.63, 125.41, 123.95, 121.12, 75.70, 58.41, 56.64, 54.55, 53.60, 43.65, 28.66, 27.75, 25.19, 20.06, 14.54, 11.39. MS (ESI) (m/z): [M+H]⁺517.30.

### Structure of (S, E)-1-(2-ethyl-4-(1-((3-methyl-4-(1-methyl-1H-pyrazol-4-yl)benzyl)oxy)imino)ethyl)benzyl)pyrrolidine-3-carboxylic acid (Compound 40)

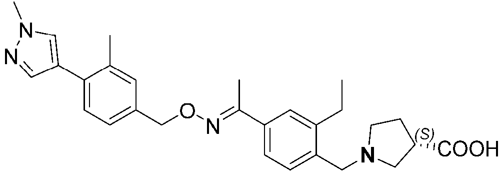

Pale yellow solid, yield 29.5%. ¹H NMR (600 MHz, CD₃OD) δ 7.74 (s, 1H), 7.62 (d, *J* = 1.9 Hz, 1H), 7.61 - 7.60 (m, 1H), 7.56 (dd, *J* = 8.1, 1.9 Hz, 1H), 7.45 (d, *J* = 8.1 Hz, 1H), 7.32 (d, *J* = 7.8 Hz, 1H), 7.28 (d, *J* = 1.8 Hz, 1H), 7.22 (dd, *J* = 7.9, 1.8 Hz, 1H), 5.18 (s, 2H), 4.41 - 4.32 (m, 2H), 3.93 (s, 3H), 3.49 (dd, *J* = 11.2, 5.8 Hz, 1H), 3.35 (dd, *J* = 11.2, 7.0 Hz, 2H), 3.26 (dt, *J* = 10.8, 7.3 Hz, 1H), 3.12 - 3.02 (m, 1H), 2.80 (q, *J* = 7.5 Hz, 2H), 2.38 (s, 3H), 2.30 (ddd, *J* = 15.7, 8.4, 6.2 Hz, 1H), 2.25 (s, 3H), 2.24 - 2.18 (m, 1H), 1.24 (t, *J* = 7.5 Hz, 3H). ¹³C NMR (151 MHz, CD₃OD) δ 177.95, 154.26, 143.69, 138.10, 137.85, 136.50, 134.86, 131.45, 130.44, 130.23, 129.63, 128.60, 126.66, 125.64, 123.96, 121.92, 75.66, 56.60, 54.49, 53.58, 43.61, 37.44, 27.73, 25.22, 20.08, 14.53, 11.38. MS (ESI) (m/z): [M+H]⁺ 475.30.

### Structure of (S, E)-1-(2-ethyl-4-(1-(((3-methyl-4-(thiophen-3-yl)benzyl)oxy)imino)ethyl)benzyl)pyrrolidine-3-carboxylic acid (Compound 41)

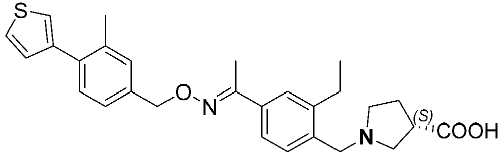

Pale yellow solid, yield 9.5%. ¹H NMR (600 MHz, CD₃OD) δ 7.63 (d, *J* = 1.9 Hz, 1H), 7.56 (dd, *J* = 8.1, 1.9 Hz, 1H), 7.48 - 7.46 (m, 1H), 7.43 (dd, *J* = 4.9, 3.0 Hz, 1H), 7.29 - 7.27 (m, 1H), 7.27 (dd, *J* = 3.0, 1.3 Hz, 1H), 7.26 - 7.22 (m, 2H), 7.13 (dd, *J* = 5.0, 1.3 Hz, 1H), 5.19 (s, 2H), 4.38 (dd, *J* = 6.1, 3.1 Hz, 2H), 3.53 - 3.49 (m, 1H), 3.36 (dd, *J* = 10.8, 4.3 Hz, 2H), 3.28 (dd, *J* = 10.9, 7.2 Hz, 1H), 3.09 (td, *J* = 8.6, 4.2 Hz, 1H), 2.81 - 2.78 (m, 2H), 2.31 (s, 4H), 2.25 (s, 3H), 2.24 - 2.20 (m, 1H), 1.24 (t, *J* = 7.5 Hz, 3H). ¹³C NMR (151 MHz, CD₃OD) δ 178.04, 154.24, 143.72, 141.88, 137.88, 137.04, 136.10, 135.31, 130.50, 129.99, 129.30, 128.45, 126.68, 125.40, 124.71, 123.99, 122.26, 75.67, 56.42, 54.43, 53.60, 43.54, 27.71, 25.26, 19.58, 14.56, 11.41.

### Structure of (S, E)-1-(2-ethyl-4-(1-(((2-(trifluoromethyl)-[1,1'-biphenyl]-4-yl)methoxy)imino)ethyl)benzyl)pyrrolidine-3-carboxylic acid (Compound 42)

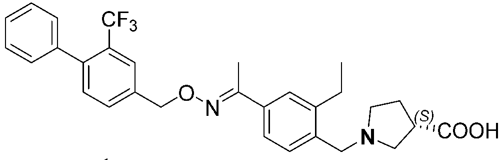

Pale yellow solid, yield 31.8%. ¹H NMR (600 MHz, CD₃OD) δ 7.81 (s, 1H), 7.66 (d, *J* = 7.8 Hz, 1H), 7.63 (d, *J* = 1.9 Hz, 1H), 7.56 (dd, *J* = 8.0, 1.9 Hz, 1H), 7.48 (d, *J* = 8.1 Hz, 1H), 7.38 (dd, *J* = 5.1, 1.9 Hz, 3H), 7.34 (d, *J* = 7.8 Hz, 1H), 7.28 (dd, *J* = 6.7, 2.9 Hz, 2H), 5.32 (s, 2H), 4.41 - 4.32 (m, 2H), 3.49 (dd, *J* = 11.2, 5.8 Hz, 1H), 3.34 (t, *J* = 8.1 Hz, 2H), 3.29 - 3.23 (m, 1H), 3.08 (ddd, *J* = 14.4, 8.7, 5.9 Hz, 1H), 2.81 (q, *J* = 7.5 Hz, 2H), 2.34 - 2.29 (m, 1H), 2.29 (s, 3H), 2.23 (tt, *J* = 13.5, 6.2 Hz, 1H), 1.24 (t, *J* = 7.5 Hz, 3H). ¹³C NMR (151 MHz, CD₃OD) δ 179.63, 154.93, 143.74, 140.81, 139.60, 138.23, 137.55, 132.04, 130.96, 130.48, 128.64, 128.62, 127.45, 127.36, 126.66, 125.35, 125.31, 123.97, 74.69, 56.58, 54.49, 53.60, 43.68, 27.75, 25.23, 14.52, 11.41. MS (ESI) (m/z): [M+H]⁺ 525.25.

### Structure of (S, E)-1-(2-ethyl-4-(1-((3-methyl-4-(1-methyl-1H-pyrrol-3-yl)benzyl)oxy)imino)ethyl)benzyl)pyrrolidine-3-carboxylic acid (Compound 43)

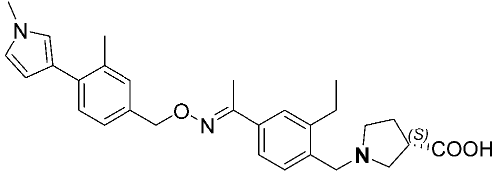

Pale yellow solid, yield 21.5%. ¹H NMR (600 MHz, CD₃OD) δ 7.61 (s, 1H), 7.54 (d, *J* = 7.8 Hz, 1H), 7.44 (d, *J* = 8.1 Hz, 1H), 7.29 (d, *J* = 8.0 Hz, 1H), 7.20 (s, 1H), 7.15 (d, *J* = 7.8 Hz, 1H), 6.74 (s, 1H), 6.64 (s, 1H), 6.21 (s, 1H), 5.15 (s, 2H), 4.35 (t, *J* = 10.7 Hz, 2H), 3.66 (s, 3H), 3.48 (dd, *J* = 11.3, 5.7 Hz, 1H), 3.34 (s, 2H), 3.24 (d, *J* = 9.9 Hz, 1H), 3.07 (d, *J* = 8.1 Hz, 1H), 2.79 (q, *J* = 7.5 Hz, 2H), 2.39 (s, 3H), 2.30 - 2.24 (m, 1H), 2.23 (s, 3H), 2.21 (s, 1H), 1.23 (t, *J* = 7.6 Hz, 3H). ¹³C NMR (151 MHz, CD₃OD) δ 178.09, 154.09, 143.65, 137.89, 135.78, 134.80, 134.36, 130.45, 130.22, 130.11, 128.42, 126.64, 125.44, 123.94, 123.77, 121.28, 120.43, 108.50, 75.92, 56.53, 54.45, 53.57, 43.63, 34.81, 27.73, 25.22, 20.45, 14.53, 11.40. MS (ESI) (m/z): [M+H]⁺ 474.25.

### Structure of (S, E)-1-(2-ethyl-4-(1-((3-methyl-4-(1-methyl-1H-imidazol-5-yl)benzyl)oxy)imino)ethyl)benzyl)pyrrolidine-3-carboxylic acid (Compound 44)

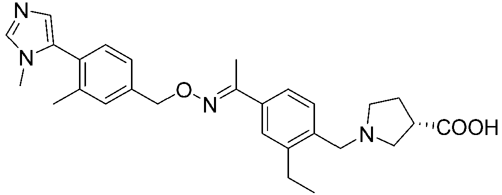

Pale yellow solid, yield 27.1%. ¹H NMR (600 MHz, CD₃OD) δ7.72 (s, 1H), 7.62 (s, 1H), 7.56 (d, *J* = 7.9 Hz, 1H), 7.46 (d, *J* = 8.1 Hz, 1H), 7.38 (s, 1H), 7.32 (d, *J* = 7.8 Hz, 1H), 7.21 (d, *J* = 7.8 Hz, 1H), 6.88 (s, 1H), 5.24 (s, 2H), 4.34 (d, *J* = 4.2 Hz, 2H), 3.45 (s, 4H), 3.34 (s, 2H), 3.26 (s, 1H), 3.07 (q, *J* = 6.0 Hz, 1H), 2.81 (q, *J* = 7.6 Hz, 2H), 2.27 (s, 3H), 2.23 (t, *J* = 6.7 Hz, 2H), 2.18 (s, 3H), 1.24 (t, *J* = 7.5 Hz, 3H). ¹³C NMR (151 MHz, CD₃OD) δ 178.17, 154.52, 143.68, 139.34, 138.14, 137.90, 137.62, 132.16, 130.91, 130.41, 129.62, 128.07, 126.63, 126.33, 125.24, 123.94, 75.41, 56.69, 54.56, 53.60, 43.72, 30.75, 27.78, 25.23, 18.73, 14.54, 11.42. MS (ESI) (m/z): [M+H]⁺ 475.25.

### Structure of (S, E)-1-(2-ethyl-4-(1-(((3,5-dimethylisoxazol-4-yl)-3-methylbenzyl)oxy)imino)ethyl)benzyl)pyrrolidine-3-carboxylic acid (Compound 45)

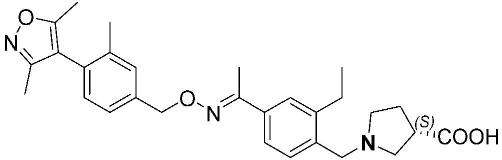

Pale yellow solid, yield 20.7%. ¹H NMR (600 MHz, CD₃OD) δ 7.64 (s, 1H), 7.57 (d, *J* = 8.1 Hz, 1H), 7.48 (d, *J* = 8.1 Hz, 1H), 7.39 (s, 1H), 7.31 (d, *J* = 7.8 Hz, 1H), 7.12 (dd, *J* = 7.8, 4.1 Hz, 1H), 5.23 (s, 2H), 4.40 (d, *J* = 13.6 Hz, 2H), 3.50 (dd, *J* = 11.3, 5.8 Hz, 1H), 3.36 (q, *J* = 10.0, 9.1 Hz, 2H), 3.30 - 3.25 (m, 1H), 3.12 - 3.05 (m, 1H), 2.82 (q, *J* = 7.5 Hz, 2H), 2.31 (dd, *J* = 14.6, 6.8 Hz, 1H), 2.27 (s, 3H), 2.26 - 2.23 (m, 1H), 2.21 (s, 3H), 2.14 (s, 3H), 2.05 (s, 3H), 1.25 (t, *J* = 7.5 Hz, 3H). ¹³C NMR (151 MHz, CD₃OD) δ 178.08, 165.70, 159.25, 154.41, 143.72, 138.64, 137.45, 130.52, 130.46, 129.72, 128.36, 126.66, 125.58, 123.98, 115.90, 75.51, 56.54, 54.47, 53.59, 43.60, 27.73, 25.24, 18.47, 14.55, 11.43, 9.83, 8.99. MS (ESI) (m/z): [M+H]⁺ 490.30.

### Structure of (S, E)-1-(2-ethyl-4-(1-(((3-methylbenzyl)oxy)imino)ethyl)benzyl)pyrrolidine-3-carboxylic acid (Compound 46)

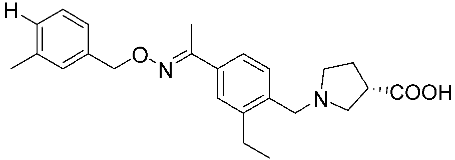

Pale yellow solid, yield 50.1%. ¹H NMR (600 MHz, CD₃OD) δ 7.62 (d, *J* = 1.9 Hz, 1H), 7.55 (dd, *J* = 8.1, 1.9 Hz, 1H), 7.47 (d, *J* = 8.1 Hz, 1H), 7.25 - 7.19 (m, 2H), 7.17 (d, *J* = 7.5 Hz, 1H), 7.10 (d, *J* = 7.5 Hz, 1H), 5.16 (s, 2H), 4.42 - 4.32 (m, 2H), 3.50 (dd, *J* = 11.3, 5.9 Hz, 1H), 3.38 - 3.33 (m, 2H), 3.28 (dd, *J* = 10.9, 7.5 Hz, 1H), 3.09 (ddd, *J* = 14.6, 8.5, 5.8 Hz, 1H), 2.81 (q, *J* = 7.5 Hz, 2H), 2.33 (s, 3H), 2.32 - 2.28 (m, 1H), 2.24 (s, 4H), 1.24 (t, *J* = 7.5 Hz, 3H). ¹³C NMR (151 MHz, CD₃OD) δ 178.42, 154.23, 143.69, 137.94, 137.83, 137.66, 130.47, 128.47, 128.09, 127.88, 126.65, 124.91, 123.96, 75.94, 56.52, 54.52, 53.64, 43.66, 27.75, 25.25, 20.06, 14.55, 11.40. MS (ESI) (m/z): [M+H]⁺ 395.25.

### Structure of (S, E)-1-(2-ethyl-4-(1-(((2',4'-difluoro-2-methyl-[1,1'-biphenyl]-4-yl)methoxy)imino)ethyl)benzyl)pyrrolidine-3-carboxylic acid (Compound 47)

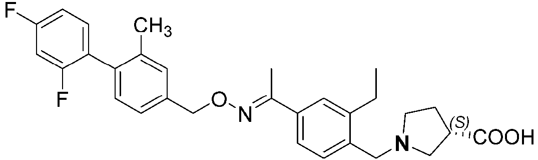

Pale yellow solid, yield 19.0%. ¹H NMR (600 MHz, CD₃OD) δ 7.63 (s, 1H), 7.56 (d, *J* = 7.9 Hz, 1H), 7.47 (d, *J* = 8.1 Hz, 1H), 7.33 (s, 1H), 7.28 (d, *J* = 8.0 Hz, 1H), 7.25 (d, *J* = 7.5 Hz, 1H), 7.14 (d, *J* = 7.9 Hz, 1H), 7.01 (t, *J* = 8.8 Hz, 2H), 5.22 (s, 2H), 4.41 - 4.28 (m, 2H), 3.48 (dd, *J* = 11.1, 5.7 Hz, 1H), 3.37 - 3.32 (m, 2H), 3.25 (q, *J* = 8.0 Hz, 1H), 3.07 (t, *J* = 7.8 Hz, 1H), 2.80 (q, *J* = 7.5 Hz, 2H), 2.34 - 2.28 (m, 1H), 2.26 (s, 3H), 2.22 (d, *J* = 14.8 Hz, 1H), 2.15 (s, 3H), 1.24 (t, *J* = 7.5 Hz, 3H). ¹³C NMR (151 MHz, CD₃OD) δ 177.31, 162.53, 160.90, 159.67, 158.03, 153.59, 142.87, 137.32, 136.91, 135.57, 133.46, 131.48, 131.44, 129.64, 129.00, 128.62, 125.84, 124.47, 123.14, 110.15, 109.98, 102.59, 102.42, 102.24, 74.75, 55.85, 53.67, 52.76, 42.92, 26.96, 24.42, 17.83, 13.73, 10.62. MS (ESI) (m/z): [M+H]⁺ 507.25.

### Structure of (S, E)-1-(2-ethyl-4-(1-(((3-methyl-4-(pyrrolidin-1-yl)benzyl)oxy)imino)ethyl)benzyl)pyrrolidine-3-carboxylic acid (Compound 48)

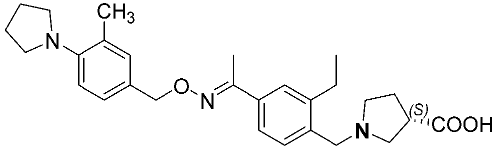

Pale yellow solid, yield 17.5%. ¹H NMR (600 MHz, CD₃OD) δ 7.61 (d, *J* = 1.9 Hz, 1H), 7.54 (dd, *J* = 8.0, 1.9 Hz, 1H), 7.46 (d, *J* = 8.1 Hz, 1H), 7.14 (d, *J* = 2.1 Hz, 1H), 7.11 (dd, *J* = 8.2, 2.2 Hz, 1H), 6.87 (d, *J* = 8.2 Hz, 1H), 5.08 (s, 2H), 4.36 (d, *J* = 8.0 Hz, 2H), 3.50 (dd, *J* = 11.2, 5.7 Hz, 1H), 3.38 - 3.32 (m, 2H), 3.26 (dt, *J* = 10.9, 7.3 Hz, 1H), 3.15 - 3.10 (m, 4H), 3.07 (td, *J* = 8.8, 4.4 Hz, 1H), 2.80 (q, *J* = 7.5 Hz, 2H), 2.33 - 2.30 (m, 1H), 2.29 (s, 3H), 2.26 - 2.22 (m, 1H), 2.20 (s, 3H), 1.94 - 1.88 (m, 4H), 1.24 (t, *J* = 7.5 Hz, 3H). ¹³C NMR (151 MHz, CD₃OD) δ 178.01, 153.87, 149.09, 143.66, 137.93, 131.74, 130.47, 129.79, 128.56, 126.61, 126.49, 123.93, 115.53, 75.99, 56.52, 54.41, 53.55, 50.82, 43.67, 27.75, 25.24, 24.30, 19.25, 14.57, 11.40. MS (ESI) (m/z): [M+H]⁺ 464.30.

### Structure of (S, E)-1-(2-ethyl-4-(1-(((3-methyl-4-(piperidin-1-yl)benzyl)oxy)imino)ethyl)benzyl)pyrrolidine-3-carboxylic acid (Compound 49)

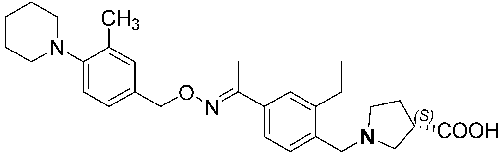

Pale yellow solid, yield 19.4%. ¹H NMR (500 MHz, CD₃OD) δ 7.62 (d, *J* = 1.9 Hz, 1H), 7.55 (dd, *J* = 8.1, 1.9 Hz, 1H), 7.47 (d, *J* = 8.1 Hz, 1H), 7.20 (d, *J* = 2.1 Hz, 1H), 7.17 (dd, *J* = 8.1, 2.1 Hz, 1H), 6.99 (d, *J* = 8.1 Hz, 1H), 5.11 (s, 2H), 4.39 - 4.29 (m, 2H), 3.48 (dd, *J* = 11.1, 5.8 Hz, 1H), 3.37 (s, 1H), 3.35 - 3.31 (m, 1H), 3.29 - 3.22 (m, 1H), 3.12 - 3.04 (m, 1H), 2.86 - 2.77 (m, 6H), 2.32 (dd, *J* = 13.2, 7.4 Hz, 1H), 2.29 (s, 3H), 2.25 (d, *J* = 12.9 Hz, 1H), 2.23 (s, 3H), 1.72 (p, *J* = 5.7 Hz, 4H), 1.59 (q, *J* = 5.8, 5.4 Hz, 2H), 1.25 (t, *J* = 7.5 Hz, 3H). ¹³C NMR (126 MHz, CD₃OD) δ 178.42, 154.03, 152.53, 143.62, 137.78, 132.13, 132.04, 130.84, 130.42, 126.59, 126.44, 123.91, 118.45, 75.89, 56.59, 54.54, 53.60, 53.20, 43.76, 27.78, 26.29, 25.24, 24.08, 16.69, 14.55, 11.42. MS (ESI) (m/z): [M+H]⁺ 478.30.

### Structure of (S, E)-1-(2-ethyl-4-(1-(((3-methyl-4-morpholinylbenzyl)oxy)imino)ethyl)benzyl)pyrrolidine-3-carboxylic acid (Compound 50)

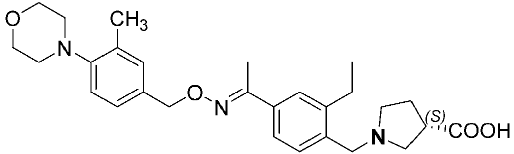

Pale yellow solid, yield 20.7%. ¹H NMR (500 MHz, CD₃OD) δ 7.64 (d, *J* = 1.8 Hz, 1H), 7.57 (dd, *J* = 8.1, 1.9 Hz, 1H), 7.48 (d, *J* = 8.1 Hz, 1H), 7.26 - 7.18 (m, 2H), 7.04 (dt, *J* = 8.1, 1.9 Hz, 1H), 5.13 (s, 2H), 4.43 - 4.33 (m, 2H), 3.88 - 3.77 (m, 4H), 3.55 - 3.47 (m, 1H), 3.37 (dd, *J* = 9.5, 6.3 Hz, 2H), 3.32 - 3.26 (m, 1H), 3.12 (tt, *J* = 8.7, 6.0 Hz, 1H), 2.88 (dq, *J* = 4.8, 2.6 Hz, 4H), 2.82 (q, *J* = 7.6 Hz, 2H), 2.39 - 2.29 (m, 4H), 2.28 - 2.20 (m, 4H), 1.29 - 1.23 (m, 3H). ¹³C NMR (126 MHz, CD₃OD) δ 178.88, 154.10, 151.00, 143.69, 137.88, 132.82, 132.22, 130.99, 130.47, 130.17, 126.64, 126.57, 123.96, 118.46, 75.76, 67.06, 56.48, 54.55, 53.67, 52.11, 43.64, 27.74, 25.25, 16.72, 14.56, 11.41. MS (ESI) (m/z): [M+H]⁺ 480.30.

### Structure of (S, E)-1-(2-ethyl-4-(1-(((2', 3', 4'-trifluoro-[1,1'-biphenyl]-4-yl)methoxy)imino)ethyl)benzyl)pyrrolidine-3-carboxylic acid (Compound 51)

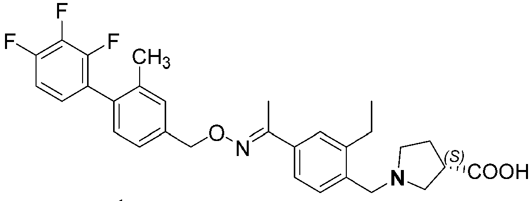

Pale yellow solid, yield 20.1%. ¹H NMR (600 MHz, CD₃OD) δ 7.63 (s, 1H), 7.56 (d, *J* = 8.0 Hz, 1H), 7.48 (d, *J* = 8.2 Hz, 1H), 7.34 (s, 1H), 7.30 (d, *J* = 7.9 Hz, 1H), 7.16 (d, *J* = 7.5 Hz, 2H), 7.03 (q, *J* = 6.3 Hz, 1H), 5.22 (s, 2H), 4.38 (t, *J* = 10.0 Hz, 2H), 3.50 (s, 1H), 3.36 (d, *J* = 8.6 Hz, 2H), 3.28 (d, *J* = 10.5 Hz, 1H), 3.09 (s, 1H), 2.80 (q, *J* = 7.5 Hz, 2H), 2.26 (s, 5H), 2.16 (s, 3H), 1.24 (t, *J* = 7.5 Hz, 3H). ¹³C NMR (151 MHz, CD₃OD) δ 154.43, 143.70, 138.70, 137.73, 136.31, 133.12, 133.11, 130.48, 130.25, 129.75, 129.50, 126.65, 125.36, 125.14, 125.12, 125.06, 123.96, 111.81, 111.76, 111.76, 75.44, 56.57, 56.51, 56.48, 54.74, 54.43, 53.59, 53.01, 43.63, 43.61, 43.56, 43.45, 43.30, 43.11, 27.74, 25.24, 18.57, 18.55, 14.53, 11.42. MS (ESI) (m/z): [M+H]⁺ 525.20.

### The structure of (S, E)-1-(2-ethyl-4-(1-(((2',4',5'-trifluoro-2-methyl-[1,1'-biphenyl]-4-yl)methoxy)imino)ethyl)benzyl)pyrrolidine-3-carboxylic acid (Compound 52)

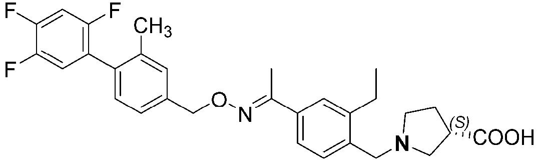

Pale yellow solid, yield 17.9%. ¹H NMR (600 MHz, CD₃OD) δ 7.62 (s, 1H), 7.55 (d, *J* = 8.0 Hz, 1H), 7.51 (d, *J* = 8.3 Hz, 1H), 7.33 (s, 1H), 7.28 (d, *J* = 7.6 Hz, 1H), 7.22 (d, *J* = 6.7 Hz, 1H), 7.14 (d, *J* = 7.3 Hz, 2H), 5.21 (s, 2H), 4.38 (t, *J* = 8.9 Hz, 2H), 3.49 (s, 1H), 3.35 (s, 2H), 3.29 (s, 1H), 3.10 (s, 1H), 2.81 (d, *J* = 7.5 Hz, 2H), 2.30 (d, *J* = 7.4 Hz, 1H), 2.25 (s, 3H), 2.23 (s, 1H), 2.16 (s, 3H), 1.23 (t, *J* = 7.5 Hz, 3H). ¹³C NMR (151 MHz, CD₃OD) δ 155.58, 154.43, 153.96, 150.21, 148.51, 147.31, 145.70, 143.71, 138.63, 137.70, 136.34, 133.18, 130.53, 130.33, 129.70, 129.49, 126.64, 125.34, 123.95, 118.93, 118.76, 105.40, 105.26, 105.20, 105.06, 75.46, 56.40, 54.49, 53.66, 43.71, 27.79, 25.30, 18.59, 18.57, 14.56, 11.43. MS (ESI) (m/z): [M+H]⁺ 525.25.

### Structure of (S, E)-1-(2-ethyl-4-(1-(((4-(6-fluoropyridin-3-yl)-3-methylbenzyl)oxy)imino)ethyl)benzyl)pyrrolidine-3-carboxylic acid (Compound 53)

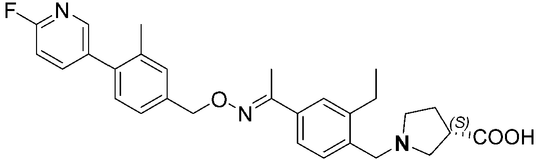

Pale yellow solid, yield 25.7%. ¹H NMR (500 MHz, CD₃OD) δ 8.12 - 8.08 (m, 1H), 7.88 (ddd, *J* = 8.5, 7.8, 2.6 Hz, 1H), 7.62 (d, *J* = 1.9 Hz, 1H), 7.55 (dd, *J* = 8.0, 1.9 Hz, 1H), 7.47 (d, *J* = 8.1 Hz, 1H), 7.35 (d, *J* = 1.7 Hz, 1H), 7.31 (dd, *J* = 7.8, 1.8 Hz, 1H), 7.19 (d, *J* = 7.8 Hz, 1H), 7.11 (ddd, *J* = 8.5, 2.6, 0.7 Hz, 1H), 5.22 (s, 2H), 4.39 - 4.31 (m, 2H), 3.48 (dd, *J* = 11.2, 5.7 Hz, 1H), 3.36 (s, 1H), 3.33 (d, *J* = 2.6 Hz, 1H), 3.29 - 3.21 (m, 1H), 3.08 (tt, *J* = 8.5, 5.8 Hz, 1H), 2.80 (q, *J* = 7.5 Hz, 2H), 2.34 - 2.27 (m, 1H), 2.25 (s, 3H), 2.24 (s, 3H), 2.21 (dd, *J* = 13.5, 6.4 Hz, 1H), 1.23 (t, *J* = 7.6 Hz, 3H). ¹³C NMR (126 MHz, CD₃OD) δ 179.54, 165.00, 163.11, 155.80, 148.23, 148.12, 145.08, 143.92, 143.86, 139.83, 139.04, 137.56, 136.94, 136.90, 136.84, 131.85, 131.43, 130.95, 128.01, 127.07, 125.32, 110.23, 109.93, 76.82, 57.91, 55.84, 54.97, 45.08, 29.14, 26.63, 20.46, 15.94, 12.80. MS (ESI) (m/z): [M+H]⁺ 490.25.

### Structure of (S, E)-1-(2-ethyl-4-(1-(((2,4'-difluoro-[1,1'-biphenyl]-4-yl)methoxy)imino)ethyl)benzyl)pyrrolidine-3-carboxylic acid (Compound 54)

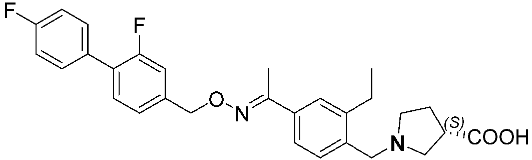

Pale yellow solid, yield 28.1%. ¹H NMR (600 MHz, CD₃OD) δ 7.62 (s, 1H), 7.56 (s, 2H), 7.52 (dd, *J* = 7.3, 5.3 Hz, 2H), 7.42 (t, *J* = 8.0 Hz, 1H), 7.27 (d, *J* = 7.9 Hz, 1H), 7.21 (d, *J* = 11.6 Hz, 1H), 7.15 (t, *J* = 8.8 Hz, 2H), 5.24 (s, 2H), 4.46 - 4.38 (m, 2H), 3.54 (dd, *J* = 11.3, 6.2 Hz, 1H), 3.44 - 3.37 (m, 2H), 3.36 (s, 1H), 3.16 - 3.10 (m, 1H), 2.82 (q, *J* = 7.5 Hz, 2H), 2.34 (dd, *J* = 14.3, 7.3 Hz, 1H), 2.28 (s, 3H), 2.27 - 2.23 (m, 1H), 1.23 (t, *J* = 7.5 Hz, 3H).¹³C NMR (151 MHz, CD₃OD) δ 179.36, 163.26, 161.63, 160.25, 158.62, 154.74, 143.80, 140.37, 137.67, 131.67, 130.67, 130.55, 130.53, 130.49, 130.47, 130.32, 130.29, 130.06 127.05, 126.67, 123.98, 123.75, 123.72, 115.11, 114.95, 114.81, 74.67, 56.19, 54.41, 53.74, 43.59, 27.72, 25.37, 14.56, 11.43. MS (ESI) (m/z): [M+H]⁺ 493.25.

### Structure of (S, E)-1-(2-ethyl-4-(1-((4-(1-methyl-1H-pyrazol-4-yl)benzyl)oxy)imino)ethyl)benzyl)pyrrolidine-3-carboxylic acid (Compound 55)

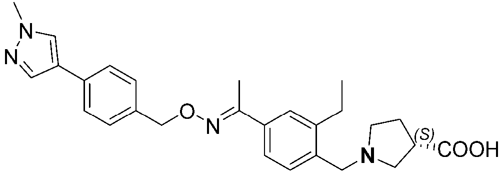

Pale yellow solid, yield 34.3%. ¹H NMR (500 MHz, CD₃OD) δ 7.94 - 7.88 (m, 1H), 7.79 (dd, *J* = 2.7, 1.2 Hz, 1H), 7.62 (q, *J* = 1.8 Hz, 1H), 7.56 (dt, *J* = 8.1, 2.2 Hz, 1H), 7.52 (dt, *J* = 8.3, 2.0 Hz, 2H), 7.45 (d, *J* = 8.1 Hz, 1H), 7.39 (dd, *J* = 8.2, 1.9 Hz, 2H), 5.20 (s, 2H), 4.43 - 4.33 (m, 2H), 3.92 - 3.87 (m, 3H), 3.52 (dd, *J* = 11.3, 5.7 Hz, 1H), 3.37 (dt, *J* = 10.6, 7.1 Hz, 2H), 3.28 (d, *J* = 9.4 Hz, 1H), 3.14 - 3.05 (m, 1H), 2.79 (dtt, *J* = 7.6, 3.9, 2.0 Hz, 2H), 2.31 (dt, *J* = 15.7, 7.4 Hz, 1H), 2.26 - 2.24 (m, 3H), 2.22 (dd, *J* = 13.7, 6.9 Hz, 1H), 1.25 - 1.22 (m, 3H). ¹³C NMR (126 MHz, CD₃OD) δ 177.66, 154.30, 143.73, 137.91, 136.13, 135.97, 132.02, 130.48, 129.94, 128.49, 127.75, 126.69, 124.90, 123.99, 122.83, 75.65, 56.45, 54.38, 53.56, 43.44, 37.53, 27.67, 25.22, 14.54, 11.39. MS (ESI) (m/z): [M+H]⁺ 461.30.

### Structure of (S, E)-1-(2-ethyl-4-(1-((4-(thiophen-3-yl)benzyl)oxy)imino)ethyl)benzyl)pyrrolidine-3-carboxylic acid (Compound 56)

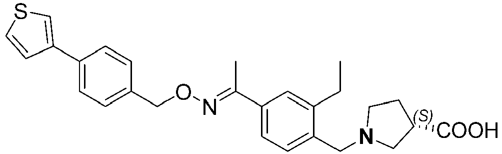

Pale yellow solid, yield 19.7%. ¹H NMR (500 MHz, CD₃OD) δ 7.64 (dd, *J* = 8.5, 2.0 Hz, 3H), 7.61 (dd, *J* = 2.8, 1.5 Hz, 1H), 7.58 (dd, *J* = 8.1, 1.9 Hz, 1H), 7.48 - 7.40 (m, 5H), 5.23 (s, 2H), 4.40 (d, *J* = 2.5 Hz, 2H), 3.53 (dd, *J* = 11.3, 5.8 Hz, 1H), 3.37 (dd, *J* = 12.5, 5.6 Hz, 2H), 3.27 (d, *J* = 10.5 Hz, 1H), 3.10 (ddd, *J* = 11.8, 8.6, 5.7 Hz, 1H), 2.81 (q, *J* = 7.5 Hz, 2H), 2.32 (dq, *J* = 15.8, 7.9 Hz, 1H), 2.27 (s, 3H), 2.22 (dd, *J* = 13.4, 6.7 Hz, 1H), 1.25 (t, *J* = 7.5 Hz, 3H). ¹³C NMR (126 MHz, CD₃OD) δ 154.35, 143.75, 141.83, 137.95, 136.87, 135.39, 130.47, 128.38, 126.72, 125.90, 125.85, 125.71, 124.02, 119.91, 75.59, 56.53, 54.43, 53.55, 43.44, 27.68, 25.21, 14.53, 11.39. MS (ESI) (m/z): [M+H]⁺ 463.20

### Structure of (S, E)-1-(2-ethyl-4-(1-((2-fluoro-4-(thiophen-3-yl)benzyl)oxy)imino)ethyl)benzyl)pyrrolidine-3-carboxylic acid (Compound 57)

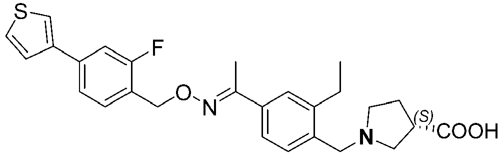

Pale yellow solid, yield 27.7%. ¹H NMR (500 MHz, CD₃OD) δ 7.67 (dd, *J* = 2.9, 1.4 Hz, 1H), 7.63 (d, *J* = 2.0 Hz, 1H), 7.56 (dd, *J* = 8.1, 1.9 Hz, 1H), 7.51 - 7.45 (m, 4H), 7.44 (dd, *J* = 5.1, 1.4 Hz, 1H), 7.40 (dd, *J* = 11.4, 1.6 Hz, 1H), 5.28 (d, *J* = 1.1 Hz, 2H), 4.43 - 4.34 (m, 2H), 3.53 (dd, *J* = 11.3, 5.6 Hz, 1H), 3.43 - 3.34 (m, 2H), 3.28 (dt, *J* = 11.0, 7.4 Hz, 1H), 3.15 - 3.05 (m, 1H), 2.80 (q, *J* = 7.5 Hz, 2H), 2.37 - 2.29 (m, 1H), 2.28 - 2.19 (m, 4H), 1.24 (t, *J* = 7.5 Hz, 3H). ¹³C NMR (126 MHz, CD₃OD) δ 177.72, 162.31, 160.35, 154.57, 143.71, 140.56, 137.91, 137.84, 137.72, 131.06, 130.50, 130.02, 126.66, 126.26, 125.60, 123.97, 123.49, 123.37, 121.53, 121.51, 121.01, 112.54, 112.35, 69.22, 69.19, 56.44, 54.29, 53.52, 43.54, 27.69, 25.22, 14.51, 11.30. MS (ESI) (m/z): [M+H]⁺ 481.20.

### Structure of (S, E)-1-(2-ethyl-4-(1-((2-fluoro-4-(1-methyl-1H-pyrazol-4-yl)benzyl)oxy)imino)ethyl)benzyl)pyrrolidine-3-carboxylic acid (Compound 58)

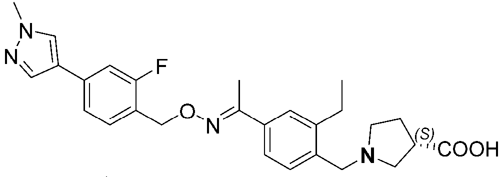

Pale yellow solid, yield 31.6%. ¹H NMR (500 MHz, CD₃OD) δ 8.00 - 7.93 (m, 1H), 7.82 (d, *J* = 0.8 Hz, 1H), 7.63 (d, *J* = 2.0 Hz, 1H), 7.56 (dd, *J* = 8.1, 1.9 Hz, 1H), 7.50 - 7.41 (m, 2H), 7.34 (dd, *J* = 7.8, 1.7 Hz, 1H), 7.30 (dd, *J* = 11.3, 1.7 Hz, 1H), 5.26 (s, 2H), 4.44 - 4.34 (m, 2H), 3.91 (s, 3H), 3.53 (dd, *J* = 11.2, 5.7 Hz, 1H), 3.43 - 3.34 (m, 2H), 3.28 (dt, *J* = 11.0, 7.4 Hz, 1H), 3.10 (tt, *J* = 8.7, 5.7 Hz, 1H), 2.81 (q, *J* = 7.6 Hz, 2H), 2.37 - 2.29 (m, 1H), 2.25 (s, 4H), 1.25 (t, *J* = 7.6 Hz, 3H). ¹³C NMR (126 MHz, CD₃OD) δ 177.73, 162.41, 160.45, 154.51, 143.71, 137.73, 136.16, 134.81, 134.74, 131.22, 131.18, 130.49, 130.06, 128.17, 126.65, 123.95, 122.64, 122.52, 121.79, 121.77, 120.55, 120.52, 111.53, 111.35, 69.24, 69.22, 56.48, 54.31, 53.53, 43.56, 37.61, 27.69, 25.21, 14.50, 11.28 MS (ESI) (m/z): [M+H]⁺ 479.30.

### Structure of (S, E)-1-(2-ethyl-4-(1-(((2'-fluoro-2-methyl-[1,1'-biphenyl]-4-yl)methoxy)imino)ethyl)benzyl)pyrrolidine-3-carboxylic acid (Compound 59)

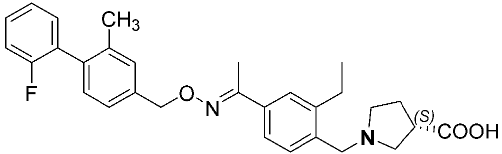

Pale yellow solid, yield 33.5%. ¹H NMR (500 MHz, CD₃OD) δ 7.63 (s, 1H), 7.56 (d, *J* = 8.0 Hz, 1H), 7.49 (d, *J* = 8.1 Hz, 1H), 7.38 (td, *J* = 8.9, 8.2, 3.6 Hz, 1H), 7.32 (s, 1H), 7.28 (d, *J* = 7.7 Hz, 1H), 7.22 (d, *J* = 4.6 Hz, 2H), 7.15 (t, *J* = 8.0 Hz, 2H), 5.22 (s, 2H), 4.38 - 4.31 (m, 2H), 3.48 (dd, *J* = 11.3, 6.1 Hz, 1H), 3.35 (s, 2H), 3.29 - 3.24 (m, 1H), 3.09 (ddd, *J* = 14.7, 8.6, 6.1 Hz, 1H), 2.81 (q, *J* = 7.5 Hz, 2H), 2.31 (dd, *J* = 14.5, 7.0 Hz, 1H), 2.27 (s, 3H), 2.23 (dd, *J* = 13.4, 6.8 Hz, 1H), 2.16 (s, 3H), 1.24 (t, *J* = 7.5 Hz, 3H). ¹³C NMR (126 MHz, CD₃OD) δ 179.07, 160.43, 158.81, 154.38, 143.68, 137.85, 137.75, 136.26, 135.28, 131.31, 131.28, 130.47, 129.67, 129.35, 129.09, 129.04, 128.96, 126.66, 125.19, 123.95, 123.92, 123.89, 115.08, 114.93, 75.63, 56.46, 54.60, 53.70, 43.65, 27.75, 25.28, 18.68, 14.54, 11.44. MS (ESI) (m/z): [M+H]⁺ 489.25.

### Structure of (S, E)-1-(2-ethyl-4-(1-(((4-cyclohexyl-3-methylbenzyl)oxy)imino)ethyl)benzyl)pyrrolidine-3-carboxylic acid (Compound 60)

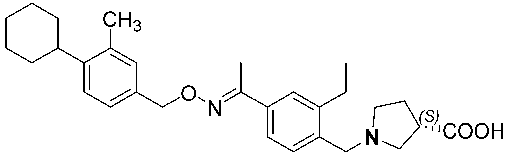

Pale yellow solid, yield 34.5%. ¹H NMR (500 MHz, CD₃OD) δ 7.62 (d, *J* = 2.0 Hz, 1H), 7.55 (dd, *J* = 8.1, 1.9 Hz, 1H), 7.48 (d, *J* = 8.1 Hz, 1H), 7.20 - 7.13 (m, 3H), 5.13 (s, 2H), 4.42 - 4.30 (m, 2H), 3.50 (dd, *J* = 11.2, 5.8 Hz, 1H), 3.36 (dd, *J* = 11.7, 7.9 Hz, 2H), 3.27 (dt, *J* = 10.9, 7.3 Hz, 1H), 3.09 (ddd, *J* = 14.6, 8.9, 5.8 Hz, 1H), 2.81 (q, *J* = 7.5 Hz, 2H), 2.74 (ddd, *J* = 11.5, 9.7, 3.2 Hz, 1H), 2.32 (s, 4H), 2.23 (s, 4H), 1.91 - 1.82 (m, 2H), 1.81 - 1.73 (m, 3H), 1.52 - 1.39 (m, 4H), 1.33 - 1.28 (m, 1H), 1.25 (t, *J* = 7.6 Hz, 3H). ¹³C NMR (126 MHz, CD₃OD) δ 178.14, 154.05, 145.25, 143.65, 137.82, 134.85, 134.63, 130.45, 130.24, 129.88, 126.62, 125.87, 124.97, 123.93, 75.90, 56.52, 54.43, 53.57, 43.69, 39.82, 33.49, 27.76, 26.83, 26.02, 25.24, 18.11, 14.56, 11.42. MS (ESI) (m/z): [M+H]⁺ 477.30.

### Structure of (S, E)-1-(2-ethyl-4-(1-((2-methyl-2', 3', 4' ,5'-tetrahydro-[1,1'-biphenyl]-4-yl)methoxy)imino)ethyl)benzyl)pyrrolidine-3-carboxylic acid (Compound 61)

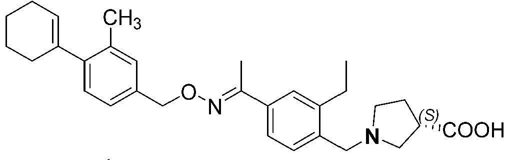

Pale yellow solid, yield 31.2%. ¹H NMR (500 MHz, CD₃OD) δ 7.60 (d, *J* = 2.0 Hz, 1H), 7.52 (dd, *J* = 8.1, 1.9 Hz, 1H), 7.46 (d, *J* = 8.1 Hz, 1H), 7.15 (d, *J* = 1.7 Hz, 1H), 7.11 (dd, *J* = 7.8, 1.8 Hz, 1H), 6.97 (d, *J* = 7.7 Hz, 1H), 5.47 (td, *J* = 3.8, 2.0 Hz, 1H), 5.11 (s, 2H), 4.39 - 4.28 (m, 2H), 3.47 (dd, *J* = 11.1, 5.7 Hz, 1H), 3.37 - 3.31 (m, 2H), 3.24 (dt, *J* = 10.9, 7.3 Hz, 1H), 3.06 (tt, *J* = 8.9, 5.8 Hz, 1H), 2.78 (q, *J* = 7.5 Hz, 2H), 2.33 - 2.21 (m, 5H), 2.20 (s, 3H), 2.16 - 2.10 (m, 4H), 1.73 (tdd, *J* = 8.2, 4.9, 2.6 Hz, 2H), 1.67 (qq, *J* = 5.8, 2.6 Hz, 2H), 1.22 (t, *J* = 7.5 Hz, 3H). ¹³C NMR (126 MHz, CD₃OD) δ 178.11, 154.10, 144.02, 143.65, 138.75, 137.74, 135.99, 134.52, 130.49, 130.29, 129.59, 127.88, 126.60, 125.31, 125.22, 123.93, 75.85, 56.47, 54.35, 53.53, 43.72, 29.82, 27.78, 25.26, 24.97, 22.81, 21.92, 18.61, 14.59, 11.45. MS (ESI) (m/z): [M+H]⁺ 475.30

### Structure of (S, E)-1-(2-ethyl-4-(1-(((4-cyclopentyl-3-methylbenzyl)oxy)imino)ethyl)benzyl)pyrrolidine-3-carboxylic acid (Compound 62)

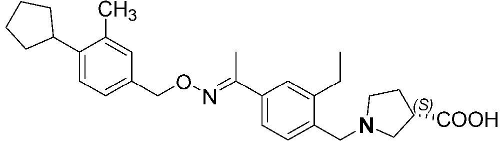

Pale yellow solid, yield 33.5%. ¹H NMR (500 MHz, CD₃OD) δ 7.61 (d, *J* = 1.9 Hz, 1H), 7.54 (dd, *J* = 8.0, 1.9 Hz, 1H), 7.47 (d, *J* = 8.1 Hz, 1H), 7.19 (d, *J* = 7.8 Hz, 1H), 7.16 (d, *J* = 1.9 Hz, 1H), 7.13 (d, *J* = 1.8 Hz, 1H), 5.11 (s, 2H), 4.45 - 4.30 (m, 2H), 3.52 (dd, *J* = 11.3, 5.8 Hz, 1H), 3.41 - 3.32 (m, 2H), 3.28 (dd, *J* = 11.0, 7.4 Hz, 1H), 3.19 (tt, *J* = 9.2, 7.5 Hz, 1H), 3.09 (ddd, *J* = 14.6, 8.8, 5.8 Hz, 1H), 2.79 (q, *J* = 7.6 Hz, 2H), 2.31 (s, 4H), 2.21 (s, 4H), 1.99 (ddddd, *J* = 12.2, 10.9, 6.7, 2.8, 1.4 Hz, 2H), 1.85 - 1.76 (m, 2H), 1.72 - 1.64 (m, 2H), 1.58 - 1.47 (m, 2H), 1.23 (t, *J* = 7.5 Hz, 3H). ¹³C NMR (126 MHz, CD₃OD) δ 178.02, 154.03, 143.86, 143.70, 137.92, 135.45, 134.86, 130.52, 129.89, 129.76, 126.65, 125.83, 124.80, 123.98, 75.90, 56.35, 54.35, 53.58, 43.56, 41.31, 33.26, 27.72, 25.27, 25.10, 18.61, 14.59, 11.43. MS (ESI) (m/z): [M+H]⁺ 463.30

### Structure of (S, E)-1-(2-ethyl-4-(1-((4-(cyclopent-1-en-1-yl)-3-methylbenzyl)oxy)imino)ethyl)benzyl)pyrrolidine-3-carboxylic acid (Compound 63)

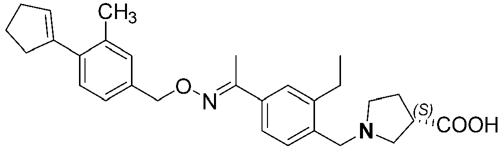

Pale yellow solid, yield 30.4%. ¹H NMR (500 MHz, CD₃OD) δ 7.60 (d, *J* = 1.9 Hz, 1H), 7.53 (dd, *J* = 8.1, 1.9 Hz, 1H), 7.46 (d, *J* = 8.1 Hz, 1H), 7.18 (d, *J* = 1.7 Hz, 1H), 7.16 - 7.10 (m, 2H), 5.73 (p, *J* = 2.2 Hz, 1H), 5.13 (s, 2H), 4.39 - 4.26 (m, 2H), 3.48 (dd, *J* = 11.2, 5.7 Hz, 1H), 3.37 - 3.32 (m, 1H), 3.29 (s, 1H), 3.26 - 3.20 (m, 1H), 3.06 (ddd, *J* = 14.6, 8.8, 5.8 Hz, 1H), 2.78 (q, *J* = 7.5 Hz, 2H), 2.67 - 2.58 (m, 2H), 2.51 (tq, *J* = 7.2, 2.4 Hz, 2H), 2.31 (s, 3H), 2.30 - 2.19 (m, 5H), 1.97 (p, *J* = 7.5 Hz, 2H), 1.22 (t, *J* = 7.5 Hz, 3H). ¹³C NMR (126 MHz, CD₃OD) δ 178.11, 154.18, 143.66, 143.14, 137.75, 137.50, 136.20, 135.06, 130.47, 130.29, 130.01, 128.84, 127.61, 126.61, 125.17, 123.93, 75.74, 56.50, 54.38, 53.54, 43.71, 36.25, 33.04, 27.76, 25.24, 23.30, 20.04, 14.57, 11.43. MS (ESI) (m/z): [M+H]⁺ 461.30.

### Structure of (S, E)-1-(2-ethyl-4-(1-((2,2'-difluoro-[1,1'-biphenyl]-4-yl)methoxy)imino)ethyl)benzyl)pyrrolidine-3-carboxylic acid (Compound 64)

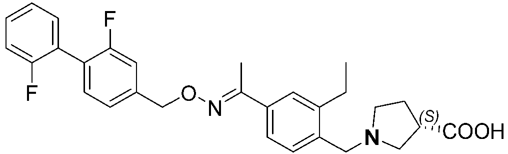

Pale yellow solid, yield 28.9 %. ¹H NMR (500 MHz, CD₃OD) δ 7.63 (d, *J* = 1.9 Hz, 1H), 7.57 (dd, *J* = 8.1, 2.0 Hz, 1H), 7.47 (d, *J* = 8.1 Hz, 1H), 7.44 - 7.40 (m, 1H), 7.39 (d, *J* = 2.5 Hz, 1H), 7.38 (d, *J* = 2.6 Hz, 1H), 7.31 (dd, *J* = 7.9, 1.6 Hz, 1H), 7.28 - 7.25 (m, 1H), 7.24 (d, *J* = 1.7 Hz, 1H), 7.19 (ddd, *J* = 9.6, 8.2, 1.2 Hz, 1H), 5.28 (s, 2H), 4.31 (d, *J* = 3.9 Hz, 2H), 3.44 (dd, *J* = 10.6, 5.7 Hz, 1H), 3.29 (d, *J* = 9.8 Hz, 2H), 3.23 (s, 1H), 3.07 (ddd, *J* = 14.5, 8.6, 6.0 Hz, 1H), 2.81 (q, *J* = 7.5 Hz, 2H), 2.31 (d, *J* = 0.8 Hz, 3H), 2.29 - 2.25 (m, 1H), 2.23 (dd, *J* = 13.6, 7.0 Hz, 1H), 1.27 - 1.23 (m, 3H). ¹³C NMR (126 MHz, CD₃OD) δ 160.79, 160.67, 158.83, 154.90, 143.63, 141.17, 141.11, 137.38, 131.32, 131.22, 130.37, 129.77, 129.71, 126.62, 123.99, 123.96, 123.90, 123.32, 123.30, 123.15, 122.70, 122.57, 115.27, 115.10, 114.64, 114.46, 74.66, 56.68, 54.68, 53.65, 43.80, 27.79, 25.22, 14.50, 11.45. MS (ESI) (m/z): [M+H]⁺ 493.20.

### Structure of (S, E)-1-(2-ethyl-4-(1-((2-fluoro-4-(6-fluoropyridin-3-yl)benzyl)oxy)imino)ethyl)benzyl)pyrrolidine-3-carboxylic acid (Compound 65)

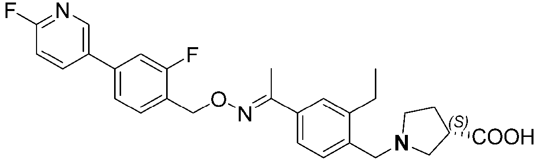

Pale yellow solid, yield 20.3%. ¹H NMR (500 MHz, CD₃OD) δ 8.49 - 8.43 (m, 1H), 8.23 - 8.16 (m, 1H), 7.64 (q, *J* = 1.8 Hz, 1H), 7.61 (dt, *J* = 7.8, 1.8 Hz, 1H), 7.58 (dq, *J* = 9.9, 1.9 Hz, 1H), 7.49 (s, 1H), 7.47 (d, *J* = 4.2 Hz, 1H), 7.44 (ddd, *J* = 10.5, 3.9, 2.0 Hz, 1H), 7.16 (dq, *J* = 8.5, 2.4 Hz, 1H), 5.34 (d, *J* = 2.2 Hz, 2H), 4.43 - 4.34 (m, 2H), 3.52 (dd, *J* = 11.2, 5.8 Hz, 1H), 3.41 - 3.37 (m, 1H), 3.36 - 3.34 (m, 1H), 3.28 (dd, *J* = 10.9, 7.3 Hz, 1H), 3.10 (tt, *J* = 8.9, 5.8 Hz, 1H), 2.85 - 2.78 (m, 2H), 2.37 - 2.29 (m, 1H), 2.27 (q, *J* = 2.6, 2.2 Hz, 3H), 2.23 (dd, *J* = 13.5, 6.7 Hz, 1H), 1.25 (tt, *J* = 7.6, 1.9 Hz, 3H). ¹³C NMR (126 MHz, CD₃OD) δ 162.39, 154.77, 145.28, 145.16, 143.72, 140.36, 140.29, 138.32, 137.59, 131.37, 131.33, 130.46, 130.34, 126.66, 125.13, 125.01, 123.96, 122.37, 113.51, 113.32, 109.52, 109.22, 69.01, 56.52, 54.43, 53.58, 43.59, 27.71, 25.21, 14.50, 11.28. MS (ESI) (m/z): [M+H]⁺ 494.20

### Structure of (S, E)-1-(2-ethyl-4-(1-(((6'-fluoro-[2,3'-bipyridin]-5-yl)methoxy)imino)ethyl)benzyl)pyrrolidine-3-carboxylic acid (Compound 66)

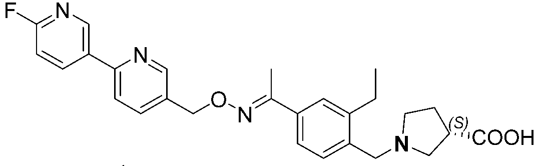

Pale yellow solid, yield 17.2%. ¹H NMR (500 MHz, CD₃OD) δ 8.84 - 8.80 (m, 1H), 8.72 (dd, *J* = 2.3, 0.9 Hz, 1H), 8.54 (ddd, *J* = 8.6, 7.6, 2.6 Hz, 1H), 7.98 (dd, *J* = 8.1, 2.2 Hz, 1H), 7.92 (dd, *J* = 8.1, 0.9 Hz, 1H), 7.64 (d, *J* = 2.0 Hz, 1H), 7.58 (dd, *J* = 8.1, 1.9 Hz, 1H), 7.49 (d, *J* = 8.1 Hz, 1H), 7.19 (ddd, *J* = 8.6, 2.6, 0.6 Hz, 1H), 5.33 (s, 2H), 4.45 - 4.35 (m, 2H), 3.53 (dd, *J* = 11.2, 5.7 Hz, 1H), 3.42 - 3.37 (m, 1H), 3.37 - 3.34 (m, 1H), 3.29 (dd, *J* = 11.0, 7.3 Hz, 1H), 3.11 (ddt, *J* = 11.2, 8.4, 5.9 Hz, 1H), 2.82 (q, *J* = 7.5 Hz, 2H), 2.34 (dt, *J* = 15.1, 7.7 Hz, 1H), 2.29 (s, 3H), 2.28 - 2.21 (m, 1H), 1.25 (t, *J* = 7.5 Hz, 3H). ¹³C NMR (126 MHz, CD₃OD) δ 177.73, 164.91, 162.99, 155.08, 152.89, 149.35, 145.86, 145.74, 143.78, 140.31, 140.25, 137.56, 137.50, 133.65, 132.98, 132.94, 130.50, 130.31, 126.69, 123.99, 120.45, 109.42, 109.12, 72.79, 56.49, 54.38, 53.57, 43.52, 27.69, 25.22, 14.53, 11.38. MS (ESI) (m/z): [M+H]⁺ 477.20.

### Structure of (S, E)-1-(2-ethyl-4-(1-(((4-(5-fluoropyrazin-2-yl)-3-methylbenzyl)oxy)imino)ethyl)benzyl)pyrrolidine-3-carboxylic acid (Compound 67)

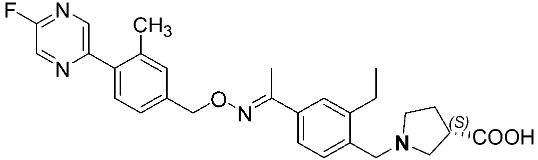

Pale yellow solid, yield 16.3%. ¹H NMR (500 MHz, CD₃OD) δ 8.59 (dd, *J* = 8.3, 1.4 Hz, 1H), 8.38 (d, *J* = 1.6 Hz, 1H), 7.66 (d, *J* = 1.9 Hz, 1H), 7.60 (dd, *J* = 8.1, 1.9 Hz, 1H), 7.51 (d, *J* = 8.1 Hz, 1H), 7.43 (d, *J* = 7.7 Hz, 1H), 7.40 (s, 1H), 7.38 - 7.35 (m, 1H), 5.27 (s, 2H), 4.49 - 4.41 (m, 2H), 3.58 (dd, *J* = 11.4, 6.0 Hz, 1H), 3.45 (d, *J* = 8.2 Hz, 1H), 3.41 (d, *J* = 7.7 Hz, 1H), 3.39 - 3.34 (m, 1H), 3.20 - 3.12 (m, 1H), 2.83 (q, *J* = 7.5 Hz, 2H), 2.38 (s, 3H), 2.35 (dd, *J* = 9.1, 6.9 Hz, 1H), 2.30 (s, 3H), 2.28 - 2.24 (m, 1H), 1.29 - 1.25 (m, 3H). ¹³C NMR (126 MHz, CD₃OD) δ 179.74, 154.46, 152.91, 143.82, 141.30, 141.23, 139.46, 137.93, 136.38, 135.09, 131.95, 131.65, 130.58, 130.32, 129.70, 129.58, 126.73, 125.50, 124.04, 75.35, 56.22, 54.40, 53.64, 43.26, 27.61, 25.28, 19.04, 14.57, 11.40. MS (ESI) (m/z): [M+H]⁺ 491.25.

### Structure of (S, E)-1-(2-ethyl-4-(1-(((4-(6-fluoropyridin-3-yl)benzyl)oxy)imino)ethyl)benzyl)pyrrolidine-3-carboxylic acid (Compound 68)

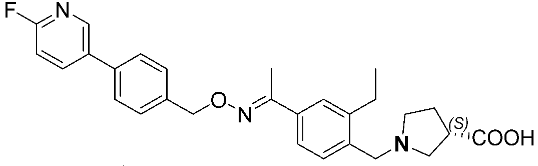

Pale yellow solid, yield 27.6%. ¹H NMR (500 MHz, CD₃OD) δ 8.41 (dt, *J* = 2.7, 0.8 Hz, 1H), 8.16 (ddd, *J* = 8.5, 7.6, 2.6 Hz, 1H), 7.63 (d, *J* = 1.9 Hz, 1H), 7.62 (d, *J* = 1.8 Hz, 1H), 7.61 (d, *J* = 2.0 Hz, 1H), 7.57 (dd, *J* = 8.1, 1.9 Hz, 1H), 7.54 (d, *J* = 1.8 Hz, 1H), 7.52 (d, *J* = 1.8 Hz, 1H), 7.48 (d, *J* = 8.1 Hz, 1H), 7.13 (ddd, *J* = 8.5, 2.6, 0.7 Hz, 1H), 5.28 (s, 2H), 4.43 - 4.34 (m, 2H), 3.52 (dd, *J* = 11.2, 5.7 Hz, 1H), 3.37 (ddd, *J* = 11.0, 8.0, 3.1 Hz, 2H), 3.29 (dt, *J* = 11.0, 7.3 Hz, 1H), 3.10 (tt, *J* = 8.5, 5.7 Hz, 1H), 2.81 (q, *J* = 7.5 Hz, 2H), 2.38 - 2.30 (m, 1H), 2.28 (s, 3H), 2.24 (dt, *J* = 13.6, 6.8 Hz, 1H), 1.24 (t, *J* = 7.5 Hz, 3H). ¹³C NMR (126 MHz, CD₃OD) δ 177.94, 163.99, 162.09, 154.52, 145.03, 144.92, 143.73, 140.27, 140.21, 138.46, 137.72, 135.77, 134.78, 134.74, 130.48, 130.19, 128.57, 126.66, 126.64, 123.97, 109.39, 109.09, 75.30, 56.45, 54.36, 53.56, 43.56, 27.71, 25.24, 14.55, 11.42.MS (ESI) (m/z): [M+H]⁺ 476.20.

### Structure of (S, E)-1-(2-ethyl-4-(1-(((3-fluoro-4-(6-fluoropyridin-3-yl)benzyl)oxy)imino)ethyl)benzyl)pyrrolidine-3-carboxylic acid (Compound 69)

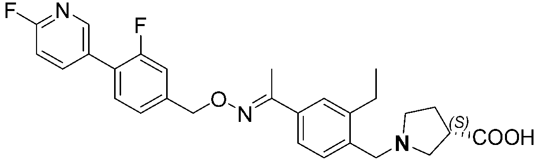

Pale yellow solid, yield 29.2%. ¹H NMR (500 MHz, CD₃OD) δ 8.39 (s, 1H), 8.14 (d, *J* = 8.5 Hz, 1H), 7.64 (d, *J* = 4.0 Hz, 1H), 7.60 - 7.55 (m, 1H), 7.53 (dd, *J* = 8.0, 2.3 Hz, 1H), 7.47 (dd, *J* = 8.2, 2.1 Hz, 1H), 7.38 - 7.33 (m, 1H), 7.32 (d, *J* = 11.2 Hz, 1H), 7.19 (dd, *J* = 8.9, 2.9 Hz, 1H), 5.29 (d, *J* = 4.3 Hz, 2H), 4.41 - 4.30 (m, 2H), 3.48 (dd, *J* = 10.8, 5.4 Hz, 1H), 3.37 (s, 1H), 3.26 (q, *J* = 9.3, 8.4 Hz, 2H), 3.09 (tt, *J* = 8.5, 5.9 Hz, 1H), 2.83 (q, *J* = 5.8, 4.4 Hz, 2H), 2.34 - 2.22 (m, 5H), 1.27 - 1.22 (m, 3H). ¹³C NMR (126 MHz, CD₃OD) δ 178.47, 163.95, 162.05, 160.52, 158.55, 154.93, 146.85, 146.82, 146.74, 146.70, 143.71, 142.17, 142.11, 141.75, 141.69, 137.46, 130.72, 130.44, 130.22, 130.19, 129.71, 126.64, 123.99, 123.97, 123.94, 123.35, 123.24, 115.19, 115.01, 109.24, 108.94, 74.50, 56.56, 54.53, 53.63, 43.69, 27.74, 25.23, 14.52, 11.43.. MS (ESI) (m/z): [M+H]⁺ 494.20

### Structure of (S, E)-1-(2-ethyl-4-(1-(((4-(pyrimidin-5-yl)benzyl)oxy)imino)ethyl)benzyl)pyrrolidine-3-carboxylic acid (Compound 70)

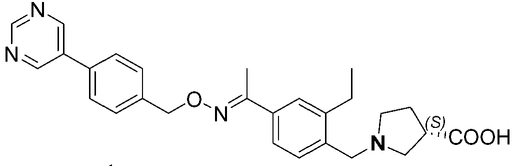

Pale yellow solid, yield 19.8%. ¹H NMR (500 MHz, CD₃OD) δ 9.17 - 9.12 (m, 1H), 9.11 - 9.05 (m, 2H), 7.75 (dd, *J* = 9.5, 3.5 Hz, 2H), 7.65 - 7.55 (m, 4H), 7.47 (d, *J* = 8.1 Hz, 1H), 5.32 (d, *J* = 4.0 Hz, 2H), 4.33 (s, 2H), 3.43 (dd, *J* = 10.8, 6.1 Hz, 1H), 3.31 - 3.20 (m, 3H), 3.08 (q, *J* = 7.5, 7.0 Hz, 1H), 2.88 - 2.74 (m, 2H), 2.31 - 2.29 (m, 3H), 2.28 - 2.19 (m, 2H), 1.25 (ddd, *J* = 9.6, 4.3, 2.7 Hz, 3H). ¹³C NMR (126 MHz, CD₃OD) δ 178.39, 156.47, 154.70, 154.64, 143.64, 139.57, 137.42, 134.33, 133.16, 130.98, 130.38, 128.74, 126.71, 126.58, 123.88, 75.16, 56.68, 54.58, 53.59, 43.81, 27.79, 25.22, 14.53, 11.43. MS (ESI) (m/z): [M+H]⁺ 459.20

### Structure of (S, E)-1-(2-ethyl-4-(1-(((3-fluoro-4-(pyrimidin-5-yl)benzyl)oxy)imino)ethyl)benzyl)pyrrolidine-3-carboxylic acid (Compound 71)

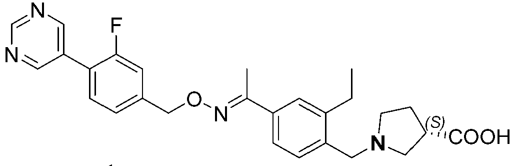

Pale yellow solid, yield 30.0%. ¹H NMR (600 MHz, CD₃OD) δ 9.39 - 9.24 (m, 1H), 9.14 (d, *J* = 8.3 Hz, 2H), 7.84 - 7.46 (m, 6H), 5.45 (d, *J* = 7.7 Hz, 2H), 4.58 - 4.42 (m, 2H), 3.67 - 3.57 (m, 1H), 3.53 (s, 1H), 3.45 (s, 1H), 3.39 (d, *J* = 9.6 Hz, 1H), 3.28 - 3.19 (m, 1H), 2.96 (q, *J* = 7.5 Hz, 2H), 2.49 - 2.44 (m, 2H), 2.42 (d, *J* = 2.3 Hz, 1H), 2.37 (q, *J* = 6.8 Hz, 1H), 1.40 - 1.37 (m, 3H). ¹³C NMR (126 MHz, CD₃OD) δ 178.30, 160.69, 158.72, 156.78, 156.20, 156.16, 155.06, 143.69, 142.91, 142.85, 137.34, 130.42, 130.09, 130.07, 129.97, 129.95, 126.61, 124.22, 124.20, 123.92, 120.82, 120.71, 115.26, 115.07, 74.40, 56.66, 54.53, 53.59, 43.77, 27.77, 25.22, 14.52, 11.43. MS (ESI) (m/z): [M+H]⁺ 477.20

### Structure of (S, E)-1-(2-ethyl-4-(1-(((3-methyl-4-(thiophen-2-yl)benzyl)oxy)imino)ethyl)benzyl)pyrrolidine-3-carboxylic acid (Compound 72)

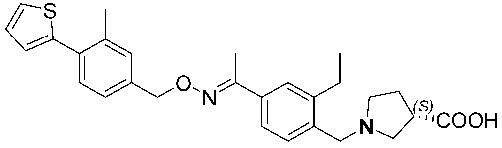

Pale yellow solid, yield 18.3%. ¹H NMR (500 MHz, CD₃OD) δ 7.62 (s, 1H), 7.54 (d, *J* = 7.8 Hz, 1H), 7.47 (d, *J* = 8.0 Hz, 1H), 7.39 (d, *J* = 4.6 Hz, 1H), 7.38 - 7.28 (m, 2H), 7.23 (d, *J* = 7.8 Hz, 1H), 7.14 - 7.01 (m, 2H), 5.19 (s, 2H), 4.34 (d, *J* = 6.8 Hz, 2H), 3.48 (dd, *J* = 11.2, 5.8 Hz, 1H), 3.35 (s, 1H), 3.31 (s, 1H), 3.25 (q, *J* = 10.5, 8.7 Hz, 1H), 3.13 - 3.02 (m, 1H), 2.79 (q, *J* = 7.3 Hz, 2H), 2.37 (s, 3H), 2.24 (q, *J* = 6.5 Hz, 5H), 1.23 (t, *J* = 7.5 Hz, 3H). ¹³C NMR (126 MHz, CD₃OD) δ 178.28, 154.41, 143.66, 142.51, 137.70, 135.65, 133.55, 130.47, 130.21, 129.96, 126.81, 126.62, 126.22, 125.44, 124.87, 123.95, 75.48, 56.50, 54.42, 53.56, 43.76, 27.79, 25.25, 20.01, 14.58, 11.49. MS (ESI) (m/z): [M+H]⁺ 477.20

### Structure of (S, E)-1-(2-ethyl-4-(1-((4-(thiophen-2-yl)benzyl)oxy)imino)ethyl)benzyl)pyrrolidine-3-carboxylic acid (Compound 73)

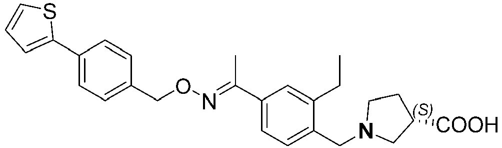

Pale yellow solid, yield 21.2%. ¹H NMR (500 MHz, CD₃OD) δ 7.64 - 7.59 (m, 3H), 7.56 (dd, *J* = 8.1, 2.0 Hz, 1H), 7.47 (d, *J* = 8.1 Hz, 1H), 7.43 - 7.39 (m, 2H), 7.36 (ddd, *J* = 8.2, 4.4, 1.2 Hz, 2H), 7.08 (dd, *J* = 5.1, 3.6 Hz, 1H), 5.22 (s, 2H), 4.40 - 4.30 (m, 2H), 3.49 (dd, *J* = 11.2, 5.8 Hz, 1H), 3.35 (d, *J* = 7.7 Hz, 1H), 3.32 (s, 1H), 3.26 (dt, *J* = 10.9, 7.3 Hz, 1H), 3.12 - 3.04 (m, 1H), 2.80 (q, *J* = 7.5 Hz, 2H), 2.34 - 2.18 (m, 5H), 1.24 (t, *J* = 7.5 Hz, 3H). ¹³C NMR (126 MHz, CD₃OD) δ 178.13, 154.46, 143.72, 143.69, 137.71, 137.42, 133.91, 130.48, 130.27, 128.47, 127.75, 126.64, 125.28, 124.49, 123.95, 122.92, 75.43, 56.49, 54.41, 53.56, 43.70, 27.76, 25.25, 14.55, 11.46. MS (ESI) (m/z): [M+H]⁺ 463.20

### Structure of (S, E)-1-(2-ethyl-4-(1-((4-(isothiazol-4-yl)benzyl)oxy)imino)ethyl)benzyl)pyrrolidine-3-carboxylic acid (Compound 74)

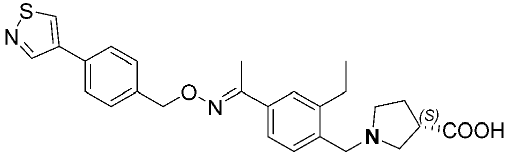

Pale yellow solid, yield 11.6%. ¹H NMR (500 MHz, CD₃OD) δ 9.07 (s, 1H), 8.86 (s, 1H), 7.72 - 7.68 (m, 2H), 7.63 (d, *J* = 1.9 Hz, 1H), 7.57 (dd, *J* = 8.1, 1.9 Hz, 1H), 7.52 (d, *J* = 8.1 Hz, 1H), 7.50 - 7.46 (m, 2H), 5.26 (s, 2H), 4.46 - 4.37 (m, 2H), 3.52 (dd, *J* = 11.3, 6.0 Hz, 1H), 3.44 - 3.39 (m, 1H), 3.39 (d, *J* = 4.0 Hz, 1H), 3.35 (d, *J* = 6.9 Hz, 1H), 3.12 (ddd, *J* = 14.4, 8.7, 5.9 Hz, 1H), 2.82 (q, *J* = 7.5 Hz, 2H), 2.37 - 2.22 (m, 5H), 1.24 (t, *J* = 7.5 Hz, 3H). ¹³C NMR (126 MHz, CD₃OD) δ 178.11, 155.89, 154.49, 143.75, 143.06, 139.51, 138.10, 137.76, 131.87, 130.56, 130.04, 128.57, 126.66, 126.46, 123.98, 75.38, 56.36, 54.37, 53.59, 43.65, 27.77, 25.29, 14.57, 11.44. MS (ESI) (m/z): [M+H]⁺ 464.20

### Structure of (S, E)-1-(2-ethyl-4-(1-((4-(isothiazol-5-yl)benzyl)oxy)imino)ethyl)benzyl)pyrrolidine-3-carboxylic acid (Compound 75)

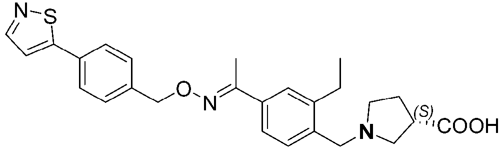

Pale yellow solid, yield 14.9%. ¹H NMR (500 MHz, CD₃OD) δ 8.48 (d, *J* = 1.8 Hz, 1H), 7.72 - 7.66 (m, 2H), 7.63 (d, *J* = 1.9 Hz, 1H), 7.60 (d, *J* = 1.8 Hz, 1H), 7.57 (dd, *J* = 8.0, 1.9 Hz, 1H), 7.52 (s, 2H), 7.51 (s, 1H), 5.27 (s, 2H), 4.46 - 4.36 (m, 2H), 3.52 (dd, *J* = 11.3, 5.9 Hz, 1H), 3.43 - 3.38 (m, 1H), 3.36 (dd, *J* = 12.2, 5.6 Hz, 2H), 3.12 (dq, *J* = 11.3, 4.4, 2.9 Hz, 1H), 2.82 (q, *J* = 7.5 Hz, 2H), 2.38 - 2.23 (m, 5H), 1.24 (t, *J* = 7.5 Hz, 3H). ¹³C NMR (126 MHz, CD₃OD) δ 177.97, 167.11, 158.52, 154.68, 143.76, 140.25, 137.69, 130.55, 130.11, 129.81, 128.63, 126.67, 126.35, 123.99, 119.97, 75.14, 56.38, 54.37, 53.58, 43.64, 27.77, 25.28, 14.56, 11.44. MS (ESI) (m/z): [M+H]⁺ 464.20

### Structure of (S, E)-1-(2-ethyl-4-(1-(((3-methyl-4-(pyrimidin-4-yl)benzyl)oxy)imino)ethyl)benzyl)pyrrolidine-3-carboxylic acid (Compound 76)

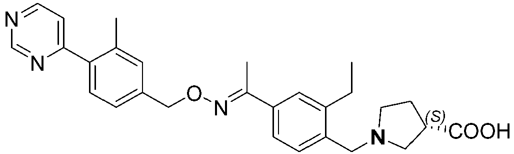

Pale yellow solid, yield 19.9%. ¹H NMR (500 MHz, CD₃OD) δ 9.21 (d, *J* = 1.4 Hz, 1H), 8.83 (d, *J* = 5.3 Hz, 1H), 7.65 (dt, *J* = 3.4, 1.5 Hz, 2H), 7.59 (dd, *J* = 8.1, 1.9 Hz, 1H), 7.49 (dd, *J* = 7.8, 6.3 Hz, 2H), 7.39 (d, *J* = 8.6 Hz, 2H), 5.28 (s, 2H), 4.49 - 4.39 (m, 2H), 3.56 (dd, *J* = 11.3, 5.6 Hz, 1H), 3.45 - 3.34 (m, 2H), 3.30 (t, *J* = 5.6 Hz, 1H), 3.13 (p, *J* = 7.2 Hz, 1H), 2.83 (q, *J* = 7.5 Hz, 2H), 2.43 (s, 3H), 2.41 - 2.32 (m, 1H), 2.30 (s, 3H), 2.29 - 2.23 (m, 1H), 1.26 (t, *J* = 7.5 Hz, 3H). ¹³C NMR (126 MHz, CD₃OD) δ 167.30, 157.63, 156.90, 154.54, 143.78, 140.16, 137.83, 136.66, 136.11, 130.53, 130.43, 129.94, 129.44, 126.71, 125.53, 124.02, 121.62, 75.26, 56.40, 54.34, 53.54, 29.38, 27.65, 25.24, 19.11, 14.57, 11.41. MS (ESI) (m/z): [M+H]⁺ 473.20

### Structure of (S, E)-1-(2-ethyl-4-(1-(((4-(5-fluoropyrimidin-2-yl)-3-methylbenzyl)oxy)imino)ethyl)benzyl)pyrrolidine-3-carboxylic acid (Compound 77)

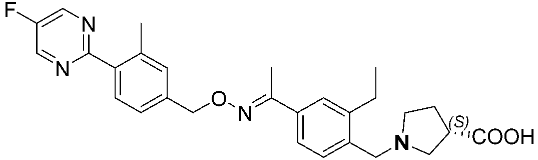

Pale yellow solid, yield 15.4%. ¹H NMR (500 MHz, CD₃OD) δ 8.82 (d, *J* = 1.9 Hz, 2H), 7.76 (d, *J* = 7.7 Hz, 1H), 7.64 (d, *J* = 2.0 Hz, 1H), 7.57 (dd, *J* = 8.1, 2.0 Hz, 1H), 7.48 (d, *J* = 8.1 Hz, 1H), 7.35 (d, *J* = 8.9 Hz, 2H), 5.26 (s, 2H), 4.44 - 4.34 (m, 2H), 3.52 (dd, *J* = 11.3, 5.4 Hz, 1H), 3.41 - 3.33 (m, 2H), 3.32 - 3.25 (m, 1H), 3.10 (s, 1H), 2.82 (q, *J* = 7.6 Hz, 2H), 2.51 (s, 3H), 2.29 (d, *J* = 1.4 Hz, 5H), 1.25 (td, *J* = 7.5, 1.7 Hz, 3H). ¹³C NMR (126 MHz, CD₃OD) δ 163.34, 157.57, 155.48, 154.51, 144.66, 144.50, 143.73, 139.67, 137.77, 137.07, 136.48, 130.50, 130.46, 130.20, 130.14, 126.67, 125.12, 123.98, 75.35, 56.58, 54.40, 53.55, 29.35, 27.76, 25.23, 19.92, 14.55, 11.43. MS (ESI) (m/z): [M+H]⁺ 491.20

### Structure of (S, E)-1-(2-ethyl-4-(1-(((3-methyl-4-(pyrimidin-2-yl)benzyl)oxy)imino)ethyl)benzyl)pyrrolidine-3-carboxylic acid (Compound 78)

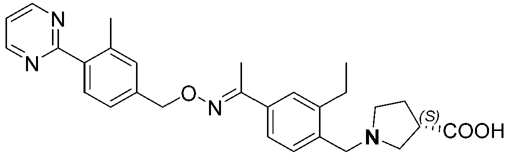

Pale yellow solid, yield 21.4%. ¹H NMR (500 MHz, CD₃OD) δ 8.89 (d, *J* = 4.9 Hz, 2H), 7.69 (d, *J* = 7.9 Hz, 1H), 7.65 (d, *J* = 1.9 Hz, 1H), 7.59 (dd, *J* = 8.0, 1.9 Hz, 1H), 7.52 (d, *J* = 8.1 Hz, 1H), 7.44 (t, *J* = 5.0 Hz, 1H), 7.37 (d, *J* = 7.2 Hz, 2H), 5.27 (s, 2H), 4.47 - 4.39 (m, 2H), 3.56 (dd, *J* = 11.3, 5.9 Hz, 1H), 3.42 (dt, *J* = 11.2, 7.4 Hz, 2H), 3.35 (d, *J* = 7.0 Hz, 1H), 3.15 (td, *J* = 8.6, 4.1 Hz, 1H), 2.83 (q, *J* = 7.5 Hz, 2H), 2.48 (s, 3H), 2.35 (dd, *J* = 14.4, 7.4 Hz, 1H), 2.30 (s, 4H), 1.26 (t, *J* = 7.5 Hz, 3H). ¹³C NMR (126 MHz, CD₃OD) δ 167.16, 157.00, 154.46, 143.78, 139.72, 137.86, 137.42, 136.88, 130.58, 130.37, 129.95, 129.87, 126.70, 125.17, 124.01, 119.06, 75.37, 56.32, 54.38, 53.60, 43.49, 27.71, 25.29, 19.54, 14.57, 11.41. MS (ESI) (m/z): [M+H]⁺ 473.20

### Structure of (S, E)-1-(2-ethyl-4-(1-(((2-fluoro-4-(2-fluoropyrimidin-5-yl)benzyl)oxy)imino)ethyl)benzyl)pyrrolidine-3-carboxylic acid (Compound 79)

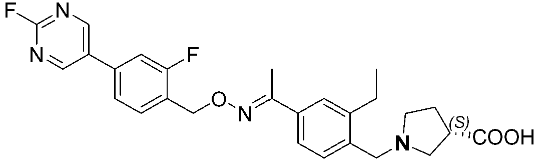

Pale yellow solid, yield 14.3%. ¹H NMR (500 MHz, CD₃OD) δ 8.98 (d, *J* = 1.4 Hz, 2H), 7.67 - 7.62 (m, 2H), 7.57 (dd, *J* = 8.0, 1.9 Hz, 1H), 7.52 (t, *J* = 8.6 Hz, 3H), 5.34 (s, 2H), 4.47 - 4.37 (m, 2H), 3.54 (dd, *J* = 11.4, 5.8 Hz, 1H), 3.43 - 3.36 (m, 2H), 3.35 (s, 1H), 3.18 - 3.09 (m, 1H), 2.83 (q, *J* = 7.5 Hz, 2H), 2.39 - 2.29 (m, 1H), 2.27 (s, 4H), 1.25 (t, *J* = 7.5 Hz, 3H). ¹³C NMR (126 MHz, CD₃OD) δ 163.34, 162.29, 161.60, 160.32, 159.08, 158.99, 154.85, 143.77, 137.60, 131.55, 131.52, 130.54, 130.13, 126.66, 126.05, 125.93, 123.98, 122.45, 113.63, 113.45, 68.93, 56.36, 54.37, 53.60, 43.53, 27.71, 25.25, 14.52, 11.28. MS (ESI) (m/z): [M+H]⁺ 495.20

### Structure of (S, E)-1-(2-ethyl-4-(1-(((4-(2-fluoropyrimidin-5-yl)-3-methylbenzyl)oxy)imino)ethyl)benzyl)pyrrolidine-3-carboxylic acid (Compound 80)

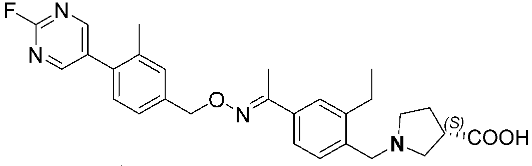

Pale yellow solid, yield 22.2%. ¹H NMR (500 MHz, CD₃OD) δ 8.69 (d, *J* = 1.6 Hz, 2H), 7.64 (d, *J* = 1.9 Hz, 1H), 7.57 (dd, *J* = 8.1, 1.9 Hz, 1H), 7.49 (d, *J* = 8.1 Hz, 1H), 7.41 (s, 1H), 7.38 (dd, *J* = 7.8, 1.8 Hz, 1H), 7.27 (d, *J* = 7.8 Hz, 1H), 5.25 (s, 2H), 4.42 - 4.34 (m, 2H), 3.51 (dd, *J* = 11.1, 5.7 Hz, 1H), 3.35 (t, *J* = 3.5 Hz, 2H), 3.31 - 3.24 (m, 1H), 3.10 (td, *J* = 8.7, 8.0, 4.3 Hz, 1H), 2.82 (q, *J* = 7.5 Hz, 2H), 2.30 (s, 4H), 2.28 (s, 4H), 1.25 (t, *J* = 7.5 Hz, 3H). ¹³C NMR (126 MHz, CD₃OD) δ 177.99, 162.77, 161.05, 160.69, 160.59, 157.18, 154.51, 143.71, 139.35, 137.62, 135.80, 133.62, 133.58, 132.50, 130.47, 130.44, 130.16, 129.72, 126.62, 125.87, 123.94, 75.29, 56.58, 54.39, 53.55, 43.69, 27.75, 25.24, 18.95, 14.56, 11.40. MS (ESI) (m/z): [M+H]⁺ 491.20

### Structure of (E)-1-(2-ethyl-4-(1-(((3-methyl-4-(pyrimidin-5-yl)benzyl)oxy)imino)ethyl)benzyl)azetidine-3-carboxylic acid (Compound 81)

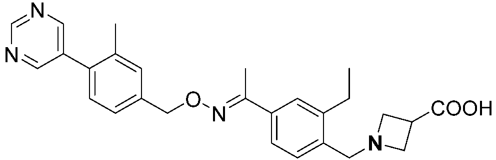

Pale yellow solid, yield 35.6%. ¹H NMR (500 MHz, CD₃OD) δ 9.15 (s, 1H), 8.85 - 8.75 (m, 2H), 7.63 (s, 1H), 7.56 (d, *J* = 8.1 Hz, 1H), 7.45 - 7.35 (m, 3H), 7.26 (t, *J* = 6.5 Hz, 1H), 5.25 (d, *J* = 3.0 Hz, 2H), 4.40 (s, 2H), 4.16 (p, *J* = 9.5 Hz, 4H), 3.41 (p, *J* = 8.3 Hz, 1H), 2.80 (q, *J* = 7.5 Hz, 2H), 2.30 (s, 3H), 2.27 (s, 3H), 1.27 - 1.22 (m, 3H). ¹³C NMR (126 MHz, CD₃OD) δ 176.27, 156.56, 156.36, 154.50, 143.54, 139.32, 137.57, 135.63, 133.39, 130.18, 129.64, 129.54, 126.62, 125.90, 123.97, 75.30, 57.16, 54.94, 34.92, 25.19, 18.97, 14.46, 11.41. MS (ESI) (m/z): [M+H]⁺ 459.20

### Structure of (E)-1-(2-ethyl-4-(1-(((3-methyl-4-(pyrimidin-5-yl)benzyl)oxy)imino)ethyl)benzyl)piperidine-4-carboxylic acid (Compound 82)

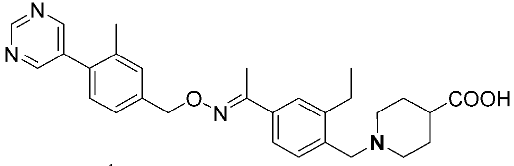

Pale yellow solid, yield 30.3%. ¹H NMR (500 MHz, CD₃OD) δ 9.15 (s, 1H), 8.80 (s, 2H), 7.63 (d, *J* = 1.9 Hz, 1H), 7.56 (dd, *J* = 8.1, 1.9 Hz, 1H), 7.47 (d, *J* = 8.1 Hz, 1H), 7.42 (s, 1H), 7.39 (dd, *J* = 7.8, 1.7 Hz, 1H), 7.27 (d, *J* = 7.8 Hz, 1H), 5.26 (s, 2H), 4.13 (s, 2H), 3.30 - 3.27 (m, 1H), 2.90 - 2.78 (m, 4H), 2.46 - 2.32 (m, 2H), 2.31 (s, 3H), 2.29 (s, 3H), 2.05 (dd, *J* = 14.6, 4.0 Hz, 2H), 1.90 (q, *J* = 12.8, 12.2 Hz, 2H), 1.25 (t, *J* = 7.5 Hz, 3H). ¹³C NMR (126 MHz, CD₃OD) δ179.59 (d, *J* = 2.2 Hz), 156.57, 156.35, 154.63, 144.37, 139.34, 137.46, 135.63, 135.60, 133.38, 131.20, 130.18, 129.63, 126.58, 125.90, 123.73, 75.28, 57.31, 52.14, 40.79, 26.66, 25.39, 18.97, 14.65, 11.45. MS (ESI) (m/z): [M+H]⁺ 487.30

### Structure of (S, E)-1-(2-ethyl-4-(1-(((3-methyl-4-(4-methylpyrimidin-5-yl)benzyl)oxy)imino)ethyl)benzyl)pyrrolidine-3-carboxylic acid (Compound 83)

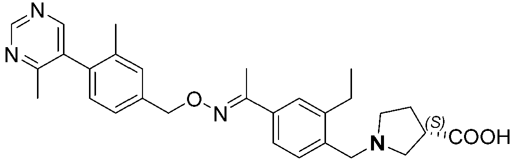

Pale yellow solid, yield 42.5%. ¹H NMR (500 MHz, Methanol-*d*₄) δ 9.03 (s, 1H), 8.48 (s, 1H), 7.66 (d, *J* = 1.9 Hz, 1H), 7.59 (dd, *J* = 8.1, 1.9 Hz, 1H), 7.51 (d, *J* = 8.1 Hz, 1H), 7.43 (d, *J* = 1.7 Hz, 1H), 7.38 (dd, *J* = 7.8, 1.7 Hz, 1H), 7.17 (d, *J* = 7.8 Hz, 1H), 5.28 (s, 2H), 4.48 - 4.36 (m, 2H), 3.53 (d, *J* = 7.7 Hz, 1H), 3.43 - 3.37 (m, 2H), 3.31 (s, 1H), 3.13 (s, 1H), 2.84 (q, *J* = 7.5 Hz, 2H), 2.33 (s, 4H), 2.30 (s, 4H), 2.11 (s, 3H), 1.27 (t, *J* = 7.5 Hz, 3H). ¹³C NMR (126 MHz, Methanol-*d*₄) δ 165.58, 156.48, 156.26, 154.49, 143.76, 139.12, 137.77, 135.87, 134.85, 134.16, 130.53, 130.18, 129.81, 129.17, 126.68, 125.69, 124.00, 75.40, 56.58, 54.41, 53.58, 48.46, 27.77, 25.25, 20.95, 18.56, 14.57, 11.43. MS (ESI) (m/z): [M+H]⁺ 487.30

### Structure of (S, E)-1-(2-ethyl-4-(1-(((3-methyl-4-(pyridazin-4-yl)benzyl)oxy)imino)ethyl)benzyl)pyrrolidine-3-carboxylic acid (Compound 84)

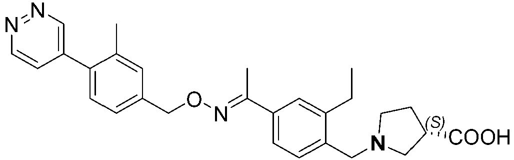

Pale yellow solid, yield 27.3%. ¹H NMR (500 MHz, CD₃OD) δ 9.27 - 9.23 (m, 2H), 7.77 (dd, *J* = 5.3, 2.4 Hz, 1H), 7.65 (d, *J* = 1.9 Hz, 1H), 7.58 (dd, *J* = 8.1, 1.9 Hz, 1H), 7.48 (d, *J* = 8.0 Hz, 1H), 7.45 (s, 1H), 7.42 (dd, *J* = 7.8, 1.7 Hz, 1H), 7.34 (d, *J* = 7.8 Hz, 1H), 5.28 (s, 2H), 4.36 (d, *J* = 2.5 Hz, 2H), 3.47 (dd, *J* = 11.1, 6.0 Hz, 1H), 3.37 - 3.34 (m, 1H), 3.31 (d, *J* = 7.1 Hz, 1H), 3.27 (q, *J* = 9.5, 8.4 Hz, 1H), 3.09 (ddd, *J* = 12.3, 8.4, 5.8 Hz, 1H), 2.83 (q, *J* = 7.5 Hz, 2H), 2.36 (s, 3H), 2.33 - 2.22 (m, 5H), 1.27 (t, *J* = 7.5 Hz, 3H). ¹³C NMR (151 MHz, CD₃OD) δ 178.13, 154.62, 151.80, 150.95, 143.69, 141.24, 139.97, 137.58, 135.47, 134.18, 130.41, 130.34, 129.36, 127.39, 126.63, 125.97, 123.94, 75.20, 56.73, 54.58, 53.60, 43.75, 27.78, 25.22, 18.89, 14.54, 11.39. MS (ESI) (m/z): [M+H]⁺ 473.30

### Structure of (S, E)-1-(2-ethyl-4-(1-(((3-fluoro-4-(thiophen-3-yl)benzyl)oxy)imino)ethyl)benzyl)pyrrolidine-3-carboxylic acid (Compound 85)

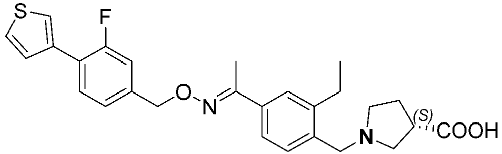

Pale yellow solid, yield 26.5%. ¹H NMR (500 MHz, CD₃OD) δ 7.66 (dt, *J* = 2.8, 1.4 Hz, 1H), 7.64 - 7.59 (m, 2H), 7.55 (dd, *J* = 8.1, 1.9 Hz, 1H), 7.49 - 7.45 (m, 2H), 7.44 (dt, *J* = 5.1, 1.4 Hz, 1H), 7.26 - 7.18 (m, 2H), 5.23 (s, 2H), 4.39 - 4.30 (m, 2H), 3.49 (dd, *J* = 11.1, 5.8 Hz, 1H), 3.35 (d, *J* = 9.5 Hz, 1H), 3.32 - 3.30 (m, 1H), 3.25 (ddd, *J* = 14.6, 7.7, 3.8 Hz, 1H), 3.08 (ddd, *J* = 14.6, 8.7, 5.8 Hz, 1H), 2.79 (q, *J* = 7.6 Hz, 2H), 2.33 - 2.20 (m, 5H), 1.23 (t, *J* = 7.6 Hz, 3H). ¹³C NMR (126 MHz, CD₃OD) δ 178.15, 160.47, 158.51, 154.74, 143.70, 139.64, 139.58, 137.55, 135.32, 135.31, 130.48, 130.44, 129.15, 129.11, 127.08, 127.05, 126.63, 125.21, 123.94, 123.69, 123.67, 123.14, 123.08, 122.77, 122.67, 115.25, 115.06, 74.68, 56.49, 54.38, 53.55, 43.69, 27.75, 25.23, 14.53, 11.45. MS (ESI) (m/z): [M+H]⁺ 481.20

### Structure of (S, E)-1-(2-ethyl-4-(1-(((2,6-difluoro-4-(6-fluoropyridin-3-yl)benzyl)oxy)imino)ethyl)benzyl)pyrrolidine-3-carboxylic acid (Compound 86)

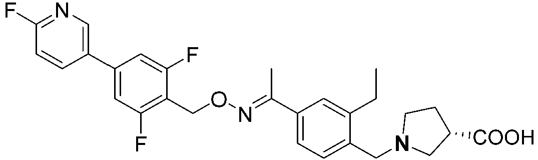

Pale yellow solid, yield 26.9%. ¹H NMR (500 MHz, CD₃OD) δ 8.49 (d, *J* = 2.6 Hz, 1H), 8.22 (ddd, *J* = 8.5, 7.5, 2.7 Hz, 1H), 7.64 (d, *J* = 1.9 Hz, 1H), 7.56 (dd, *J* = 8.1, 1.9 Hz, 1H), 7.47 (d, *J* = 8.1 Hz, 1H), 7.35 (d, *J* = 8.3 Hz, 2H), 7.19 - 7.15 (m, 1H), 5.33 (s, 2H), 4.42 - 4.34 (m, 2H), 3.51 (dd, *J* = 11.2, 5.8 Hz, 1H), 3.39 - 3.34 (m, 2H), 3.32 - 3.25 (m, 1H), 3.10 (ddd, *J* = 14.4, 8.5, 5.8 Hz, 1H), 2.81 (q, *J* = 7.6 Hz, 2H), 2.36 - 2.24 (m, 2H), 2.20 (s, 3H), 1.26 (t, *J* = 7.3 Hz, 3H). ¹³C NMR (126 MHz, CD₃OD) δ 172.97, 165.98, 164.82, 164.08, 162.83, 156.01, 146.90, 146.78, 145.01, 141.80, 141.73, 140.95, 138.87, 131.81, 127.93, 125.25, 114.64, 111.05, 111.03, 110.84, 110.74, 64.06, 57.88, 55.90, 55.04, 45.00, 29.11, 26.57, 20.85, 15.78, 12.33. MS (ESI) (m/z): [M+H]⁺ 512.20

### Structure of (S, E)-1-(2-ethyl-4-(1-(((2-fluoro-4-(5-fluoropyrazin-2-yl)benzyl)oxy)imino)ethyl)benzyl)pyrrolidine-3-carboxylic acid (Compound 87)

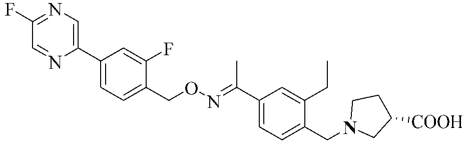

White solid, yield 78.9%. ¹H NMR (500 MHz, Methanol-*d*₄) δ 8.77 (s, 1H), 8.56 (s, 1H), 7.90 - 7.79 (m, 2H), 7.65 - 7.59 (m, 2H), 7.56 (s, 1H), 7.48 (d, *J* = 8.1 Hz, 1H), 5.34 (s, 2H), 4.44 - 4.34 (m, 2H), 3.55 - 3.46 (m, 1H), 3.36 (d, *J* = 7.7 Hz, 1H), 3.28 (dd, *J* = 10.9, 7.3 Hz, 1H), 3.13 - 3.05 (m, 1H), 2.81 (q, *J* = 8.6 Hz, 2H), 2.33 (dt, *J* = 15.2, 7.9 Hz, 1H), 2.28 (s, 3H), 2.23 (dd, *J* = 13.5, 6.8 Hz, 1H), 2.03 (s, 1H), 1.28 - 1.21 (m, 3H). ¹³C NMR (126 MHz, Methanol-*d*₄) δ 154.84, 143.72, 138.45, 138.38, 137.57, 132.58, 132.27, 131.05, 130.46, 130.30, 126.64, 123.95, 121.93, 113.21, 113.02, 69.00, 56.53, 54.37, 53.56, 43.58, 27.71, 25.21, 11.29. MS(m/z): [M+H]⁺ 495.20

### Structure of (S, E)-1-(2-ethyl-4-(1-(((3-fluoro-4-(5-fluoropyrazin-2-yl)benzyl)oxy)imino)ethyl)benzyl)pyrrolidine-3-carboxylic acid (Compound 88)

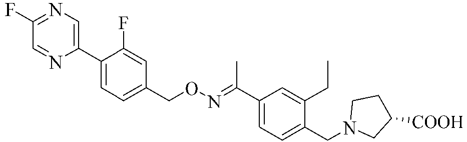

Yellow solid, yield 75.0%. ¹H NMR (500 MHz, Methanol-*d*₄) δ 8.67 (s, 1H), 8.62 (d, *J* = 8.3 Hz, 1H), 7.63 (s, 1H), 7.56 (d, *J* = 7.4 Hz, 1H), 7.49 (d, *J* = 8.1 Hz, 1H), 7.38 (d, *J* = 8.0 Hz, 1H), 7.32 (d, *J* = 12.1 Hz, 1H), 5.30 (s, 2H), 4.37 (d, *J* = 11.1 Hz, 2H), 3.29 (t, *J* = 6.7 Hz, 1H), 3.09 (t, *J* = 7.6 Hz, 1H), 2.82 (q, *J* = 7.5 Hz, 2H), 2.31 (s, 3H), 2.26 - 2.17 (m, 1H), 2.03 (s, 1H), 1.95 (s, 1H), 1.24 (t, *J* = 7.5 Hz, 3H).¹³C NMR (126 MHz, Methanol-*d*₄) δ 155.04, 143.70, 143.17, 141.41, 141.34, 141.24, 137.37, 132.95, 132.65, 130.83, 130.54, 130.52, 130.46, 126.61, 123.93, 123.82, 115.16, 114.98, 74.39, 56.65, 54.53, 53.57, 43.85, 25.24, 14.52, 11.44. MS(m/z): [M+H]⁺ 495.20

### Structure of (S, E)-1-(2-ethyl-4-(1-((4-(5-fluoropyrazin-2-yl)benzyl)oxy)imino)ethyl)benzyl)pyrrolidine-3-carboxylic acid (Compound 89)

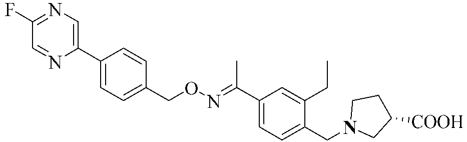

White solid, yield 76.6%. ¹H NMR (500 MHz, Methanol-*d*₄) δ 8.79 - 8.69 (m, 1H), 8.58 - 8.51 (m, 1H), 8.03 (dd, *J* = 16.8, 5.3 Hz, 2H), 7.63 (d, *J* = 7.0 Hz, 1H), 7.58 - 7.54 (m, 3H), 7.47 (d, *J* = 8.1 Hz, 1H), 5.30 (s, 2H), 4.41 - 4.30 (m, 2H), 3.50 (dd, *J* = 10.7, 5.5 Hz, 1H), 3.37 (s, 1H), 3.28 - 3.20 (m, 1H), 3.09 (dd, *J* = 10.2, 4.2 Hz, 1H), 2.85 - 2.78 (m, 2H), 2.36 - 2.31 (m,1H), 2.30 (s, 3H), 2.24 (q, *J* = 6.9, 6.4 Hz, 1H), 1.28 - 1.20 (m, 3H).¹³C NMR (126 MHz, Methanol-*d*₄) δ 154.62, 143.69, 140.08, 138.24, 138.17, 132.34, 132.04, 130.43, 128.28, 126.65, 126.44, 123.94, 75.24, 56.57, 54.45, 53.58, 43.66, 27.73, 25.22, 14.53, 11.42. MS(m/z): [M+H]⁺ 477.20

### Structure of (E)-1-(2-ethyl-4-(1-(((3-methyl-4-(5-fluoropyrazin-2-yl)-benzyl)oxy)imino)ethyl)benzyl)piperidine-4-carboxylic acid (Compound 90)

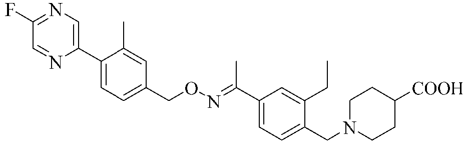

White solid, yield 28.8%. ¹H NMR (500 MHz, Methanol-*d*₄) δ 8.58 (d, *J* = 8.2 Hz, 1H), 8.37 (s, 1H), 7.60 (s, 1H), 7.52 (d, *J* = 10.0 Hz, 1H), 7.41 (dd, *J* = 18.7, 7.9 Hz, 4H), 5.26 (s, 2H), 3.96 (s, 2H), 3.18 (d, *J* = 11.6 Hz, 2H), 2.80 (q, *J* = 7.6 Hz, 2H), 2.66 (d, *J* = 11.5 Hz, 2H), 2.38 (s, 3H), 2.33 (d, *J* = 15.1 Hz, 1H), 2.28 (s, 3H), 2.02 - 1.93 (m, 2H), 1.84 (d, *J* = 12.0 Hz, 2H), 1.24 (t, *J* = 7.6 Hz, 3H). ¹³C NMR (126 MHz, Methanol-*d*₄) δ 158.27, 154.80, 144.11, 141.30, 139.50, 136.91, 136.35, 131.93, 131.63, 130.86, 130.33, 129.57, 126.42, 125.50, 123.52, 75.26, 57.89, 52.46, 27.35, 25.33, 19.05, 14.60, 11.48. MS(m/z): [M+H]⁺ 505.20

### Structure of (E)-1-(2-ethyl-4-(1-(((3-methyl-4-(5-fluoropyrazin-2-yl)-benzyl)oxy)imino)ethyl)benzyl)azetidine-3-carboxylic acid (Compound 91)

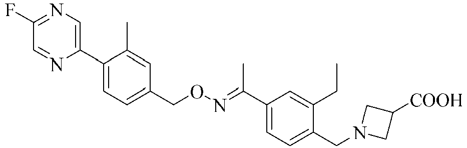

White solid, yield 62.5%. ¹H NMR (500 MHz, Methanol-*d*₄) δ 8.58 (d, *J* = 8.3 Hz, 1H), 8.37 (s, 1H), 7.63 (s, 1H), 7.56 (d, *J* = 8.0 Hz, 1H), 7.40 (dd, *J* = 19.3, 7.9 Hz, 4H), 5.26 (s, 2H), 4.40 (s, 2H), 4.16 (d, *J* = 8.3 Hz, 4H), 2.79 (q, *J* = 7.5 Hz, 3H), 2.37 (s, 7H), 2.28 (s, 3H), 1.25 (t, *J* = 7.5 Hz, 3H). ¹³C NMR (126 MHz, Methanol-*d*₄) δ 176.26, 160.27, 158.28, 154.47, 152.86, 143.53, 141.29, 141.22, 139.45, 137.66, 136.37, 131.94, 131.64, 130.32, 129.57, 129.51, 126.66, 125.50, 123.98, 75.33, 57.18, 54.93, 34.86, 25.16, 19.04, 14.45, 11.39. MS(m/z): [M+H]⁺ 477.20

### Structure of (E)-1-(2-ethyl-4-(1-(((3-methyl-4-(5-fluoropyrazin-2-yl)-benzyl)oxy)imino)ethyl)benzyl)piperidine-3-carboxylic acid (Compound 92)

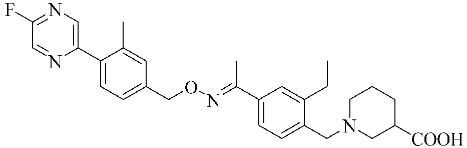

White solid, yield 83.5%. ¹H NMR (500 MHz, Methanol-*d*₄) δ 8.58 (d, *J* = 7.0 Hz, 1H), 8.37 (s, 1H), 7.65 (s, 1H), 7.57 (d, *J* = 8.0 Hz, 1H), 7.48 (d, *J* = 8.1 Hz, 2H), 7.45 - 7.35 (m, 6H), 5.26 (s, 5H), 4.23 (d, *J* = 3.6 Hz, 5H), 3.37 (s, 4H), 3.12 (d, *J* = 10.1 Hz, 5H), 2.82 (dq, *J* = 14.9, 7.3 Hz, 3H), 2.65 (s, 2H), 2.38 (s, 7H), 2.29 (s, 6H), 1.85 (d, *J* = 48.6 Hz, 11H), 1.27 (t, *J* = 7.5 Hz, 6H).¹³C NMR (126 MHz, Methanol-*d*₄) δ 160.27, 158.28, 154.54, 152.92, 144.11, 141.30, 141.22, 139.48, 137.76, 136.37, 135.06, 131.94, 131.64, 130.98, 130.33, 129.58, 126.60, 125.51, 123.93, 75.33, 54.53, 52.79, 48.46, 25.20, 19.04, 14.45, 11.41. MS(m/z): [M+H]⁺ 505.20

### Structure of (S, E)-1-(2-ethyl-4-(1-(((2-fluoro-4-(5-fluoropyrazin-2-yl)-3-methylbenzyl)oxy)imino)ethyl)benzyl)pyrrolidine-3-carboxylic acid (Compound 93)

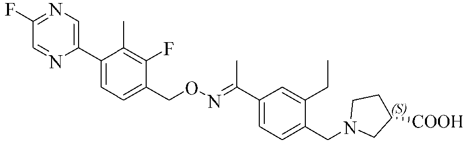

White solid, yield 70.1 %. ¹H NMR (500 MHz, Methanol-*d*₄) δ 8.59 (d, *J* = 8.2 Hz, 1H), 8.37 (s, 1H), 7.63 (s, 1H), 7.56 (d, *J* = 8.0 Hz, 1H), 7.49 (d, *J* = 8.1 Hz, 1H), 7.42 (t, *J* = 7.5 Hz, 1H), 7.25 (d, *J* = 7.9 Hz, 1H), 5.33 (s, 2H), 4.44 - 4.33 (m, 2H), 3.52 (dd, *J* = 11.2, 5.6 Hz, 1H), 3.41 - 3.35 (m, 2H), 3.28 (dt, *J* = 10.8, 7.3 Hz, 1H), 3.10 (dq, *J* = 8.7, 4.3, 2.9 Hz, 1H), 2.81 (q, *J* = 7.5 Hz, 2H), 2.27 (s, 8H), 1.24 (t, *J* = 7.4 Hz, 3H). ¹³C NMR (126 MHz, Methanol-*d*₄) δ 177.94, 160.40, 160.38, 158.43, 158.40, 154.73, 151.66, 143.71, 141.43, 141.35, 137.54, 132.19, 131.88, 130.49, 130.34, 127.45, 127.41, 126.63, 125.98, 125.85, 124.93, 124.90, 123.94, 123.68, 123.54, 69.41, 69.38, 56.46, 54.32, 53.54, 43.62, 27.73, 25.23, 14.53, 11.32, 10.54, 10.49. MS(m/z): [M+H]⁺ 509.19

### Structure of (S, E)-1-(2-ethyl-4-(1-(((2-fluoro-4-(5-fluoropyrazin-2-yl)-5-methylbenzyl)oxy)imino)ethyl)benzyl)pyrrolidine-3-carboxylic acid (Compound 94)

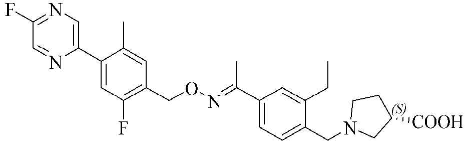

White solid, yield 71.5 %. ¹H NMR (500 MHz, Methanol-*d*₄) δ 8.60 (dd, *J* = 8.2, 1.4 Hz, 1H), 8.41 (t, *J* = 1.5 Hz, 1H), 7.65 (d, *J* = 1.9 Hz, 1H), 7.58 (dd, *J* = 8.1, 1.9 Hz, 1H), 7.48 (d, *J* = 8.1 Hz, 1H), 7.45 (d, *J* = 7.2 Hz, 1H), 7.25 (d, *J* = 10.3 Hz, 1H), 5.31 (s, 2H), 4.43 - 4.35 (m, 2H), 3.52 (dd, *J* = 11.2, 5.8 Hz, 1H), 3.39 - 3.35 (m, 2H), 3.29 (dd, *J* = 12.9, 5.5 Hz, 1H), 3.11 (ddd, *J* = 14.4, 8.7, 5.8 Hz, 1H), 2.82 (q, *J* = 7.5 Hz, 2H), 2.35 (s, 3H), 2.33 - 2.30 (m, 1H), 2.27 (s, 3H), 2.24 (dd, *J* = 13.7, 6.5 Hz, 1H), 1.25 (d, *J* = 7.5 Hz, 3H). ¹³C NMR (126 MHz, Methanol-*d*₄) δ 178.22, 160.41, 160.09, 158.41, 158.14, 154.72, 151.50, 143.70, 141.35, 141.27, 137.60, 136.95, 133.09, 133.05, 132.26, 132.08, 131.96, 130.46, 130.37, 126.65, 125.86, 125.74, 123.96, 116.17, 115.99, 69.06, 56.50, 54.48, 53.62, 43.57, 27.71, 25.21, 18.32, 14.50, 11.27. MS(m/z): [M+H]⁺ 509.20

### Structure of (R, E)-1-(2-ethyl-4-(1-(((4-(5-fluoropyrazin-2-yl)-3-methylbenzyl)oxy)imino)ethyl)benzyl)pyrrolidine-3-carboxylic acid (Compound 95)

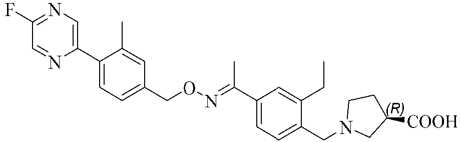

Pale yellow solid, yield 21.4%. ¹H NMR (500 MHz, Methanol-*d*₄) δ 8.57 (dd, *J* = 8.3, 1.3 Hz, 1H), 8.36 (t, *J* = 1.5 Hz, 1H), 7.64 (d, *J* = 1.9 Hz, 1H), 7.57 (dd, *J* = 8.1, 1.9 Hz, 1H), 7.49 (d, *J* = 8.1 Hz, 1H), 7.41 (d, *J* = 7.8 Hz, 1H), 7.39 - 7.34 (m, 2H), 5.26 (s, 2H), 4.44 - 4.34 (m, 2H), 3.52 (dd, *J* = 11.2, 5.7 Hz, 1H), 3.37 (td, *J* = 9.1, 8.2, 3.3 Hz, 2H), 3.28 (dt, *J* = 11.0, 7.4 Hz, 1H), 3.10 (ddd, *J* = 14.5, 8.8, 5.7 Hz, 1H), 2.82 (q, *J* = 7.5 Hz, 2H), 2.37 (s, 3H), 2.32 (td, *J* = 7.9, 3.6 Hz, 1H), 2.28 (s, 3H), 2.27 - 2.22 (m, 1H), 1.25 (t, *J* = 7.5 Hz, 3H). ¹³C NMR (126 MHz, Methanol-*d*₄) δ 177.86, 160.26, 158.26, 154.49, 152.89, 152.85, 143.73, 141.28, 141.21, 139.46, 137.71, 136.37, 135.07, 135.06, 131.94, 131.64, 130.50, 130.33, 130.21, 129.58, 126.65, 125.50, 123.96, 75.34, 56.48, 54.34, 53.54, 43.60, 27.72, 25.24, 19.06, 14.55, 11.42. MS (ESI) (m/z): [M+H]⁺ 491.25.

### Example 2. Bioactivity assay of S1PR4 agonist

### I. Experimental Principle

The Tango assay detects ligand-dependent GPCR activity through the interaction between β-arrestin and GPCRs. The TEV separation technology relies on the complementation of two inactive fragments of TEV protease (NTEV, residues 1-118, and CTEV, residues 119-221) to reconstitute functional protease. The NTEV fragment and the tetracycline transactivator (tTA), an artificial transcription factor, are fused at the C-terminus of the GPCR, while the CTEV fragment is fused to human β-arrestin 2. To enhance the stability of the interaction between GPCR and β-arrestin, a short sequence from the C-terminus of the human arginine vasopressin receptor 2 (V2 tail) is inserted between the GPCR and NTEV. The V2 tail contains multiple GRK phosphorylation sites, which strengthen β-arrestin binding upon phosphorylation. The CTEV fragment carries the stabile point mutation S219P and is truncated at residue 221 to remove the autoinhibitory C-terminal tail. Meanwhile, β-arrestin 2 is truncated to lack the entire C-terminal tail (residues 383-410 deleted). This truncation confers robust stimulus-dependent receptor desensitization, thereby improving detection sensitivity compared with wild-type β-arrestin 2. The UAS reporter construct consists of upstream activating sequence (UAS) elements, a minimal CMV promoter, and a tTA-dependent luciferase reporter gene. Therefore, constructing Tango cells requires the co-transfection of the above three plasmids into host cells. Upon GPCR activation and β-arrestin recruitment, the TEV enzyme at the β-arrestin terminus cleaves tTA from the GPCR C-terminus. The released tTA initiates tTA-dependent luciferase transcription, and compound activity is ultimately determined by measuring luminescence intensity.

### II. Experimental Procedure

1) Tango cell line from was digested from the culture dish respectively. After cell counting, the cells were plated at a density of 10,000 cells per well with 700 µL/well into a white opaque 96-well plate (add 60 µL D'Hanks to the surrounding wells), and incubated overnight (20 h) in a 5% CO₂ incubator at 37°C. The cell density will reach approximately 90% the next day.
2) After 20 h, 30 µL of serially diluted compound solution was added to each well. The control group was supplemented with 30 µL F12K complete medium. Each group had three replicate wells.
3) Samples were collected after 20 h of drug treatment. The cells were taken from the incubator and subjected to luciferase assay using the Bright Glo^{™} Luciferase Assay System: 5 µL of reagent per well was added, gently shaken in darkness at room temperature for 10 min, then detected.
4) The plate was read on a microplate reader, the Relative Light Unit (RLU) was measured, the luminescence values were recorded, the data was processed with GraphPad Prism software, and the EC₅₀ of the compound was calculated.

### III. Test Results:

(All compounds were tested at a final concentration of 45 µM, with 9 serial dilutions at 5-fold intervals. 1 µM BAF-312 was used as the positive control for S1PR1, and 25 µM BAF-312 was used as the positive control for S1PR4.

| No. of compound | Structural formula | Tango-hS1P1-CHO EC₅₀(nM ) | Tango-hS1P4-CHO EC₅₀(nM) | Tango-hS1P4-R-79C-CHO EC₅₀(nM) |
|---|---|---|---|---|
| BAF312 | 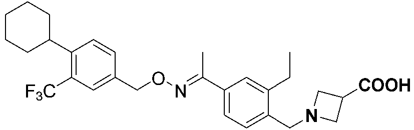 | 1.04+0.1 0 | 155.44+2 1.75 | 157.15+7.9 0 |
| 1 | 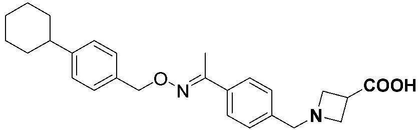 | 698.90+ 157.79 | 2969.00+ 471.63 | 1908.00+79 8.15 |
| 2 | 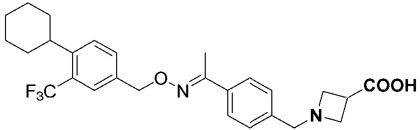 | 4.48+0.2 6 | 324.45+2 4.55 | 236.94+54. 45 |
| 3 | 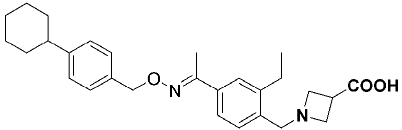 | 578.00+ 102.33 | 2157.00+ 771.50 | 1338.00+27 8.75 |
| 4 | 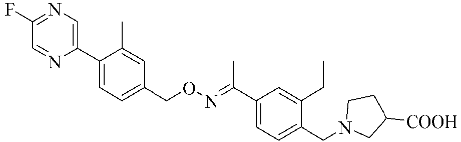 | **162.2±2 3.44** | **10.59±1.1 9** | |
| 5 | 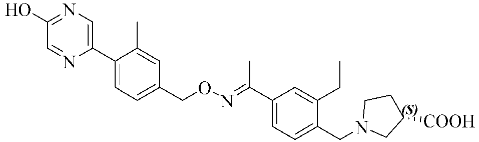 | **133.2±2 3.44** | **4.22±1.19** | |
| 6 | 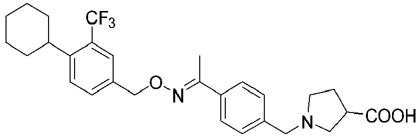 | 21.77±3. 02 | 318.40+8 1.40 | 291.50±95. 50 |
| 7 | 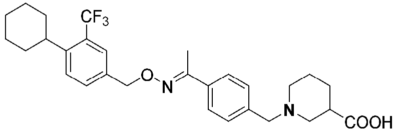 | 508.40+ 86.94 | 2504.00+ 518.88 | 3822.00±13 20.00 |
| 8 | 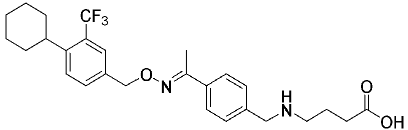 | 51.64±0. 38 | 2399.00+ 157.50 | 728.70+64. 50 |
| 9 | 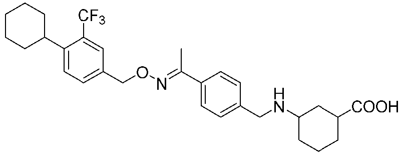 | 455.50+ 36.20 | 2352.00+ 239.04 | 1426.00+37 3.84 |
| 10 | 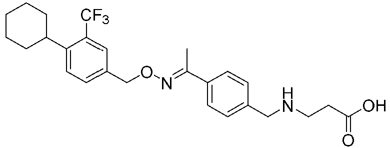 | 10.30+0. 38 | 261.60+3 7.30 | 159.60+4.9 0 |
| 11 | 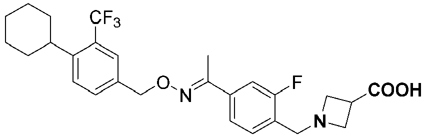 | 5.63+0.1 5 | 573.50+1 06.45 | 254.80+10. 90 |
| 12 | 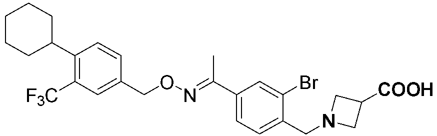 | 17.73±3. 25 | 2251.00+ 701.50 | 1678.00+45 5.00 |
| 13 | 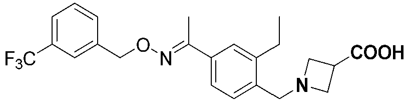 | 5099.00 +1511.4 8 | 3238.00+ 1129.35 | 3111.00+25 8.91 |
| 14 | 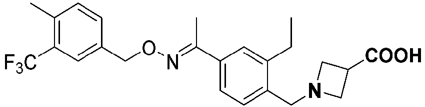 | 5991.00 +830.82 | 3665.00+ 387.61 | 7543.00±90 1.31 |
| 15 | 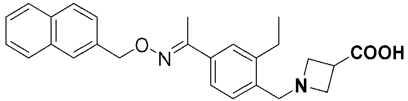 | 806.60+ 241.09 | 560.10+1 84.62 | 560.50+134 .72 |
| 16 | 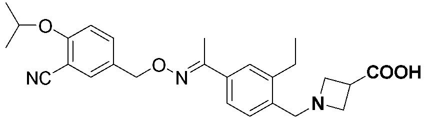 | 23.17+1 0.86 | 3315.00+ 863.14 | 1270.00+45 6.82 |
| 17 | 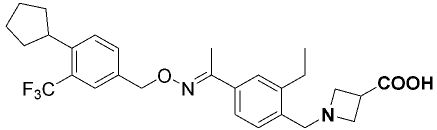 | 2.21±0.5 8 | 389.50+1 95.42 | 95.57+42.0 8 |
| 18 | 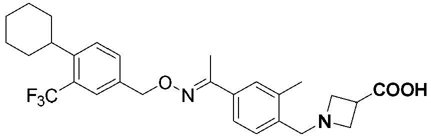 | 2.95+0.6 9 | 266.40±6 2.77 | 63.06+21.7 6 |
| 19 | 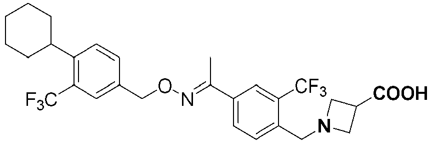 | 8.63+0.3 9 | 563.90+1 84.36 | 121.40+30. 39 |
| 20 | 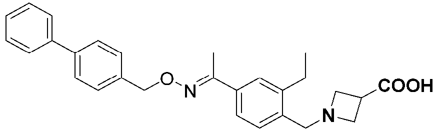 | 781.80± 24.90 | 234.60+5 4.29 | 273.10+54. 21 |
| 21 | 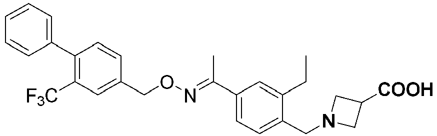 | 11.96+0. 03 | 192.20±6 4.93 | 70.78+27.0 7 |
| 22 | 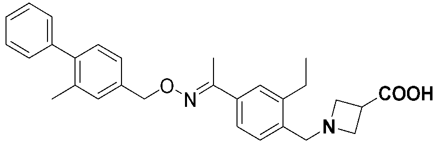 | 55.47±7. 82 | 114.50+83 .50 | 27.03+6.93 |
| 23 | 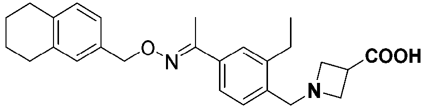 | 560.50+ 24.80 | 569.50+1 57.62 | 585.90+80. 57 |
| 24 | 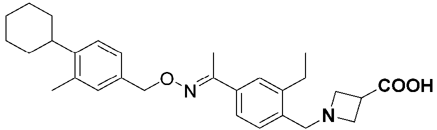 | 11.64+2. 84 | 257.10+5 7.40 | 219.20+80. 40 |
| 25 | 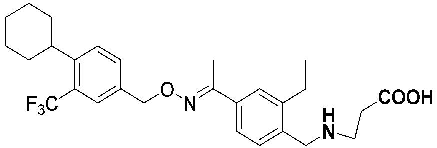 | 1.06+0.2 8 | 269.60±9 9.60 | 157.10+55. 19 |
| 26 | 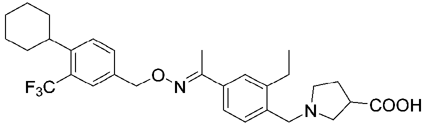 | 0.31±0.0 2 | 146.90+3 0.79 | 57.40+17.4 2 |
| 27 | 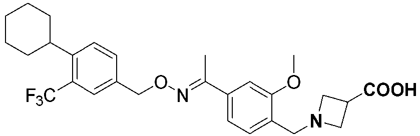 | 55.50±3. 43 | 1697.00+ 588.45 | 1130.00+74 .84 |
| 28 | 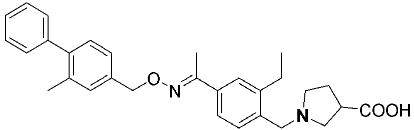 | 99.07+1 6.20 | 23.56+7.4 3 | 57.19±16.7 8 |
| 29 | 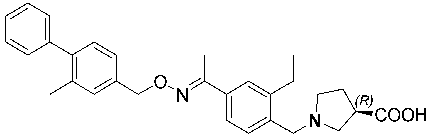 | 169.20+ 32 | 54.41±0.7 2 | 87.17+4.08 |
| 30 | 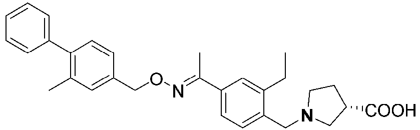 | 87.64+3. 35 | 9.09+0.67 | 14.97+3.78 |
| 31 | 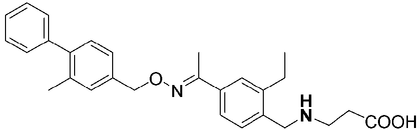 | 128.30+ 10.00 | 77.07+3.0 4 | 73.10+4.91 |
| 32 | 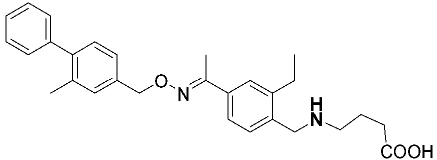 | 88.02±3. 66 | 116.00+2 0.29 | 110.50+6.8 5 |
| 33 | 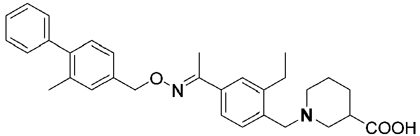 | 454.20+ 84.20 | 127.10±9. 85 | 175.40+36. 50 |
| 34 | 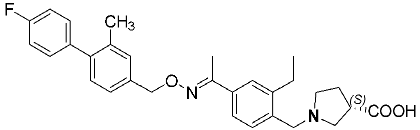 | 86.87 | 2.405 | |
| 35 | 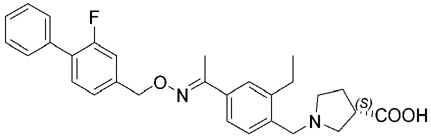 | 103.6 | 3.791 | |
| 36 | 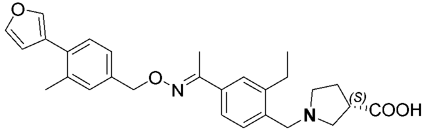 | 103.3 | 1.667 | |
| 37 | 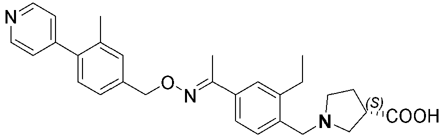 | 643.8 | 15.59 | |
| 38 | 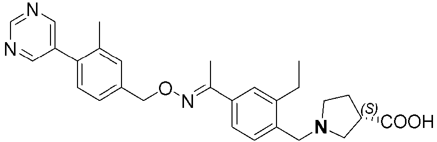 | 2974±21 6.74 | 13.99+2.3 9 | |
| 39 | 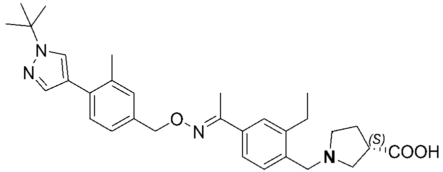 | > 10000 | 311.40±4 9.28 | |
| 40 | 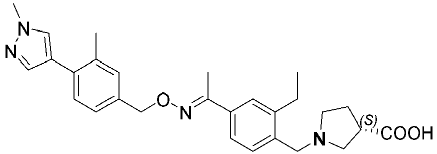 | > 10000 | 73.63+12. 88 | |
| 41 | 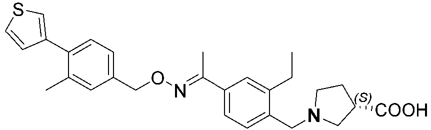 | 51.55+1. 82 | 2.91±0.01 | |
| 42 | 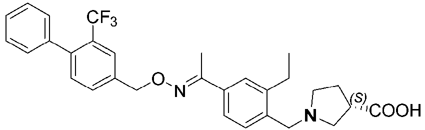 | 3.825±0. 24 | 20.53+0.9 8 | |
| 43 | 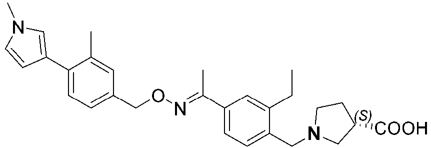 | 2092±22 3.54 | 55.33±8.3 2 | |
| 44 | 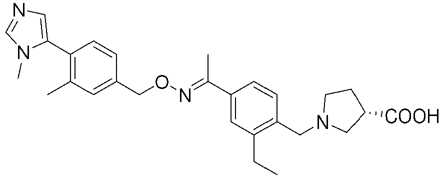 | > 10000 | 878.5±45. 87 | |
| 45 | 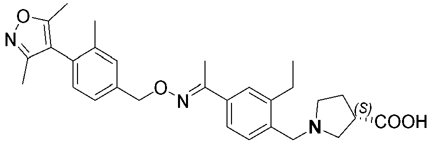 | > 10000 | 407.40+7 4.54 | |
| 46 | 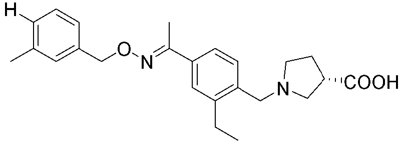 | 5606+42 0.18 | 487.50+1 8.97 | |
| 47 | 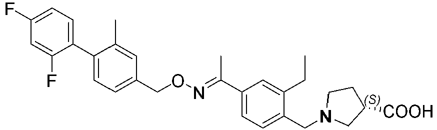 | 131.7±4. 40 | 4.67+0.71 | |
| 48 | 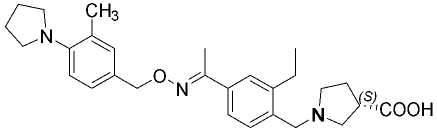 | **276.4±2 6.98** | **210.8±10 0.8** | |
| 49 | 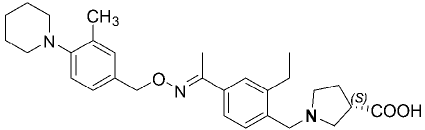 | **66.07±8. 58** | **181.3±10 0.8** | |
| 50 | 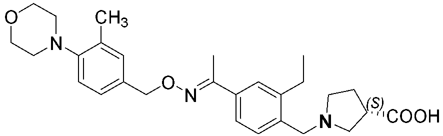 | **3365±39 7.2** | **101.9±97. 13** | |
| 51 | 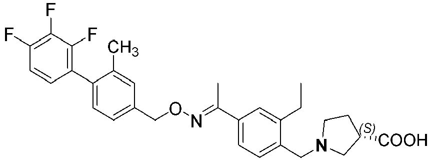 | **195±27.** 65 | **118±46.2 1** | |
| 52 | 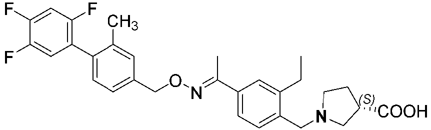 | **218.8±6 6.2** | **26.31±3.2 6** | |
| 53 | 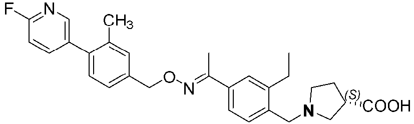 | **238.9±9 7.56** | **7.02±0.59** | HYY20 |
| 54 | 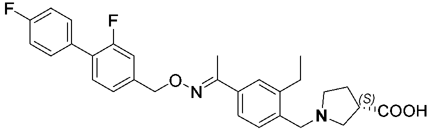 | **765.3±8 1.76** | **13.53±8.5 8** | |
| 55 | 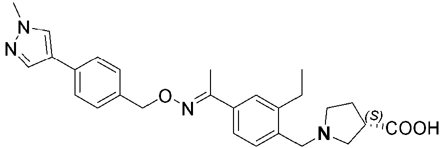 | **>10000** | **268.7** | |
| 56 | 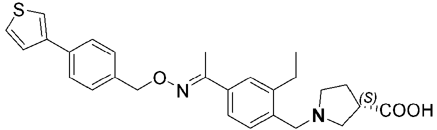 | **686.1±4 9.07** | **5.05±1.84** | |
| 57 | 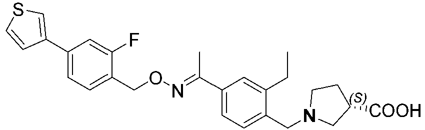 | **664.1±1 19** | **6.52±0.07 3** | |
| 58 | 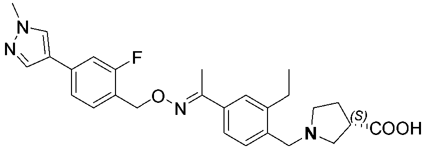 | **510.1±2 50.2** | **>10000** | .1 |
| 59 | 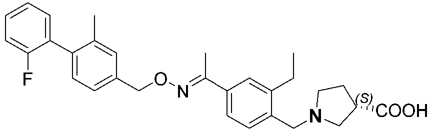 | **86.08±2 0.7** | **44.25±23. 21** | |
| 60 | 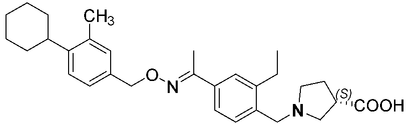 | **3.50±1.6 4** | **71.69** | |
| 61 | 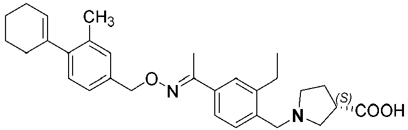 | **4.75±1.8 8** | **3.33±2.5** | |
| 62 | 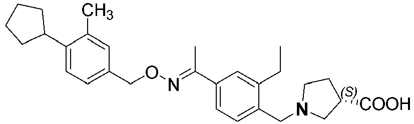 | **1.13±0.3 818** | **3.31±0.24** | |
| 63 | 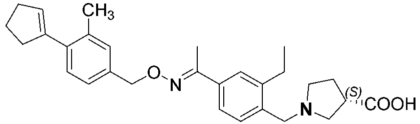 | **21.89±1. 03** | **5.86±0.27** | |
| 64 | 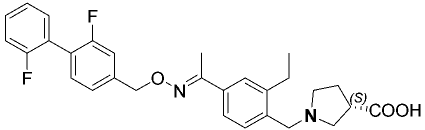 | **239.1±1. 63** | **7.77±1.02** | |
| 65 | 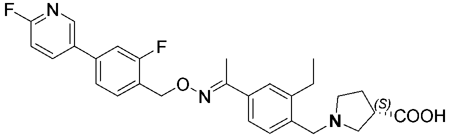 | **>10000** | **10.51±0.2 8** | |
| 66 | 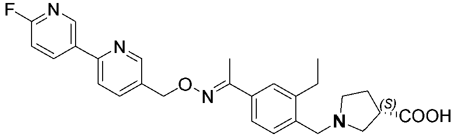 | **>10000** | **421.9±10. 61** | |
| 67 | 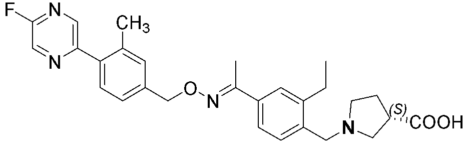 | **194.8±1 6.05** | **1.80±0.43** | |
| 68 | 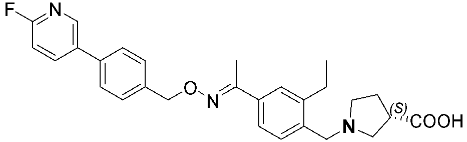 | **7536±94 1.2** | **33.69±12. 04** | |
| 69 | 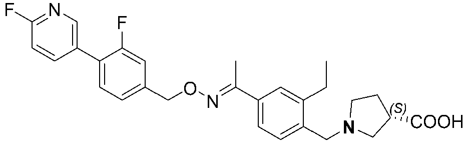 | **4981±52 5.4** | **8.28±0.38** | |
| 70 | 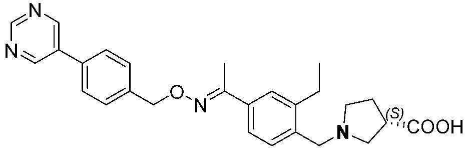 | **>10000** | **36.17±6.5 8** | |
| 71 | 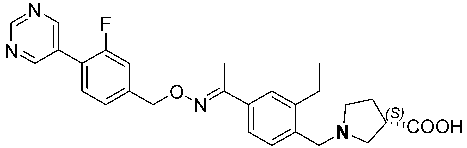 | **>10000** | **34.06±6.0 8** | |
| 72 | 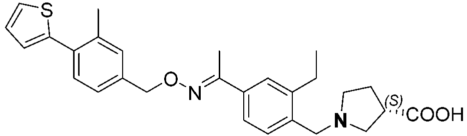 | **112.1±0. 85** | **1.51±0.64** | |
| 73 | 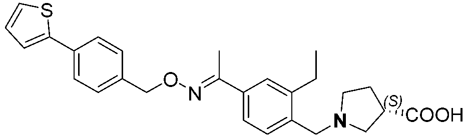 | **1113±55** .86 | **10.68±0.1 1** | |
| 74 | 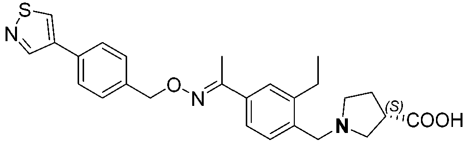 | **> 10000** | **13.13±0.0 2** | |
| 75 | 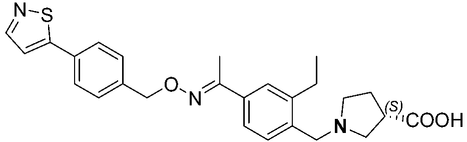 | **> 10000** | **34.17±0.2 2** | |
| 76 | 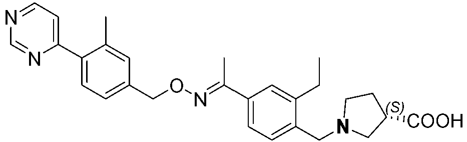 | **549.1±7 7.78** | **4.39±0.91** | |
| 77 | 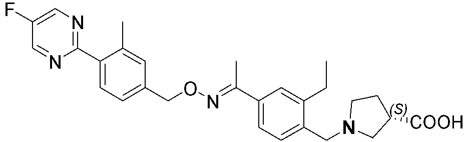 | **270.9±8. 77** | **5.93±4.12** | |
| 78 | 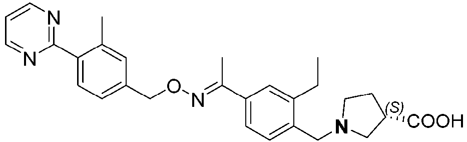 | **1888±12 7.3** | **215.8±64. 91** | |
| 79 | 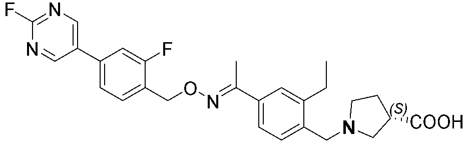 | **>10000** | **444.7±88. 81** | |
| 80 | 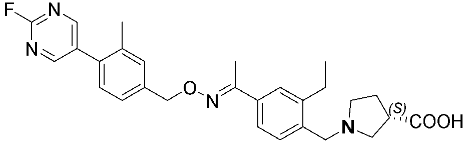 | **2082±40 .31** | **18.05±0.5 2** | |
| 81 | 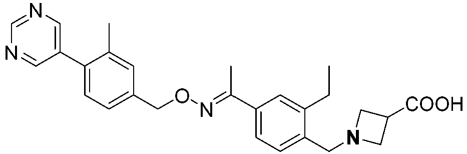 | **2254±23 6.9** | **102.7±9.1** | |
| 82 | 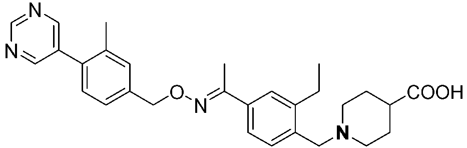 | **> 10000** | **42.7±2.28** | |
| 83 | 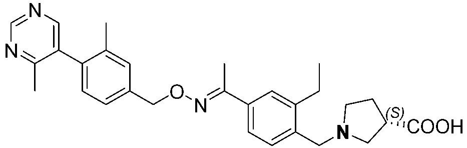 | **> 10000** | **40.95±0.2 2** | |
| 84 | 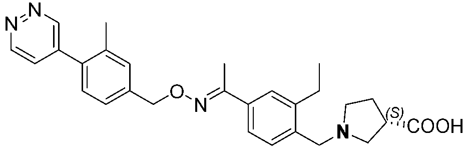 | **> 10000** | **254.1±72. 34** | |
| 85 | 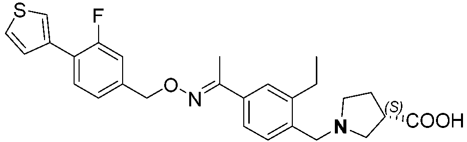 | **173.1±9. 69** | **8.52±0.21** | |
| 86 | 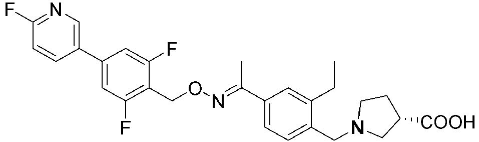 | **> 10000** | **42.22±10. 71** | |
| 87 | 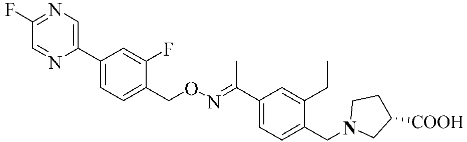 | **> 10000** | **17.4±0.39** | |
| 88 | 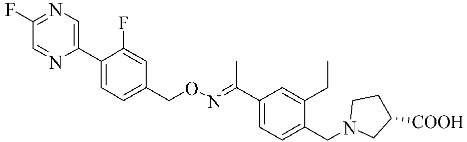 | **3207±11 1.7** | **27.38±1.1 2** | |
| 89 | 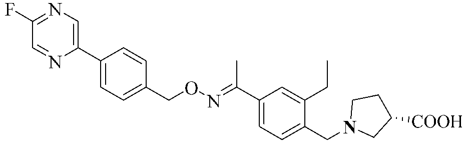 | **5250±23 4.8** | **135.2±17. 75** | |
| 90 | 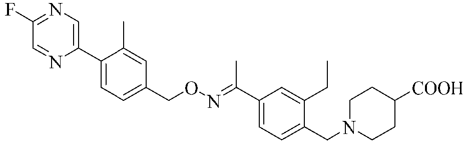 | **5950±72 .83** | **> 10000** | |
| 91 | 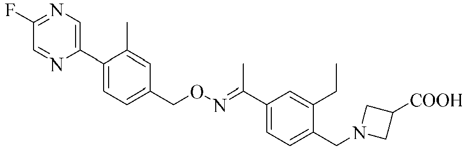 | **54.45±0. 20** | **> 10000** | |
| 92 | 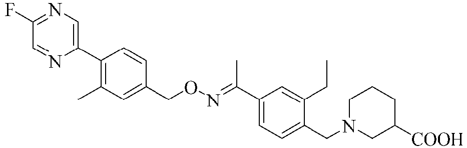 | **> 10000** | **> 10000** | |
| 93 | 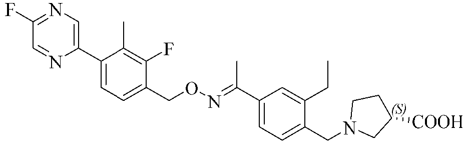 | **347.9±4 0.44** | **12.03±1.0 0** | |
| 94 | 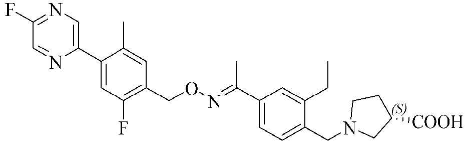 | **613.9±5 0.25** | **13.94±1.4 8** | |
| 95 | 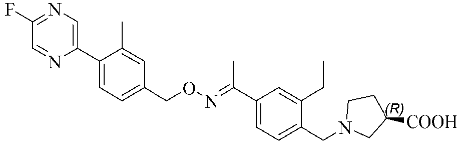 | **125.0±1 4.50** | **75.38±5.7 2** | |

Although some S1PR4 regulators have been reported, most of these compounds have unclear mechanisms, lack detailed structure-activity relationship analysis, and have no *in vivo* pharmacokinetic parameters available, making it impossible to evaluate their *in vivo* efficacy. Starting from BAF312, an S1PR1/5 agonist, as the lead compound, the inventors modified the polar head and hydrophobic moiety of BAF312 based on a β-arrestin recruitment assay system for S1PR1 and S1PR4, synthesized and evaluated a series of compounds. Through comprehensive structure-activity relationship studies on BAF312 at S1PR1/4, the inventors found that replacing the cyclohexyl group of BAF312 with a benzene ring and the trifluoromethyl group with a methyl group to yield Compound 22 significantly reduced S1PR1 activity while retaining S1PR4 activity. Subsequently, the inventors modified the polar head region of Compound 22 and discovered that the introduction of chiral pyrrolidine carboxylic acid further enhanced the compound's activity on S1PR4, leading to the identification of the selective S1PR4 agonist Compound 30. Test results showed that Compound 30 exhibited a β-arrestin recruitment activity on S1PR4 with an EC₅₀ value of 9.09 nM. Further selectivity profiling across S1PR1-5 revealed that, compared with the endogenous ligand S1P, Compound 30 showed no agonist activity on S1PR2/3/5 and approximately 10-fold selectivity over S1PR1, further confirming it as a selective S1PR4 agonist with markedly higher agonistic activity on S1PR4 than S1P. Meanwhile, Compound 67 displayed superior S1PR4 selectivity and activity relative to Compound 30, with an S1PR4 activity of 1.80 nM and more than 100-fold selectivity over S1PR1.

### Selectivity of Compounds 30 and 67 on S1PRs

| Receptor | EC₅₀/nM (Emax/%) | EC₅₀/nM (Emax/%) | EC₅₀/nM |
|---|---|---|---|
| | **30** | **67** | S1P |
| S1PR1 | 87.64±3.35 (101.66)*^{a}* | 194.8± 16.05 nM*^{a}* | 8.52±1.06*^{a}* |
| S1PR2 | >10000 (-6.90)*^{b}* | >10000 nM*^{a}* | 2.95±1.37*^{b}* |
| S1PR3 | >10000 (2.25)*^{c}* | >10000 nM*^{a}* | 20.60±3.37*^{c}* |
| S1PR4 | 9.09±0.67 (103.51)*^{a}* | 1.80±0.43 nM*^{a}* | 100.00±21.85*^{a}* |
| S1PR5 | >10000 (19.51)*^{b}* | 3740±221.3 nM*^{a}* | 2.00±1.90*^{b}* |

| | | | |
|---|---|---|---|
| *^{a}* β-arrestin recruitment activity based on Tango-S1PR1-CHO-K1 and Tango-S1PR4-CHO-K1 cells, expressed as mean EC₅₀ values ± SD. *^{b}* FLIPR assays were performed using S1PR2-CHO and S1PR5-CHO cell lines, expressed as mean EC₅₀ values ± SD. *^{c}* β-arrestin recruitment activity based on PathHunter CHO-K1-S1PR3 cells, expressed as mean EC₅₀ values ± SD. Data are the mean of at least two independent determinations, reported as mean ± SD (standard deviation). | | | |

### Example 3. Pharmacokinetic Property Testing of Compounds

In the structure-activity relationship discussion, the inventors obtained key compound 22. Based on compound 22, the polar head of the compound was modified as chiral pyrrolidine carboxylic acid, yielding selective S1PR4 agonists 30 and 67. We conducted pharmacokinetic property analyses on compounds 22, 30, and 67, respectively. It was found that compound 22 retained a cyclobutanoic acid polar head similar to BAF312, but exhibited dual activity on S1PR1 and S1PR4, with a bioavailability of only 23.5%, resulting in poor druggability. In contrast, the newly discovered compounds 30 and 67 not only showed excellent S1PR4 agonist activity but also outstanding oral absorption properties, with significantly improved *in vivo* exposure. Their oral bioavailabilities reached 119.7% and 76.21%, respectively, demonstrating greater druggable potential.

### Pharmacokinetic parameters of Compound 22 ^{a}

| Parameter | Compound **22** | |
|---|---|---|
| | IV (1 mg/kg) | PO (10 mg/kg) |
| Tmax (h) | 0.08±0 | 0.83±0.29 |
| T_{1/2} (h) | 0.42±0.03 | 2.16±0.96 |
| Cmax (ng/mL) | 995.32±53.20 | 544.44±221.09 |
| AUC₀₋ₜ (h*ng/mL) | 475.87±43.64 | 1118.59±399.59 |
| CL_obs (L/h/kg) | 2.08±0.19 | 8.90±3.86 |
| Vss_obs (L/kg) | 0.88±0.07 | 27.73±16.85 |
| *F* (%) | -- | 23.5 |

| **Pharmacokinetic parameters of Compound 30** *^{a}* | | |
|---|---|---|
| Parameter | Compound **30** | |
| | IV (1 mg/kg) | PO (10 mg/kg) |
| Tmax (h) | 0.08±0 | 2.00±0 |
| T_{1/2} (h) | 2.15±0.06 | 2.78±0.12 |
| Cmax (ng/mL) | 437.70±82.58 | 1031.52±91.90 |
| AUC₀₋ₜ (h*ng/mL) | 823.51±284.02 | 9860.13±794.15 |
| CL_obs (L/h/kg) | 1.21±0.44 | 1.02±0.08 |
| Vss_obs (L/kg) | 3.59±1.16 | 4.08±0.32 |
| *F* (%) | -- | 119.7 |

| | | |
|---|---|---|
| *^{a}* SD rats weighing 200-220 g (male, 3 rats per group) were used for the study. | | |

### Pharmacokinetic parameters of Compound 67 ^{a}

| Parameter | Compound 67 | |
|---|---|---|
| | IV (1 mg/kg) | PO (10 mg/kg) |
| Tmax (h) | 0.08±0.00 | 1.83±1.89 |
| T_{1/2} (h) | 1.18±0.01 | 2.17±0.11 |
| Cmax (ng/mL) | 958.08±45.87 | 986.13±277.10 |
| AUC₀₋ₜ (h*ng/mL) | 836.22±26.98 | 6372.51±1509.83 |
| CL_obs (L/h/kg) | 1.19±0.04 | 1.55±0.27 |
| Vss_obs (L/kg) | 1.52±0.02 | 4.88±1.17 |
| *F* (%) | -- | 76.21 |

| | | |
|---|---|---|
| *^{a}* SD rats weighing 200-220 g (male, 3 rats per group) were used for the study. | | |

### Example 4. Internalization Function Test

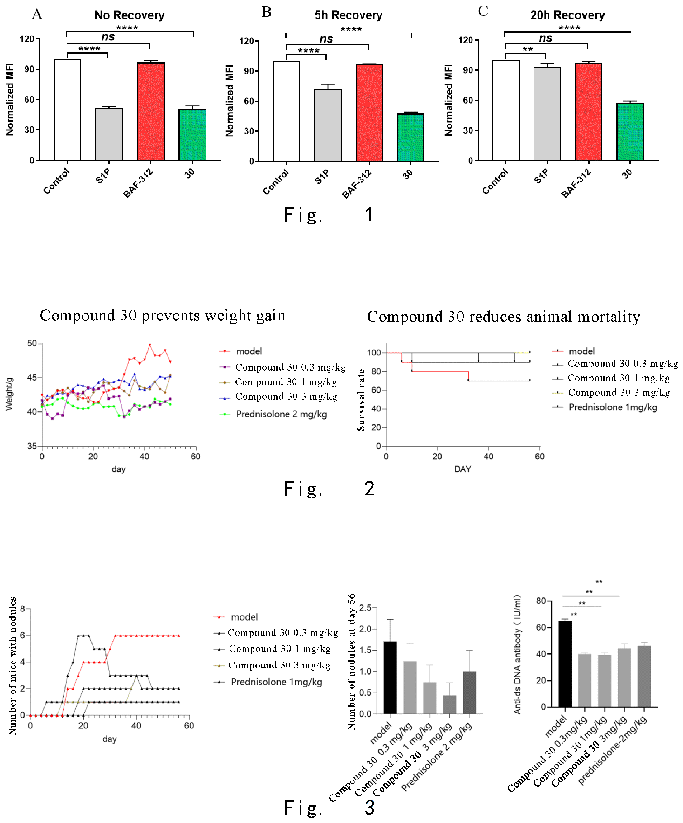

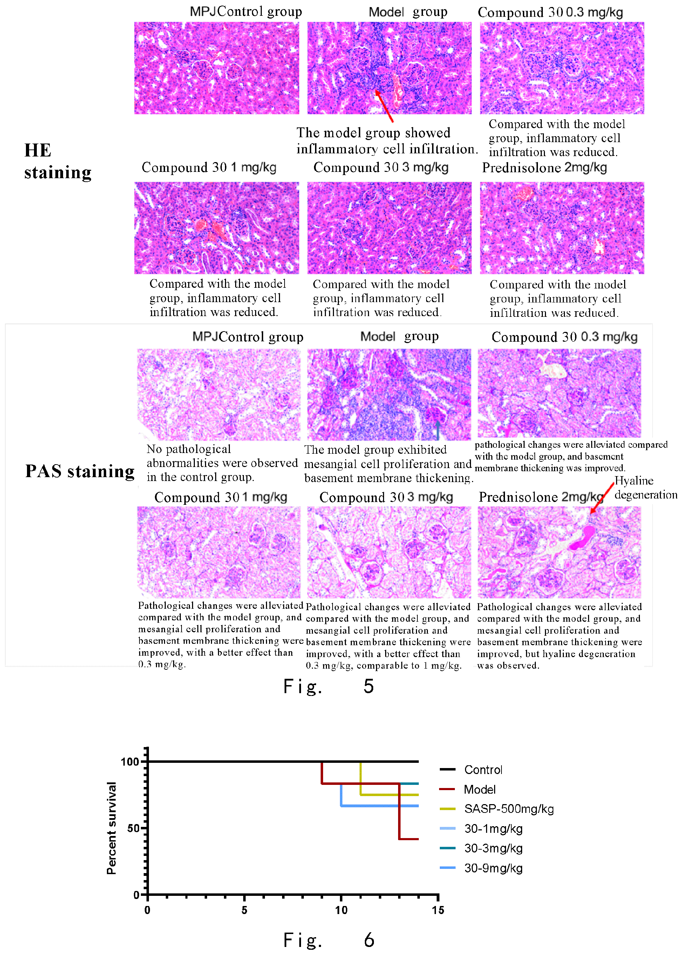

S1P can induce receptor internalization and inhibit the egress of lymphocytes from secondary lymphoid tissues, thereby reducing inflammatory responses in body tissues, which is the key biological effect underlying the therapeutic activity for diseases of S1P-targeted drugs. Therefore, the recovery degree of S1PR4 after internalization caused by Compound 30 was evaluated, and the recovery effect following receptor internalization caused by Compound 30 was assessed in cells with high S1PR4 expression. S1PR4-293T cells were treated with 1 µM S1P, BAF312, and Compound 30 at 37 °C for 1.5 hours, and the expression of S1PR4 receptor on the cell surface was detected immediately, or at 5 hours or 20 hours after treatment. As shown in Figure 5, BAF312, an agonist of S1PR1 and S1PR5, cannot induce S1PR4 internalization due to its lack of agonistic activity on S1PR4. S1P can induce S1PR4 internalization, with receptor expression recovering after 20 hours. The selective S1PR4 agonist Compound 30 exhibits sustained internalization of S1PR4 for up to 20 hours, demonstrating that Compound 30 is a potent and selective S1PR4 agonist. The recovery effect of S1PR4 after internalization induced by Compound 30 is shown in Figure 1.

### Example 5. In Vivo Pharmacodynamic Test of Compound 30 in Systemic Lupus Erythematosus (SLE)

Systemic lupus erythematosus (SLE) is a typical autoimmune disease that affects multiple organs and tissues. Its main features include the production of various specific autoantibodies and abnormal activation of the immune system, often accompanied by coagulation and fibrinolysis abnormalities. It predominantly affects young and middle-aged women, with an incidence rate of nearly 70 per 100,000 people in China. The etiology and pathogenesis of SLE are not yet fully elucidated. Existing studies suggest that its pathogenesis is related to the combined effects of genetic, hormonal, environmental and other comprehensive factors, which induce antigen accumulation, autoantibody production, and excessive immune activation in the body, disrupting immune balance and impairing immune tolerance, thereby leading to organ damage, inflammatory responses, and abnormalities in immune cells. Hyperactivation of T and B lymphocytes is considered a key factor in the pathogenesis of autoimmune diseases. Activated T lymphocytes secrete relevant cytokines that act on B lymphocytes, promoting their proliferation and differentiation to produce autoantibodies, forming immune complexes that deposit in various organs and tissues throughout the body, causing inflammatory responses and dysfunction of the immune system.

The kidneys are the most commonly affected organs in SLE, known as lupus nephritis. Pathological changes in the kidneys include proliferation of capillaries, mesangial cells and matrix, inflammatory cell infiltration, and immune complex deposition, which further lead to renal interstitial fibrosis, glomerular sclerosis, and abnormal renal function.

There is no effective radical cure for SLE. Currently, the clinical treatment of SLE still relies on high-dose glucocorticoids. Although they can control disease progression to a certain extent, a radical cure cannot be achieved. Moreover, a long-term medication tends to induce drug resistance in the body, with severe adverse drug reactions. Common complications seriously affect the quality of life of SLE patients.

Compound 30, an S1PR4 agonist designed and synthesized by the inventor, demonstrates excellent therapeutic effects on a variety of inflammation- and immune-related diseases. Based on this, the inventor conducted efficacy evaluation on this compound using MRL/lpr mice, a classic model of lupus erythematosus. The results showed that after 8 weeks, mice in the SLE model group exhibited a significant increase in body weight (indicating renal function impairment) and mortality. Continuous administration of Compound 30 for 8 weeks prevented body weight gain and reduced mortality in a dose-dependent manner, with an efficacy superior to that of prednisolone (as shown in Figure 2).

Studies on the SLE phenotype showed that Compound 30 significantly reduced subcutaneous nodules in SLE mice and caused the nodules to disappear after a period of treatment. It also markedly decreased serum levels of anti-nuclear antibody dsDNA, with effects superior to prednisolone and a favorable dose-dependent relationship (as shown in Figure 3).

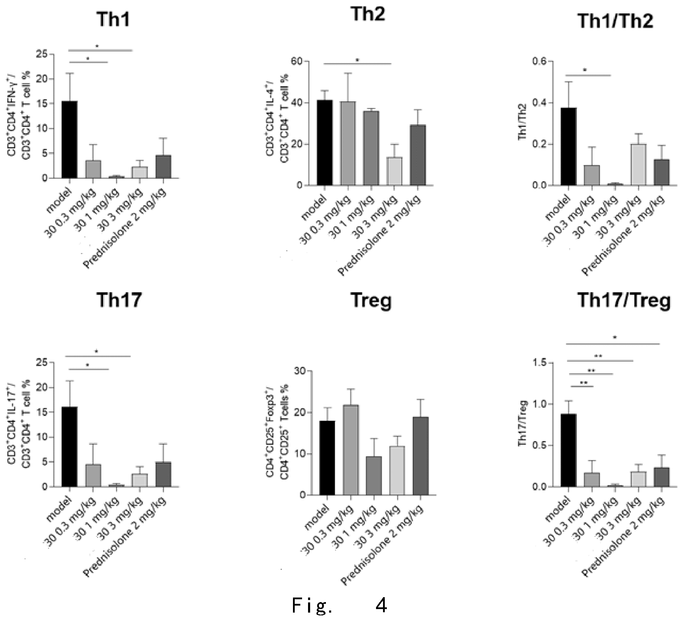

Immunological studies have shown that the numbers of Th1 and Th17 cells in the spleens of SLE mice are significantly higher than those in the normal control group. Compound 30 can significantly reduce the number of Th1- and Th17-positive cells, as well as the Th1/Th2 and Th17/Treg ratios, suggesting that its therapeutic effect is related to the regulation on the number and function of T lymphocytes (as shown in Figure 4).

Given that lupus nephritis is the most frequently affected organ in SLE, the inventors performed HE and PAS staining on the kidneys of mice in each group. The results showed that SLE mice exhibited severe renal interstitial inflammatory cell infiltration, as well as mesangial cell proliferation and basement membrane thickening. After 8 weeks of treatment with Compound 30, renal interstitial inflammatory cell infiltration was significantly reduced, and the pathological changes including mesangial cell proliferation and basement membrane thickening were alleviated, with a better effect than prednisolone (as shown in Figure 5).

In summary, Compound 30 demonstrates excellent efficacy against SLE and is superior to the positive control drug prednisolone. As a selective S1PR4 agonist, Compound 30 has a target and mechanism of action distinct from currently clinically used therapeutic agents. It is an immunomodulator with a novel target and is expected to become a drug for the treatment of lupus erythematosus with a new target and new mechanism.

### Example 6. Pharmacodynamic Evaluation of Compound 30 on Mice with Acute/Chronic Ulcerative Colitis

UC is an inflammatory bowel disease (IBD) that commonly affects adults aged 30 to 40. The disease usually starts in the rectum and extends to the sigmoid colon, with a high tendency to relapse. Its main clinical manifestations include diarrhea, abdominal pain, and mucoid bloody stools. Pathological features are mainly inflammatory cell infiltration and ulcers, which in severe cases can lead to colon cancer.

The etiology and pathogenesis of ulcerative colitis remain unclear. Factors such as persistent intestinal infection, impaired intestinal mucosal barrier, abnormal intestinal mucosal immune regulation, heredity, and environment are jointly involved in the disease process. Immune factors are widely recognized as a crucial component in the pathogenesis of IBD. As an important part of the barrier, intestinal epithelium acts as a key hub between immune defense and immune activation, maintaining mucosal homeostasis through various mechanisms to protect the host. Under normal physiological conditions, molecular communication among epithelial cells, immune cells, and stromal cells sustains epithelial cell regeneration and mucosal healing. Various cell types, including antigen-presenting cells, helper T cells, regulatory T cells, and natural killer T cells, play vital roles in the pathogenesis of UC by regulating, maintaining, or suppressing inflammation.

Commonly used medications for the treatment of UC include 5-aminosalicylic acid, glucocorticoids, and immunosuppressants. Although these drugs can effectively reduce disease activity, they each have their own limitations and potential complications. For example, glucocorticoids have severe side effects and a wide range of contraindications, making them unsuitable for long-term use, while biological agents carry the risk of inducing infection and promoting tumorigenesis. In addition, with the increasing incidence of UC in recent years, some patients have shown poor or no response to conventional drug therapy, leading to protracted and recurrent long-term disease. This has driven efforts to develop new therapeutic approaches and methods, with immunosuppressant and biological agent therapy currently being the focus of attention. By establishing mouse models of acute and chronic ulcerative colitis, the inventors evaluated the therapeutic effect of S1PR4 agonist Compound 30 in this disease. The specific experimental methods and results are presented below.

### 1. Experimental Methods

### 1.1 Acute Ulcerative Colitis

From Day 0 to Day 14 of the experiment, the mice were weighed daily and their blood and stool conditions were observed, scored and recorded according to the following table (Table 1). Random grouping was performed on Day 1, and modeling was conducted from Day 1 to Day 7. All groups except the Control group were given 3.5% DSS water daily. Re-grouping was performed on Day 8. The Control group and Model group received no medication and were given tap water; the other groups were administered intragastrically with 1 mg/kg, 3 mg/kg, 9 mg/kg, and SASP (Sulfasalazine or Salicylazosulfapyridine) 500 mg/kg, respectively. On Day 15, blood and colon samples were collected, colon length was measured, and a 1-2 cm segment of the colon was fixed with formaldehyde.

### 1.2 Chronic Ulcerative Colitis

BALB/c mice were acclimatized for 7 days before the experiment. From Day 7 to Day 50 of the experiment, the mice were weighed daily and their blood and stool conditions were observed, scored and recorded according to the following table (Table 1). After random grouping, all groups except the Control group were given free access to 2-3% DSS solution for modeling from Day 7 to Day 14, while the Control group received normal drinking water during the same period. One cycle lasted 14 days and was repeated 3 times. Re-grouping was performed at the start of the final cycle to initiate drug administration. The Control group and Model group received no drug but the same solvent. The other groups were intragastrically administered 1 mg/kg, 3 mg/kg, 9 mg/kg, and SASP 500 mg/kg respectively. On Day 45, blood and colon samples were collected, colon length was measured, and a 1-2 cm colon segment was fixed with formaldehyde.

### 2. Experimental materials include:

BALB/c mice, male, 20-22 g
Dextran sulfate sodium MW: 36,000-50,000 (DSS)
30 1 mg/kg, 3 mg/kg, 9 mg/kg, SASP (500 mg/kg) indicators were observed.

### 3. Therapeutic Effect

### 3.1 Pharmacodynamic Effect

(1) Clinical Symptom Score: The mice were weighed daily and their blood and stool were observed during the experiment for DAI scoring, which was graded and recorded according to the following table (Table 1).

| DAI score | | | | | |
|---|---|---|---|---|---|
| Rectal bleeding | | Stool consistency | | Weight loss | |
| 0 | normal | 0 | Normal, loose stools | 0 | none |
| 1 | Slight bleeding | 1 | loose stools | 1 | 1%~4.99% |
| 2 | bleeding | 2 | loose watery stools | 2 | 5%~10 |
| 3 | Massive bleeding | 3 | watery diarrhea | 3 | >10% |

(2) After the experiment, the colon was removed, its length measured and weight recorded. The colon-to-body weight index (colon weight/body weight × 100%) and colon swelling rate (colon weight/length × 100%) were calculated.

### 3.2 Pathological Morphological Changes

Colonic tissue approximately 1 cm from the anal margin (0.5 cm) and ileocecal region tissue were collected for histopathological examination. After H&E staining, the tissues were scored under single-blind conditions to evaluate the level of distal colon inflammation and tissue damage; scoring was performed according to the Ameho criteria and calculated as follows:
Score 0: No abnormal findings in the texture.
Score 1: Mild inflammatory infiltration of the mucosa and/or submucosa (mixed with neutrophils), submucosal edema, punctate mucosal erosion, accompanied by capillary proliferation. The muscularis mucosae is intact.
Score 2: Changes of Score 1 account for more than 50%.
Score 3: Obvious inflammatory infiltration and edema (neutrophils are often predominant); ulcers usually penetrate the muscularis mucosae to reach the submucosa. There is little inflammatory cell infiltration in the muscularis propria and no muscle necrosis.
Score 4: Changes of Score 3 account for more than 50%.
Score 5: Large confluent ulcers with coagulative necrosis, massive neutrophil infiltration at the margins and few monocytes. Necrotic tissue penetrates the muscularis propria.
Score 6: Changes of Score 5 account for more than 50% of the specimen.

### 3.3 Changes in Colonic Mucosal Barrier

The fence function of Occludin separates the apical and basolateral sides of the epithelial cell plasma membrane, restricting the free flow of lipids on the cell membrane, facilitating the formation of cell polarity, maintaining the impermeability and rigidity of the intestinal mucosa, and preventing the entry of pathogenic microorganisms. The barrier function of Occludin can regulate the transport of small molecules and maintain tissue homeostasis. The expression and distribution of related proteins are detected by immunohistochemistry.

### 4. Experimental Results

### 4.1 Observation on the Efficacy of Compound 30 in Mice with DSS-induced Acute Ulcerative Colitis

Seven days after model establishment in this experiment, the mice were re-divided into groups with 8 mice per group. Beginning on Day 8, some mice died successively. Among them, no death occurred in the Control group; 2 mice died in the model group, with a survival rate of 75%; 1 mouse died in the positive drug SASP group, with a survival rate of 87.5%; 1 mouse died in the 30 1 mg/kg group, with a survival rate of 87.5%; 1 mouse died in the 30 3 mg/kg group, with a survival rate of 87.5%; 2 mice died in the 30 9 mg/kg group, with a survival rate of 75%. Compared with the model group, both the positive drug SASP and Compound 30 administration improved the survival rate of UC model mice. The survival curve of acute UC model mice administered with Compound 30 is shown in Figure 6.

### 4.2 Effect of Compound 30 on Body Weight and DAI Score in Acute UC Model Mice

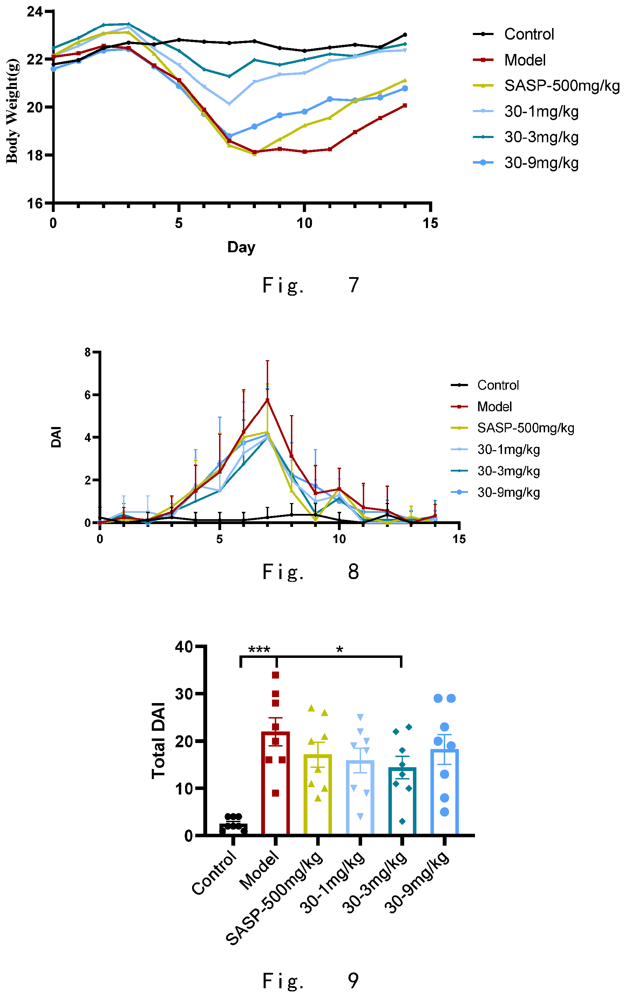

Body weight: On the Day 4 of model establishment in this experiment, the body weight of the mice began to decrease generally, reaching the lowest point on Day 8, after which the body weight started to rebound. Figure 7 shows that compared with the control group, the body weight of the other groups decreased significantly, indicating successful model establishment. Compared with the model group, the SASP group and the 30-3 mg/kg administration group exhibited faster body weight recovery with a greater rebound amplitude.

DAI score: The DAI (Disease Activity Index) score gradually increased with the modeling time and decreased gradually after modeling was stopped. Compared with the model group, the DAI scores of the SASP and 30 treatment groups decreased more rapidly (as shown in Figure 8);
The total DAI scores over 14 days (as shown in Figure 9) were significantly lower in the 30 3 mg/kg treatment group than in the model group, indicating that **30** at 3 mg/kg can alleviate DSS-induced symptoms such as weight loss, hematochezia, and diarrhea in mice.

### 4.3 Effects of Compound 30 on Colon Length, Weight and Swelling Rate in Acute UC Model Mice

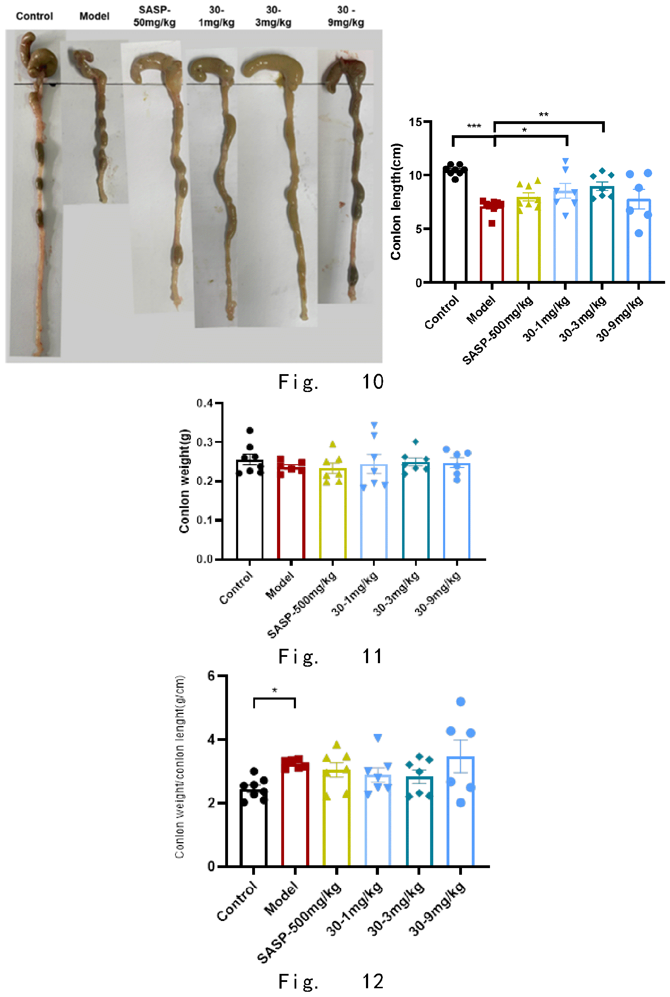

Patients with UC and mouse models typically exhibit colon shortening. As shown in Figure 10, compared with the Control group, the colon length of the Model group was significantly shortened (*p<0.05). 30 1 mg/kg and 3 mg/kg significantly inhibited DSS-induced colon shortening (30 1 mg/kg, *p<0.05; 30 3 mg/kg, **p<0.01, vs. Model group), which was generally consistent with the disease activity index results. Figure 11 shows the colon weight, with no statistically significant difference. To exclude the factor of weight increase due to colon lengthening, the ratio of colon weight to length was used to represent the degree of colon swelling. As shown in Figure 12, compared with the Control group, the colon weight-to-length ratio of the Model group was significantly increased.

### 4.4 Pathological Morphological Changes

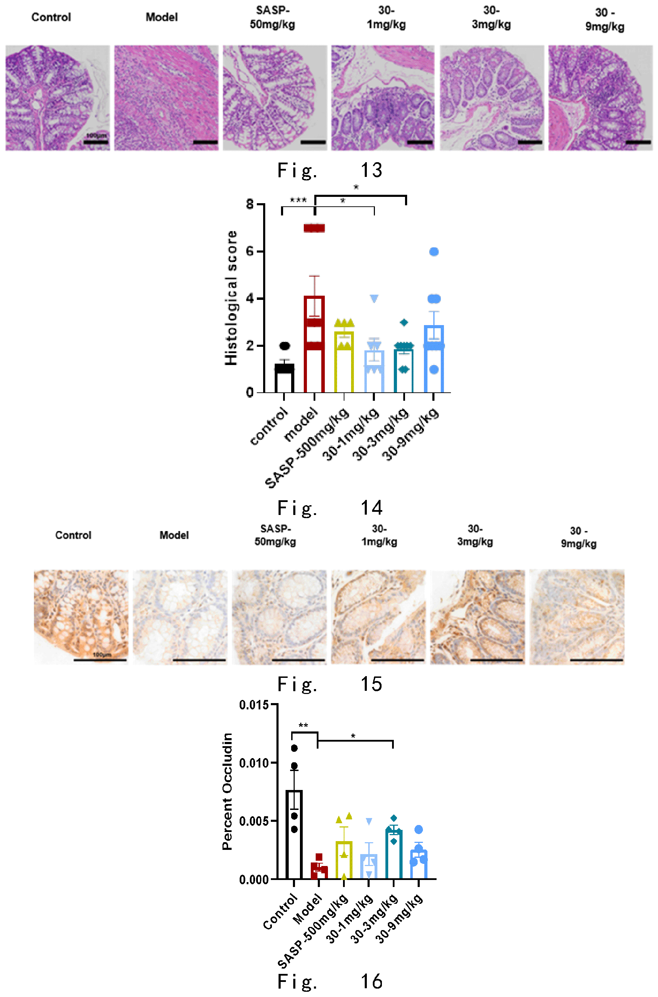

Histopathological examination is an important method for the clinical diagnosis of UC. To further confirm the effect of Compound 30 on UC, we fixed the colon with PFA, prepared paraffin sections, and observed the results by H&E staining. In H&E staining, hematoxylin is alkaline and can stain chromatin in the nucleus and nucleic acids in the cytoplasm purple-blue; eosin is acidic and can stain the cytoplasm and extracellular matrix red. H&E staining allows observation of pathological features of UC such as structural changes, inflammation, and abscesses in tissues. As shown in Figure 13, the Control group mice had intact colonic mucosal structure and intestinal epithelium, with numerous goblet cells arranged orderly and no inflammatory cell infiltration. After DSS modeling, the Model group showed obvious inflammatory cell infiltration in the colonic mucosal lamina propria and submucosa, extending to the muscular layer, along with significantly reduced goblet cells, thinned mucosa, and loss of mucosal epithelial cells, indicating successful modeling. The positive drug SASP group still had inflammatory cell infiltration, but to a lesser extent than the Model group, with more goblet cells and relatively orderly arrangement. The 30 at 9 mg/kg group showed obvious inflammatory cell infiltration and reduced goblet cells. After administration of Compound 30 at 1 mg/kg and 3 mg/kg, the mucosal structure was normal, goblet cells increased and were arranged neatly, no obvious inflammatory cell infiltration was observed, and the loss of epithelial cells was also improved. Figure 14 shows the pathological scores of each group. Overall, compared with the Model group, both SASP and Compound 30 exerted certain ameliorative effects on colonic mucosal injury, and the protective effects of Compound 30 at 1 mg/kg and 3 mg/kg were superior to those of other groups.

### Changes in the colonic mucosal barrier

The colonic mucosal barrier is the most effective barrier in the intestine. Colonic epithelial cells adhere to each other through adhesion molecules and attach to the extracellular matrix. Colonic epithelial cells are connected to one another via tight junctions (TJs), adherens junctions, gap junctions, and other structures, among which TJs are the most important. TJs serve two key functions: first, a selective barrier function that helps maintain the selective permeability of the intestinal epithelium, allowing only ions and small soluble molecules to pass through; second, a fence function. This unique physiological function enables the colonic mucosa not only to absorb water, nutrients, and molecules required for cellular metabolism but also to prevent bacteria and antigens from invading from the intestinal lumen. TJ proteins are located at the apex of cell-cell junctional complexes. Alterations in their expression and distribution can modify colonic inflammation by regulating colonic mucosal homeostasis and intestinal permeability. DSS can damage the colonic barrier in mice, although the specific mechanism remains unclear. It may be that the cytotoxicity of DSS disrupts TJs in colonic epithelium and alters their localization in the colonic mucosa, allowing bacteria to penetrate intestinal tissue more easily and trigger inflammation. Occludin is an important TJ protein. Therefore, immunohistochemistry was used to detect the expression of related proteins.

The fence function of Occludin can separate the apical and basolateral sides of the epithelial cell plasma membrane, restrict the free flow of lipids on the cell membrane, facilitate the formation of cell polarity, maintain the impermeability and rigidity of the intestinal mucosa, and prevent the entry of pathogenic microorganisms. The barrier function of Occludin can regulate the transport of small molecules and maintain tissue homeostasis.

The expression of Occludin in the colonic mucosa was determined by immunohistochemistry, and the results are shown in Figure 15. Compared with the Control group, the expression of Occludin was decreased in the Model group, indicating that the intestinal barrier of mice was impaired after DSS modeling. After treatment with SASP and Compound 30, the expression of Occludin was increased. The expression of Occludin protein in the colon was detected by Western blotting in Figure 16. Compared with the Model group, Compound 30 could increase the expression of Occludin, which was basically consistent with the immunohistochemical results.

### Observation on the efficacy of Compound 30 in mice with DSS-induced chronic ulcerative colitis

### 5.1 Effect of Compound 30 on body weight in mice with chronic ulcerative colitis

During modeling, the body weight of mice in the control group increased steadily. In the model group, during the first modeling period, mice were given 3% DSS drinking water *ad libitum* for 7 days, followed by tap water ad libitum for 7 days. On Day 4 of modeling, the body weight of mice began to decrease generally, reaching the lowest point on Day 8, and then started to rise. During the second modeling period, mice were given 2% DSS drinking water ad libitum for 7 days, followed by tap water ad libitum for 7 days. During the 7 days of DSS administration, the body weight of mice did not increase and was significantly lower than that of the control group; body weight began to recover after switching to tap water. Before the third modeling, mice in the model group were evenly divided into model, sasp-500mg/kg, 30-1mg/kg, 30-3mg/kg, and 30-9mg/kg groups according to their body weight and DAI score, with 11 mice in each group. They were given 2.75% DSS drinking water ad libitum for 7 days, followed by tap water ad libitum for 7 days. Compared with the model group, the weight loss in the treatment groups was milder, and body weight recovered faster after resuming normal drinking water.

Compared with the control group, the body weight of the other groups decreased significantly, indicating successful modeling. Compared with the model group, the body weight recovered rapidly and increased substantially in the SASP group and the 30 treatment groups. Among them, the body weight of mice remained relatively stable during administration in the 30-1 mg/kg and 30-3 mg/kg groups.

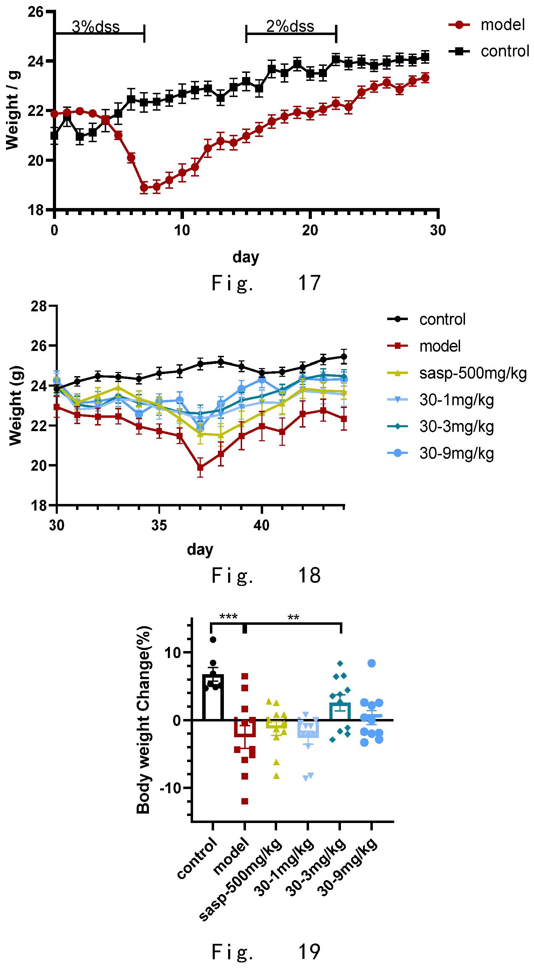

Compared with the control group, the body weight change rate of the model group was significantly decreased. The administration groups inhibited the weight loss of model mice to a certain extent, and the body weight change rate of the 30-3 mg/kg administration group was significantly increased compared with the model group. The body weight changes of each group during modeling are shown in Figure 17; the body weight changes of each group after administration are shown in Figure 18; the body weight change rates of each group before and after administration are shown in Figure 19.

### 5.2 Effect of Compound 30 on DAI Scores in Chronic UC Model Mice

DAI Score: The DAI (Disease Activity Index) gradually increased with the prolongation of modeling time and decreased gradually after modeling was stopped. Compared with the model group, the DAI scores of the SASP group and the 30 treatment group decreased more rapidly.

The total DAI scores of the administration for 14 days were significantly lower in the 30 3 mg/kg treatment group than in the model group, indicating that 30 at 3 mg/kg can alleviate DSS-induced symptoms such as weight loss, hematochezia, and diarrhea in mice.

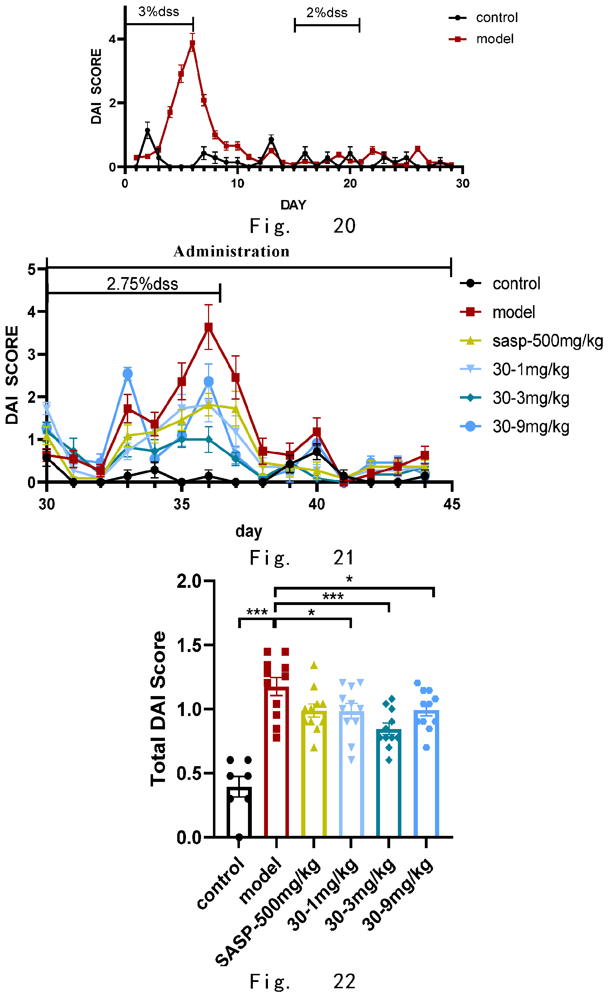

The daily DAI scores of the chronic UC model during modeling are shown in Figure 20; the daily DAI scores during administration are shown in Figure 21; the total daily DAI scores of each group during administration are presented in Figure 22.

### 5.3 Effects of Compound 30 on Colon Length, Weight and Swelling Rate in Chronic UC Model Mice

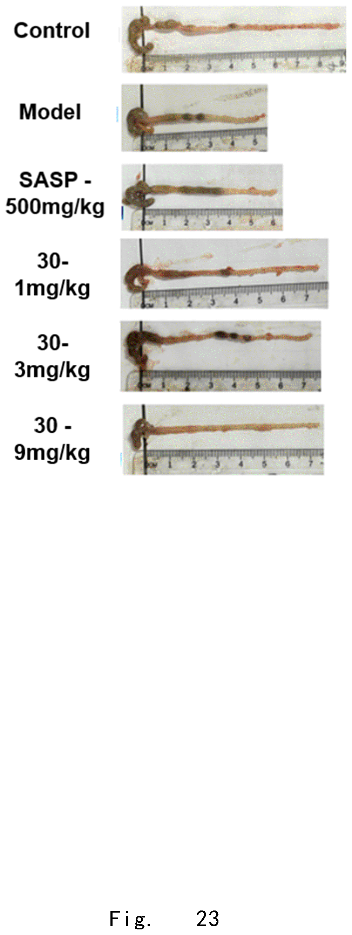

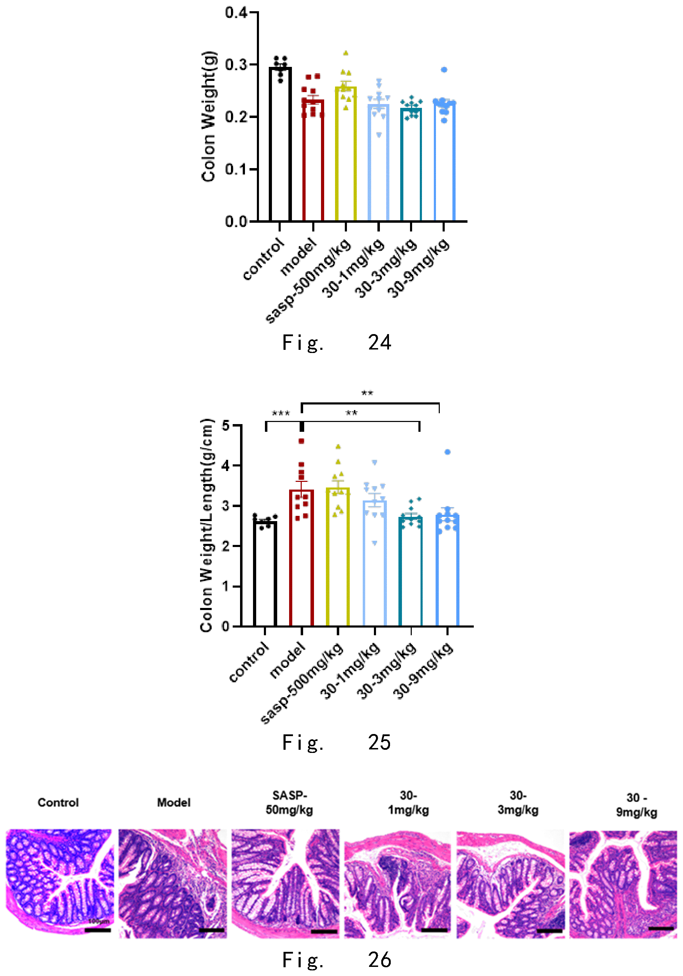

UC patients and mouse models typically exhibit colon shortening. In Figure 23, compared with the Control group, the colon length of mice in the Model group was significantly shortened, and Compound 30 at 3 mg/kg and 9 mg/kg significantly inhibited DSS-induced colon shortening. Figure 24 shows the colon weight. To exclude the factor of weight increase caused by colon lengthening, the ratio of colon weight to length was used to represent the degree of colon swelling. As shown in Figure 25, compared with the Control group, the ratio of colon weight to length in the Model group was significantly increased.

### 5.4 Pathological Morphological Changes

Histopathological examination is an important method for the clinical diagnosis of UC. To further confirm the effect of P30 on UC, the inventors fixed the colon with PFA, prepared paraffin sections, and observed the results by H&E staining. In H&E staining, hematoxylin is an alkaline stain that colors chromatin in the nucleus and nucleic acids in the cytoplasm purple-blue; eosin is an acidic stain that colors the cytoplasm and extracellular matrix red. H&E staining allows observation of pathological features of UC such as structural changes, inflammation, and abscesses in tissues.

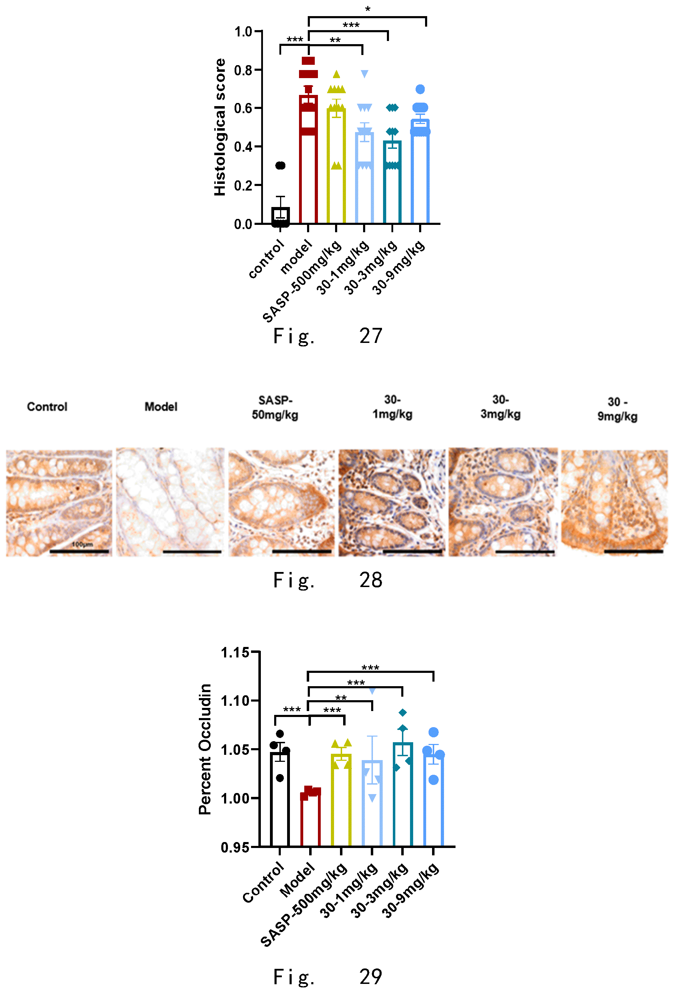

The results are shown in Figure 26. In the Control group, the colonic mucosa structure of mice was intact with a complete intestinal epithelium, a large number of orderly arranged goblet cells, and no inflammatory cell infiltration. After chronic DSS modeling, the model group showed significant inflammatory cell infiltration in the colonic mucosa lamina propria and submucosa, extending to the muscular layer, accompanied by a marked reduction in goblet cells, thinning of the mucosa, and loss of mucosal epithelial cells, indicating successful modeling. The positive drug SASP group exhibited inflammatory cell infiltration, but to a lesser extent than the Model group, with more goblet cells and relatively orderly arrangement. After administration of 30 at 1 mg/kg and 3 mg/kg, the mucosal structure was normal, goblet cells increased and were arranged neatly, no obvious inflammatory cell infiltration was observed, and the loss of epithelial cells was improved. Figure 27 presents the pathological scores of each group. Overall, compared with the Model group, both SASP and 30 exerted certain ameliorative effects on colonic mucosal injury, and the protective effects of 30 at 1 mg/kg and 3 mg/kg were superior to those of other groups.

### Changes in the colonic mucosal barrier

The colonic mucosal barrier is the most effective barrier in the intestinal tract. Colonic epithelial cells adhere to each other through adhesion molecules and to the extracellular matrix. Colonic epithelial cells are connected to one another via tight junctions (TJs), adherens junctions, gap junctions and other means, among which TJs are the most important connection mode. TJs have two functions: first, the selective barrier function, which helps maintain the selective permeability of the intestinal epithelium and only allows ions and small molecular soluble substances to pass through; second, the barrier function. This special physiological function enables the colonic mucosa to not only absorb water, nutrients and molecules required for cellular metabolism, but also prevent bacteria and antigens from invading from the intestinal lumen. TJ proteins are located at the top of the cell-cell junction complex, and changes in their expression and distribution can alter the inflammatory level of the colon by regulating the colonic mucosal homeostasis and intestinal permeability. DSS causes damage to the colonic barrier in mice, but the specific mechanism remains unclear. It may be that the cytotoxicity of DSS leads to the destruction of TJs in colonic epithelium, and the localization of TJs in the colonic mucosa is altered, making it easier for bacteria to penetrate into the intestinal tissue and induce inflammation. Occludin, an important TJ protein, is one of the key components. Therefore, immunohistochemistry is used to detect the expression of related proteins.

The fence function of Occludin separates the apical and basolateral sides of the epithelial cell plasma membrane, restricting the free movement of lipids on the cell membrane, facilitating the formation of cell polarity, maintaining the impermeability and rigidity of the intestinal mucosa, and preventing the entry of pathogenic microorganisms. The barrier function of Occludin regulates the transport of small molecules and maintains tissue homeostasis.

The expression of Occludin in the colonic mucosa was determined by immunohistochemistry, and the results are shown in Figures 28 and 29. Compared with the Control group, the expression of Occludin in the Model group was decreased, indicating that the intestinal barrier of mice was impaired after DSS modeling. After treatment with SASP and 30, the expression of Occludin was increased.

### Example 7. Pharmacodynamic effects of Compounds 30/38/53/67/77/80 in a mouse model of acute ulcerative colitis (UC)

In addition to Compound 30, the inventors further evaluated several other S1PR4 compounds, namely 38, 53, 67, 77, and 80, further demonstrating the therapeutic potential of S1PR4 agonists in the treatment of ulcerative colitis.

Mouse models were established using DSS, with the protocol being 2.5% DSS in drinking water for 5 days followed by *ad libitum* normal drinking water for 2 days. Intragastric administration was performed concurrently with modeling, and RPC1063 was used as the positive control drug. The specific groupings were as follows: RPC1063 was administered intragastrically at 1 mg/kg. Compound 30 was tested at three doses: 0.3 mg/kg, 1 mg/kg, and 3 mg/kg. Compound 38 was administered at 5 mg/kg, while Compounds 53, 67, 77, and 80 were each administered at 1 mg/kg.

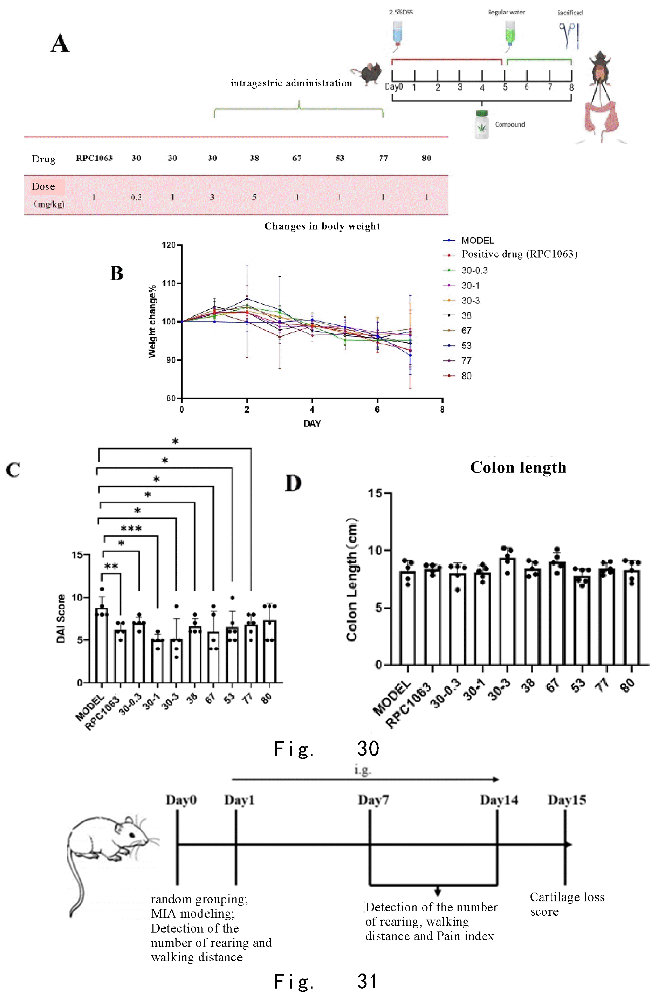

The experimental results (as shown in Figure 30) revealed that Compound 67 induced less body weight loss, and Compound 30 exhibited concentration-dependent recovery. DAI scores indicated that Compounds 30 and 67 produced favorable effects. Regarding colon length, Compounds 30 and 67 showed significant efficacy, whereas the positive control drug had no obvious effect.

### Example 8. Pharmacodynamic effect of Compound 30 in osteoarthritis (OA) model rat

### I. Animals and Main Reagents:

### Experimental Animals:

SD rats, approximately 200 g, male. Purchased from Shanghai Bikai Animal Co., Ltd. Adaptive feeding was conducted for about 7 days.

### Experimental Drug: Compound 30.

### II. Experimental Grouping:

| No. of group | Grouping | Number of rats |
|---|---|---|
| 1 | Control | 10 |
| 2 | Model | 10 |
| 3 | Diclofenac Sodium 2.5 mg/kg | 10 |
| 4 | Compound 30 0.3 mg/kg | 10 |
| 5 | Compound **30** 1mg/kg | 10 |
| 6 | Compound **30** 3mg/kg | 10 |

### III. Experimental Protocol:

### 3.1. Modeling method:

3.1.1 Anesthesia: the rat was placed into the anesthesia box, the O₂ gas source and the gas delivery pump were turned on, and the gas adjustment knob was rotated to set the ratio of O₂ to isoflurane at 8:2. The anesthesia depth knob was adjusted: first the indicator was turned to 3 to induce deep anesthesia in the rat; during the subsequent surgical procedure, the indicator was adjusted to 1.5 to maintain anesthesia. The three-way valve was opened to deliver gas into both the anesthesia box and the rat anesthesia mask.

3.1.2 Needle Insertion: the skin on the right knee of the rat was disinfected with 75% ethanol, the knee was bent to 90°, the knee joint was gently touched to locate the patella, and the tibial plateau was found by slowly probing medially from the inferior border of the patella. The syringe needle was inserted at a 45° angle to the plateau along the lateral side of the patellar ligament, as shown in the figure below. The Control group is injected with 50 µl of 0.9% sterile normal saline, while the other groups are injected with 50 µl of sterile normal saline containing 3 mg of MIA. After the procedure, the rat was removed, placed in a breeding cage, and irradiated with an incandescent lamp to help restore body temperature until the rat recovers from anesthesia.

### 3.2. Drug Preparation:

The Control group was administered 5% DMSO + 35% PEG400 + 60% normal saline. The compounds were dissolved in a system consisting of 5% dimethyl sulfoxide (DMSO), 35% polyethylene glycol 400 (PEG 400), and 60% normal saline. The drug solutions were prepared on the evening one day before administration.

### 3.3. Administration

The rats were acclimated for 1 week and randomly divided into groups, with a total of 10 rats. The Control group was administered 5% DMSO + 35% PEG400 + 60% normal saline, while the other groups were given the test compounds dissolved in 5% DMSO + 35% PEG400 + 60% normal saline.

### 3.4. Experimental procedure: as shown in Figure 31 above.

3.4.1. From Day 1 to Day 14 of the experiment, the rats were administered intragastrically once daily, with a gavage volume of 5 mL/kg. The Control group and Model group were given 5% DMSO + 35% PEG400 + 60% normal saline throughout the experiment, while the other groups were administered the corresponding drugs.

### 3.4.2. OA Disease Activity Index Score

On Day 7 and Day14 of the experiment, behavioral and joint pain tests were performed on the rats. To minimize confounding factors, the tests were conducted 1 hour after administration at a fixed time, preventing variations in the rats' condition at different time points from affecting the experimental results. The rats were allowed to acclimate to the environment for 20 minutes before the start of the experiment.
(1) Observation of walking distance and rearing times within five minutes: the dimensions of the resin box were measured and a paper sheet of the corresponding size was prepared. 1 cm × 1 cm grids were drawn on the paper sheet. The paper sheet was sealed with a transparent tape to prevent damage from rat excrement. Then the paper sheet with 1 cm × 1 cm grids was placed into the transparent resin box, ensuring a flat bottom. A camera device was used to record the walking process and trajectory of the rat within 5 minutes, and the walking distance and rearing times of the rat were counted.
(2) Joint Pain Assessment: After fixing the rat and allowing it to calm down, its knee joint was gently bent. Score 0 if the rat shows no reaction; score 1 if it exhibits a brief, distinct withdrawal or vocalization. The knee joint pain severity in rats is reflected by the scores of leg withdrawal and vocalization. The joint pain assessment was performed every 5 seconds for a total of 5 times.

### 3.4.3. Tissue Collection

On Day 15 of the experiment, the rats were anesthetized, and blood was collected from their eyeballs. The serum was left to stand overnight at 4°C, centrifuged at 3600 rpm for 15 minutes at 4°C. The serum was aspirated and stored at -80°C. The rats were sacrificed by cervical dislocation. An incision was made along the knee joint on ice to remove fur, muscle, and other tissues. For each group, 5 rats were used; bilateral knee joints were dissected, and the color, roughness, and integrity of the femoral condyle surface were observed. The knee joint cartilage was then harvested and cryopreserved at -80°C. The joint specimens from the remaining 5 rats were fixed in formaldehyde fixative.

### 3.4.4. Safranin O-Fast Green Staining

The inventors further evaluated the pathological changes in the knee joints of OA model rats using Safranin O-Fast Green staining and OARSI scoring. Safranin O is basic and can bind to acidic proteoglycans, staining the cartilage matrix red; Fast Green is acidic, while the amino acids abundant in collagen fibers are basic, so Fast Green can stain the superficial cartilage and subchondral bone green. Proteoglycans in cartilage are released after cartilage injury, resulting in disordered arrangement of the uniformly dispersed matrix and loss of Safranin O staining. Therefore, Safranin O can indirectly reflect the content of proteoglycans in the matrix.

### 3.4.5. OARIS Score

The pathological changes in the knee joints of rats were observed under a microscope. The severity of cartilage damage was assessed using the OARIS Osteoarthritis Cartilage Histopathology Assessment System (OOCHAS) grading and scoring system (grade = 6 levels, score = 0-24) (see table), to determine whether the drug could alleviate the severity of osteoarthritis.

| **Grading** | **Main features** | **Sub-level (may not be evaluated)** | **Relevant Standards (Tissue Response)** |
|---|---|---|---|
| 0 | The articular surface is intact. | None | All structures are intact, and the cartilage is not involved. |
| | The cartilage is intact | | |
| 1 | Intact surface | 1.0 Intact cells | Matrix: Intact surface, swelling or fibrosis |
| | | 1.5 cell necrosis | Cells: proliferate and cluster, hyperplasia and hypertrophy ditto |
| 2 | Surface discontinuity | 2.0 Fibrosis exceeds the cartilage surface | |
| | | | The accompanying surface layer is incomplete; |
| | | 2.5 Articular cartilage wear | |
| | | with loss of superficial matrix | With or without matrix cation staining (Safranin O or Toluidine Blue staining), the upper 1/3 of the cartilage (i.e., down to the mid-cartilage layer) shows pale staining; |
| 3 | Vertical fissure | 3.0 Simple Fissure | ditto |
| | | 3.5 Complex or branching fissures | with or without matrix cation staining (Safranin O or Toluidine Blue staining), pale staining of the subchondral region (i.e., extending deep |
| | | | into the mid-cartilage layer); |
| 4 | Erosion | 4.0 Surface stratification | Loss of cartilage matrix and formation of intramatrix cysts |
| | | 4.5 Cystic degeneration in the middle layer | |
| 5 | Defect | 5.0 The bone surface is intact. | Sclerosis of the bone surface or the presence of reparative tissue including fibrocartilage |
| | | 5.5 Tissue repair occurs | |
| 6 | Deformation | 6.0 Osteophyte formation at the joint margins | Bone remodeling: changes in the shape of the joint surface (not just osteophyte formation), including microfractures and the appearance of repair tissue. |
| | | 6.5 Osteophyte formation at the joint margins and centers | |

| **Stage** | **Percentage of damage in the sample (surface, area and volume) (%)** |
|---|---|
| 0 | No manifestations of osteoarthritis |
| 1 | <10 |
| 2 | 10-25 |
| 3 | 25-50 |
| 4 | >50 |

| **Grade** | **Stage** | | | |
|---|---|---|---|---|
| | S1 | S2 | S3 | S4 |
| G1 | 1 | 2 | 3 | 4 |
| G2 | 2 | 4 | 6 | 8 |
| G3 | 3 | 6 | 9 | 12 |
| G4 | 4 | 8 | 12 | 16 |
| G5 | 5 | 10 | 15 | 20 |
| G6 | 6 | 12 | 18 | 24 |

### 4. Experimental Results:

4.1. Before model establishment, the rats had normal food and water intake and shiny fur. After MIA injection for model establishment, during OA modeling, the knee joints of rats injected with MIA into the articular cavity began to swell from the needle insertion site. The swelling gradually spread to the entire knee joint over time, accompanied by redness, swelling, heat and pain in the joint. The rats showed reduced activity, flexion and contraction of the left hind limb, lameness, and listlessness. The swelling degree decreased with time.

### 4.2. Pain Index:

On DAY7, compared with the Model group, the pain indices of rats in the diclofenac sodium-2.5 mg/kg, Compound 30-0.3 mg/kg, and Compound 30-3 mg/kg treatment groups were all significantly decreased.

On DAY14, compared with the Model group, the pain indices of rats in the Compound 30-1 mg/kg and Compound 30-3 mg/kg treatment groups were all significantly decreased.

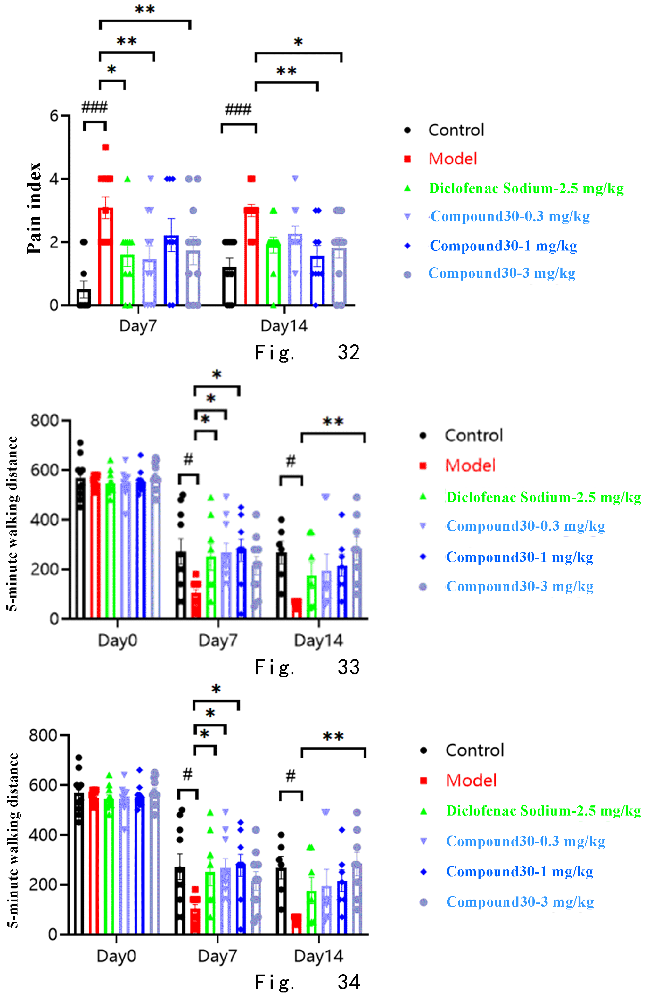

The statistics of the pain index are shown in Figure 32.

### 4.3. Five-minute walking distance and rearing frequency:

On DAY7, compared with the Model group, the walking distance of rats in the diclofenac sodium-2.5mg/kg, Compound 30-0.3mg/kg, and Compound 30-1mg/kg treatment groups increased significantly.

On DAY14, compared with the Model group, the walking distance of rats in the Compound 30-0.3mg/kg and Compound 30-3mg/kg treatment groups increased significantly. The statistics of 5-minute walking distance are shown in Figure 33.

On DAY7 and DAY14, compared with the Model group, there was no statistically significant difference in rearing frequency. The statistics of 5-minute rearing frequency are shown in Figure 34.

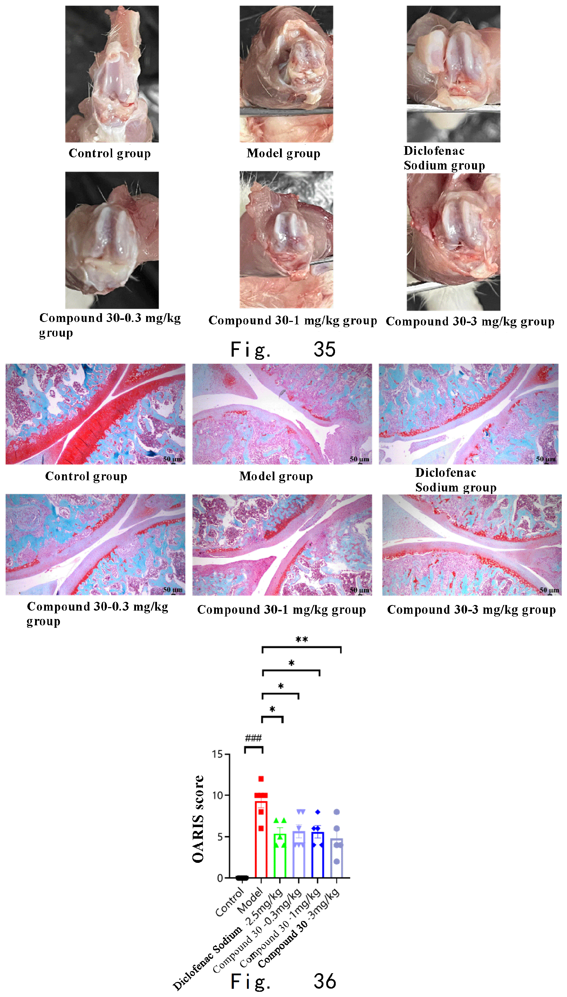

4.4. After sacrificing 5 rats in each group, the knee joints were dissected to expose the femoral condyles of the rats, followed by photography and observation. We conducted an overall observation of the color, roughness, and integrity of the femoral condylar surface of the rat knee joints, with the results shown in Figure 35:
In the Control group, there was no hyperplasia of the synovial bursa in the joint cavity; the cartilage surface was smooth and intact, with a bright color and no cracks; no osteophytes were formed at the joint margins.

In the Model group, the articular cartilage surface of the rats was slightly rough with partial erosion, small fissures, and a dark gray color; the cartilage lost its normal luster, showed moth-eaten changes on the surface, and osteophytes had formed at the margins.

Following treatment with diclofenac sodium and Compound 30, the knee cartilage in most rats became smooth and glossy, with a significant improvement in wear. Although mild moth-eaten changes were observed in some cartilage areas, no obvious osteophyte formation was seen. Therefore, Compound 30 is considered to alleviate knee articular cartilage lesions in OA model rats.

### 4.5. Effects of Compound 30 on Safranin O-Fast Green Staining and OARSI Scoring of Knee Joints in OA Rats

As can be seen from Figure 36, the cartilage matrix in the Control group is bright red; the superficial cartilage and subchondral bone are stained green by fast green. The cartilage structure is intact, with flattened chondrocytes in the superficial layer and round chondrocytes in the middle layer, and chondrocyte proliferation is observed in the deep layer. In contrast, the Model group shows slight loss of safranin staining and local non-staining; superficial chondrocytes become swollen, reduced in number and disorganized; the tidemark extends irregularly with blurred boundaries and partial disappearance.

After treatment with diclofenac sodium and Compound 30, the knee joints of OA model mice are improved, with enhanced safranin staining; chondrocytes increase in number and are arranged relatively regularly.

Next, the inventor used the OARIS score to evaluate the severity of osteoarthritis in each group of rats. The results are shown in Figure 36. The score of the Model group was significantly higher than that of the Control group. After treatment with diclofenac sodium and Compound 30, the knee osteoarthritis in rats was significantly milder than that in the Model group. Rats in the Compound 30-0.3 mg/kg, Compound 30-1 mg/kg, and Compound 30-3 mg/kg treatment groups showed significant differences, effectively alleviating knee joint injury in OA rats. This indicates that Compound 30 can delay the progression of OA.

### Experimental results:

The inventor established a rat OA model by intra-articular injection of MIA into the knee joint to investigate the therapeutic effect of Compound 30 on rats in this model. It was found that Compound 30 could improve the locomotor ability of rats with joint injury and alleviate pain in the rats. Observation of the lesion sites revealed that Compound 30 could ameliorate cartilage wear, erosion and fracture in the knee joints of OA rats.

### Conclusion:

(1) The experimental results indicate that the walking distance of rats in the Compound 30 treatment group increased significantly. It exhibited excellent therapeutic effects in the MIA-induced rat OA model, effectively alleviating pain in rats and improving their walking condition.
(2) Gross observation results show that the Compound 30 treatment group effectively alleviated knee joint damage in OA rats.
(3) The results of pathological sections and OARSI scoring demonstrate that the Compound 30 treatment group effectively relieved knee joint injury in OA rats.

### Example 9. Pharmacodynamic effect of Compound 67 in osteoarthritis (OA) model rat

The rats were acclimatized for 1 week and randomly divided into groups of 10 rats each. Monosodium iodoacetate (MIA) was used to induce the model. The Control group received 5% DMSO + 35% PEG400 + 60% normal saline, while the other groups were administered the test compounds dissolved in 5% DMSO + 35% PEG400 + 60% normal saline. The specific grouping of the administration groups is as follows: diclofenac sodium at 2.5 mg/kg served as the positive control group. Compound 67 was divided into 3 dose groups: 0.3 mg/kg, 1 mg/kg, and 3 mg/kg, respectively.

Before modeling, the rats had normal food and water intake with shiny fur. After intra-articular injection of MIA to establish the OA model, swelling began at the needle insertion site in the knee joints of the rats, which gradually spread to the entire knee joint over time. The joints exhibited redness, swelling, heat, and pain; the rats showed reduced activity, flexion and contraction of the left hind limb, lameness, and listlessness. The swelling subsided gradually over time.

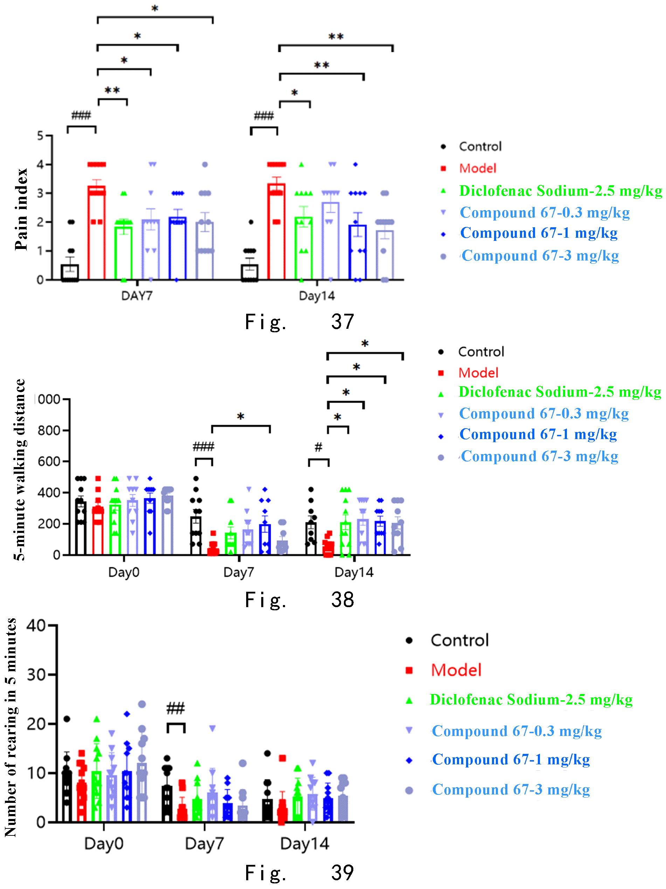

### The pain index of Compound 67 is shown in Figure 37.

On DAY7, compared with the Model group, the pain indices of rats in the diclofenac sodium-2.5 mg/kg, Compound 67-0.3 mg/kg, Compound 67-1 mg/kg, and Compound 67-3 mg/kg treatment groups were all significantly decreased.

On DAY14, compared with the Model group, the pain indices of rats in the Compound 67-1 mg/kg and Compound 67-3 mg/kg treatment groups were all significantly decreased.

The five-minute walking distance and the number of upright stands are shown in Figures 38 and 39:
The experimental results of the five-minute walking distance in rats showed that on DAY7, compared with the Model group, the walking distance of rats in the diclofenac sodium-2.5 mg/kg and Compound 67-1 mg/kg treatment groups increased significantly.

On DAY14, compared with the Model group, the walking distance of rats in the Compound 67-0.3 mg/kg, 67-1 mg/kg, and 67-3 mg/kg treatment groups increased significantly.

The experimental results of the number of times rats rearing within five minutes showed that:
On DAY7 and DAY14, there was no statistically significant difference in the number of rearing compared with the Model group.

Five rats in each group were sacrificed, and the knee joints were dissected to expose the femoral condyles of the rats, followed by photography and observation. The color, roughness and integrity of the femoral condyle surface of the rat knee joints were generally observed.

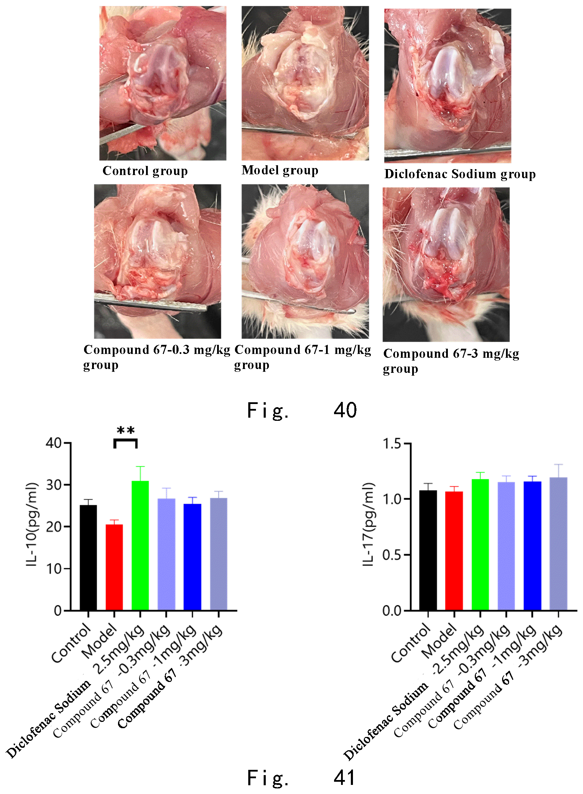

The results are shown in Figure 40: In the Control group, the synovial bursa of the rat joint cavity showed no hyperplasia; the cartilage surface was smooth, intact, bright in color, and free of cracks; no osteophytes formed at the joint margins. In contrast, the Model group exhibited slightly rough and partially eroded articular cartilage surfaces with small fissures and a dull color; cartilage color was lost, the surface showed erosive changes, and osteophytes formed at the margins.

Following treatment with diclofenac sodium and Compound 67, the cartilage of most rat knee joints became smooth and bright, with a significant improvement in wear. Although slight cartilage erosion was observed in some areas, no obvious osteophyte formation was noted. Therefore, Compound 67 can alleviate articular cartilage lesions in the knee joints of OA model rats.

The effect of Compound 67 on serum cytokines in OA rats is shown in Figure 41:
The level of the anti-inflammatory factor IL-10 in the serum of rats in the Model group was decreased. Compared with the Model group, the level of IL-10 was significantly increased in the diclofenac sodium group, while Compound 67 tended to upregulate the levels of IL-10 and IL-17.

There was no difference in the serum level of the anti-inflammatory factor IL-17 among all groups of rats.

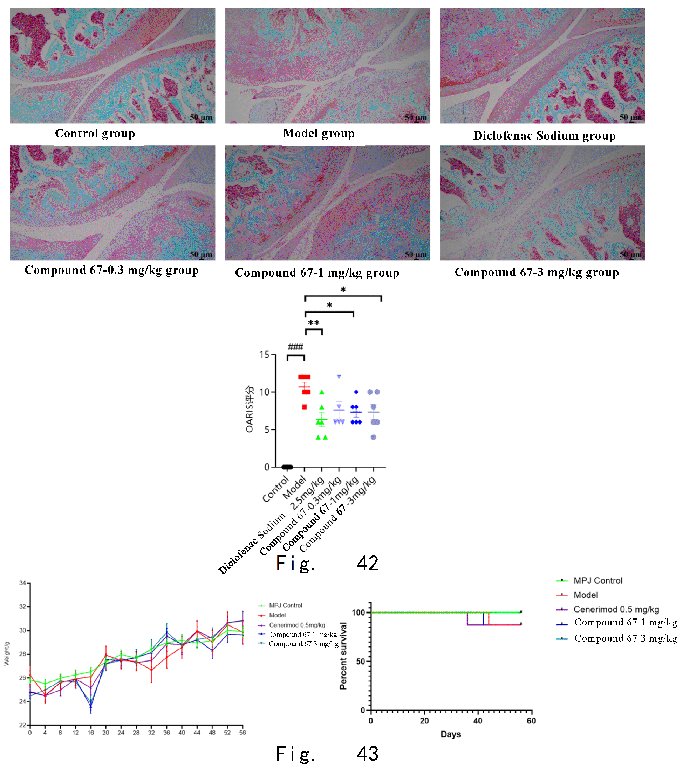

The effects of Compound 67 on safranin O-fast green staining and OARSI scores of knee joints in OA rats are shown in Figure 42: In the Control group, the cartilage matrix exhibited bright red staining; the superficial cartilage and subchondral bone were stained green by fast green. The cartilage structure was intact, with flattened and round chondrocytes in the superficial and middle layers respectively, and chondrocyte proliferation observed in the deep layer. In contrast, the Model group showed slight loss of safranin O staining and regional non-staining; superficial chondrocytes became swollen, reduced in number and disorganized; the tidemark extended irregularly with blurred boundaries and partial disappearance.

After treatment with diclofenac sodium and Compound 67, the knee joints of OA model rats showed improvement, with enhanced Safranin O staining; chondrocytes increased and were arranged more regularly and neatly.

Next, the severity of osteoarthritis in each group of rats was evaluated using the OARIS score. The results are shown in Figure 42. The score of the Model group was significantly higher than that of the Control group. After treatment with diclofenac sodium and Compound 67, the knee osteoarthritis in rats was significantly milder than that in the Model group. In addition, the rats in the Compound 67-1 mg/kg and 3 mg/kg treatment groups showed significant effects, which effectively alleviated the knee joint injury in OA rats.

The inventors established a rat OA model by intra-articular injection of MIA into the knee joint and investigated the therapeutic effect of Compound 67 on rats in this model. It was found that Compound 67 could improve the locomotor ability of rats with joint injury and alleviate pain in the rats. Observation of the lesion sites revealed that Compound 67 could ameliorate cartilage wear, erosion and fracture in the knee joints of OA rats.

### Results:

(1) The experimental results indicate that the walking distance of rats in the Compound 67 treatment group increased significantly. It exhibited excellent therapeutic effects in the MIA-induced rat OA model, effectively alleviating pain in rats and improving their walking condition.
(2) Gross observation results show that Compound 67 treatment effectively alleviated knee joint damage in OA rats.
(3) The results of pathological sections and OARSI scoring demonstrate that Compound 67 treatment significantly relieved knee joint injury in OA rats.
(4) Elisa results reveal that the Compound 67 treatment group tended to upregulate IL-10.

In summary, as a selective S1PR4 agonist, Compound 67 exhibits significant efficacy in the treatment of rat osteoarthritis models.

### Example 10. Pharmacodynamic Evaluation of Compound 67 in MRL/Lpr Model Mice with Systemic Lupus Erythematosus (SLE)

The efficacy of Compound 67 was evaluated using MRL/Lpr lupus model mice.

The specific groups were as follows: MPJ control group, model group, positive drug Cenerimod group at 0.5 mg/kg, and Compound 67 at two doses: 1 mg/kg and 3 mg/kg.

The statistical results of body weight and survival rate within 8 weeks are shown in Figure 43.

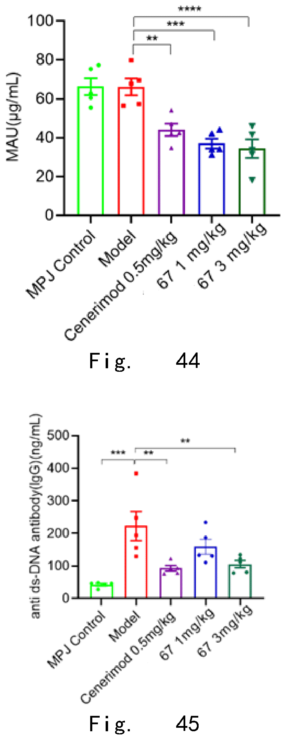

MAU refers to microalbuminuria, which reflects the abnormal leakage of protein from the kidneys. Measurement of MAU can indicate early kidney disease and renal damage. The results showed that after 8 weeks, the urinary microalbumin level in mice of the SLE model group was increased (suggesting renal function injury). Continuous administration of Compound 67 for 8 weeks significantly reduced the urinary microalbumin level in mice, and its effect was superior to that of the Cenerimod 0.5 mg/kg group (as shown in Figure 44).

Anti-double-stranded DNA antibody (anti-ds-DNA antibody (IgG)) has high sensitivity for SLE and serves as a diagnostic indicator and activity marker for SLE. The results showed that the level of anti-double-stranded DNA antibody in the SLE model group mice was significantly increased after 8 weeks, and continuous administration of Compound 67 for 8 weeks could significantly reduce the level of anti-double-stranded DNA antibody in mice (as shown in Figure 45).

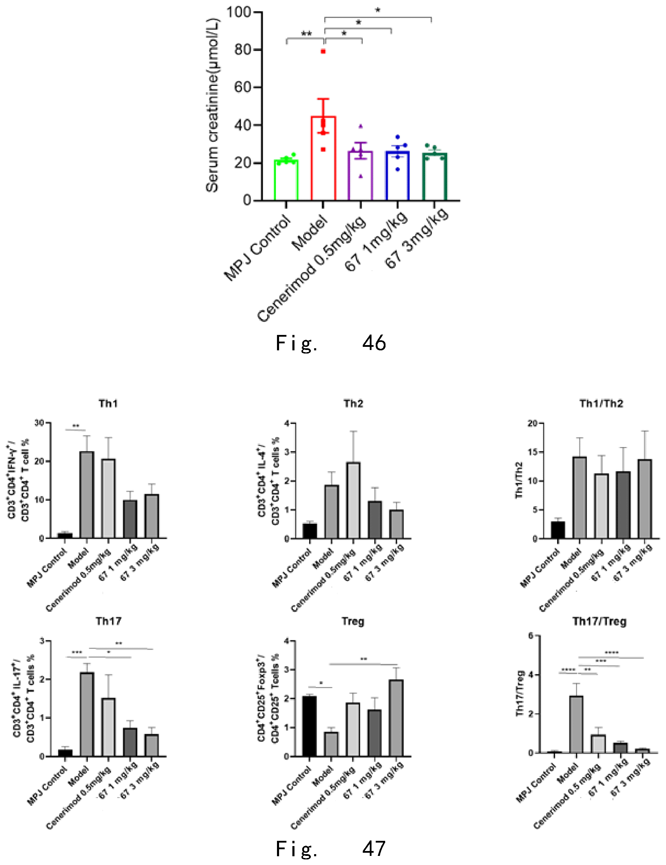

Serum creatinine is a product of human muscle metabolism. Under normal renal function, most creatinine is filtered out through the glomeruli, so it can be used as a diagnostic and screening indicator for glomerular filtration function. When glomerular filtration function decreases, serum creatinine accumulates in the blood and increases. The results showed that the serum creatinine level of mice in the SLE model group was significantly increased after 8 weeks, and continuous administration of Compound 67 for 8 weeks could significantly reduce the serum creatinine level of mice (as shown in Figure 46).

Immunological studies have shown that the numbers of Th1 and Th17 cells in the spleens of SLE mice are significantly higher than those in the normal control group. Compound 67 can significantly reduce the number of Th1- and Th17-positive cells, as well as the Th1/Th2 and Th17/Treg ratios, suggesting that its therapeutic effect is related to regulating the number and function of T lymphocytes (as shown in Figure 47).

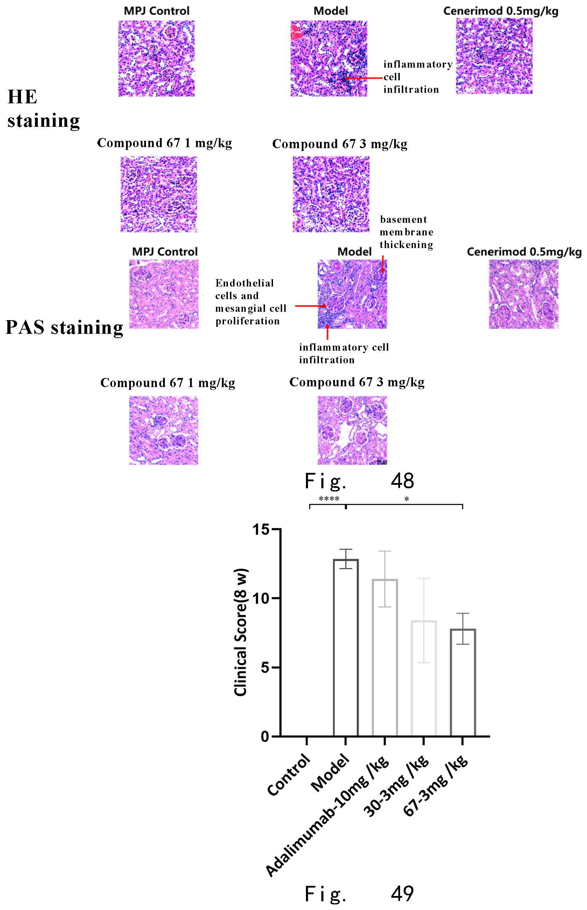

Given that lupus nephritis is the most frequently affected organ in SLE, we performed HE and PAS staining on the kidneys of mice in each group. The results showed that the SLE model group exhibited severe inflammatory cell infiltration, mesangial cell proliferation, basement membrane thickening, and capillary endothelial cell proliferation. After 8 weeks of treatment with Compound 67, inflammatory cell infiltration was significantly reduced, and mesangial cell proliferation, basement membrane thickening, and capillary endothelial cell proliferation were improved, with a better effect than the Cenerimod 0.5 mg/kg group (as shown in Figure 48).

In conclusion, Compound 67 exhibits excellent therapeutic efficacy against SLE and is superior to the positive control drug Cenerimod.

### Example 11. Pharmacodynamic effects of Compounds 30 and 67 in a mouse model of ankylosing spondylitis (AS)

Ankylosing Spondylitis (AS) is a chronic, progressive, inflammatory disease that primarily affects the spine and sacroiliac joints, and may cause pain and stiffness in these areas.

### 1. Establishment of the AS mouse model and administration method

After adaptive feeding, SKG mice were used for the experiment. Except for 5 mice as the blank control group, the other 20 mice were intraperitoneally injected with Curdlan (3 mg per mouse) at week 0 and week 2 of the experiment to establish an AS mouse model, and were randomly divided into 4 groups: model group, positive control group treated with adalimumab (10 mg/kg), compound 30 group (3 mg/kg), and compound 67 group (3 mg/kg), with 5 mice in each group. Mice in the compound 30 and 67 groups were intragastrically administered compound 30 and 67 daily starting from week 3.3 until sacrifice; the positive control group received intraperitoneal injection of adalimumab injection (first administration at week 3.3, second at week 9). Body weight was measured every week to adjust the dosage.

### 2. Detection Indicators

### 2.1 Peripheral Clinical Symptoms

Clinical symptom observation of the limbs and toes were conducted, and their redness and swelling were scored, with a total score of 16 points (4 points per foot).

### Scoring criteria:

0 = No swelling
1 = Mild swelling and redness on the top of the foot
2 = Severe swelling and redness on the top of the foot
3 = Severe swelling and redness in the wrist or ankle joint
4 = Severe swelling in the wrist or ankle joint and toes.

### 2.2 Spinal Joint Lesion Status

CT scans were performed on mice at Week 13.4 and 14.3 of the experiment to obtain imaging data of the mice's bones and joints. Image WL: 1400; WW: 2000.

### Scoring criteria:

1. Spine (total 2 points): 1 point for narrowed intervertebral space, 2 points for complete fusion.
2. Sacroiliac joints (1 point for each side, total 2 points): 0.5 points for blurred and sclerotic articular surface, 0.5 points for narrowed joint space.
3. Zygapophyseal joints of the spine (1 point for each side, total 2 points): 0.5 points for narrowed joint space, 1 point for complete fusion.

### 2.3 Statistical Processing

Analysis of variance (ANOVA) was performed using IBM SPSS Statistics software to obtain statistical results of the dataset, with P < 0.05 considered statistically significant.

### 3. Experimental Results

### 3.1 Peripheral Clinical Symptoms

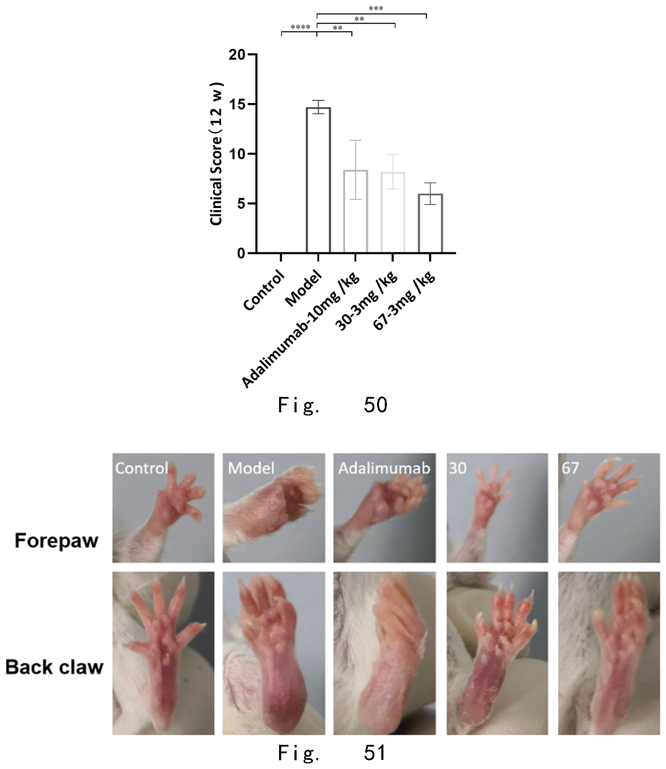

AS mice began to exhibit peripheral inflammatory symptoms at the 4th week: redness and swelling of the feet and toes; redness and swelling of the ears; scaling and redness of the tail; mild hair loss and dermatitis near the eyes and nose; and ear redness. Symptoms in the model group gradually worsened over time. Symptoms in the Compound 30 group, Compound 67 group, and positive control (adalimumab) group were milder than those in the model group. Clinical symptom scores showed that at Week 12, the scores of the Compound 30 group, Compound 67 group, and positive control (adalimumab) group were significantly lower than those of the model group (P < 0.05), as shown in Figures 49 and 50. Compound 30 and Compound 67 also significantly reduced swelling of the wrist and ankle joints (as shown in Figure 51).

### 3.2 Spinal Joint Lesion Status

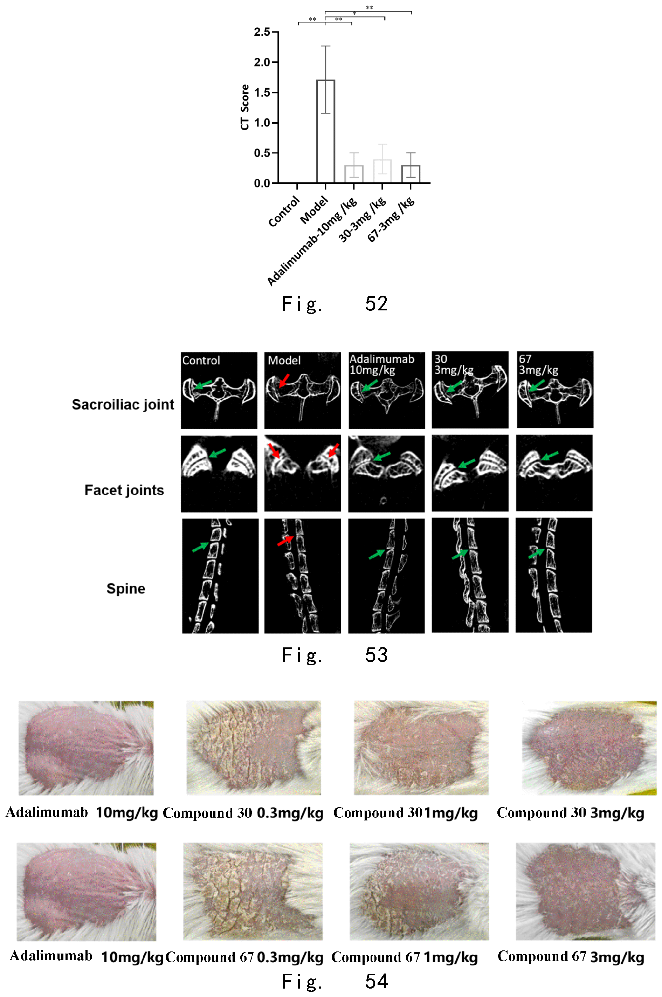

CT scan results showed that some mice in the model group had unclear articular surfaces on one side of the sacroiliac joint with certain damage; obvious fusion was observed on one side of the superior and inferior zygapophyseal joints, while the other side exhibited narrowed joint space and blurred articular surfaces. Mild fusion was also present in the spinal segment, and the joint space of the spinal zygapophyseal joints was narrowed. Mice treated with Compound 30 and Compound 67, as well as those in the positive control (adalimumab) group, showed milder spinal joint lesions compared with the model group, with significantly lower imaging scores (P<0.05), as presented in Figures 52 and 53.

Conclusion: In mouse models of ankylosing spondylitis, both Compound 30 and Compound 67 achieve therapeutic effects comparable to or even superior to the positive control drug adalimumab in reducing clinical scores and alleviating skeletal and joint pathological changes. This fully demonstrates that Compound 30 and Compound 67 possess therapeutic effects against ankylosing arthritis.

### Example 12: Pharmacodynamic Evaluation of Compounds 30 and 67 in a Mouse Psoriasis Model

The imiquimod-induced mouse psoriasis model is a commonly used model for psoriasis research. This model uses the topical immune modulator imiquimod (IMQ) to induce lesions similar to human psoriasis on the skin of mice.

### Experimental Materials

Mice: Female BALB/c mice, aged 6-8 weeks.
Imiquimod cream: 5% IMQ cream.
Experimental Procedures
Animal preparation: Mice were acclimated to the laboratory environment for at least one week.

Random grouping: Generally, 5 mice were included in each group. Specifically, the groups were as follows: adalimumab injection group for positive control; compounds 30 and 67 administered by gavage, divided into 3 dosage groups: 0.3, 1, and 3 mg/kg.

### Application of Imiquimod Cream (Model Establishment):

The hair on the backs of the mice in each group was shaved with a razor, covering an area of approximately 2 × 3 cm².

5% IMQ cream was applied daily to the shaved area on the mouse backs at a dose of 62.5 mg of IMQ cream for 6-7 consecutive days. Skin reactions of the mice were observed after 7 days, including erythema, scaling, and skin thickening. The specific experimental results are shown in Figure 54.

In terms of clinical manifestations, in a BALB/c female mouse model of psoriasis induced by imiquimod, Compound 30 and Compound 67 exerted significant alleviating effects at all concentrations in a dose-dependent manner. Among them, Compound 30 and Compound 67 exhibited the best efficacy at a high dose of 3 mg/kg, achieving therapeutic effects similar to those of adalimumab. These results demonstrate that Compound 30 and Compound 67 possess the potential for treating psoriasis.

### Discussion:

Through extensive and in-depth research, the inventors designed and synthesized a series of small-molecule S1P receptor regulators that have not been reported in the literature. The obtained compounds were subjected to cell-level activity testing, yielding a number of compounds capable of regulating S1PR4 and the S1PR4 mutant protein. Furthermore, *in vivo* pharmacokinetic parameters were determined for several S1PR4 agonists with excellent activity, which were found to possess favorable oral absorption properties and exhibit certain development potential.

The inventors evaluated the invented S1PR4 agonist compounds in disease models including ulcerative colitis, systemic lupus erythematosus, osteoarthritis, and ankylosing spondylitis. These compounds exhibited *in vivo* efficacy in various autoimmune diseases and were superior to S1PR1 agonists or antibody drugs targeting the same target.

The above work lays the foundation for treating autoimmune diseases mediated by the S1PR4 receptor.

All documents mentioned in the present invention are incorporated herein by reference as if each document were individually incorporated by reference. Furthermore, it should be understood that after reading the above teachings of the present invention, those skilled in the art may make various alterations or modifications to the present invention, and such equivalent forms shall also fall within the scope defined by the appended claims of the present application.

## Claims

1. A compound of formula I, or an optical isomer or a pharmaceutically acceptable salt thereof: 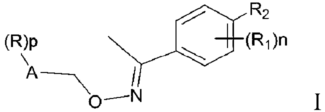
A is selected from: a substituted or unsubstituted C₅-C₈ aromatic ring (such as phenyl) or C₈-C₁₄ fused aromatic ring (such as naphthyl), substituted or unsubstituted five-membered or six-membered heterocyclic group containing 1-3 heteroatoms selected from N or O;
R is independently selected from the group consisting of a hydrogen, halogen, cyano, substituted or unsubstituted C₁-C₁₀ alkyl (for example, a methyl, trifluoromethyl, trifluoroethyl), substituted or unsubstituted C₃-C₈ cycloalkyl or cycloalkenyl, substituted or unsubstituted C₁-C₅ alkoxy, substituted or unsubstituted C₅-C₈ aryl (for example, phenyl), substituted or unsubstituted five-membered or six-membered heterocyclic group or heteroaryl containing 1-2 heteroatoms selected from N, O or S, substituted or unsubstituted C₁-C₁₀ alkyl formyl, substituted or unsubstituted C₅-C₈ aryl formyl; p is an integer from 1 to 3;
R¹ is selected from the group consisting of a hydrogen, halogen, cyano, substituted or unsubstituted C₁-C₅ alkoxy, substituted or unsubstituted C₁-C₁₀ alkyl, substituted or unsubstituted C₃-C₈ cycloalkyl, substituted or unsubstituted C₂-C₆ alkenyl, substituted or unsubstituted C₃-C₆ cycloalkenyl, substituted or unsubstituted C₃-C₈ lactone group, substituted or unsubstituted C₁-C₁₀ amide group, substituted or unsubstituted C₅-C₈ aryl, substituted or unsubstituted five-membered or six-membered heterocyclic group containing 1-2 heteroatoms selected from N, O or S, substituted or unsubstituted C₅-C₈ aromatic heterocyclic group;
n is an integer selected from 1-3;
R² is selected from the following group: 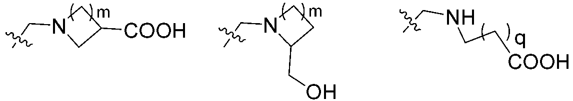
m is an integer selected from 1-4;
q is an integer selected from 1-2.

2. The compound of claim 1, wherein the compound is represented by the following formula II: 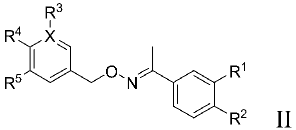 wherein,
R¹ is selected from the group consisting of a hydrogen, halogen (preferably F, Cl or Br), cyano, substituted or unsubstituted C₁-C₅ alkoxy (preferably methoxy), substituted or unsubstituted C₁-C₁₀ alkyl (preferably a substituted or unsubstituted C₁-C₆ alkyl, such as a methyl, ethyl, trifluoromethyl), substituted or unsubstituted C₃-C₈ cycloalkyl, substituted or unsubstituted C₂-C₆ alkenyl, substituted or unsubstituted C₃-C₆ cycloalkenyl, substituted or unsubstituted C₅-C₈ aryl, substituted or unsubstituted five-membered or six-membered heterocyclic group containing 1-2 heteroatoms selected from N, O or S, and substituted or unsubstituted C₅-C₈ aromatic heterocyclic group;
R² is selected from the following group: 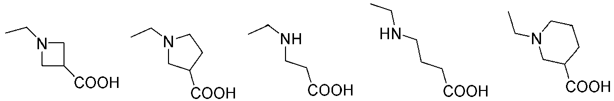
X is selected from C or N;
R³ is not present or is a substituent selected from the following group: a hydrogen, cyano, halogen, substituted or unsubstituted C₁-C₁₀ alkyl (for example, a methyl, trifluoromethyl, trifluoroethyl), substituted or unsubstituted C₃-C₈ cycloalkyl, cyano, substituted or unsubstituted C₁-C₅ alkoxy, substituted or unsubstituted C₁-C₁₀ alkyl formyl, substituted or unsubstituted C₅-C₈ aryl formyl, substituted or unsubstituted C₅-C₈ aryl, substituted or unsubstituted five-membered or six-membered heterocyclic group containing 1-2 heteroatoms selected from N, O or S;
R⁵ is selected from the group consisting of a hydrogen, cyano, halogen, substituted or unsubstituted C₁-C₁₀ alkyl (for example, a methyl, trifluoromethyl, trifluoroethyl), substituted or unsubstituted C₃-C₈ cycloalkyl, cyano, substituted or unsubstituted C₁-C₅ alkoxy, substituted or unsubstituted C₁-C₁₀ alkyl formyl, substituted or unsubstituted C₅-C₈ aryl formyl, substituted or unsubstituted C₅-C₈ aryl, substituted or unsubstituted five-membered or six-membered heterocyclic group containing 1-2 heteroatoms selected from N, O or S;
R⁴ is selected from the group consisting of a hydrogen, halogen, substituted or unsubstituted C₁-C₁₀ alkyl, substituted or unsubstituted C₃-C₈ cycloalkyl or cycloalkenyl, substituted or unsubstituted C₅-C₈ aryl, substituted or unsubstituted five-membered or six-membered heterocyclic group or heteroaryl groups containing 1-2 heteroatoms selected from N, O or S, substituted or unsubstituted C₁-C₁₀ alkyl formyl, and substituted or unsubstituted aryl formyl.

3. The compound of claim 1, wherein the compound is represented by the following formula III: 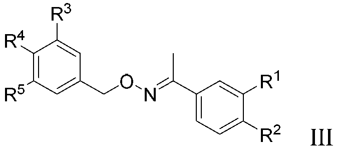 wherein,
R¹ is selected from the following group: a hydrogen, halogen (preferably F, Cl, or Br), cyano, substituted or unsubstituted C₁-C₅ alkoxy (preferably a methoxy), substituted or unsubstituted C₁-C₁₀ alkyl (preferably a substituted or unsubstituted C₁-C₆ alkyl, such as a methyl, ethyl, trifluoromethyl), substituted or unsubstituted C₃-C₈ cycloalkyl, substituted or unsubstituted C₂-C₆ alkenyl, substituted or unsubstituted C₃-C₆ cycloalkenyl, substituted or unsubstituted C₅-C₈ aryl, substituted or unsubstituted five-membered or six-membered heterocyclic group containing 1-2 heteroatoms selected from N, O, or S, substituted or unsubstituted C₅-C₈ aromatic heterocyclic group;
R² is selected from the following group: 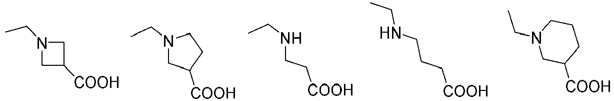
R³ is selected from the group consisting of a hydrogen, cyano, halogen, substituted or unsubstituted C₁-C₁₀ alkyl (for example, a methyl, trifluoromethyl, trifluoroethyl), substituted or unsubstituted C₃-C₈ cycloalkyl, cyano, substituted or unsubstituted C₁-C₅ alkoxy, substituted or unsubstituted C₁-C₁₀ alkyl formyl, substituted or unsubstituted C₅-C₈ aryl formyl, substituted or unsubstituted C₅-C₈ aryl, substituted or unsubstituted five-membered or six-membered heterocyclic group containing 1-2 heteroatoms selected from N, O or S;
R⁵ is selected from the following group: a hydrogen, cyano, halogen, substituted or unsubstituted C₁-C₁₀ alkyl (for example, a methyl, trifluoromethyl, trifluoroethyl), substituted or unsubstituted C₃-C₈ cycloalkyl, cyano, substituted or unsubstituted C₁-C₅ alkoxy, substituted or unsubstituted C₁-C₁₀ alkyl formyl, substituted or unsubstituted C₅-C₈ aryl formyl, substituted or unsubstituted C₅-C₈ aryl, substituted or unsubstituted five-membered or six-membered heterocyclic group containing 1-2 heteroatoms selected from N, O or S;
R⁴ is selected from the group consisting of a hydrogen, halogen, substituted or unsubstituted C₁-C₁₀ alkyl, substituted or unsubstituted C₃-C₈ cycloalkyl or cycloalkenyl, substituted or unsubstituted C₅-C₈ aryl, substituted or unsubstituted five-membered or six-membered heterocyclic group or heteroaryl containing 1-2 heteroatoms selected from N, O or S, substituted or unsubstituted C₁-C₁₀ alkyl formyl, and substituted or unsubstituted aryl formyl.

4. A compound selected from the following group, or an optical isomer or a pharmaceutically acceptable salt thereof:

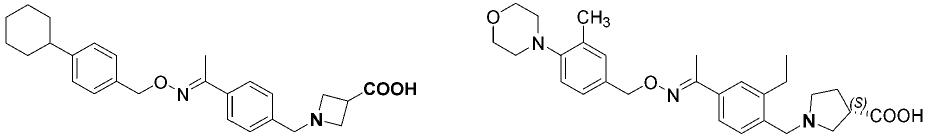

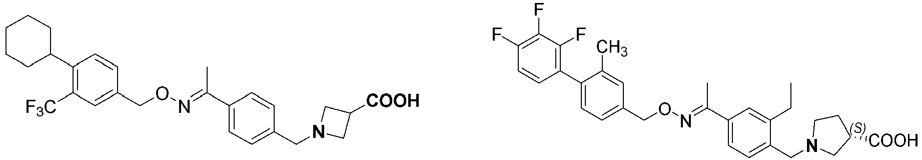

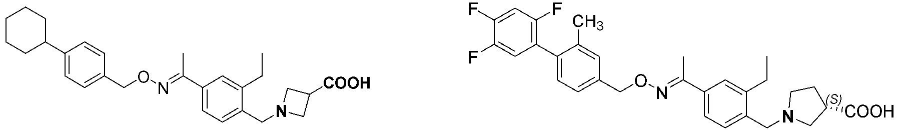

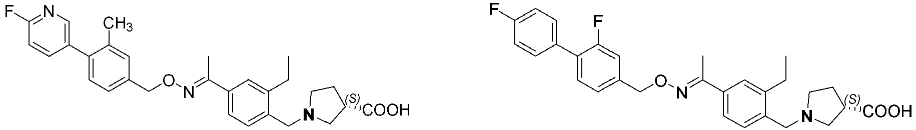

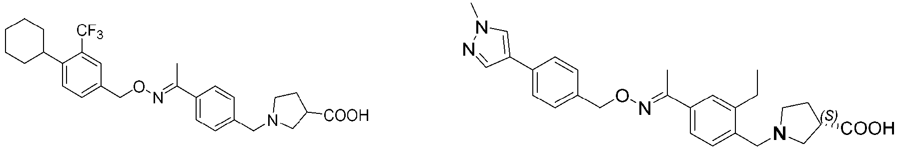

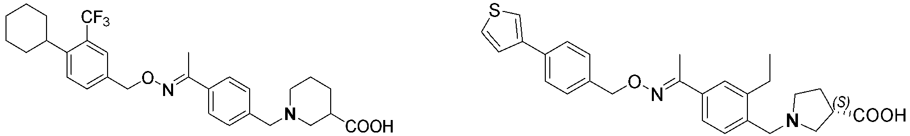

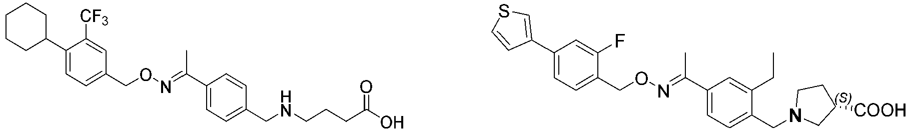

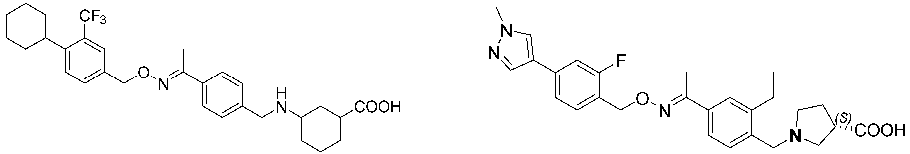

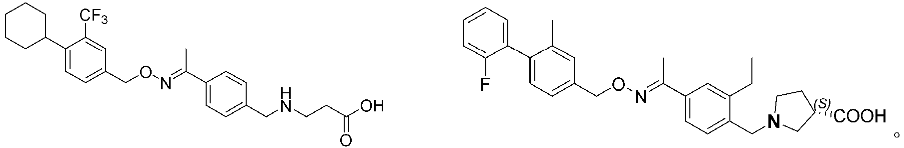

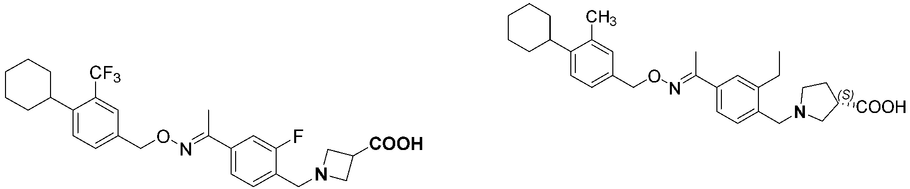

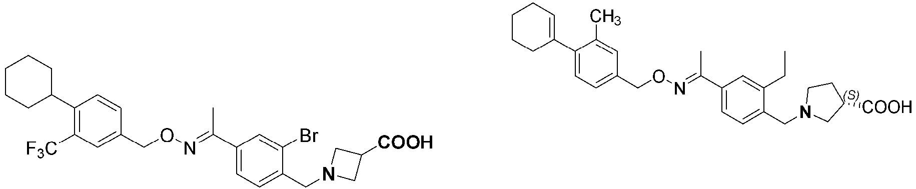

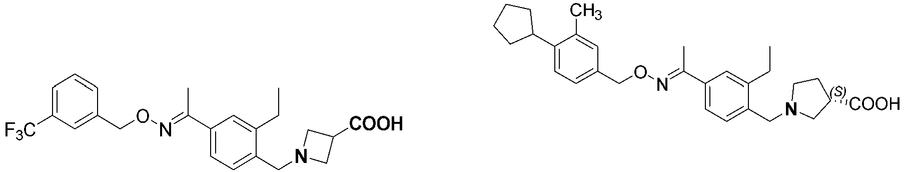

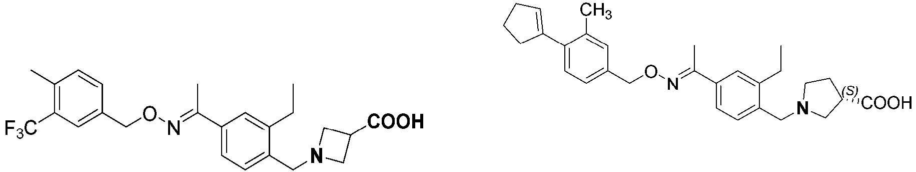

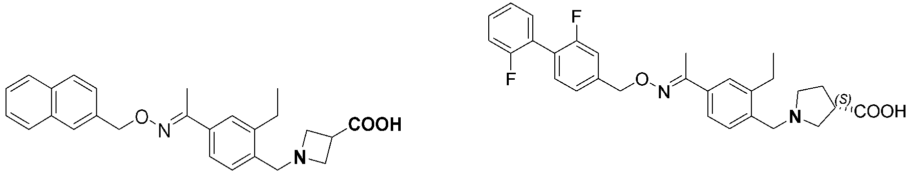

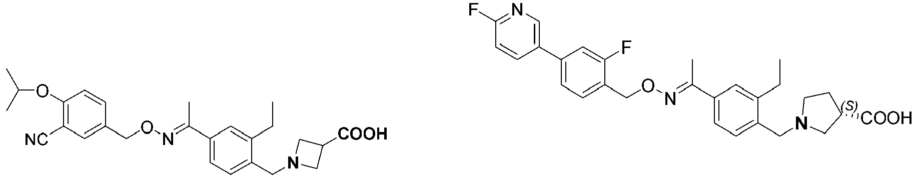

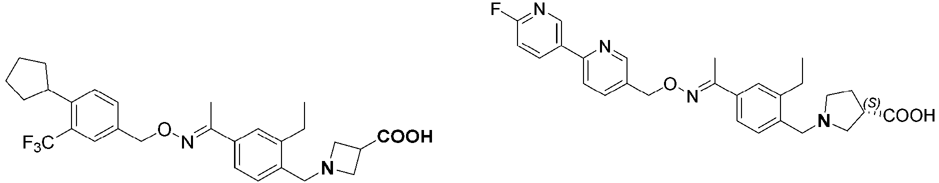

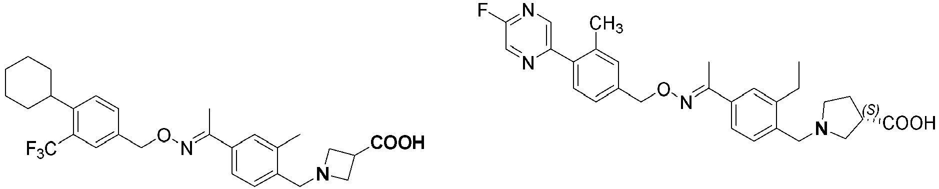

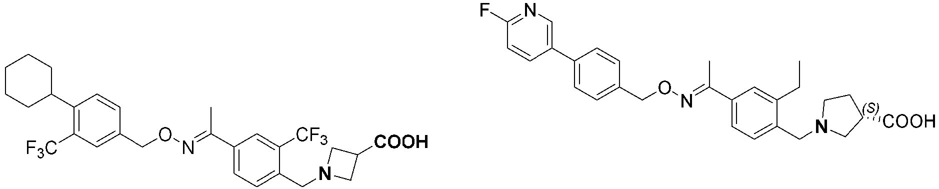

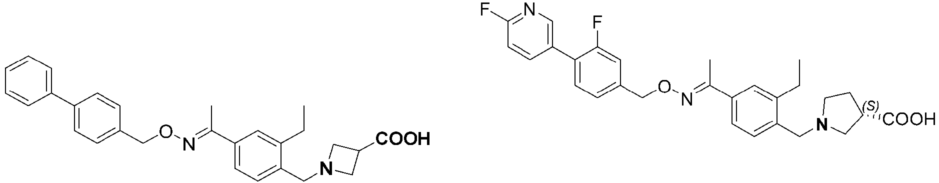

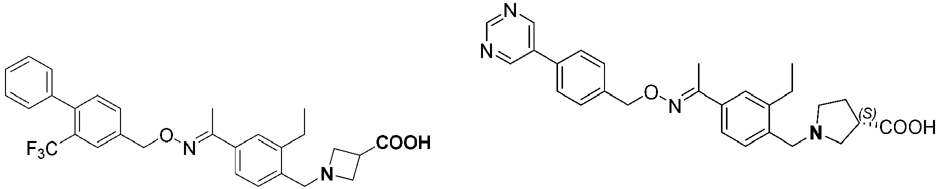

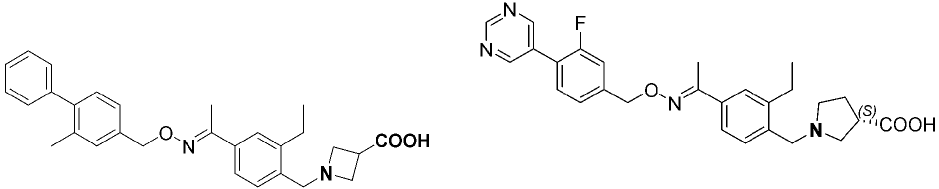

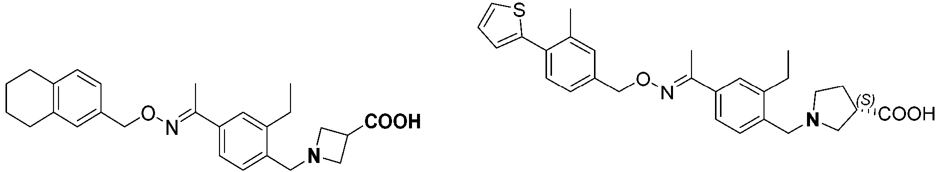

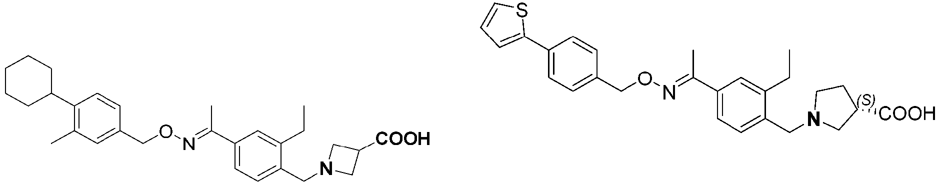

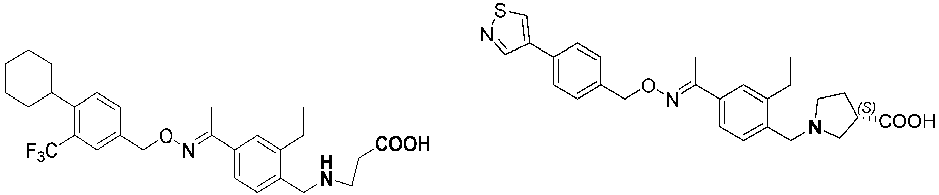

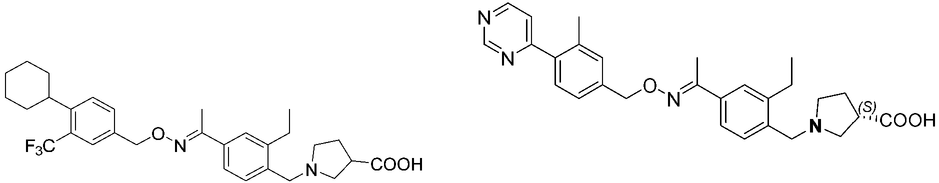

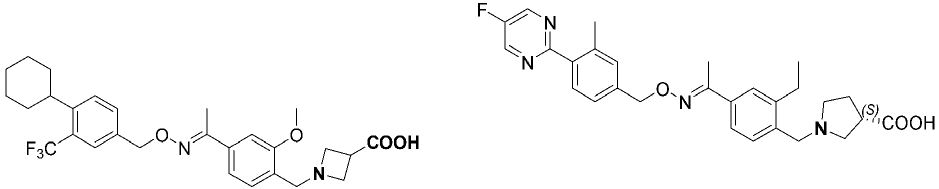

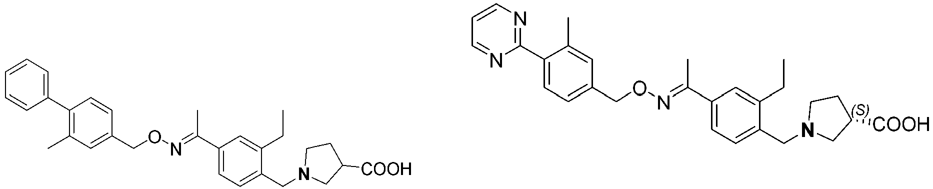

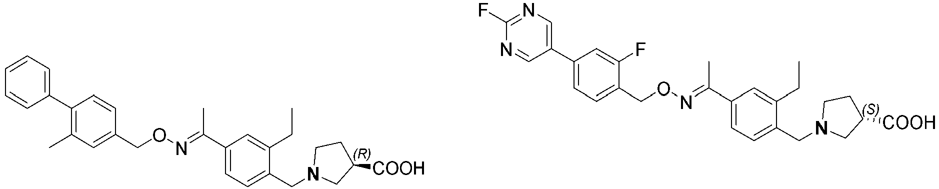

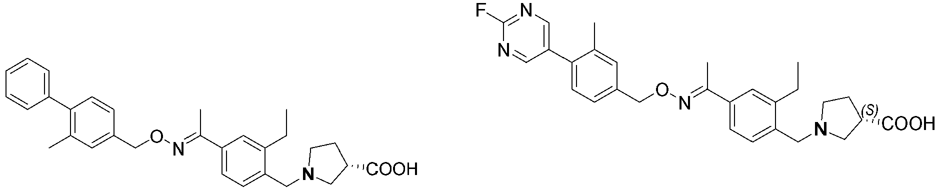

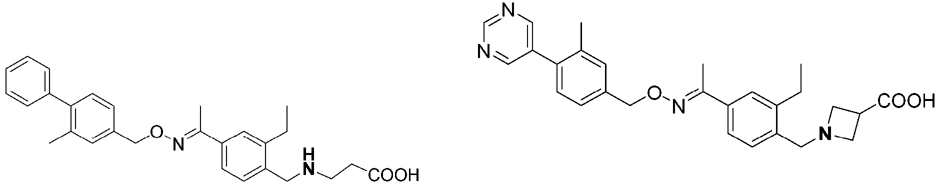

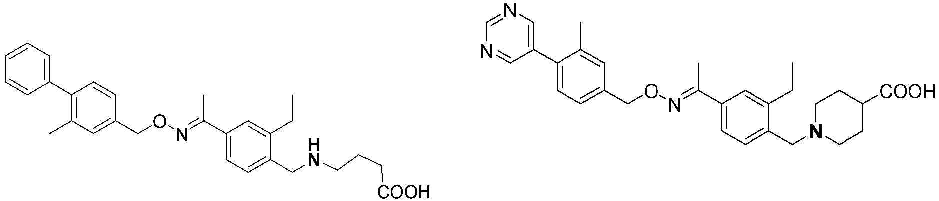

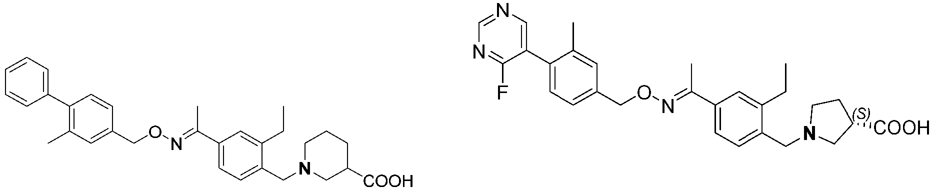

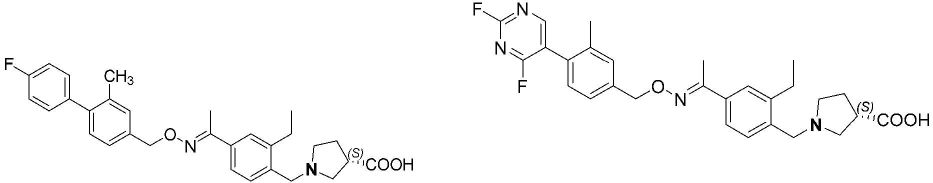

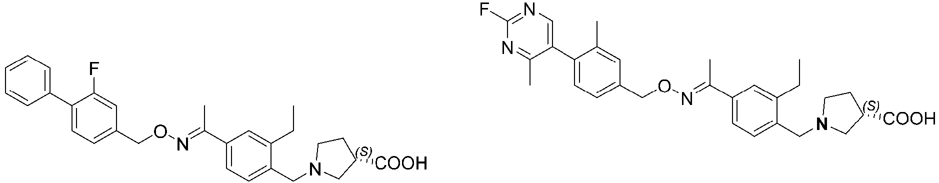

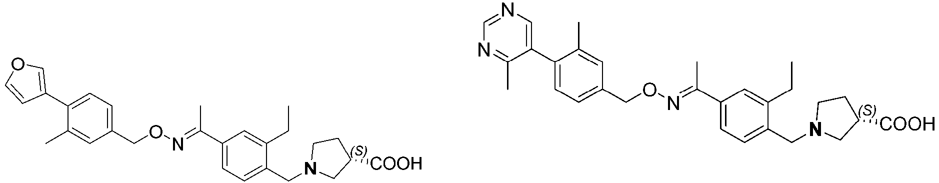

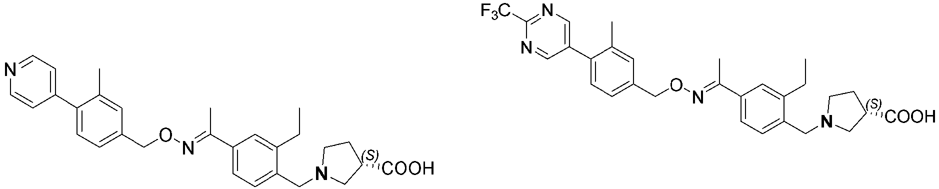

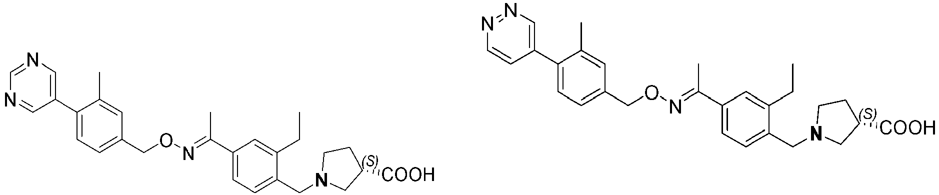

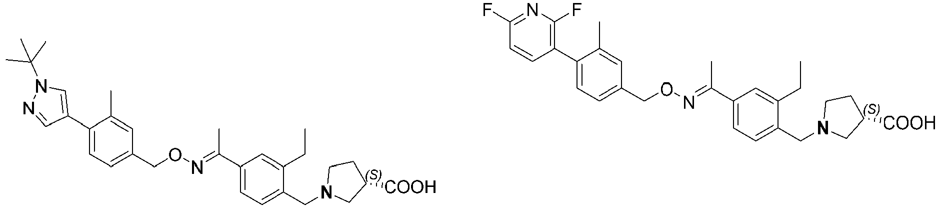

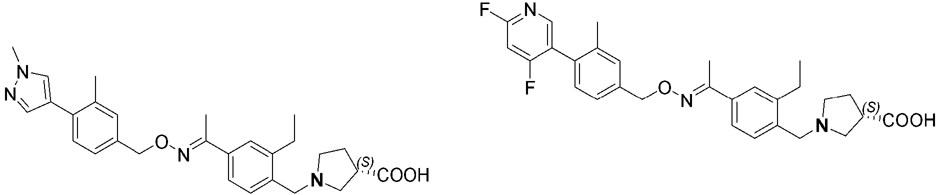

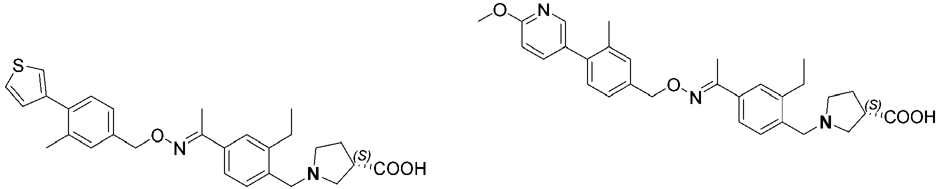

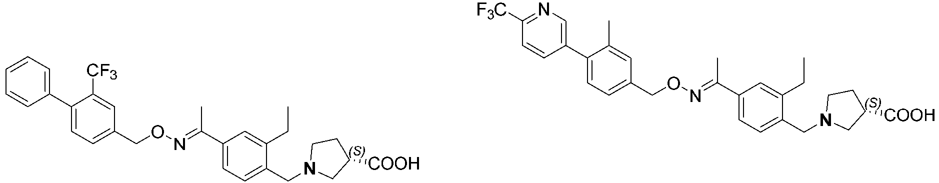

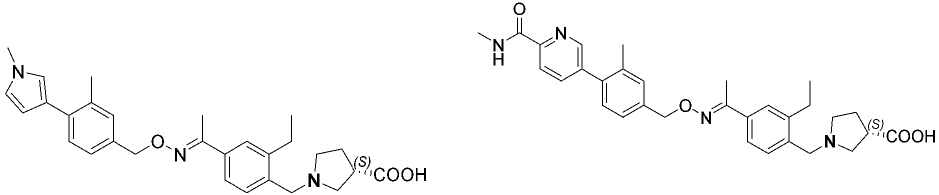

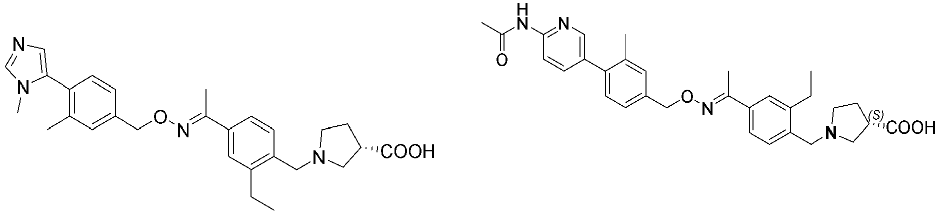

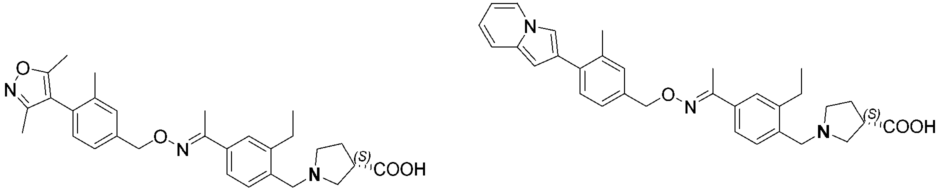

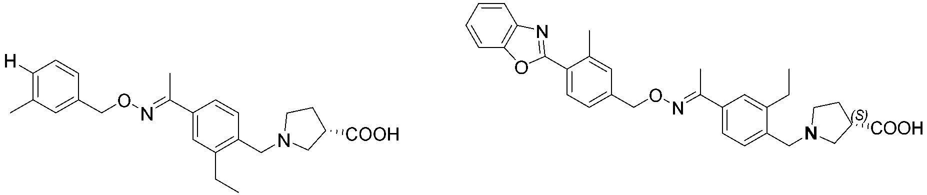

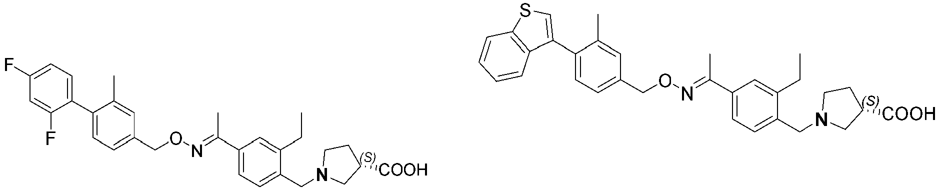

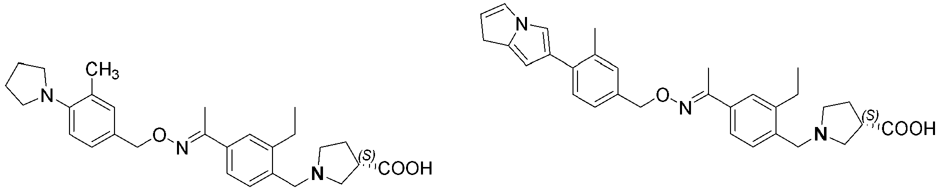

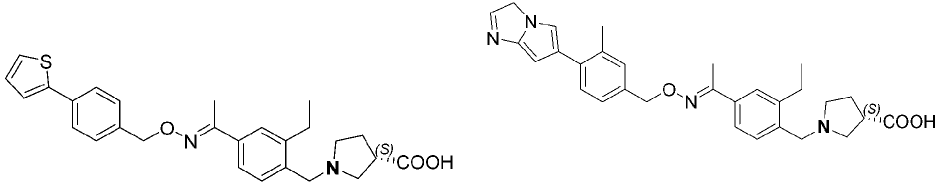

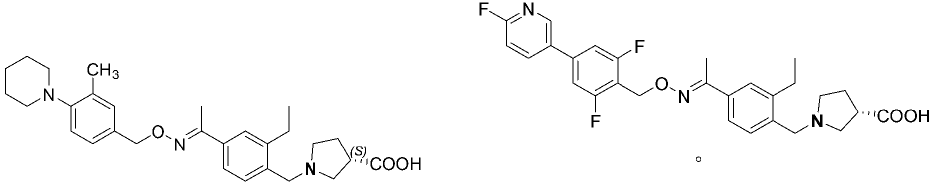

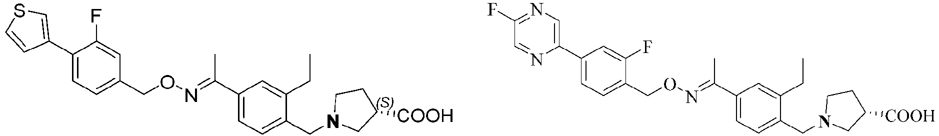

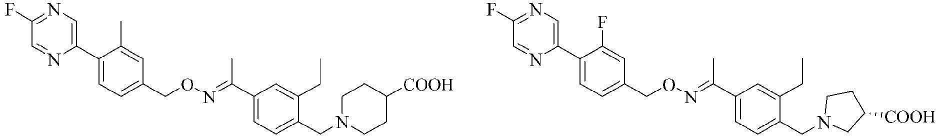

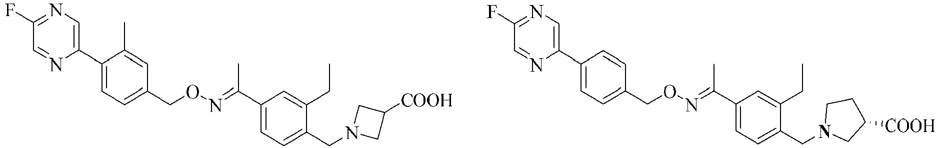

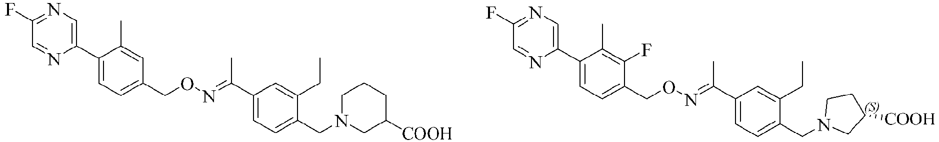

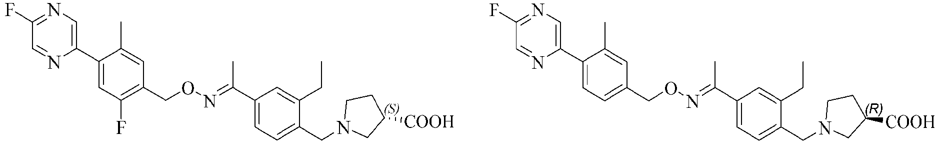

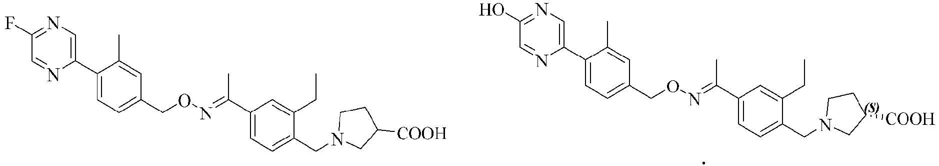

5. The compound of claim 3, wherein in Formula III, R¹ is selected from the group consisting of: a hydrogen, halogen (preferably F, Cl, or Br), cyano, substituted or unsubstituted C₁-C₅ alkoxy (preferably a methoxy), substituted or unsubstituted C₁-C₆ alkyl (preferably a methyl, ethyl, propyl);
R² is: 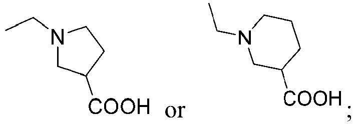
R³ and R⁵ are independently selected from the group consisting of a hydrogen, substituted or unsubstituted C₁-C₆ alkyl (preferably a methyl, ethyl or propyl), and halogen (preferably F);
R⁴ is selected from the group consisting of a substituted or unsubstituted C₅-C₈ aryl (preferably a phenyl or F-substituted phenyl), substituted or unsubstituted five-membered or six-membered heterocyclic group or heteroaryl containing 1-2 heteroatoms selected from N, O or S.

6. A compound selected from the following group, or an optical isomer or a pharmaceutically acceptable salt thereof:

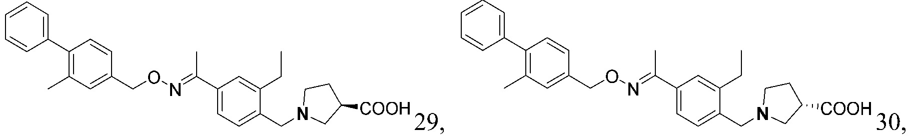

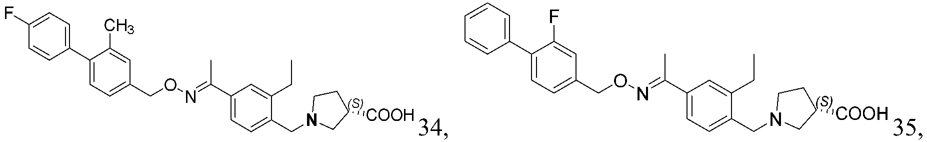

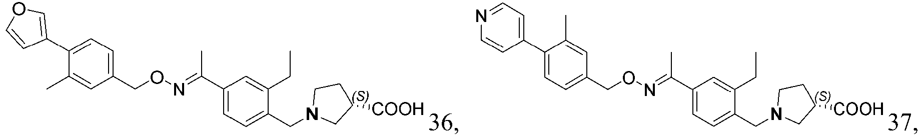

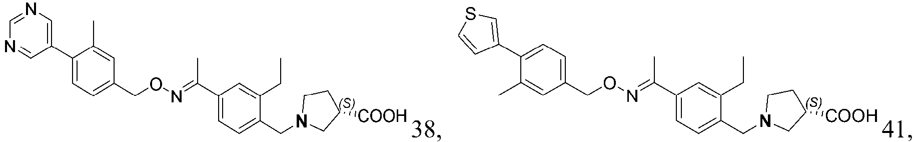

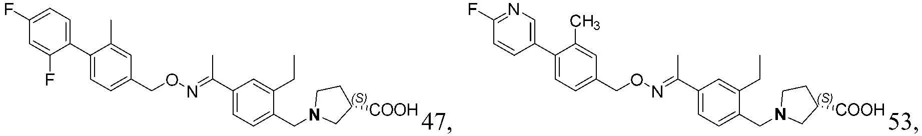

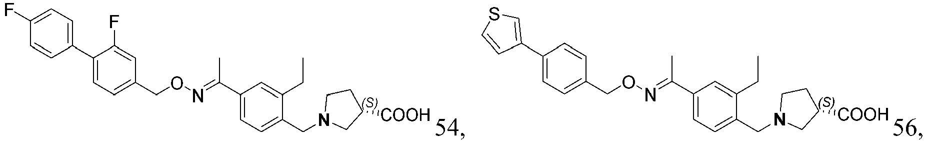

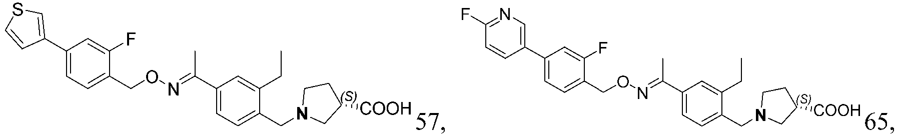

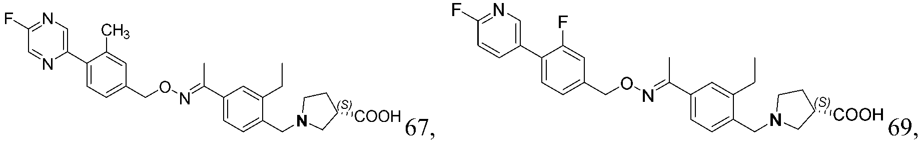

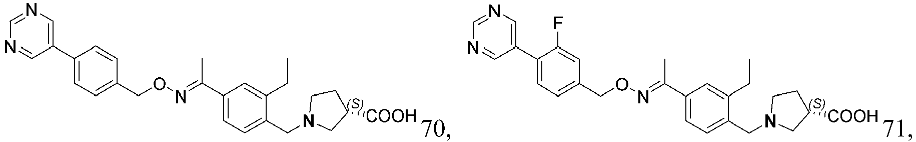

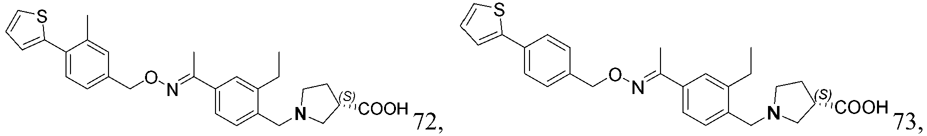

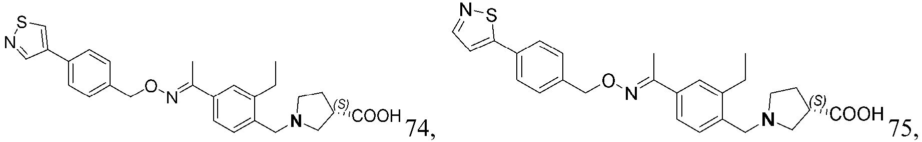

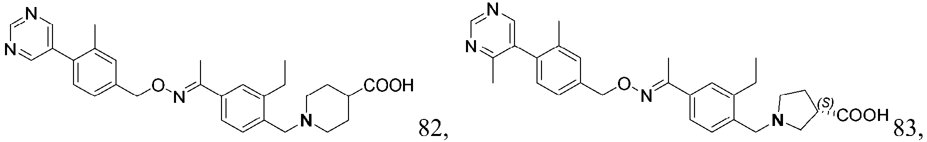

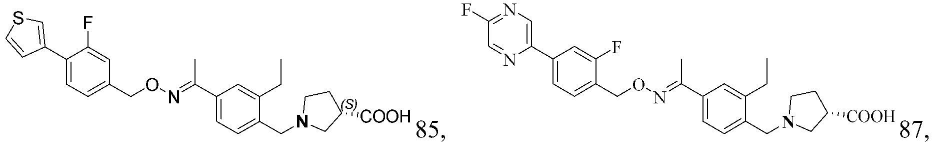

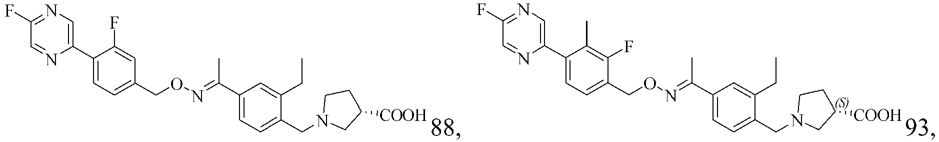

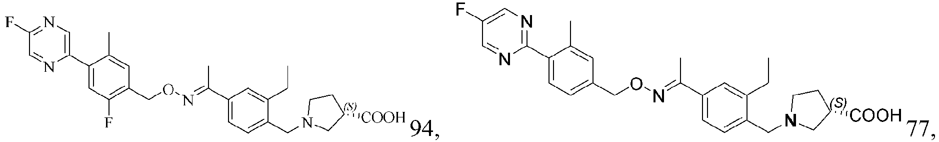

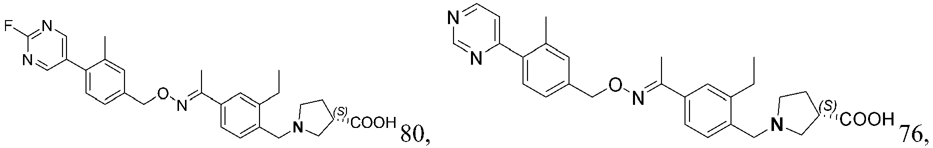

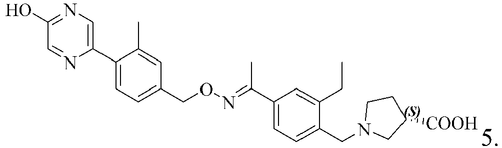

7. The compound of claim 3, wherein in Formula III,
R¹ is selected from the group consisting of: a cyano, substituted or unsubstituted C₁-C₅ alkoxy (preferably a methoxy), substituted or unsubstituted C₁-C₆ alkyl (preferably a methyl, ethyl, propyl);
R² is: 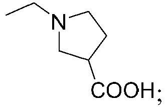
R³ and R⁵ are independently selected from the group consisting of a hydrogen, substituted or unsubstituted C₁-C₃ alkyl (preferably a methyl, ethyl or propyl), and halogen (preferably F);
R⁴ is selected from the group consisting of a substituted or unsubstituted C₅-C₈ aryl (preferably a phenyl or F-substituted phenyl), and substituted or unsubstituted five-membered or six-membered heteroaryl containing 1-2 heteroatoms selected from N, O or S.

8. A compound selected from the following group, or an optical isomer or a pharmaceutically acceptable salt thereof:

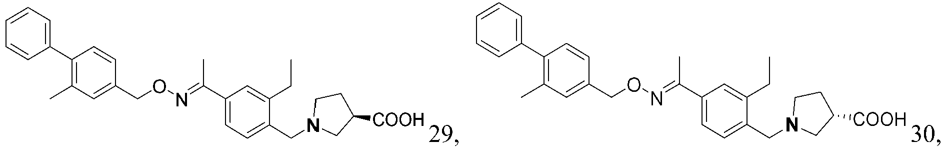

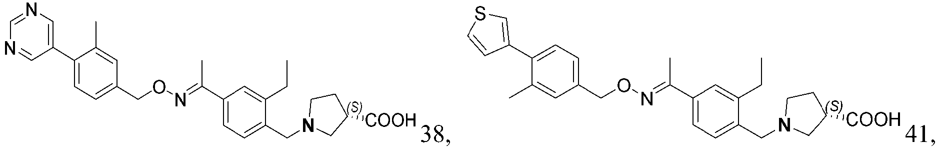

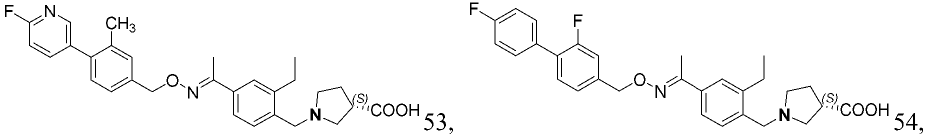

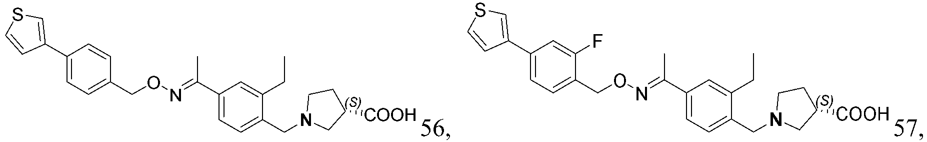

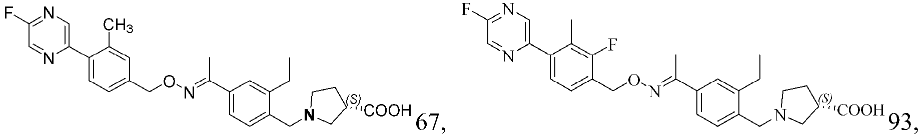

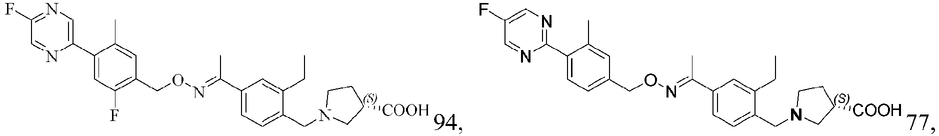

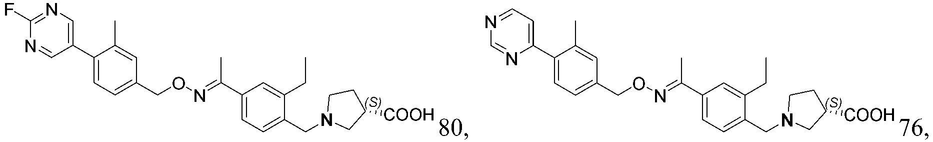

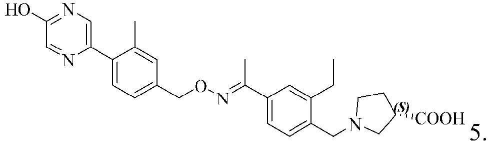

9. A pharmaceutical composition, comprising the compound of any one of claims 1-8, or an optical isomer or a pharmaceutically acceptable salt thereof, as well as a pharmaceutically acceptable carrier or excipient.

10. Use of the compound of any one of claims 1-8, or an optical isomer thereof or a pharmaceutically acceptable salt thereof, in the preparation of an S1PR regulator. In a preferred embodiment, the S1PR regulator is a selective agonist of SIPR4 or a selective agonist of S1PR4 mutant.

11. A method for regulating S1PR in a subject, comprising the step of administering an effective amount of the compound of any one of claims 1-8, or an optical isomer thereof, or a pharmaceutically acceptable salt thereof, or the pharmaceutical composition of claim 9 to a subject in need thereof.

12. The compound of any one of claims 1-8, or an optical isomer, pharmaceutically acceptable salt thereof, or the pharmaceutical composition of claim 9, for treating or preventing diseases mediated by S1PR4.

13. A medicament comprising the compound of any one of claims 1-8, or an optical isomer thereof, a pharmaceutically acceptable salt thereof, or the pharmaceutical composition of claim 9, for treating or preventing diseases mediated by S1PR4.
